# EUROPEAN PATENT APPLICATION

(11) **EP 1 870 466 A2**
(43) Date of publication of application: **26.12.2007**
(21) Application number: 07015347.3
(22) Date of filing: 14.07.1999
(51) Int. Cl.: C12N 15/12, C07K 14/47, C12Q 1/68, A61K 39/395, G01N 33/68, G01N 33/574, C07K 16/30, C12N 15/62, C12N 5/02, A61P 35/00

(54) **Compositions and methods for therapy and diagnosis of prostate cancer**

(30) Priority: 14.07.1998 US 115453; 14.07.1998 US 116134; 23.09.1998 US 159822; 23.09.1998 US 159812; 15.01.1999 US 232880; 15.01.1999 US 232149; 09.04.1999 US 288946
(62) Divisional of application: 99938735.0
(71) Applicant: CORIXA CORPORATION, Hamilton, Montana 59840-3131 (US)
(72) Inventor: Dillon, Davin, Clifford, Redmond WA 98053 (US); Harlocker, Susan, Louise, Seattle WA 98107 (US); Yuqiu, Jiang, Kent WA 98032 (US); Xu, Jiangchun, Bellevue WA 98006 (US); Mitcham, Jennifer, Lynn, Redmond WA 98052 (US)
(74) Representative: Thornton, Neil

(57) **Abstract**

Compositions and methods for the therapy and diagnosis of cancer, such as prostate cancer, are disclosed. Compositions may comprise one or more prostate tumor proteins, immunogenic portions thereof, or polynucleotides that encode such portions. Alternatively, a therapeutic composition may comprise an antigen presenting cell that expresses a prostate tumor protein, or a T cell that is specific for cells expressing such a protein. Such compositions may be used, for example, for the prevention and treatment of diseases such as prostate cancer. Diagnostic methods based on detecting a prostate tumor protein, or mRNA encoding such a protein, in a sample are also provided.

SEQUENCE LISTING

<110> Corixa Corporation

<120> COMPOUNDS FOR IMMUNOTHERAPY AND DIAGNOSIS
  OF PROSTATE CANCER AND METHODS FOR THEIR USE

<130> 210121.42701PC

<140> PCT
<141> 1999-07-08

<160> 472

<170> FastSEQ for Windows Version 3.0

<210> 1
<211> 814
<212> DNA
<213> Homo sapien

<220>
<221> misc_feature
<222> (1)...(814)
<223> n = A,T,C or G

<400> 1
tttttttttt tttttcacag tataacagct ctttatttct gtgagttcta ctaggaaatc 60
atcaaatctg agggttgtct ggaggacttc aatacacctc cccccatagt gaatcagctt 120
ccagggggtc cagtccctct ccttacttca tccccatccc atgccaaagg aagaccctcc 180
ctccttggct cacagccttc tctaggcttc ccagtgcctc caggacagag tgggttatgt 240
tttcagctcc atccttgctg tgagtgtctg gtgcgttgtg cctccagctt ctgctcagtg 300
cttcatggac agtgtccagc acatgtcact ctccactctc tcagtgtgga tccactagtt 360
ctagagcggc cgccaccgcg gtggagctcc agcttttgtt ccctttagtg agggttaatt 420
gcgcgcttgg cgtaatcatg gtcataactg tttcctgtgt gaaattgtta tccgctcaca 480
attccacaca acatacgagc cggaagcata aagtgtaaag cctggggtgc ctaatgagtg 540
anctaactca cattaattgc gttgcgctca ctgnccgctt tccagtcngg aaaactgtcg 600
tgccagctgc attaatgaat cggccaacgc ncggggaaaa gcggtttgcg ttttgggggc 660
tcttccgctt ctcgctcact nantcctgcg ctcggtcntt cggctgcggg gaacggtatc 720
actcctcaaa ggnggtatta cggttatccn naaatcnggg gatacccngg aaaaaanttt 780
aacaaaaggg cancaaaggg cngaaacgta aaaa 814

<210> 2
<211> 816
<212> DNA
<213> Homo sapien

<220>
<221> misc_feature
<222> (1)...(816)
<223> n = A,T,C or G

<400> 2
acagaaatgt tggatggtgg agcacctttc tatacgactt acaggacagc agatggggaa 60
ttcatggctg ttggagcaat agaaccccag ttctacgagc tgctgatcaa aggacttgga 120
ctaaagtctg atgaacttcc caatcagatg agcatggatg attggccaga aatgaagaag 180
aagtttgcag atgtatttgc aaagaagacg aaggcagagt ggtgtcaaat ctttgacggc 240
acagatgcct gtgtgactcc ggttctgact tttgaggagg ttgttcatca tgatcacaac 300
aaggaacggg gctcgtttat caccagtgag gagcaggacg tgagcccccg ccctgcacct 360
ctgctgttaa acaccccagc catcccttct ttcaaaaggg atccactagt tctagaagcg 420
gccgccaccg cggtggagct ccagcttttg ttccctttag tgagggttaa ttgcgcgctt 480
ggcgtaatca tggtcatagc tgtttcctgt gtgaaattgt tatccgctca caattccccc 540

aacatacgag ccggaacata aagtgttaag cctggggtgc ctaatgantg agctaactcn 600
cattaattgc gttgcgctca ctgcccgctt tccagtcggg aaaactgtcg tgccactgcn 660
ttantgaatc ngccaccccc cgggaaaagg cggttgcntt ttgggcctct tccgctttcc 720
tcgctcattg atcctngcnc ccggtcttcg gctgcggnga acggttcact cctcaaaggc 780
ggtntnccgg ttatccccaa acnggggata cccnga 816

<210> 3
<211> 773
<212> DNA
<213> Homo sapien

<220>
<221> misc_feature
<222> (1)...(773)
<223> n = A,T,C or G

<400> 3
cttttgaaag aagggatggc tggggtgttt aacagcagag gtgcagggcg ggggctcacg 60
tcctgctcct cactggtgat aaacgagccc cgttccttgt tgtgatcatg atgaacaacc 120
tcctcaaaag tcagaaccgg agtcacacag gcatctgtgc cgtcaaagat ttgacaccac 180
tctgccttcg tcttctttgc aaatacatct gcaaacttct tcttcatttc tggccaatca 240
tccatgctca tctgattggg aagttcatca gactttagtc canntccttt gatcagcagc 300
tcgtagaact ggggttctat tgctccaaca gccatgaatt ccccatctgc tgtcctgtaa 360
gtcgtataga aaggtgctcc accatccaac atgttctgtc ctcgaggggg ggcccggtac 420
ccaattcgcc ctatantgag tcgtattacg cgcgctcact ggccgtcgtt ttacaacgtc 480
gtgactggga aaaccctggg cgttaccaac ttaatcgcct tgcagcacat ccccctttcg 540
ccagctgggc gtaatancga aaaggcccgc accgatcgcc cttccaacag ttgcgcacct 600
gaatgggnaa atgggacccc cctgttaccg cgcattnaac ccccgcnggg tttngttgtt 660
acccccacnt nnaccgctta cactttgcca gcgccttanc gcccgctccc tttcnccttt 720
cttcccttcc tttcncnccn ctttcccccg gggtttcccc cntcaaaccc cna 773

<210> 4
<211> 828
<212> DNA
<213> Homo sapien

<220>
<221> misc_feature
<222> (1)...(828)
<223> n = A,T,C or G

<400> 4
   cctcctgagt cctactgacc tgtgctttct ggtgtggagt ccagggctgc taggaaaagg 60
   aatgggcaga cacaggtgta tgccaatgtt tctgaaatgg gtataatttc gtcctctcct 120
   tcggaacact ggctgtctct gaagacttct cgctcagttt cagtgaggac acacacaaag 180
   acgtgggtga ccatgttgtt tgtggggtgc agagatggga ggggtggggc ccaccctgga 240
   agagtggaca gtgacacaag gtggacactc tctacagatc actgaggata agctggagcc 300
   acaatgcatg aggcacacac acagcaagga tgacnctgta aacatagccc acgctgtcct 360
   gngggcactg ggaagcctan atnaggccgt gagcanaaag aaggggagga tccactagtt 420
   ctanagcggc cgccaccgcg gtgganctcc ancttttgtt ccctttagtg agggttaatt 480
   gcgcgcttgg cntaatcatg gtcatanctn tttcctgtgt gaaattgtta tccgctcaca 540
   attccacaca acatacganc cggaaacata aantgtaaac ctggggtgcc taatgantga 600
   ctaactcaca ttaattgcgt tgcgctcact gcccgctttc caatcnggaa acctgtcttg 660
   ccncttgcat tnatgaatcn gccaaccccc ggggaaaagc gtttgcgttt tgggcgctct 720
   tccgcttcct cnctcantta ntccctncnc tcggtcattc cggctgcngc aaaccggttc 780
   accncctcca aagggggtat tccggtttcc ccnaatccgg gganancc 828

<210> 5
<211> 834
<212> DNA
<213> Homo sapien

<220>
<221> misc_feature
<222> (1)...(834)
<223> n = A,T,C or G

<400> 5
tttttttttt tttttactga tagatggaat ttattaagct tttcacatgt gatagcacat 60
agttttaatt gcatccaaag tactaacaaa aactctagca atcaagaatg gcagcatgtt 120
attttataac aatcaacacc tgtggctttt aaaatttggt tttcataaga taatttatac 180
tgaagtaaat ctagccatgc ttttaaaaaa tgctttaggt cactccaagc ttggcagtta 240
acatttggca taaacaataa taaaacaatc acaatttaat aaataacaaa tacaacattg 300
taggccataa tcatatacag tataaggaaa aggtggtagt gttgagtaag cagttattag 360
aatagaatac cttggcctct atgcaaatat gtctagacac tttgattcac tcagccctga 420
cattcagttt tcaaagtagg agacaggttc tacagtatca ttttacagtt tccaacacat 480
tgaaaacaag tagaaaatga tgagttgatt tttattaatg cattacatcc tcaagagtta 540
tcaccaaccc ctcagttata aaaaattttc aagttatatt agtcatataa cttggtgtgc 600
ttattttaaa ttagtgctaa atggattaag tgaagacaac aatggtcccc taatgtgatt 660
gatattggtc atttttacca gcttctaaat ctnaactttc aggcttttga actggaacat 720
tgnatnacag tgttccanag ttncaaccta ctggaacatt acagtgtgct tgattcaaaa 780
tgttattttg ttaaaaatta aattttaacc tggtggaaaa ataatttgaa atna 834

<210> 6
<211> 818
<212> DNA
<213> Homo sapien

<220>
<221> misc_feature
<222> (1)...(818)
<223> n = A,T,C or G

<400> 6
tttttttttt tttttttttt aagaccctca tcaatagatg gagacataca gaaatagtca 60
aaccacatct acaaaatgcc agtatcaggc ggcggcttcg aagccaaagt gatgtttgga 120
tgtaaagtga aatattagtt ggcggatgaa gcagatagtg aggaaagttg agccaataat 180
gacgtgaagt ccgtggaagc ctgtggctac aaaaaatgtt gagccgtaga tgccgtcgga 240
aatggtgaag ggagactcga agtactctga ggcttgtagg agggtaaaat agagacccag 300
taaaattgta ataagcagtg cttgaattat ttggtttcgg ttgttttcta ttagactatg 360
gtgagctcag gtgattgata ctcctgatgc gagtaatacg gatgtgttta ggagtgggac 420
ttctagggga tttagcgggg tgatgcctgt tgggggccag tgccctccta gttggggggt 480
aggggctagg ctggagtggt aaaaggctca gaaaaatcct gcgaagaaaa aaacttctga 540
ggtaataaat aggattatcc cgtatcgaag gcctttttgg acaggtggtg tgtggtggcc 600
ttggtatgtg ctttctcgtg ttacatcgcg ccatcattgg tatatggtta gtgtgttggg 660
ttantanggc ctantatgaa gaacttttgg antggaatta aatcaatngc ttggccggaa 720
gtcattanga nggctnaaaa ggccctgtta ngggtctggg ctnggtttta cccnacccat 780
ggaatncncc ccccggacna ntgnatccct attcttaa 818

<210> 7
<211> 817
<212> DNA
<213> Homo sapien

<220>
<221> misc_feature
<222> (1)...(817)
<223> n = A,T,C or G

<400> 7
tttttttttt tttttttttt tggctctaga gggggtagag ggggtgctat agggtaaata 60
cgggccctat ttcaaagatt tttaggggaa ttaattctag gacgatgggt atgaaactgt 120
ggtttgctcc acagatttca gagcattgac cgtagtatac ccccggtcgt gtagcggtga 180
aagtggtttg gtttagacgt ccgggaattg catctgtttt taagcctaat gtggggacag 240

ctcatgagtg caagacgtct tgtgatgtaa ttattatacn aatgggggct tcaatcggga 300
gtactactcg attgtcaacg tcaaggagtc gcaggtcgcc tggttctagg aataatgggg 360
gaagtatgta ggaattgaag attaatccgc cgtagtcggt gttctcctag gttcaatacc 420
attggtggcc aattgatttg atggtaaggg gagggatcgt tgaactcgtc tgttatgtaa 480
aggatncctt ngggatggga aggcnatnaa ggactangga tnaatggcgg gcangatatt 540
tcaaacngtc tctanttcct gaaacgtctg aaatgttaat aanaattaan tttngttatt 600
gaatnttnng gaaaagggct tacaggacta gaaaccaaat angaaaanta atnntaangg 660
cnttatcntn aaaggtnata accnctccta tnatcccacc caatngnatt ccccacncnn 720
acnattggat nccccanttc canaaanggc cnccccccgg tgnannccnc cttttgttcc 780
cttnantgan ggttattcnc ccctngcntt atcancc 817

<210> 8
<211> 799
<212> DNA
<213> Homo sapien

<220>
<221> misc_feature
<222> (1)...(799)
<223> n = A,T,C or G

<400> 8
catttccggg tttactttct aaggaaagcc gagcggaagc tgctaacgtg ggaatcggtg 60
cataaggaga actttctgct ggcacgcgct agggacaagc gggagagcga ctccgagcgt 120
ctgaagcgca cgtcccagaa ggtggacttg gcactgaaac agctgggaca catccgcgag 180
tacgaacagc gcctgaaagt gctggagcgg gaggtccagc agtgtagccg cgtcctgggg 240
tgggtggccg angcctganc cgctctgcct tgctgccccc angtgggccg ccaccccctg 300
acctgcctgg gtccaaacac tgagccctgc tggcggactt caaggaanac ccccacangg 360
ggattttgct cctanantaa ggctcatctg ggcctcggcc cccccacctg gttggccttg 420
tctttgangt gagccccatg tccatctggg ccactgtcng gaccaccttt ngggagtgtt 480
ctccttacaa ccacannatg cccggctcct cccggaaacc antcccancc tgngaaggat 540
caagncctgn atccactnnt nctanaaccg gccnccnccg cngtggaacc cnccttntgt 600
tccttttcnt tnagggttaa tnncgccttg gccttnccan ngtcctncnc nttttccnnt 660
gttnaaattg ttangcnccc nccnntcccn cnncnncnan cccgacccnn annttnnann 720
ncctgggggt nccnncngat tgacccnncc nccctntant tgcnttnggg nncnntgccc 780
ctttccctct ngggannccg 799

<210> 9
<211> 801
<212> DNA
<213> Homo sapien

<220>
<221> misc_feature
<222> (1)...(801)
<223> n = A,T,C or G

<400> 9
acgccttgat cctcccaggc tgggactggt tctgggagga gccgggcatg ctgtggtttg 60
taangatgac actcccaaag gtggtcctga cagtggccca gatggacatg gggctcacct 120
caaggacaag gccaccaggt gcgggggccg aagcccacat gatccttact ctatgagcaa 180
aatcccctgt gggggcttct ccttgaagtc cgccancagg gctcagtctt tggacccang 240
caggtcatgg ggttgtngnc caactggggg ccncaacgca aaanggcnca gggcctcngn 300
cacccatccc angacgcggc tacactnctg gacctcccnc tccaccactt tcatgcgctg 360
ttcntacccg cgnatntgtc ccanctgttt cngtgccnac tccancttct nggacgtgcg 420
ctacatacgc ccggantcnc nctcccgctt tgtccctatc cacgtnccan caacaaattt 480
cnccntantg caccnattcc cacntttnnc agntttccnc nncgngcttc cttntaaaag 540
ggttganccc cggaaaatnc cccaaagggg gggggccngg tacccaactn ccccctnata 600
gctgaantcc ccatnaccnn gnctcnatgg anccntccnt tttaannacn ttctnaactt 660
gggaanancc ctcgnccntn cccccnttaa tcccnccttg cnangnncnt cccccnntcc 720
ncccnnntng gcntntnann cnaaaaaggc ccnnnancaa tctcctnncn cctcanttcg 780
ccanccctcg aaatcggccn c 801

<210> 10
<211> 789
<212> DNA
<213> Homo sapien

<220>
<221> misc_feature
<222> (1)...(789)
<223> n = A,T,C or G

<400> 10
cagtctatnt ggccagtgtg gcagctttcc ctgtggctgc cggtgccaca tgcctgtccc 60
acagtgtggc cgtggtgaca gcttcagccg ccctcaccgg gttcaccttc tcagccctgc 120
agatcctgcc ctacacactg gcctccctct accaccggga gaagcaggtg ttcctgccca 180
aataccgagg ggacactgga ggtgctagca gtgaggacag cctgatgacc agcttcctgc 240
caggccctaa gcctggagct cccttcccta atggacacgt gggtgctgga ggcagtggcc 300
tgctcccacc tccacccgcg ctctgcgggg cctctgcctg tgatgtctcc gtacgtgtgg 360
tggtgggtga gcccaccgan gccagggtgg ttccgggccg gggcatctgc ctggacctcg 420
ccatcctgga tagtgcttcc tgctgtccca ngtggcccca tccctgttta tgggctccat 480
tgtccagctc agccagtctg tcactgccta tatggtgtct gccgcaggcc tgggtctggt 540
cccatttact ttgctacaca ggtantattt gacaagaacg anttggccaa atactcagcg 600
ttaaaaaatt ccagcaacat tgggggtgga aggcctgcct cactgggtcc aactccccgc 660
tcctgttaac cccatggggc tgccggcttg gccgccaatt tctgttgctg ccaaantnat 720
gtggctctct gctgccacct gttgctggct gaagtgcnta cngcncanct nggggggtng 780
ggngttccc 789

<210> 11
<211> 772
<212> DNA
<213> Homo sapien

<220>
<221> misc_feature
<222> (1)...(772)
<223> n = A,T,C or G

<400> 11
cccaccctac ccaaatatta gacaccaaca cagaaaagct agcaatggat tcccttctac 60
tttgttaaat aaataagtta aatatttaaa tgcctgtgtc tctgtgatgg caacagaagg 120
accaacaggc cacatcctga taaaaggtaa gaggggggtg gatcagcaaa aagacagtgc 180
tgtgggctga ggggacctgg ttcttgtgtg ttgcccctca ggactcttcc cctacaaata 240
actttcatat gttcaaatcc catggaggag tgtttcatcc tagaaactcc catgcaagag 300
ctacattaaa cgaagctgca ggttaagggg cttanagatg ggaaaccagg tgactgagtt 360
tattcagctc ccaaaaaccc ttctctaggt gtgtctcaac taggaggcta gctgttaacc 420
ctgagcctgg gtaatccacc tgcagagtcc ccgcattcca gtgcatggaa cccttctggc 480
ctccctgtat aagtccagac tgaaaccccc ttggaaggnc tccagtcagg cagccctana 540
aactggggaa aaaagaaaag gacgccccan cccccagctg tgcanctacg cacctcaaca 600
gcacagggtg gcagcaaaaa aaccacttta ctttggcaca aacaaaaact ngggggggca 660
accccggcac cccnangggg gttaacagga ancngggnaa cntggaaccc aattnaggca 720
ggcccnccac cccnaatntt gctgggaaat ttttcctccc ctaaattntt tc 772

<210> 12
<211> 751
<212> DNA
<213> Homo sapien

<220>
<221> misc_feature
<222> (1)...(751)
<223> n = A,T,C or G

<400> 12
gccccaattc cagctgccac accacccacg gtgactgcat tagttcggat gtcatacaaa      60
agctgattga agcaaccctc tactttttgg tcgtgagcct tttgcttggt gcaggtttca     120
ttggctgtgt tggtgacgtt gtcattgcaa cagaatgggg gaaaggcact gttctctttg     180
aagtanggtg agtcctcaaa atccgtatag ttggtgaagc cacagcactt gagccctttc     240
atggtggtgt tccacacttg agtgaagtct tcctgggaac cataatcttt cttgatggca     300
ggcactacca gcaacgtcag ggaagtgctc agccattgtg gtgtacacca aggcgaccac     360
agcagctgcn acctcagcaa tgaagatgan gaggangatg aagaagaacg tcncgagggc     420
acacttgctc tcagtcttan caccatanca gcccntgaaa accaananca aagaccacna     480
cnccggctgc gatgaagaaa tnaccccncg ttgacaaact tgcatggcac tggganccac     540
agtggcccna aaaatcttca aaaaggatgc cccatcnatt gacccccccaa atgcccactg     600
ccaacagggg ctgccccacn cncnnaacga tganccnatt gnacaagatc tncntggtct     660
tnatnaacnt gaaccctgcn tngtggctcc tgttcaggnc cnnggcctga cttctnaann     720
aangaactcn gaagnccccca cnggananne g                                  751

<210> 13
<211> 729
<212> DNA
<213> Homo sapien

<220>
<221> misc_feature
<222> (1)...(729)
<223> n = A,T,C or G

<400> 13
gagccaggcg tccctctgcc tgcccactca gtggcaacac ccgggagctg ttttgtcctt      60
tgtggancct cagcagtncc ctctttcaga actcantgcc aaganccctg aacaggagcc     120
accatgcagt gcttcagctt cattaagacc atgatgatcc tcttcaattt gctcatcttt     180
ctgtgtggtg cagccctgtt ggcagtgggc atctgggtgt caatcgatgg ggcatccttt     240
ctgaagatct tcgggccact gtcgtccagt gccatgcagt ttgtcaacgt gggctacttc     300
ctcatcgcag ccggcgttgt ggtcttagct ctaggtttcc tgggctgcta tggtgctaag     360
actgagagca agtgtgccct cgtgacgttc ttcttcatcc tcctcctcat cttcattgct     420
gaggttgcaa tgctgtggtc gccttggtgt acaccacaat ggctgagcac ttcctgacgt     480
tgctggtaat gcctgccatc aanaaaagat tatgggttcc caggaanact tcactcaagt     540
gttggaacac caccatgaaa gggctcaagt gctgtggctt cnnccaacta tacggatttt     600
gaagantcac ctacttcaaa gaaaanagtg cctttccccc atttctgttg caattgacaa     660
acgtccccaa cacagccaat tgaaaacctg cacccaaccc aaangggtcc ccaaccanaa     720
attnaaggg                                                           729

<210> 14
<211> 816
<212> DNA
<213> Homo sapien

<220>
<221> misc_feature
<222> (1)...(816)
<223> n = A,T,C or G

<400> 14
tgctcttcct caaagttgtt cttgttgcca taacaaccac cataggtaaa gcgggcgcag      60
tgttcgctga aggggttgta gtaccagcgc gggatgctct ccttgcagag tcctgtgtct     120
ggcaggtcca cgcagtgccc tttgtcactg gggaaatgga tgcgctggag ctcgtcaaag     180
ccactcgtgt atttttcaca ggcagcctcg tccgacgcgt cggggcagtt gggggtgtct     240
tcacactcca ggaaactgtc natgcagcag ccattgctgc agcggaactg ggtgggctga     300
cangtgccag agcacactgg atggcgcctt tccatgnnan gggccctgng ggaaagtccc     360
tganccccan anctgcctct caaangcccc accttgcaca ccccgacagg ctagaatgga     420
atcttcttcc cgaaaggtag ttnttcttgt tgcccaancc anccccntaa acaaactctt     480
gcanatctgc tccgnggggg tcntantacc ancgtgggaa aagaacccca ggcngcgaac     540
caancttgtt tggatncgaa gcnataatct nctnttctgc ttggtggaca gcaccantna     600
ctgtnnanct ttagnccntg gtcctcntgg gttgnncttg aacctaatcn ccnntcaact     660

gggacaaggt aantngccnt cctttnaatt cccnancntn ccccctggtt tggggttttn 720
cncnctccta ccccagaaan nccgtgttcc cccccaacta ggggccnaaa ccnnttnttc 780
cacaaccctn ccccacccac gggttcngnt ggttng 816

<210> 15
<211> 783
<212> DNA
<213> Homo sapien

<220>
<221> misc_feature
<222> (1)...(783)
<223> n = A,T,C or G

<400> 15
ccaaggcctg ggcaggcata nacttgaagg tacaacccca ggaacccctg gtgctgaagg 60
atgtggaaaa cacagattgg cgcctactgc ggggtgacac ggatgtcagg gtagagagga 120
aagacccaaa ccaggtggaa ctgtggggac tcaaggaang cacctacctg ttccagctga 180
cagtgactag ctcagaccac ccagaggaca cggccaacgt cacagtcact gtgctgtcca 240
ccaagcagac agaagactac tgcctcgcat ccaacaangt gggtcgctgc cggggctctt 300
tcccacgctg gtactatgac cccacggagc agatctgcaa gagtttcgtt tatggaggct 360
gcttgggcaa caagaacaac taccttcggg aagaagagtg cattctancc tgtcngggtg 420
tgcaaggtgg gcctttgana ngcanctctg gggctcangc gactttcccc cagggcccct 480
ccatggaaag gcgccatcca ntgttctctg gcacctgtca gcccacccag ttccgctgca 540
ncaatggctg ctgcatcnac antttcctng aattgtgaca acacccccca ntgcccccaa 600
ccctcccaac aaagcttccc tgttnaaaaa tacnccantt ggcttttnac aaacncccgg 660
cncctccntt ttccccnntn aacaaagggc nctngcnttt gaactgcccn aacccnggaa 720
tctnccnngg aaaaantncc ccccctggtt cctnnaancc cctccncnaa anctnccccc 780
ccc 783

<210> 16
<211> 801
<212> DNA
<213> Homo sapien

<220>
<221> misc_feature
<222> (1)...(801)
<223> n = A,T,C or G

<400> 16
gccccaattc cagctgccac accacccacg gtgactgcat tagttcggat gtcatacaaa 60
agctgattga agcaaccctc tactttttgg tcgtgagcct tttgcttggt gcaggtttca 120
ttggctgtgt tggtgacgtt gtcattgcaa cagaatgggg gaaaggcact gttctctttg 180
aagtagggtg agtcctcaaa atccgtatag ttggtgaagc cacagcactt gagccctttc 240
atggtggtgt tccacacttg agtgaagtct tcctgggaac cataatcttt cttgatggca 300
ggcactacca gcaacgtcag gaagtgctca gccattgtgg tgtacaccaa ggcgaccaca 360
gcagctgcaa cctcagcaat gaagatgagg aggaggatga agaagaacgt cncgagggca 420
cacttgctct ccgtcttagc accatagcag cccangaaac caagagcaaa gaccacaacg 480
ccngctgcga atgaaagaaa ntacccacgt tgacaaactg catggccact ggacgacagt 540
tggcccgaan atcttcagaa aagggatgcc ccatcgattg aacacccana tgcccactgc 600
cnacagggct gcnccncncn gaaagaatga gccattgaag aaggatcntc ntggtcttaa 660
tgaactgaaa ccntgcatgg tggcccctgt tcagggctct tggcagtgaa ttctganaaa 720
aaggaacngc ntnagccccc ccaaangana aaacaccccc gggtgttgcc ctgaattggc 780
ggccaaggan ccctgccccn g 801

<210> 17
<211> 740
<212> DNA
<213> Homo sapien

<220>

<221> misc_feature
<222> (1)...(740)
<223> n = A,T,C or G

<400> 17
gtgagagcca ggcgtccctc tgcctgccca ctcagtggca acacccggga gctgttttgt 60
cctttgtgga gcctcagcag ttccctcttt cagaactcac tgccaagagc cctgaacagg 120
agccaccatg cagtgcttca gcttcattaa gaccatgatg atcctcttca atttgctcat 180
ctttctgtgt ggtgcagccc tgttggcagt gggcatctgg gtgtcaatcg atggggcatc 240
ctttctgaag atcttcgggc cactgtcgtc cagtgccatg cagtttgtca acgtgggcta 300
cttcctcatc gcagccggcg ttgtggtctt tgctcttggt ttcctgggct gctatggtgc 360
taagacggag agcaagtgtg ccctcgtgac gttcttcttc atcctcctcc tcatcttcat 420
tgctgaagtt gcagctgctg tggtcgcctt ggtgtacacc acaatggctg aaccattcct 480
gacgttgctg gtantgcctg ccatcaanaa agattatggg ttcccaggaa aaattcactc 540
aantntggaa caccnccatg aaaaggctcc caatttctgn tggcttcccc aactataccg 600
gaattttgaa agantcnccc tacttccaaa aaaaaanant tgcctttncc cccnttctgt 660
tgcaatgaaa acntcccaan acngccaatn aaaacctgcc cnnncaaaaa ggntcncaaa 720
caaaaaaant nnaagggttn 740

<210> 18
<211> 802
<212> DNA
<213> Homo sapien

<220>
<221> misc_feature
<222> (1)...(802)
<223> n = A,T,C or G

<400> 18
ccgctggttg cgctggtcca gngnagccac gaagcacgtc agcatacaca gcctcaatca 60
caaggtcttc cagctgccgc acattacgca gggcaagagc ctccagcaac actgcatatg 120
ggatacactt tactttagca gccagggtga caactgagag gtgtcgaagc ttattcttct 180
gagcctctgt tagtggagga agattccggg cttcagctaa gtagtcagcg tatgtcccat 240
aagcaaacac tgtgagcagc cggaaggtag aggcaaagtc actctcagcc agctctctaa 300
cattgggcat gtccagcagt tctccaaaca cgtagacacc agnggcctcc agcacctgat 360
ggatgagtgt ggccagcgct gcccccttgg ccgacttggc taggagcaga aattgctcct 420
ggttctgccc tgtcaccttc acttccgcac tcatcactgc actgagtgtg ggggacttgg 480
gctcaggatg tccagagacg tggttccgcc ccctcnctta atgacaccgn ccanncaacc 540
gtcggctccc gccgantgng ttcgtcgtnc ctgggtcagg gtctgctggc cnctacttgc 600
aancttcgtc nggcccatgg aattcaccnc accggaactn gtangatcca ctnnttctat 660
aaccggncgc caccgcnnnt ggaactccac tcttnttncc tttacttgag ggttaaggtc 720
acccttnncg ttaccttggt ccaaaccntn ccntgtgtcg anatngtnaa tcnggnccna 780
tnccanccnc atangaagcc ng 802

<210> 19
<211> 731
<212> DNA
<213> Homo sapien

<220>
<221> misc_feature
<222> (1)...(731)
<223> n = A,T,C or G

<400> 19
cnaagcttcc aggtnacggg ccgcnaancc tgacccnagg tancanaang cagncngcgg 60
gagcccaccg tcacgnggng gngtctttat nggagggggc ggagccacat cnctggacnt 120
cntgacccca actccccncc ncncantgca gtgatgagtg cagaactgaa ggtnacgtgg 180
caggaaccaa gancaaannc tgctccnntc caagtcggcn nagggggcgg ggctggccac 240
gcncatccnt cnagtgctgn aaagccccnn cctgtctact tgtttggaga acngcnnnga 300
catgcccagn gttanataac nggcngagag tnantttgcc tctcccttcc ggctgcgcan 360

cgngtntgct tagnggacat aacctgacta cttaactgaa cccnngaatc tnccncccct 420
ccactaagct cagaacaaaa aacttcgaca ccactcantt gtcacctgnc tgctcaagta 480
aagtgtaccc catncccaat gtntgctnga ngctctgncc tgcnttangt tcggtcctgg 540
gaagacctat caattnaagc tatgtttctg actgcctctt gctccctgna acaancnacc 600
cnncnntcca agggggggnc ggcccccaat ccccccaacc ntnaattnan tttanccccn 660
cccccnggcc cggcctttta cnancntcnn nnacngggna aaaccnnngc tttncccaac 720
nnaatccncc t 731

<210> 20
<211> 754
<212> DNA
<213> Homo sapien

<220>
<221> misc_feature
<222> (1)...(754)
<223> n = A,T,C or G

<400> 20
   tttttttttt tttttttttt taaaaacccc ctccattnaa tgnaaacttc cgaaattgtc 60
   caaccccctc ntccaaatnn ccntttccgg gngggggttc caaacccaan ttanntttgg 120
   annttaaatt aaatnttnnt tggnggnnna anccnaatgt nangaaagtt naacccanta 180
   tnancttnaa tncctggaaa ccngtngntt ccaaaaatnt ttaaccctta antccctccg 240
   aaatngttna nggaaaaccc aanttctcnt aaggttgttt gaaggntnaa tnaaaanccc 300
   nnccaattgt ttttngccac gcctgaatta attggnttcc gntgttttcc nttaaaanaa 360
   ggnnancccc ggttantnaa tccccccnnc cccaattata ccganttttt ttngaattgg 420
   gancccncgg gaattaacgg ggnnnntccc tnttgggggg cnggnncccc ccccntcggg 480
   ggttngggnc aggncnnaat tgtttaaggg tccgaaaaat ccctccnaga aaaaaanctc 540
   ccaggntgag nntngggttt nccccccccc canggcccct ctcgnanagt tggggtttgg 600
   ggggcctggg atttntnttc ccctnttncc tccccccccc ccnggganag aggttngngt 660
   tttgntcnnc ggccccnccn aaganctttn ccganttnan ttaaatccnt gcctnggcga 720
   agtccnttgn agggntaaan ggccccctnn cggg 754

<210> 21
<211> 755
<212> DNA
<213> Homo sapien

<220>
<221> misc_feature
<222> (1)...(755)
<223> n = A,T,C or G

<400> 21
atcancccat gaccccnaac nngggaccnc tcanccggnc nnncnaccnc cggccnatca 60
nngtnagnnc actncnnttn natcacnccc cnccnactac gcccncnanc cnacgcncta 120
nncanatncc actganngcg cgangtngan ngagaaanct nataccanag ncaccanacn 180
ccagctgtcc nanaangcct nnnatacngg nnnatccaat ntgnancctc cnaagtattn 240
nncnncanat gattttcctn anccgattac ccntnccccc tancccctcc cccccaacna 300
cgaaggcnct ggnccnaagg nngcgncncc ccgctagntc cccnncaagt cncncnccta 360
aactcanccn nattacncgc ttcntgagta tcactccccg aatctcaccc tactcaactc 420
aaaaanatcn gatacaaaat aatncaagcc tgnttatnac actntgactg ggtctctatt 480
ttagnggtcc ntnaancntc ctaatacttc cagtctncct tcnccaattt ccnaanggct 540
ctttcngaca gcatnttttg gttcccnntt gggttcttan ngaattgccc ttcntngaac 600
gggctcntct tttccttcgg ttancctggn ttcnnccggc cagttattat ttcccntttt 660
aaattcntnc cntttanttt tggcnttcna aacccccggc cttgaaaacg gccccctggt 720
aaaaggttgt tttganaaaa tttttgtttt gttcc 755

<210> 22
<211> 849
<212> DNA
<213> Homo sapien

<220>
<221> misc_feature
<222> (1)...(849)
<223> n = A,T,C or G

<400> 22
   tttttttttt tttttangtg tngtcgtgca ggtagaggct tactacaant gtgaanacgt 60
   acgctnggan taangcgacc cganttctag ganncnccct aaaatcanac tgtgaagatn 120
   atcctgnnna cggaanggtc accggnngat nntgctaggg tgnccnctcc cannncnttn 180
   cataactcng nggccctgcc caccaccttc ggcggcccng ngnccgggcc cgggtcattn 240
   gnnttaaccn cactnngcna ncggtttccn nccccnncng acccnggcga tccggggtnc 300
   tctgtcttcc cctgnagncn anaaantggg ccncggnccc ctttacccct nnacaagcca 360
   cngccntcta nccncngccc cccctccant nngggggact gccnanngct ccgttnctng 420
   nnaccccnnn gggtncctcg gttgtcgant cnaccgnang ccanggattc cnaaggaagg 480
   tgcgttnttg gcccctaccc ttcgctncgg nncacccttc ccgacnanga nccgctcccg 540
   cncnncgnng cctcncctcg caacacccgc nctcntcngt ncggnnnccc ccccacccgc 600
   nccctcncnc ngncgnancn ctccnccncc gtctcannca ccaccccgcc ccgccaggcc 660
   ntcanccacn ggnngacnng nagcncnntc gcnccgcgcn gcgncnccct cgccncngaa 720
   ctncntcngg ccantnncgc tcaanccnna cnaaacgccg ctgcgcggcc cgnagcgncc 780
   ncctccncga gtcctcccgn cttccnaccc angnnttccn cgaggacacn nnaccccgcc 840
   nncangcgg 849

<210> 23
<211> 872
<212> DNA
<213> Homo sapien

<220>
<221> misc_feature
<222> (1)...(872)
<223> n = A,T,C or G

<400> 23
   gcgcaaacta tacttcgctc gnactcgtgc gcctcgctnc tcttttcctc cgcaaccatg 60
   tctgacnanc ccgattnggc ngatatcnan aagntcganc agtccaaact gantaacaca 120
   cacacncnan aganaaatcc nctgccttcc anagtanacn attgaacnng agaaccangc 180
   nggcgaatcg taatnaggcg tgcgccgcca atntgtcncc gtttattntn ccagcntcnc 240
   ctnccnaccc tacntcttcn nagctgtcnn acccctngtn cgnacccccc naggtcggga 300
   tcgggtttnn nntgaccgng cnncccctcc ccccntccat nacganccnc ccgcaccacc 360
   nanngcncgc nccccgnnct cttcgccncc ctgtcctntn cccctgtngc ctggcncngn 420
   accgcattga ccctcgccnn ctncnngaaa ncgnanacgt ccgggttgnn annancgctg 480
   tgggnnngcg tctgcnccgc gttccttccn ncnncttcca ccatcttcnt tacngggtct 540
   ccncgccntc tcnnncacnc cctgggacgc tntcctntgc cccccttnac tccccccctt 600
   cgncgtgncc cgnccccacc ntcatttnca nacgntcttc acaannncct ggntnnctcc 660
   cnancngncn gtcanccnag ggaagggngg ggnnccnntg nttgacgttg nggngangtc 720
   cgaanantcc tcnccntcan cnctacccct cgggcgnnct ctcngttncc aacttancaa 780
   ntctcccccg ngngcncntc tcagcctcnc ccnccccnct ctctgcantg tnctctgctc 840
   tnaccnntac gantnttcgn cnccctcttt cc 872

<210> 24
<211> 815
<212> DNA
<213> Homo sapien

<220>
<221> misc_feature
<222> (1)...(815)
<223> n = A,T,C or G

<400> 24
gcatgcaagc ttgagtattc tatagngtca cctaaatanc ttggcntaat catggtcnta 60

nctgncttcc tgtgtcaaat gtatacnaan tanatatgaa tctnatntga caaganngta 120
tcntncatta gtaacaantg tnntgtccat cctgtcngan canattccca tnnattncgn 180
cgcattcncn gcncantatn taatngggaa ntcnnntnnn ncaccnncat ctatcntncc 240
gcnccctgac tggnagagat ggatnanttc tnntntgacc nacatgttca tcttggattn 300
aananccccc cgcngnccac cggttngnng cnagccnntc ccaagacctc ctgtggaggt 360
aacctgcgtc aganncatca aacntgggaa acccgcnncc angtnnaagt ngnnncanan 420
gatcccgtcc aggnttnacc atcccttcnc agcgccccct ttngtgcctt anagngnagc 480
gtgtccnanc cnctcaacat ganacgcgcc agnccanccg caattnggca caatgtcgnc 540
gaacccccta gggggantna tncaaanccc caggattgtc cncncangaa atcccncanc 600
cccnccctac ccnnctttgg gacngtgacc aantcccgga gtnccagtcc ggccngnctc 660
ccccaccggt nnccntgggg gggtgaanct cngnntcanc cngncgaggn ntcgnaagga 720
accggncctn ggncgaanng ancnntcnga agngccncnt cgtataaccc cccctcncca 780
nccnacngnt agntcccccc cngggtncgg aangg 815

<210> 25
<211> 775
<212> DNA
<213> Homo sapien

<220>
<221> misc_feature
<222> (1)...(775)
<223> n = A,T,C or G

<400> 25
ccgagatgtc tcgctccgtg gccttagctg tgctcgcgct actctctctt tctggcctgg 60
aggctatcca gcgtactcca aagattcagg tttactcacg tcatccagca gagaatggaa 120
agtcaaattt cctgaattgc tatgtgtctg ggtttcatcc atccgacatt gaanttgact 180
tactgaagaa tgganagaga attgaaaaag tggagcattc agacttgtct ttcagcaagg 240
actggtcttt ctatctcntg tactacactg aattcacccc cactgaaaaa gatgagtatg 300
cctgccgtgt gaaccatgtg actttgtcac agcccaagat agttaagtgg gatcgagaca 360
tgtaagcagn cnncatggaa gtttgaagat gccgcatttg gattggatga attccaaatt 420
ctgcttgctt gcnttttaat antgatatgc ntatacaccc taccctttat gnccccaaat 480
tgtaggggtt acatnantgt tcncntngga catgatcttc ctttataant ccnccnttcg 540
aattgcccgt cncccngttn ngaatgtttc cnnaaccacg gttggctccc ccaggtcncc 600
tcttacggaa gggcctgggc cnctttncaa ggttggggga accnaaaatt tcncttntgc 660
ccncccncca cnntcttgng nncncanttt ggaacccttc cnattcccct tggcctcnna 720
nccttnncta anaaaacttn aaancgtngc naaannttn acttcccccc ttacc 775

<210> 26
<211> 820
<212> DNA
<213> Homo sapien

<220>
<221> misc_feature
<222> (1)...(820)
<223> n = A,T,C or G

<400> 26
anattantac agtgtaatct tttcccagag gtgtgtanag ggaacggggc ctagaggcat 60
cccanagata ncttatanca acagtgcttt gaccaagagc tgctgggcac atttcctgca 120
gaaaaggtgg cggtccccat cactcctcct ctcccatagc catcccagag gggtgagtag 180
ccatcangcc ttcggtggga gggagtcang gaaacaacan accacagagc anacagacca 240
ntgatgacca tgggcgggag cgagcctctt ccctgnaccg gggtggcana nganagccta 300
nctgaggggt cacactataa acgttaacga ccnagatnan cacctgcttc aagtgcaccc 360
ttcctacctg acnaccagng accnnnaact gcngcctggg gacagcnctg ggancagcta 420
acnnagcact cacctgcccc cccatggccg tncgcntccc tggtcctgnc aagggaagct 480
ccctgttgga attncgggga naccaaggga nccccctcct ccanctgtga aggaaaaann 540
gatggaattt tncccttccg gccnntcccc tcttccttta cacgccccct nntactcntc 600
tccctctntt ntcctgncnc acttttnacc ccnnnatttc ccttnattga tcggannctn 660
ganattccac tnncgcctnc cntcnatcng naanacnaaa nactntctna cccnggggat 720

gggnncctcg ntcatcctct ctttttcnct accnccnntt ctttgcctct ccttngatca
780tccaaccntc gntggccntn cccccccnnn tcctttnccc
820

<210> 27
<211> 818
<212> DNA
<213> Homo sapien

<220>
<221> misc_feature
<222> (1)...(818)
<223> n = A,T,C or G

<400> 27
tctgggtgat ggcctcttcc tcctcaggga cctctgactg ctctgggcca aagaatctct 60
tgtttcttct ccgagcccca ggcagcggtg attcagccct gcccaacctg attctgatga 120
ctgcggatgc tgtgacggac ccaaggggca aatagggtcc cagggtccag ggaggggcgc 180
ctgctgagca cttccgcccc tcaccctgcc cagcccctgc catgagctct gggctgggtc 240
tccgcctcca gggttctgct cttccangca ngccancaag tggcgctggg ccacactggc 300
ttcttcctgc cccntccctg gctctgantc tctgtcttcc tgtcctgtgc angcnccttg 360
gatctcagtt tccctcnctc anngaactct gtttctgann tcttcantta actntgantt 420
tatnaccnan tggnctgtnc tgtcnnactt taatgggccn gaccggctaa tccctccctc 480
nctcccttcc anttcnnnna accngcttnc cntcntctcc ccntancccg ccngggaanc 540
ctcctttgcc ctnaccangg gccnnnaccg cccntnnctn ggggggcnng gtnnctncnc 600
ctgntnnccc cnctcncnnt tncctcgtcc cnncnncgcn nngcannttc ncngtcccnn 660
tnnctcttcn ngtntcgnaa ngntcncntn tnnnnngncn ngntnntncn tccctctcnc 720
cnnntgnang tnnttnnnnc ncngnncccc nnnncnnnnn nggnnntnnn tctncncngc 780
cccnnccccc ngnattaagg cctccnntct ccggccnc 818

<210> 28
<211> 731
<212> DNA
<213> Homo sapien

<220>
<221> misc_feature
<222> (1)...(731)
<223> n = A,T,C or G

<400> 28
aggaagggcg gagggatatt gtangggatt gagggatagg agnataangg gggaggtgtg 60
tcccaacatg anggtgnngt tctcttttga angagggttg ngtttttann ccnggtgggt 120
gattnaaccc cattgtatgg agnnaaaggn tttnagggat ttttcggctc ttatcagtat 180
ntanattcct gtnaatcgga aaatnatntt tcnncnggaa aatnttgctc ccatccgnaa 240
attnctcccg ggtagtgcat nttngggggn cngccangtt tcccaggctg ctanaatcgt 300
actaaagntt naagtgggan tncaaatgaa aacctnncac agagnatccn tacccgactg 360
tnnnttncct tcgccctntg actctgcnng agcccaatac ccnngngnat gtcncccngn 420
nnngcgncnc tgaaannnnc tcgnggctnn gancatcang gggtttcgca tcaaaagcnn 480
cgtttcncat naaggcactt tngcctcatc caaccnctng ccctcnncca tttngccgtc 540
nggttcncct acgctnntng cncctnnntn ganattttnc ccgcctnggg naancctcct 600
gnaatgggta gggncttntc ttttnaccnn gnggtntact aatcnnctnc acgcntnctt 660
tctcnacccc cccccttttt caatcccanc ggcnaatggg gtctccccnn cgangggggg 720
nnncccannc c 731

<210> 29
<211> 822
<212> DNA
<213> Homo sapien

<220>
<221> misc_feature

<222> (1)...(822)
<223> n = A,T,C or G

<400> 29
actagtccag tgtggtggaa ttccattgtg ttggggncnc ttctatgant antnttagat 60
cgctcanacc tcacancctc ccnacnangc ctataangaa nannaataga nctgtncnnt 120
atntntacnc tcatanncct cnnnacccac tccctcttaa cccntactgt gcctatngcn 180
tnnctantct ntgccgcctn cnanccaccn gtgggccnac cncnngnatt ctcnatctcc 240
tcnccatntn gcctananta ngtncatacc ctatacctac nccaatgcta nnnctaancn 300
tccatnantt annntaacta ccactgacnt ngactttcnc atnanctcct aatttgaatc 360
tactctgact cccacngcct annnattagc ancntccccc nacnatntct caaccaaatc 420
ntcaacaacc tatctanctg ttcnccaacc nttncctccg atccccnnac aacccccctc 480
ccaaataccc nccacctgac ncctaacccn caccatcccg gcaagccnan ggncatttan 540
ccactggaat cacnatngga naaaaaaaac ccnaactctc tancncnnat ctccctaana 600
aatnctcctn naatttactn ncantnccat caancccacn tgaaacnnaa cccctgtttt 660
tanatccctt ctttcgaaaa ccnacccttt annncccaac ctttngggcc cccccnctnc 720
ccnaatgaag gncncccaat cnangaaacg nccntgaaaa ancnaggcna anannntccg 780
canatcctat cccttanttn ggggnccctt ncccngggcc cc 822

<210> 30
<211> 787
<212> DNA
<213> Homo sapien

<220>
<221> misc_feature
<222> (1)...(787)
<223> n = A,T,C or G

<400> 30
cggccgcctg ctctggcaca tgcctcctga atggcatcaa aagtgatgga ctgcccattg 60
ctagagaaga ccttctctcc tactgtcatt atggagccct gcagactgag ggctcccctt 120
gtctgcagga tttgatgtct gaagtcgtgg agtgtggctt ggagctcctc atctacatna 180
gctggaagcc ctggagggcc tctctcgcca gcctccccct tctctccacg ctctccangg 240
acaccagggg ctccaggcag cccattattc ccagnangac atggtgtttc tccacgcgga 300
cccatggggc ctgnaaggcc agggtctcct ttgacaccat ctctcccgtc ctgcctggca 360
ggccgtggga tccactantt ctanaacggn cgccaccncg gtgggagctc cagcttttgt 420
tcccnttaat gaaggttaat tgcncgcttg gcgtaatcat nggtcanaac tntttcctgt 480
gtgaaattgt ttntcccctc ncnattccnc ncnacatacn aacccggaan cataaagtgt 540
taaagcctgg gggtngcctn nngaatnaac tnaactcaat taattgcgtt ggctcatggc 600
ccgctttccn ttcnggaaaa ctgtcntccc ctgcnttnnt gaatcggcca cccccngggg 660
aaaagcggtt tgcnttttng ggggntcctt ccncttcccc cctcnctaan ccctncgcct 720
cggtcgttnc nggtngcggg gaangggnat nnnctcccnc naagggggng agnnngntat 780
ccccaaa 787

<210> 31
<211> 799
<212> DNA
<213> Homo sapien

<220>
<221> misc_feature
<222> (1)...(799)
<223> n = A,T,C or G

<400> 31
tttttttttt tttttttggc gatgctactg tttaattgca ggaggtgggg gtgtgtgtac 60
catgtaccag ggctattaga agcaagaagg aaggagggag ggcagagcgc cctgctgagc 120
aacaaaggac tcctgcagcc ttctctgtct gtctcttggc gcaggcacat ggggaggcct 180
cccgcagggt gggggccacc agtccagggg tgggagcact acanggggtg ggagtgggtg 240
gtggctggtn cnaatggcct gncacanatc cctacgattc ttgacacctg gatttcacca 300
ggggaccttc tgttctccca nggnaacttc ntnnatctcn aaagaacaca actgtttctt 360

cngcanttct ggctgttcat ggaaagcaca ggtgtccnat ttnggctggg acttggtaca 420
tatggttccg gcccacctct cccntcnaan aagtaattca cccccccccn ccntctnttg 480
cctgggccct taantaccca caccggaact canttantta ttcatcttng gntgggcttg 540
ntnatcnccn cctgaangcg ccaagttgaa aggccacgcc gtncccnctc cccatagnan 600
nttttnncnt canctaatgc ccccccnggc aacnatccaa tccccccccn tgggggcccc 660
agcccanggc ccccgnctcg ggnnnccngn cncgnantcc ccaggntctc ccantcngnc 720
ccnnngcncc cccgcacgca gaacanaagg ntngagccnc cgcannnnnn nggtnncnac 780
ctcgcccccc ccnncgnng 799

<210> 32
<211> 789
<212> DNA
<213> Homo sapien

<220>
<221> misc_feature
<222> (1)...(789)
<223> n = A,T,C or G

<400> 32
   tttttttttt tttttttttt tttttttttt tttttttttt tttttttttt tttttttttt 60
   ttttnccnag ggcaggttta ttgacaacct cncgggacac aancaggctg gggacaggac 120
   ggcaacaggc tccggcggcg gcggcggcgg ccctacctgc ggtaccaaat ntgcagcctc 180
   cgctcccgct tgatnttcct ctgcagctgc aggatgccnt aaaacagggc ctcggccntn 240
   ggtgggcacc ctgggatttn aatttccacg ggcacaatgc ggtcgcancc cctcaccacc 300
   nattaggaat agtggtntta cccnccnccg ttggcncact ccccntggaa accacttntc 360
   gcggctccgg catctggtct taaaccttgc aaacnctggg gccctctttt tggttantnt 420
   nccngccaca atcatnactc agactggcnc gggctggccc caaaaaancn ccccaaaacc 480
   ggnccatgtc ttnncggggt tgctgcnatn tncatcacct cccgggcnca ncaggncaac 540
   ccaaaagttc ttgnggcccn caaaaaanct ccggggggnc ccagtttcaa caaagtcatc 600
   ccccttggcc cccaaatcct ccccccgntt nctgggtttg ggaacccacg cctctnnctt 660
   tggnnggcaa gntggntccc ccttcgggcc cccggtgggc ccnnctctaa ngaaaacncc 720
   ntcctnnnca ccatcccccc nngnnacgnc tancaangna tccctttttt tanaaacggg 780
   ccccccncg 789

<210> 33
<211> 793
<212> DNA
<213> Homo sapien

<220>
<221> misc_feature
<222> (1)...(793)
<223> n = A,T,C or G

<400> 33
gacagaacat gttggatggt ggagcacctt tctatacgac ttacaggaca gcagatgggg 60
aattcatggc tgttggagca atanaacccc agttctacga gctgctgatc aaaggacttg 120
gactaaagtc tgatgaactt cccaatcaga tgagcatgga tgattggcca gaaatgaana 180
agaagtttgc agatgtattt gcaaagaaga cgaaggcaga gtggtgtcaa atctttgacg 240
gcacagatgc ctgtgtgact ccggttctga cttttgagga ggttgttcat catgatcaca 300
acaangaacg gggctcgttt atcaccantg aggagcagga cgtgagcccc cgccctgcac 360
ctctgctgtt aaacacccca gccatccctt ctttcaaaag ggatccacta cttctagagc 420
ggncgccacc gcggtggagc tccagctttt gttcccttta gtgagggtta attgcgcgct 480
tggcgtaatc atggtcatan ctgtttcctg tgtgaaattg ttatccgctc acaattccac 540
acaacatacg anccggaagc atnaaatttt aaagcctggn ggtngcctaa tgantgaact 600
nactcacatt aattggcttt gcgctcactg cccgctttcc agtccggaaa acctgtcctt 660
gccagctgcc nttaatgaat cnggccaccc cccggggaaa aggcngtttg cttnttgggg 720
cgcncttccc gctttctcgc ttcctgaant ccttcccccc ggtctttcgg cttgcggcna 780
acggtatcna cct 793

<210> 34

<211> 756
<212> DNA
<213> Homo sapien

<220>
<221> misc_feature
<222> (1)...(756)
<223> n = A,T,C or G

<400> 34
gccgcgaccg gcatgtacga gcaactcaag ggcgagtgga accgtaaaag ccccaatctt 60
ancaagtgcg gggaanagct gggtcgactc aagctagttc ttctggagct caacttcttg 120
ccaaccacag ggaccaagct gaccaaacag cagctaattc tggcccgtga catactggag 180
atcggggccc aatggagcat cctacgcaan gacatcccct ccttcgagcg ctacatggcc 240
cagctcaaat gctactactt tgattacaan gagcagctcc ccgagtcagc ctatatgcac 300
cagctcttgg gcctcaacct cctcttcctg ctgtcccaga accgggtggc tgantnccac 360
acggganttgg ancggctgcc tgcccaanga catacanacc aatgtctaca tcnaccacca 420
gtgtcctgga gcaatactga tgganggcag ctaccncaaa gtnttcctgg ccnagggtaa 480
catcccccgc cgagagctac accttcttca ttgacatcct gctcgacact atcagggatg 540
aaaatcgcng ggttgctcca gaaaggctnc aanaanatcc ttttcnctga aggcccccgg 600
atncnctagt nctagaatcg gcccgccatc gcggtgganc ctccaacctt tcgttnccct 660
ttactgaggg ttnattgccg cccttggcgt tatcatggtc acnccngttn cctgtgttga 720
aattnttaac cccccacaat tccacgccna cattng 756

<210> 35
<211> 834
<212> DNA
<213> Homo sapien

<220>
<221> misc_feature
<222> (1)...(834)
<223> n = A,T,C or G

<400> 35
ggggatctct anatcnacct gnatgcatgg ttgtcggtgt ggtcgctgtc gatgaanatg 60
aacaggatct tgcccttgaa gctctcggct gctgtnttta agttgctcag tctgccgtca 120
tagtcagaca cnctcttggg caaaaaacan caggatntga gtcttgattt cacctccaat 180
aatcttcngg gctgtctgct cggtgaactc gatgacnang ggcagctggt tgtgtntgat 240
aaantccanc angttctcct tggtgacctc cccttcaaag ttgttccggc cttcatcaaa 300
cttctnnaan angannancc canctttgtc gagctggnat ttgganaaca cgtcactgtt 360
ggaaactgat cccaaatggt atgtcatcca tcgcctctgc tgcctgcaaa aaacttgctt 420
ggcncaaatc cgactccccn tccttgaaag aagccnatca cacccccctc cctggactcc 480
nncaangact ctnccgctnc cccntccnng cagggttggt ggcannccgg gcccntgcgc 540
ttcttcagcc agttcacnat nttcatcagc ccctctgcca gctgttntat tccttggggg 600
ggaanccgtc tctcccttcc tgaannaact ttgaccgtng gaatagccgc gcntcnccnt 660
acntnctggg ccgggttcaa antccctccn ttgncnntcn cctcgggcca ttctggattt 720
nccnaacttt ttccttcccc cnccccncgg ngtttggntt tttcatnggg ccccaactct 780
gctnttggcc antcccctgg gggcntntan cnccccctnt ggtcccntng ggcc 834

<210> 36
<211> 814
<212> DNA
<213> Homo sapien

<220>
<221> misc_feature
<222> (1)...(814)
<223> n = A,T,C or G

<400> 36
cggncgcttt ccngccgcgc cccgtttcca tgacnaaggc tcccttcang ttaaatacnn 60

cctagnaaac attaatgggt tgctctacta atacatcata cnaaccagta agcctgccca 120
naacgccaac tcaggccatt cctaccaaag gaagaaaggc tggtctctcc accccctgta 180
ggaaaggcct gccttgtaag acaccacaat ncggctgaat ctnaagtctt gtgttttact 240
aatggaaaaa aaaaataaac aanaggtttt gttctcatgg ctgcccaccg cagcctggca 300
ctaaaacanc ccagcgctca cttctgcttg ganaaatatt ctttgctctt ttggacatca 360
ggcttgatgg tatcactgcc acntttccac ccagctgggc ncccttcccc catntttgtc 420
antganctgg aaggcctgaa ncttagtctc caaaagtctc ngcccacaag accggccacc 480
aggggangtc ntttncagtg gatctgccaa anantacccn tatcatcnnt gaataaaaag 540
gcccctgaac ganatgcttc cancancctt taagacccat aatcctngaa ccatggtgcc 600
cttccggtct gatccnaaag gaatgttcct gggtcccant ccctcctttg ttncttacgt 660
tgtnttggac ccntgctngn atnacccaan tganatcccc ngaagcaccc tncccctggc 720
atttganttt cntaaattct ctgccctacn nctgaaagca cnattccctn ggcnccnaan 780
ggngaactca agaaggtctn ngaaaaacca cncn 814

<210> 37
<211> 760
<212> DNA
<213> Homo sapien

<220>
<221> misc_feature
<222> (1)...(760)
<223> n = A,T,C or G

<400> 37
gcatgctgct cttcctcaaa gttgttcttg ttgccataac aaccaccata ggtaaagcgg 60
gcgcagtgtt cgctgaaggg gttgtagtac cagcgcggga tgctctcctt gcagagtcct 120
gtgtctggca ggtccacgca atgccctttg tcactgggga aatggatgcg ctggagctcg 180
tcnaanccac tcgtgtattt ttcacangca gcctcctccg aagcntccgg gcagttgggg 240
gtgtcgtcac actccactaa actgtcgatn cancagccca ttgctgcagc ggaactgggt 300
gggctgacag gtgccagaac acactggatn ggcctttcca tggaagggcc tgggggaaat 360
cncctnancc caaactgcct ctcaaaggcc accttgcaca ccccgacagg ctagaaatgc 420
actcttcttc ccaaaggtag ttgttcttgt tgcccaagca ncctccanca aaccaaaanc 480
ttgcaaaatc tgctccgtgg gggtcatnnn taccanggtt ggggaaanaa acccggcngn 540
ganccncctt gtttgaatgc naaggnaata atcctcctgt cttgcttggg tggaanagca 600
caattgaact gttaacnttg ggccgngttc cnctngggtg gtctgaaact aatcaccgtc 660
actggaaaaa ggtangtgcc ttccttgaat tcccaaantt cccctngntt tgggtnnttt 720
ctcctctncc ctaaaaatcg tnttcccccc ccntanggcg 760

<210> 38
<211> 724
<212> DNA
<213> Homo sapien

<220>
<221> misc_feature
<222> (1)...(724)
<223> n = A,T,C or G

<400> 38
tttttttttt tttttttttt tttttttttt tttttaaaaa ccccctccat tgaatgaaaa 60
cttccnaaat tgtccaaccc cctcnnccaa atnnccattt ccgggggggg gttccaaacc 120
caaattaatt ttgganttta aattaaatnt tnattngggg aanaanccaa atgtnaagaa 180
aatttaaccc attatnaact taaatncctn gaaacccntg gnttccaaaa atttttaacc 240
cttaaatccc tccgaaattg ntaanggaaa accaaattcn cctaaggctn tttgaaggtt 300
ngatttaaac ccccttnant tnttttnacc cnngnctnaa ntatttngnt tccggtgttt 360
tcctnttaan cntnggtaac tcccgntaat gaannnccct aanccaatta aaccgaattt 420
tttttgaatt ggaaattccn ngggaattna ccggggtttt tcccntttgg gggccatncc 480
cccnctttcg gggtttgggn ntaggttgaa tttttnnang ncccaaaaaa ncccccaana 540
aaaaaactcc caagnnttaa ttngaatntc ccccttccca ggccttttgg gaaaggnggg 600
tttntggggg ccngggantt cnttcccccn ttnccncccc ccccccnggt aaanggttat 660
ngnntttggt ttttgggccc cttnanggac cttccggatn gaaattaaat ccccgggncg 720

gccg                                                              724

<210> 39
<211> 751
<212> DNA
<213> Homo sapien

<220>
<221> misc_feature
<222> (1)...(751)
<223> n = A,T,C or G

<400> 39
tttttttttt tttttctttg ctcacattta atttttattt tgattttttt taatgctgca      60
caacacaata tttatttcat ttgtttcttt tatttcattt tatttgtttg ctgctgctgt     120
tttatttatt tttactgaaa gtgagaggga acttttgtgg ccttttttcc tttttctgta     180
ggccgcctta agctttctaa atttggaaca tctaagcaag ctgaanggaa aagggggttt     240
cgcaaaatca ctcgggggaa nggaaaggtt gctttgttaa tcatgcccta tggtgggtga     300
ttaactgctt gtacaattac ntttcacttt taattaattg tgctnaangc tttaattana     360
cttgggggtt ccctccccan accaaccccn ctgacaaaaa gtgccngccc tcaaatnatg     420
tcccggcnnt cnttgaaaca cacngcngaa ngttctcatt ntccccncnc caggtnaaaa     480
tgaagggtta ccatntttaa cnccacctcc acntggcnnn gcctgaatcc tcnaaaancn     540
ccctcaancn aattnctnng ccccggtcnc gcntnngtcc cncccgggct ccgggaantn     600
cacccccnga anncnntnnc naacnaaatt ccgaaaatat tcccnntcnc tcaattcccc     660
cnnagactnt cctcnncnan cncaattttc ttttnntcac gaacncgnnc cnnaaaatgn     720
nnnncncctc cnctngtccn naatcnccan c                                    751

<210> 40
<211> 753
<212> DNA
<213> Homo sapien

<220>
<221> misc_feature
<222> (1)...(753)
<223> n = A,T,C or G

<400> 40
gtggtatttt ctgtaagatc aggtgttcct ccctcgtagg tttagaggaa acaccctcat      60
agatgaaaac ccccccgaga cagcagcact gcaactgcca agcagccggg gtaggagggg     120
cgccctatgc acagctgggc ccttgagaca gcagggcttc gatgtcaggc tcgatgtcaa     180
tggtctggaa gcggcggctg tacctgcgta ggggcacacc gtcagggccc accaggaact     240
tctcaaagtt ccaggcaacn tcgttgcgac acaccggaga ccaggtgatn agcttggggt     300
cggtcataan cgcggtggcg tcgtcgctgg gagctggcag ggcctcccgc aggaaggcna     360
ataaaaggtg cgcccccgca ccgttcanct cgcacttctc naanaccatg angttgggct     420
cnaacccacc accannccgg acttccttga nggaattccc aaatctcttc gntcttgggc     480
ttctnctgat gccctanctg gttgcccngn atgccaanca nccccaancc ccggggtcct     540
aaancacccn cctcctcntt tcatctgggt tnttntcccc ggaccntggt tcctctcaag     600
ggancccata tctcnaccan tactcaccnt ncccccccnt gnnacccanc cttctanngn     660
ttcccncccg ncctctggcc cntcaaanan gcttncacna cctgggtctg ccttcccccc     720
tnccctatct gnaccccncn tttgtctcan tnt                                  753

<210> 41
<211> 341
<212> DNA
<213> Homo sapien

<400> 41
actatatcca tcacaacaga catgcttcat cccatagact tcttgacata gcttcaaatg      60
agtgaaccca tccttgattt atatacatat atgttctcag tattttggga gcctttccac     120
ttctttaaac cttgttcatt atgaacactg aaaataggaa tttgtgaaga gttaaaaagt     180
tatagcttgt ttacgtagta agtttttgaa gtctacattc aatccagaca cttagttgag     240

tgttaaactg tgatttttaa aaaatatcat ttgagaatat tctttcagag gtattttcat 300
ttttactttt tgattaattg tgttttatat attagggtag t 341

<210> 42
<211> 101
<212> DNA
<213> Homo sapien

<400> 42
acttactgaa tttagttctg tgctcttcct tatttagtgt tgtatcataa atactttgat 60
gtttcaaaca ttctaaataa ataattttca gtggcttcat a 101

<210> 43
<211> 305
<212> DNA
<213> Homo sapien

<400> 43
acatctttgt tacagtctaa gatgtgttct taaatcacca ttccttcctg gtcctcaccc 60
tccagggtgg tctcacactg taattagagc tattgaggag tctttacagc aaattaagat 120
tcagatgcct tgctaagtct agagttctag agttatgttt cagaaagtct aagaaaccca 180
cctcttgaga ggtcagtaaa gaggacttaa tatttcatat ctacaaaatg accacaggat 240
tggatacaga acgagagtta tcctggataa ctcagagctg agtacctgcc cgggggccgc 300
tcgaa 305

<210> 44
<211> 852
<212> DNA
<213> Homo sapien

<220>
<221> misc_feature
<222> (1)...(852)
<223> n = A,T,C or G

<400> 44
acataaatat cagagaaaag tagtctttga aatatttacg tccaggagtt ctttgtttct 60
gattatttgg tgtgtgtttt ggtttgtgtc caaagtattg gcagcttcag ttttcatttt 120
ctctccatcc tcgggcattc ttcccaaatt tatataccag tcttcgtcca tccacacgct 180
ccagaatttc tcttttgtag taatatctca tagctcggct gagcttttca taggtcatgc 240
tgctgttgtt cttcttttta ccccatagct gagccactgc ctctgatttc aagaacctga 300
agacgccctc agatcggtct tcccatttta ttaatcctgg gttcttgtct gggttcaaga 360
ggatgtcgcg gatgaattcc cataagtgag tccctctcgg gttgtgcttt ttggtgtggc 420
acttggcagg ggggtcttgc tcctttttca tatcaggtga ctctgcaaca ggaaggtgac 480
tggtggttgt catggagatc tgagcccggc agaaagtttt gctgtccaac aaatctactg 540
tgctaccata gttggtgtca tataaatagt tctngtcttt ccaggtgttc atgatggaag 600
gctcagtttg ttcagtcttg acaatgacat tgtgtgtgga ctggaacagg tcactactgc 660
actggccgtt ccacttcaga tgctgcaagt tgctgtagag gagntgcccc gccgtccctg 720
ccgcccgggt gaactcctgc aaactcatgc tgcaaaggtg ctcgccgttg atgtcgaact 780
cntggaaagg gatacaattg gcatccagct ggttggtgtc caggaggtga tggagccact 840
cccacacctg gt 852

<210> 45
<211> 234
<212> DNA
<213> Homo sapien

<400> 45
acaacagacc cttgctcgct aacgacctca tgctcatcaa gttggacgaa tccgtgtccg 60
agtctgacac catccggagc atcagcattg cttcgcagtg ccctaccgcg gggaactctt 120
gcctcgtttc tggctggggt ctgctggcga acggcagaat gcctaccgtg ctgcagtgcg 180
tgaacgtgtc ggtggtgtct gaggaggtct gcagtaagct ctatgacccg ctgt 234

<210> 46
<211> 590
<212> DNA
<213> Homo sapien

<220>
<221> misc_feature
<222> (1)...(590)
<223> n = A,T,C or G

<400> 46
actttttatt taaatgttta taaggcagat ctatgagaat gatagaaaac atggtgtgta 60
atttgatagc aatattttgg agattacaga gttttagtaa ttaccaatta cacagttaaa 120
aagaagataa tatattccaa gcanatacaa aatatctaat gaaagatcaa ggcaggaaaa 180
tgantataac taattgacaa tggaaaatca attttaatgt gaattgcaca ttatccttta 240
aaagctttca aaanaaanaa ttattgcagt ctanttaatt caaacagtgt taaatggtat 300
caggataaan aactgaaggg canaaagaat taattttcac ttcatgtaac ncacccanat 360
ttacaatggc ttaaatgcan ggaaaaagca gtggaagtag ggaagtantc aaggtctttc 420
tggtctctaa tctgccttac tctttgggtg tggctttgat cctctggaga cagctgccag 480
ggctcctgtt atatccacaa tcccagcagc aagatgaagg gatgaaaaag gacacatgct 540
gccttccttt gaggagactt catctcactg gccaacactc agtcacatgt 590

<210> 47
<211> 774
<212> DNA
<213> Homo sapien

<220>
<221> misc_feature
<222> (1)...(774)
<223> n = A,T,C or G

<400> 47
acaagggggc ataatgaagg agtggggana gattttaaag aaggaaaaaa aacgaggccc 60
tgaacagaat tttcctgnac aacggggctt caaaataatt ttcttgggga ggttcaagac 120
gcttcactgc ttgaaactta aatggatgtg ggacanaatt ttctgtaatg accctgaggg 180
cattacagac gggactctgg gaggaaggat aaacagaaag gggacaaagg ctaatcccaa 240
aacatcaaag aaaggaaggt ggcgtcatac ctcccagcct acacagttct ccagggctct 300
cctcatccct ggaggacgac agtggaggaa caactgacca tgtccccagg ctcctgtgtg 360
ctggctcctg gtcttcagcc cccagctctg gaagcccacc ctctgctgat cctgcgtggc 420
ccacactcct tgaacacaca tccccaggtt atattcctgg acatggctga acctcctatt 480
cctacttccg agatgccttg ctccctgcag cctgtcaaaa tcccactcac cctccaaacc 540
acggcatggg aagcctttct gacttgcctg attactccag catcttggaa caatccctga 600
ttccccactc cttagaggca agatagggtg gttaagagta gggctggacc acttggagcc 660
aggctgctgg cttcaaattn tggctcattt acgagctatg ggaccttggg caagtnatct 720
tcacttctat gggcntcatt ttgttctacc tgcaaaatgg gggataataa tagt 774

<210> 48
<211> 124
<212> DNA
<213> Homo sapien

<220>
<221> misc_feature
<222> (1)...(124)
<223> n = A,T,C or G

<400> 48
canaaattga aattttataa aaaggcattt ttctcttata tccataaaat gatataattt 60
ttgcaantat anaaatgtgt cataaattat aatgttcctt aattacagct caacgcaact 120
tggt 124

<210> 49
<211> 147
<212> DNA
<213> Homo sapien

<220>
<221> misc_feature
<222> (1)...(147)
<223> n = A,T,C or G

<400> 49
gccgatgcta ctattttatt gcaggaggtg ggggtgtttt tattattctc tcaacagctt 60
tgtggctaca ggtggtgtct gactgcatna aaaanttttt tacgggtgat tgcaaaaatt 120
ttagggcacc catatcccaa gcantgt 147

<210> 50
<211> 107
<212> DNA
<213> Homo sapien

<400> 50
acattaaatt aataaaagga ctgttggggt tctgctaaaa cacatggctt gatatattgc 60
atggtttgag gttaggagga gttaggcata tgttttggga gaggggt 107

<210> 51
<211> 204
<212> DNA
<213> Homo sapien

<400> 51
gtcctaggaa gtctagggga cacacgactc tggggtcacg gggccgacac acttgcacgg 60
cgggaaggaa aggcagagaa gtgacaccgt cagggggaaa tgacagaaag gaaaatcaag 120
gccttgcaag gtcagaaagg ggactcaggg cttccaccac agccctgccc cacttggcca 180
cctccctttt gggaccagca atgt 204

<210> 52
<211> 491
<212> DNA
<213> Homo sapien

<220>
<221> misc_feature
<222> (1)...(491)
<223> n = A,T,C or G

<400> 52
acaaagataa catttatctt ataacaaaaa tttgatagtt ttaaaggtta gtattgtgta 60
gggtattttc caaaagacta aagagataac tcaggtaaaa agttagaaat gtataaaaca 120
ccatcagaca ggtttttaaa aaacaacata ttacaaaatt agacaatcat ccttaaaaaa 180
aaaacttctt gtatcaattt cttttgttca aaatgactga cttaantatt tttaaatatt 240
tcanaaacac ttcctcaaaa attttcaana tggtagcttt canatgtncc ctcagtccca 300
atgttgctca gataaataaa tctcgtgaga acttaccacc caccacaagc tttctggggc 360
atgcaacagt gtcttttctt tnctttttct tttttttttt ttacaggcac agaaactcat 420
caattttatt tggataacaa aggtctccca aattatattg aaaaataaat ccaagttaat 480
atcactcttg t 491

<210> 53
<211> 484
<212> DNA
<213> Homo sapien

<220>
<221> misc_feature
<222> (1)...(484)
<223> n = A,T,C or G

<400> 53
acataattta gcagggctaa ttaccataag atgctattta ttaanaggtn tatgatctga 60
gtattaacag ttgctgaagt ttggtatttt tatgcagcat tttctttttg ctttgataac 120
actacagaac ccttaaggac actgaaaatt agtaagtaaa gttcagaaac attagctgct 180
caatcaaatc tctacataac actatagtaa ttaaaacgtt aaaaaaaagt gttgaaatct 240
gcactagtat anaccgctcc tgtcaggata anactgcttt ggaacagaaa gggaaaaanc 300
agctttgant ttctttgtgc tgatangagg aaaggctgaa ttaccttgtt gcctctccct 360
aatgattggc aggtcnggta aatnccaaaa catattccaa ctcaacactt cttttccncg 420
tancttgant ctgtgtattc caggancagg cggatggaat gggccagccc ncggatgttc 480
cant 484

<210> 54
<211> 151
<212> DNA
<213> Homo sapien

<400> 54
actaaacctc gtgcttgtga actccataca gaaaacggtg ccatccctga acacggctgg 60
ccactgggta tactgctgac aaccgcaaca acaaaaacac aaatccttgg cactggctag 120
tctatgtcct ctcaagtgcc tttttgtttg t 151

<210> 55
<211> 91
<212> DNA
<213> Homo sapien

<400> 55
acctggcttg tctccgggtg gttcccggcg ccccccacgg tccccagaac ggacactttc 60
gccctccagt ggatactcga gccaaagtgg t 91

<210> 56
<211> 133
<212> DNA
<213> Homo sapien

<400> 56
ggcggatgtg cgttggttat atacaaatat gtcattttat gtaagggact tgagtatact 60
tggatttttg gtatctgtgg gttgggggga cggtccagga accaataccc catggatacc 120
aagggacaac tgt 133

<210> 57
<211> 147
<212> DNA
<213> Homo sapien

<220>
<221> misc_feature
<222> (1)...(147)
<223> n = A,T,C or G

<400> 57
actctggaga acctgagccg ctgctccgcc tctgggatga ggtgatgcan gcngtggcgc 60
gactgggagc tgagcccttc cctttgcgcc tgcctcagag gattgttgcc gacntgcana 120
tctcantggg ctggatncat gcagggt 147

<210> 58
<211> 198

<212> DNA
<213> Homo sapien

<220>
<221> misc_feature
<222> (1)...(198)
<223> n = A,T,C or G

<400> 58
acagggatat aggtttnaag ttattgtnat tgtaaaatac attgaatttt ctgtatactc 60
tgattacata catttatcct ttaaaaaaga tgtaaatctt aatttttatg ccatctatta 120
atttaccaat gagttacctt gtaaatgaga agtcatgata gcactgaatt ttaactagtt 180
ttgacttcta agtttggt 198

<210> 59
<211> 330
<212> DNA
<213> Homo sapien

<400> 59
acaacaaatg ggttgtgagg aagtcttatc agcaaaactg gtgatggcta ctgaaaagat 60
ccattgaaaa ttatcattaa tgattttaaa tgacaagtta tcaaaaactc actcaatttt 120
cacctgtgct agcttgctaa aatgggagtt aactctagag caaatatagt atcttctgaa 180
tacagtcaat aaatgacaaa gccagggcct acaggtggtt tccagacttt ccagacccag 240
cagaaggaat ctattttatc acatggatct ccgtctgtgc tcaaaatacc taatgatatt 300
tttcgtcttt attggacttc tttgaagagt 330

<210> 60
<211> 175
<212> DNA
<213> Homo sapien

<400> 60
accgtgggtg ccttctacat tcctgacggc tccttcacca acatctggtt ctacttcggc 60
gtcgtgggct ccttcctctt catcctcatc cagctggtgc tgctcatcga ctttgcgcac 120
tcctggaacc agcggtggct gggcaaggcc gaggagtgcg attcccgtgc ctggt 175

<210> 61
<211> 154
<212> DNA
<213> Homo sapien

<400> 61
accccacttt tcctcctgtg agcagtctgg acttctcact gctacatgat gagggtgagt 60
ggttgttgct cttcaacagt atcctcccct ttccggatct gctgagccgg acagcagtgc 120
tggactgcac agccccgggg ctccacattg ctgt 154

<210> 62
<211> 30
<212> DNA
<213> Homo sapien

<400> 62
cgctcgagcc ctatagtgag tcgtattaga 30

<210> 63
<211> 89
<212> DNA
<213> Homo sapien

<400> 63
acaagtcatt tcagcaccct ttgctcttca aaactgacca tcttttatat ttaatgcttc 60

ctgtatgaat aaaaatggtt atgtcaagt 89

<210> 64
<211> 97
<212> DNA
<213> Homo sapien

<400> 64
accggagtaa ctgagtcggg acgctgaatc tgaatccacc aataaataaa ggttctgcag 60
aatcagtgca tccaggattg gtccttggat ctggggt 97

<210> 65
<211> 377
<212> DNA
<213> Homo sapien

<220>
<221> misc_feature
<222> (1)...(377)
<223> n = A,T,C or G

<400> 65
acaacaanaa ntcccttctt taggccactg atggaaacct ggaaccccct tttgatggca 60
gcatggcgtc ctaggccttg acacagcggc tggggtttgg gctntcccaa accgcacacc 120
ccaaccctgg tctacccaca nttctggcta tgggctgtct ctgccactga acatcagggt 180
tcggtcataa natgaaatcc caanggggac agaggtcagt agaggaagct caatgagaaa 240
ggtgctgttt gctcagccag aaaacagctg cctggcattc gccgctgaac tatgaacccg 300
tgggggtgaa ctacccccan gaggaatcat gcctgggcga tgcaanggtg ccaacaggag 360
gggcgggagg agcatgt 377

<210> 66
<211> 305
<212> DNA
<213> Homo sapien

<400> 66
acgcctttcc ctcagaattc agggaagaga ctgtcgcctg ccttcctccg ttgttgcgtg 60
agaacccgtg tgccccttcc caccatatcc accctcgctc catctttgaa ctcaaacacg 120
aggaactaac tgcaccctgg tcctctcccc agtccccagt tcaccctcca tccctcacct 180
tcctccactc taagggatat caacactgcc cagcacaggg gccctgaatt tatgtggttt 240
ttatatattt tttaataaga tgcactttat gtcatttttt aataaagtct gaagaattac 300
tgttt 305

<210> 67
<211> 385
<212> DNA
<213> Homo sapien

<400> 67
actacacaca ctccacttgc ccttgtgaga cactttgtcc cagcacttta ggaatgctga 60
ggtcggacca gccacatctc atgtgcaaga ttgcccagca gacatcaggt ctgagagttc 120
cccttttaaa aaaggggact tgcttaaaaa agaagtctag ccacgattgt gtagagcagc 180
tgtgctgtgc tggagattca cttttgagag agttctcctc tgagacctga tctttagagg 240
ctgggcagtc ttgcacatga gatggggctg gtctgatctc agcactcctt agtctgcttg 300
cctctcccag ggccccagcc tggccacacc tgcttacagg gcactctcag atgcccatac 360
catagtttct gtgctagtgg accgt 385

<210> 68
<211> 73
<212> DNA
<213> Homo sapien

<400> 68
acttaaccag atatattttt accccagatg gggatattct ttgtaaaaaa tgaaaataaa 60
gtttttttaa tgg 73

<210> 69
<211> 536
<212> DNA
<213> Homo sapien

<220>
<221> misc_feature
<222> (1)...(536)
<223> n = A,T,C or G

<400> 69
actagtccag tgtggtggaa ttccattgtg ttgggggctc tcaccctcct ctcctgcagc 60
tccagctttg tgctctgcct ctgaggagac catggcccag catctgagta ccctgctgct 120
cctgctggcc accctagctg tggccctggc ctggagcccc aaggaggagg ataggataat 180
cccgggtggc atctataacg cagacctcaa tgatgagtgg gtacagcgtg cccttcactt 240
cgccatcagc gagtataaca aggccaccaa agatgactac tacagacgtc cgctgcgggt 300
actaagagcc aggcaacaga ccgttggggg ggtgaattac ttcttcgacg tagaggtggg 360
ccgaaccata tgtaccaagt cccagcccaa cttggacacc tgtgccttcc atgaacagcc 420
agaactgcag aagaaacagt tgtgctcttt cgagatctac gaagttccct ggggagaaca 480
gaangtccct gggtgaaatc caggtgtcaa gaaatcctan ggatctgttg ccaggc 536

<210> 70
<211> 477
<212> DNA
<213> Homo sapien
<400> 70
atgaccccta acaggggccc tctcagccct cctaatgacc tccggcctag ccatgtgatt 60
tcacttccac tccataacgc tcctcatact aggcctacta accaacacac taaccatata 120
ccaatgatgg cgcgatgtaa cacgagaaag cacataccaa ggccaccaca caccacctgt 180
ccaaaaaggc cttcgatacg ggataatcct atttattacc tcagaagttt ttttcttcgc 240
aggatttttt ctgagccttt taccactcca gcctagcccc taccccccaa ctaggagggc 300
actggccccc aacaggcatc accccgctaa atcccctaga agtcccactc ctaaacacat 360
ccgtattact cgcatcagga gtatcaatca cctgagctca ccatagtcta atagaaaaca 420
accgaaacca aattattcaa agcactgctt attacaattt tactgggtct ctatttt 477

<210> 71
<211> 533
<212> DNA
<213> Homo sapien

<220>
<221> misc_feature
<222> (1)...(533)
<223> n = A,T,C or G

<400> 71
agagctatag gtacagtgtg atctcagctt tgcaaacaca ttttctacat agatagtact 60
aggtattaat agatatgtaa agaaagaaat cacaccatta ataatggtaa gattggttta 120
tgtgatttta gtggtatttt tggcaccctt atatatgttt tccaaacttt cagcagtgat 180
attatttcca taacttaaaa agtgagtttg aaaaagaaaa tctccagcaa gcatctcatt 240
taaataaagg tttgtcatct ttaaaaatac agcaatatgt gactttttaa aaaagctgtc 300
aaataggtgt gaccctacta ataattatta gaaatacatt taaaaacatc gagtacctca 360
agtcagtttg ccttgaaaaa tatcaaatat aactcttaga gaaatgtaca taaaagaatg 420
cttcgtaatt ttggagtang aggttccctc ctcaattttg tatttttaaa aagtacatgg 480
taaaaaaaaa aattcacaac agtatataag gctgtaaaat gaagaattct gcc 533

<210> 72
<211> 511

<212> DNA
<213> Homo sapien

<220>
<221> misc_feature
<222> (1)...(511)
<223> n = A,T,C or G

<400> 72
tattacggaa aaacacacca cataattcaa ctancaaaga anactgcttc agggcgtgta 60
aaatgaaagg cttccaggca gttatctgat taaagaacac taaaagaggg acaaggctaa 120
aagccgcagg atgtctacac tatancaggc gctatttggg ttggctggag gagctgtgga 180
aaacatggan agattggtgc tgganatcgc cgtggctatt cctcattgtt attacanagt 240
gaggttctct gtgtgcccac tggtttgaaa accgttctnc aataatgata gaatagtaca 300
cacatgagaa ctgaaatggc ccaaacccag aaagaaagcc caactagatc ctcagaanac 360
gcttctaggg acaataaccg atgaagaaaa gatggcctcc ttgtgccccc gtctgttatg 420
atttctctcc attgcagcna naaacccgtt cttctaagca aacncaggtg atgatggcna 480
aaatacaccc cctcttgaag naccnggagg a 511

<210> 73
<211> 499
<212> DNA
<213> Homo sapien

<220>
<221> misc_feature
<222> (1)...(499)
<223> n = A,T,C or G

<400> 73
cagtgccagc actggtgcca gtaccagtac caataacagt gccagtgcca gtgccagcac 60
cagtggtggc ttcagtgctg gtgccagcct gaccgccact ctcacatttg ggctcttcgc 120
tggccttggt ggagctggtg ccagcaccag tggcagctct ggtgcctgtg gtttctccta 180
caagtgagat tttagatatt gttaatcctg ccagtctttc tcttcaagcc agggtgcatc 240
ctcagaaacc tactcaacac agcactctag gcagccacta tcaatcaatt gaagttgaca 300
ctctgcatta aatctatttg ccatttctga aaaaaaaaaa aaaaaaaggg cggccgctcg 360
antctagagg gcccgtttaa acccgctgat cagcctcgac tgtgccttct anttgccagc 420
catctgttgt ttgcccctcc cccgntgcct tccttgaccc tggaaagtgc cactcccact 480
gtcctttcct aantaaaat 499

<210> 74
<211> 537
<212> DNA
<213> Homo sapien

<220>
<221> misc_feature
<222> (1)...(537)
<223> n = A,T,C or G

<400> 74
tttcatagga gaacacactg aggagatact tgaagaattt ggattcagcc gcgaagagat 60
ttatcagctt aactcagata aaatcattga aagtaataag gtaaaagcta gtctctaact 120
tccaggccca cggctcaagt gaatttgaat actgcattta cagtgtagag taacacataa 180
cattgtatgc atggaaacat ggaggaacag tattacagtg tcctaccact ctaatcaaga 240
aaagaattac agactctgat tctacagtga tgattgaatt ctaaaaatgg taatcattag 300
ggcttttgat ttataanact ttgggtactt atactaaatt atggtagtta tactgccttc 360
cagtttgctt gatatatttg ttgatattaa gattcttgac ttatattttg aatgggttct 420
actgaaaaan gaatgatata ttcttgaaga catcgatata catttattta cactcttgat 480
tctacaatgt agaaaatgaa ggaaatgccc caaattgtat ggtgataaaa gtcccgt 537

<210> 75

<211> 467
<212> DNA
<213> Homo sapien

<220>
<221> misc_feature
<222> (1)...(467)
<223> n = A,T,C or G

<400> 75
caaanacaat tgttcaaaag atgcaaatga tacactactg ctgcagctca caaacacctc 60
tgcatattac acgtacctcc tcctgctcct caagtagtgt ggtctatttt gccatcatca 120
cctgctgtct gcttagaaga acggctttct gctgcaangg agagaaatca taacagacgg 180
tggcacaagg aggccatctt ttcctcatcg gttattgtcc ctagaagcgt cttctgagga 240
tctagttggg ctttctttct gggtttgggc catttcantt ctcatgtgtg tactattcta 300
tcattattgt ataacggttt tcaaaccngt gggcacncag agaacctcac tctgtaataa 360
caatgaggaa tagccacggt gatctccagc accaaatctc tccatgttnt tccagagctc 420
ctccagccaa cccaaatagc cgctgctatn gtgtagaaca tccctgn 467

<210> 76
<211> 400
<212> DNA
<213> Homo sapien

<220>
<221> misc_feature
<222> (1)...(400)
<223> n = A,T,C or G

<400> 76
aagctgacag cattcgggcc gagatgtctc gctccgtggc cttagctgtg ctcgcgctac 60
tctctctttc tggcctggag gctatccagc gtactccaaa gattcaggtt tactcacgtc 120
atccagcaga gaatggaaag tcaaatttcc tgaattgcta tgtgtctggg tttcatccat 180
ccgacattga agttgactta ctgaagaatg gagagagaat tgaaaaagtg gagcattcag 240
acttgtcttt cagcaaggac tggtctttct atctcttgta ctacactgaa ttcaccccca 300
ctgaaaaaga tgagtatgcc tgccgtgtga accatgtgac tttgtcacag cccaagatng 360
ttnagtggga tcganacatg taagcagcan catgggaggt 400

<210> 77
<211> 248
<212> DNA
<213> Homo sapien

<400> 77
ctggagtgcc ttggtgtttc aagcccctgc aggaagcaga atgcaccttc tgaggcacct 60
ccagctgccc cggcggggga tgcgaggctc ggagcaccct tgcccggctg tgattgctgc 120
caggcactgt tcatctcagc ttttctgtcc ctttgctccc ggcaagcgct tctgctgaaa 180
gttcatatct ggagcctgat gtcttaacga ataaaggtcc catgctccac ccgaaaaaaa 240
aaaaaaaa 248

<210> 78
<211> 201
<212> DNA
<213> Homo sapien

<400> 78
actagtccag tgtggtggaa ttccattgtg ttgggcccaa cacaatggct acctttaaca 60
tcacccagac cccgccctgc ccgtgcccca cgctgctgct aacgacagta tgatgcttac 120
tctgctactc ggaaactatt tttatgtaat taatgtatgc tttcttgttt ataaatgcct 180
gatttaaaaa aaaaaaaaaa a 201

<210> 79

<211> 552
<212> DNA
<213> Homo sapien

<220>
<221> misc_feature
<222> (1)...(552)
<223> n = A,T,C or G

<400> 79
tccttttgtt aggtttttga gacaacccta gacctaaact gtgtcacaga cttctgaatg 60
tttaggcagt gctagtaatt tcctcgtaat gattctgtta ttactttcct attctttatt 120
cctctttctt ctgaagatta atgaagttga aaattgaggt ggataaatac aaaaaggtag 180
tgtgatagta taagtatcta agtgcagatg aaagtgtgtt atatatatcc attcaaaatt 240
atgcaagtta gtaattactc agggttaact aaattacttt aatatgctgt tgaacctact 300
ctgttccttg gctagaaaaa attataaaca ggactttgtt agtttgggaa gccaaattga 360
taatattcta tgttctaaaa gttgggctat acataaanta tnaagaaata tggaatttta 420
ttcccaggaa tatggggttc atttatgaat antacccggg anagaagttt tgantnaaac 480
cngttttggt taatacgtta atatgtcctn aatnaacaag gcntgactta tttccaaaaa 540
aaaaaaaaaa aa 552

<210> 80
<211> 476
<212> DNA
<213> Homo sapien

<220>
<221> misc_feature
<222> (1)...(476)
<223> n = A,T,C or G

<400> 80
acagggattt gagatgctaa ggccccagag atcgtttgat ccaaccctct tattttcaga 60
ggggaaaatg gggcctagaa gttacagagc atctagctgg tgcgctggca cccctggcct 120
cacacagact cccgagtagc tgggactaca ggcacacagt cactgaagca ggccctgttt 180
gcaattcacg ttgccacctc caacttaaac attcttcata tgtgatgtcc ttagtcacta 240
aggttaaact ttcccaccca gaaaaggcaa cttagataaa atcttagagt actttcatac 300
tcttctaagt cctcttccag cctcactttg agtcctcctt gggggttgat aggaantntc 360
tcttggcttt ctcaataaaa tctctatcca tctcatgttt aatttggtac gcntaaaaat 420
gctgaaaaaa ttaaaatgtt ctggtttcnc tttaaaaaaa aaaaaaaaaa aaaaaa 476

<210> 81
<211> 232
<212> DNA
<213> Homo sapien

<220>
<221> misc_feature
<222> (1)...(232)
<223> n = A,T,C or G

<400> 81
   tttttttttg tatgccntcn ctgtggngtt attgttgctg ccaccctgga ggagcccagt 60
   ttcttctgta tctttctttt ctgggggatc ttcctggctc tgcccctcca ttcccagcct 120
   ctcatcccca tcttgcactt ttgctagggt tggaggcgct ttcctggtag cccctcagag 180
   actcagtcag cgggaataag tcctaggggt gggggtgtgg gcaagccggc ct 232

<210> 82
<211> 383
<212> DNA
<213> Homo sapien

<220>
<221> misc_feature
<222> (1)...(383)
<223> n = A,T,C or G

<400> 82
aggcgggagc agaagctaaa gccaaagccc aagaagagtg gcagtgccag cactggtgcc 60
agtaccagta ccaataacat gccagtgcca gtgccagcac cagtggtggc ttcagtgctg 120
gtgccagcct gaccgccact ctcacatttg ggctcttcgc tggccttggt ggagctggtg 180
ccagcaccag tggcagctct ggtgcctgtg gtttctccta caagtgagat tttagatatt 240
gttaatcctg ccagtctttc tcttcaagcc agggtgcatc ctcagaaacc tactcaacac 300
agcactctng gcagccacta tcaatcaatt gaagttgaca ctctgcatta aatctatttg 360
ccatttcaaa aaaaaaaaaa aaa 383

<210> 83
<211> 494
<212> DNA
<213> Homo sapien

<220>
<221> misc_feature
<222> (1)...(494)
<223> n = A,T,C or G

<400> 83
accgaattgg gaccgctggc ttataagcga tcatgtcctc cagtattacc tcaacgagca 60
gggagatcga gtctatacgc tgaagaaatt tgacccgatg ggacaacaga cctgctcagc 120
ccatcctgct cggttctccc cagatgacaa atactctcga caccgaatca ccatcaagaa 180
acgcttcaag gtgctcatga cccagcaacc gcgccctgtc ctctgagggt ccttaaactg 240
atgtcttttc tgccacctgt tacccctcgg agactccgta accaaactct tcggactgtg 300
agccctgatg cctttttgcc agccatactc tttggcntcc agtctctcgt ggcgattgat 360
tatgcttgtg tgaggcaatc atggtggcat cacccatnaa gggaacacat ttganttttt 420
tttcncatat tttaaattac naccagaata nttcagaata aatgaattga aaaactctta 480
aaaaaaaaaa aaaa 494

<210> 84
<211> 380
<212> DNA
<213> Homo sapien

<220>
<221> misc_feature
<222> (1)...(380)
<223> n = A,T,C or G

<400> 84
gctggtagcc tatggcgtgg ccacggangg gctcctgagg cacgggacag tgacttccca 60
agtatcctgc gccgcgtctt ctaccgtccc tacctgcaga tcttcgggca gattccccag 120
gaggacatgg acgtggccct catggagcac agcaactgct cgtcggagcc cggcttctgg 180
gcacaccctc ctggggccca ggcgggcacc tgcgtctccc agtatgccaa ctggctggtg 240
gtgctgctcc tcgtcatctt cctgctcgtg gccaacatcc tgctggtcac ttgctcattg 300
ccatgttcag ttacacattc ggcaaagtac agggcaacag cnatctctac tgggaaggcc 360
agcgttnccg cctcatccgg 380

<210> 85
<211> 481
<212> DNA
<213> Homo sapien

<220>
<221> misc_feature
<222> (1)...(481)

<223> n = A,T,C or G

<400> 85
gagttagctc ctccacaacc ttgatgaggt cgtctgcagt ggcctctcgc ttcataccgc 60
tnccatcgtc atactgtagg tttgccacca cctcctgcat cttggggcgg ctaatatcca 120
ggaaactctc aatcaagtca ccgtcnatna aacctgtggc tggttctgtc ttccgctcgg 180
tgtgaaagga tctccagaag gagtgctcga tcttccccac acttttgatg actttattga 240
gtcgattctg catgtccagc aggaggttgt accagctctc tgacagtgag gtcaccagcc 300
ctatcatgcc nttgaacgtg ccgaagaaca ccgagccttg tgtggggggt gnagtctcac 360
ccagattctg cattaccaga nagccgtggc aaaaganatt gacaactcgc ccaggnngaa 420
aaagaacacc tcctggaagt gctngccgct cctcgtccnt tggtggnngc gcntnccttt 480
t 481

<210> 86
<211> 472
<212> DNA
<213> Homo sapien

<220>
<221> misc_feature
<222> (1)...(472)
<223> n = A,T,C or G

<400> 86
aacatcttcc tgtataatgc tgtgtaatat cgatccgatn ttgtctgctg agaattcatt 60
acttggaaaa gcaacttnaa gcctggacac tggtattaaa attcacaata tgcaacactt 120
taaacagtgt gtcaatctgc tcccttactt tgtcatcacc agtctgggaa taagggtatg 180
ccctattcac acctgttaaa agggcgctaa gcatttttga ttcaacatct tttttttga 240
cacaagtccg aaaaaagcaa aagtaaacag ttnttaattt gttagccaat tcactttctt 300
catgggacag agccatttga tttaaaaagc aaattgcata atattgagct ttgggagctg 360
atatntgagc ggaagantag cctttctact tcaccagaca caactccttt catattggga 420
tgttnacnaa agttatgtct cttacagatg ggatgctttt gtggcaattc tg 472

<210> 87
<211> 413
<212> DNA
<213> Homo sapien

<220>
<221> misc_feature
<222> (1)...(413)
<223> n = A,T,C or G

<400> 87
agaaaccagt atctctnaaa acaacctctc ataccttgtg gacctaattt tgtgtgcgtg 60
tgtgtgtgcg cgcatattat atagacaggc acatcttttt tacttttgta aaagcttatg 120
cctctttggt atctatatct gtgaaagttt taatgatctg ccataatgtc ttggggacct 180
ttgtcttctg tgtaaatggt actagagaaa acacctatnt tatgagtcaa tctagttngt 240
tttattcgac atgaaggaaa tttccagatn acaacactna caaactctcc cttgactagg 300
ggggacaaag aaaagcanaa ctgaacatna gaaacaattn cctggtgaga aattncataa 360
acagaaattg ggtngtatat tgaaananng catcattnaa acgttttttt ttt 413

<210> 88
<211> 448
<212> DNA
<213> Homo sapien

<220>
<221> misc_feature
<222> (1)...(448)
<223> n = A,T,C or G

<400> 88
cgcagcgggt cctctctatc tagctccagc ctctcgcctg ccccactccc cgcgtcccgc 60
gtcctagccn accatggccg ggcccctgcg cgccccgctg ctcctgctgg ccatcctggc 120
cgtggccctg gccgtgagcc ccgcggccgg ctccagtccc ggcaagccgc cgcgcctggt 180
gggaggccca tggaccccgc gtggaagaag aaggtgtgcg gcgtgcactg gactttgccg 240
tcggcnanta caacaaaccc gcaacnactt ttaccnagcn cgcgctgcag gttgtgccgc 300
cccaancaaa ttgttactng gggtaantaa ttcttggaag ttgaacctgg gccaaacnng 360
tttaccagaa ccnagccaat tngaacaatt ncccctccat aacagcccct tttaaaaagg 420
gaancantcc tgntcttttc caaatttt 448

<210> 89
<211> 463
<212> DNA
<213> Homo sapien

<220>
<221> misc_feature
<222> (1)...(463)
<223> n = A,T,C or G

<400> 89
gaattttgtg cactggccac tgtgatggaa ccattgggcc aggatgcttt gagtttatca 60
gtagtgattc tgccaaagtt ggtgttgtaa catgagtatg taaaatgtca aaaaattagc 120
agaggtctag gtctgcatat cagcagacag tttgtccgtg tattttgtag ccttgaagtt 180
ctcagtgaca agttnnttct gatgcgaagt tctnattcca gtgttttagt cctttgcatc 240
tttnatgttn agacttgcct ctntnaaatt gcttttgtnt tctgcaggta ctatctgtgg 300
tttaacaaaa tagaannact tctctgcttn gaanatttga atatcttaca tctnaaaatn 360
aattctctcc ccatannaaa acccangccc ttggganaat ttgaaaaang gntccttcnn 420
aattcnnana anttcagntn tcatacaaca naacngganc ccc 463

<210> 90
<211> 400
<212> DNA
<213> Homo sapien

<220>
<221> misc_feature
<222> (1)...(400)
<223> n = A,T,C or G

<400> 90
agggattgaa ggtctnttnt actgtcggac tgttcancca ccaactctac aagttgctgt 60
cttccactca ctgtctgtaa gcntnttaac ccagactgta tcttcataaa tagaacaaat 120
tcttcaccag tcacatcttc taggaccttt ttggattcag ttagtataag ctcttccact 180
tcctttgtta agacttcatc tggtaaagtc ttaagttttg tagaaaggaa tttaattgct 240
cgttctctaa caatgtcctc tccttgaagt atttggctga acaacccacc tnaagtccct 300
ttgtgcatcc attttaaata tacttaatag ggcattggtn cactaggtta aattctgcaa 360
gagtcatctg tctgcaaaag ttgcgttagt atatctgcca 400

<210> 91
<211> 480
<212> DNA
<213> Homo sapien

<220>
<221> misc_feature
<222> (1)...(480)
<223> n = A,T,C or G

<400> 91
gagctcggat ccaataatct ttgtctgagg gcagcacaca tatncagtgc catggnaact 60
ggtctacccc acatgggagc agcatgccgt agntatataa ggtcattccc tgagtcagac 120

atgcctcttt gactaccgtg tgccagtgct ggtgattctc acacacctcc nnccgctctt 180
tgtggaaaaa ctggcacttg nctggaacta gcaagacatc acttacaaat tcacccacga 240
gacacttgaa aggtgtaaca aagcgactct tgcattgctt tttgtccctc cggcaccagt 300
tgtcaatact aacccgctgg tttgcctcca tcacatttgt gatctgtagc tctggataca 360
tctcctgaca gtactgaaga acttcttctt ttgtttcaaa agcaactctt ggtgcctgtt 420
ngatcaggtt cccatttccc agtccgaatg ttcacatggc atatnttact tcccacaaaa 480

<210> 92
<211> 477
<212> DNA
<213> Homo sapien

<220>
<221> misc_feature
<222> (1)...(477)
<223> n = A,T,C or G

<400> 92
atacagccca natcccacca cgaagatgcg cttgttgact gagaacctga tgcggtcact 60
ggtcccgctg tagccccagc gactctccac ctgctggaag cggttgatgc tgcactcctt 120
cccacgcagg cagcagcggg gccggtcaat gaactccact cgtggcttgg ggttgacggt 180
taantgcagg aagaggctga ccacctcgcg gtccaccagg atgcccgact gtgcgggacc 240
tgcagcgaaa ctcctcgatg gtcatgagcg ggaagcgaat gangcccagg gccttgccca 300
gaaccttccg cctgttctct ggcgtcacct gcagctgctg ccgctnacac tcggcctcgg 360
accagcggac aaacggcgtt gaacagccgc acctcacgga tgcccantgt gtcgcgctcc 420
aggaacggcn ccagcgtgtc caggtcaatg tcggtgaanc ctccgcgggt aatggcg 477

<210> 93
<211> 377
<212> DNA
<213> Homo sapien

<220>
<221> misc_feature
<222> (1)...(377)
<223> n = A,T,C or G

<400> 93
gaacggctgg accttgcctc gcattgtgct gctggcagga ataccttggc aagcagctcc 60
agtccgagca gccccagacc gctgccgccc gaagctaagc ctgcctctgg ccttcccctc 120
cgcctcaatg cagaaccant agtgggagca ctgtgtttag agttaagagt gaacactgtn 180
tgattttact tgggaatttc ctctgttata tagcttttcc caatgctaat ttccaaacaa 240
caacaacaaa ataacatgtt tgcctgttna gttgtataaa agtangtgat tctgtatnta 300
aagaaaatat tactgttaca tatactgctt gcaanttctg tatttattgg tnctctggaa 360
ataaatatat tattaaa 377

<210> 94
<211> 495
<212> DNA
<213> Homo sapien

<220>
<221> misc_feature
<222> (1)...(495)
<223> n = A,T,C or G

<400> 94
ccctttgagg ggttagggtc cagttcccag tggaagaaac aggccaggag aantgcgtgc 60
cgagctgang cagatttccc acagtgaccc cagagccctg ggctatagtc tctgacccct 120
ccaaggaaag accaccttct ggggacatgg gctggagggc aggacctaga ggcaccaagg 180
gaaggcccca ttccggggct gttccccgag gaggaaggga aggggctctg tgtgcccccc 240
acgaggaana ggccctgant cctgggatca nacacccctt cacgtgtatc cccacacaaa 300

tgcaagctca ccaaggtccc ctctcagtcc cttccctaca ccctgaacgg ncactggccc 360
acacccaccc agancancca cccgccatgg ggaatgtnct caaggaatcg cngggcaacg 420
tggactctng tcccnnaagg gggcagaatc tccaatagan gganngaacc cttgctnana 480
aaaaaaaana aaaaa 495

<210> 95
<211> 472
<212> DNA
<213> Homo sapien

<220>
<221> misc_feature
<222> (1)...(472)
<223> n = A,T,C or G

<400> 95
ggttacttgg tttcattgcc accacttagt ggatgtcatt tagaaccatt ttgtctgctc 60
cctctggaag ccttgcgcag agcggacttt gtaattgttg gagaataact gctgaatttt 120
tagctgtttt gagttgattc gcaccactgc accacaactc aatatgaaaa ctatttnact 180
tatttattat cttgtgaaaa gtatacaatg aaaattttgt tcatactgta tttatcaagt 240
atgatgaaaa gcaatagata tatattcttt tattatgttn aattatgatt gccattatta 300
atcggcaaaa tgtggagtgt atgttctttt cacagtaata tatgcctttt gtaacttcac 360
ttggttattt tattgtaaat gaattacaaa attcttaatt taagaaaatg gtangttata 420
tttanttcan taatttcttt ccttgtttac gttaattttg aaaagaatgc at 472

<210> 96
<211> 476
<212> DNA
<213> Homo sapien

<220>
<221> misc_feature
<222> (1)...(476)
<223> n = A,T,C or G

<400> 96
ctgaagcatt tcttcaaact tntctacttt tgtcattgat acctgtagta agttgacaat 60
gtggtgaaat ttcaaaatta tatgtaactt ctactagttt tactttctcc cccaagtctt 120
ttttaactca tgatttttac acacacaatc cagaacttat tatatagcct ctaagtcttt 180
attcttcaca gtagatgatg aaagagtcct ccagtgtctt gngcanaatg ttctagntat 240
agctggatac atacngtggg agttctataa actcatacct cagtgggact naaccaaaat 300
tgtgttagtc tcaattccta ccacactgag ggagcctccc aaatcactat attcttatct 360
gcaggtactc ctccagaaaa acngacaggg caggcttgca tgaaaaagtn acatctgcgt 420
tacaaagtct atcttcctca nangtctgtn aaggaacaat ttaatcttct agcttt 476

<210> 97
<211> 479
<212> DNA
<213> Homo sapien

<220>
<221> misc_feature
<222> (1)...(479)
<223> n = A,T,C or G

<400> 97
actctttcta atgctgatat gatcttgagt ataagaatgc atatgtcact agaatggata 60
aaataatgct gcaaacttaa tgttcttatg caaaatggaa cgctaatgaa acacagctta 120
caatcgcaaa tcaaaactca caagtgctca tctgttgtag atttagtgta ataagactta 180
gattgtgctc cttcggatat gattgtttct canatcttgg gcaatnttcc ttagtcaaat 240
caggctacta gaattctgtt attggatatn tgagagcatg aaatttttaa naatacactt 300
gtgattatna aattaatcac aaatttcact tatacctgct atcagcagct agaaaaacat 360

ntnnttttta natcaaagta ttttgtgttt ggaantgtnn aaatgaaatc tgaatgtggg 420
ttcnatctta ttttttcccn gacnactant tnctttttta gggnctattc tganccatc 479

<210> 98
<211> 461
<212> DNA
<213> Homo sapien

<400> 98
agtgacttgt cctccaacaa aaccccttga tcaagtttgt ggcactgaca atcagaccta 60
tgctagttcc tgtcatctat tcgctactaa atgcagactg gaggggacca aaaaggggca 120
tcaactccag ctggattatt ttggagcctg caaatctatt cctacttgta cggactttga 180
agtgattcag tttcctctac ggatgagaga ctggctcaag aatatcctca tgcagcttta 240
tgaagccact ctgaacacgc tggttatcta gatgagaaca gagaaataaa gtcagaaaat 300
ttacctggag aaaagaggct ttggctgggg accatcccat tgaaccttct cttaaggact 360
ttaagaaaaa ctaccacatg ttgtgtatcc tggtgccggc cgtttatgaa ctgaccaccc 420
tttggaataa tcttgacgct cctgaacttg ctcctctgcg a 461

<210> 99
<211> 171
<212> DNA
<213> Homo sapien

<400> 99
gtggccgcgc gcaggtgttt cctcgtaccg cagggccccc tcccttcccc aggcgtccct 60
cggcgcctct gcgggcccga ggaggagcgg ctggcgggtg gggggagtgt gacccaccct 120
cggtgagaaa agccttctct agcgatctga gaggcgtgcc ttgggggtac c 171

<210> 100
<211> 269
<212> DNA
<213> Homo sapien

<400> 100
cggccgcaag tgcaactcca gctggggccg tgcggacgaa gattctgcca gcagttggtc 60
cgactgcgac gacggcggcg gcgacagtcg caggtgcagc gcgggcgcct ggggtcttgc 120
aaggctgagc tgacgccgca gaggtcgtgt cacgtcccac gaccttgacg ccgtcgggga 180
cagccggaac agagcccggt gaagcgggag gcctcgggga gcccctcggg aagggcggcc 240
cgagagatac gcaggtgcag gtggccgcc 269

<210> 101
<211> 405
<212> DNA
<213> Homo sapien

<400> 101
tttttttttt ttttggaatc tactgcgagc acagcaggtc agcaacaagt ttattttgca 60
gctagcaagg taacagggta gggcatggtt acatgttcag gtcaacttcc tttgtcgtgg 120
ttgattggtt tgtctttatg gggggcgggt ggggtagggg aaacgaagca aataacatgg 180
agtgggtgca ccctccctgt agaacctggt tacaaagctt ggggcagttc acctggtctg 240
tgaccgtcat tttcttgaca tcaatgttat tagaagtcag gatatctttt agagagtcca 300
ctgttctgga gggagattag ggtttcttgc caaatccaac aaaatccact gaaaaagttg 360
gatgatcagt acgaataccg aggcatattc tcatatcggt ggcca 405

<210> 102
<211> 470
<212> DNA
<213> Homo sapien

<400> 102
tttttttttt tttttttttt tttttttttt tttttttttt tttttttttt tttttttttt 60
ggcacttaat ccatttttat ttcaaaatgt ctacaaattt aatcccatta tacggtattt 120

tcaaaatcta aattattcaa attagccaaa tccttaccaa ataataccca aaaatcaaaa 180
atatacttct ttcagcaaac ttgttacata aattaaaaaa atatatacgg ctggtgtttt 240
caaagtacaa ttatcttaac actgcaaaca ttttaaggaa ctaaaataaa aaaaaacact 300
ccgcaaaggt taaagggaac aacaaattct tttacaacac cattataaaa atcatatctc 360
aaatcttagg ggaatatata cttcacacgg gatcttaact tttactcact ttgtttattt 420
ttttaaacca ttgtttgggc ccaacacaat ggaatccccc ctggactagt 470

<210> 103
<211> 581
<212> DNA
<213> Homo sapien

<400> 103
   tttttttttt ttttttttga cccccctctt ataaaaaaca agttaccatt ttattttact 60
   tacacatatt tattttataa ttggtattag atattcaaaa ggcagctttt aaaatcaaac 120
   taaatggaaa ctgccttaga tacataattc ttaggaatta gcttaaaatc tgcctaaagt 180
   gaaaatcttc tctagctctt ttgactgtaa atttttgact cttgtaaaac atccaaattc 240
   atttttcttg tctttaaaat tatctaatct ttccattttt tccctattcc aagtcaattt 300
   gcttctctag cctcatttcc tagctcttat ctactattag taagtggctt ttttcctaaa 360
   agggaaaaca ggaagagaaa tggcacacaa aacaaacatt ttatattcat atttctacct 420
   acgttaataa aatagcattt tgtgaagcca gctcaaaaga aggcttagat ccttttatgt 480
   ccattttagt cactaaacga tatcaaagtg ccagaatgca aaaggtttgt gaacatttat 540
   tcaaaagcta atataagata tttcacatac tcatctttct g 581

<210> 104
<211> 578
<212> DNA
<213> Homo sapien

<400> 104
tttttttttt tttttttttt tttttctctt cttttttttt gaaatgagga tcgagttttt 60
cactctctag atagggcatg aagaaaactc atctttccag ctttaaaata acaatcaaat 120
ctcttatgct atatcatatt ttaagttaaa ctaatgagtc actggcttat cttctcctga 180
aggaaatctg ttcattcttc tcattcatat agttatatca agtactacct tgcatattga 240
gaggtttttc ttctctattt acacatatat ttccatgtga atttgtatca aacctttatt 300
ttcatgcaaa ctagaaaata atgtttcttt tgcataagag aagagaacaa tatagcatta 360
caaaactgct caaattgttt gttaagttat ccattataat tagttggcag gagctaatac 420
aaatcacatt tacgacagca ataataaaac tgaagtacca gttaaatatc caaaataatt 480
aaaggaacat ttttagcctg ggtataatta gctaattcac tttacaagca tttattagaa 540
tgaattcaca tgttattatt cctagcccaa cacaatgg 578

<210> 105
<211> 538
<212> DNA
<213> Homo sapien

<400> 105
tttttttttt tttttcagta ataatcagaa caatatttat ttttatattt aaaattcata 60
gaaaagtgcc ttacatttaa taaaagtttg tttctcaaag tgatcagagg aattagatat 120
gtcttgaaca ccaatattaa tttgaggaaa atacaccaaa atacattaag taaattattt 180
aagatcatag agcttgtaag tgaaaagata aaatttgacc tcagaaactc tgagcattaa 240
aaatccacta ttagcaaata aattactatg gacttcttgc tttaattttg tgatgaatat 300
ggggtgtcac tggtaaacca acacattctg aaggatacat tacttagtga tagattctta 360
tgtactttgc taatacgtgg atatgagttg acaagtttct ctttcttcaa tcttttaagg 420
ggcgagaaat gaggaagaaa agaaaaggat tacgcatact gttctttcta tggaaggatt 480
agatatgttt cctttgccaa tattaaaaaa ataataatgt ttactactag tgaaaccc 538

<210> 106
<211> 473
<212> DNA
<213> Homo sapien

<400> 106
tttttttttt ttttttagtc aagtttctat ttttattata attaaagtct tggtcatttc 60
atttattagc tctgcaactt acatatttaa attaaagaaa cgttttagac aactgtacaa 120
tttataaatg taaggtgcca ttattgagta atatattcct ccaagagtgg atgtgtccct 180
tctcccacca actaatgaac agcaacatta gtttaatttt attagtagat atacactgct 240
gcaaacgcta attctcttct ccatccccat gtgatattgt gtatatgtgt gagttggtag 300
aatgcatcac aatctacaat caacagcaag atgaagctag gctgggcttt cggtgaaaat 360
agactgtgtc tgtctgaatc aaatgatctg acctatcctc ggtggcaaga actcttcgaa 420
ccgcttcctc aaaggcgctg ccacatttgt ggctctttgc acttgtttca aaa 473

<210> 107
<211> 1621
<212> DNA
<213> Homo sapien

<400> 107
cgccatggca ctgcagggca tctcggtcat ggagctgtcc ggcctggccc cgggcccgtt 60
ctgtgctatg gtcctggctg acttcggggc gcgtgtggta cgcgtggacc ggcccggctc 120
ccgctacgac gtgagccgct tgggccgggg caagcgctcg ctagtgctgg acctgaagca 180
gccgcgggga gccgccgtgc tgcggcgtct gtgcaagcgg tcggatgtgc tgctggagcc 240
cttccgccgc ggtgtcatgg agaaactcca gctgggccca gagattctgc agcgggaaaa 300
tccaaggctt atttatgcca ggctgagtgg atttggccag tcaggaagct tctgccggtt 360
agctggccac gatatcaact atttggcttt gtcaggtgtt ctctcaaaaa ttggcagaag 420
tggtgagaat ccgtatgccc cgctgaatct cctggctgac tttgctggtg gtggccttat 480
gtgtgcactg ggcattataa tggctctttt tgaccgcaca cgcactgaca agggtcaggt 540
cattgatgca aatatggtgg aaggaacagc atatttaagt tcttttctgt ggaaaactca 600
gaaatcgagt ctgtgggaag cacctcgagg acagaacatg ttggatggtg gagcaccttt 660
ctatacgact tacaggacag cagatgggga attcatggct gttggagcaa tagaacccca 720
gttctacgag ctgctgatca aaggacttgg actaaagtct gatgaacttc ccaatcagat 780
gagcatggat gattggccag aaatgaagaa gaagtttgca gatgtatttg caaagaagac 840
gaaggcagag tggtgtcaaa tctttgacgg cacagatgcc tgtgtgactc cggttctgac 900
ttttgaggag gttgttcatc atgatcacaa caaggaacgg ggctcgttta tcaccagtga 960
ggagcaggac gtgagccccc gccctgcacc tctgctgtta aacaccccag ccatcccttc 1020
tttcaaaagg gatcctttca taggagaaca cactgaggag atacttgaag aatttggatt 1080
cagccgcgaa gagatttatc agcttaactc agataaaatc attgaaagta ataaggtaaa 1140
agctagtctc taacttccag gcccacggct caagtgaatt tgaatactgc atttacagtg 1200
tagagtaaca cataacattg tatgcatgga aacatggagg aacagtatta cagtgtccta 1260
ccactctaat caagaaaaga attacagact ctgattctac agtgatgatt gaattctaaa 1320
aatggttatc attagggctt ttgatttata aaactttggg tacttatact aaattatggt 1380
agttattctg ccttccagtt tgcttgatat atttgttgat attaagattc ttgacttata 1440
ttttgaatgg gttctagtga aaaaggaatg atatattctt gaagacatcg atatacattt 1500
atttacactc ttgattctac aatgtagaaa atgaggaaat gccacaaatt gtatggtgat 1560
aaaagtcacg tgaaacaaaa aaaaaaaaaa aaaaaaaaaa aaaaaaaaaa aaaaaaaaaa 1620
a 1621

<210> 108
<211> 382
<212> PRT
<213> Homo sapien

<400> 108

<210> 109
<211> 1524
<212> DNA
<213> Homo sapien

<400> 109
ggcacgaggc tgcgccaggg cctgagcgga ggcgggggca gcctcgccag cgggggcccc      60
gggcctggcc atgcctcact gagccagcgc ctgcgcctct acctcgccga cagctggaac     120
cagtgcgacc tagtggctct cacctgcttc ctcctgggcg tgggctgccg gctgaccccg     180
ggtttgtacc acctgggccg cactgtcctc tgcatcgact tcatggtttt cacggtgcgg     240
ctgcttcaca tcttcacggt caacaaacag ctggggccca agatcgtcat cgtgagcaag     300
atgatgaagg acgtgttctt cttcctcttc ttcctcggcg tgtggctggt agcctatggc     360
gtggccacgg aggggctcct gaggccacgg gacagtgact tcccaagtat cctgcgccgc     420
gtcttctacc gtccctacct gcagatcttc gggcagattc cccaggagga catggacgtg     480
gccctcatgg agcacagcaa ctgctcgtcg gagcccggct tctgggcaca ccctcctggg     540
gcccaggcgg gcacctgcgt ctcccagtat gccaactggc tggtggtgct gctcctcgtc     600
atcttcctgc tcgtggccaa catcctgctg gtcaacttgc tcattgccat gttcagttac     660
acattcggca aagtacaggg caacagcgat ctctactgga aggcgcagcg ttaccgcctc     720
atccgggaat tccactctcg gcccgcgctg gccccgccct ttatcgtcat ctcccacttg     780
cgcctcctgc tcaggcaatt gtgcaggcga ccccggagcc cccagccgtc ctccccggcc     840
ctcgagcatt tccgggttta cctttctaag gaagccgagc ggaagctgct aacgtgggaa     900
tcggtgcata aggagaactt tctgctggca cgcgctaggg acaagcggga gagcgactcc     960
gagcgtctga agcgcacgtc ccagaaggtg gacttggcac tgaaacagct gggacacatc    1020
cgcgagtacg aacagcgcct gaaagtgctg gagcgggagg tccagcagtg tagccgcgtc    1080

ctggggtggg tggccgaggc cctgagccgc tctgccttgc tgcccccagg tgggccgcca 1140
ccccctgacc tgcctgggtc caaagactga gccctgctgg cggacttcaa ggagaagccc 1200
ccacagggga ttttgctcct agagtaaggc tcatctgggc ctcggccccc gcacctggtg 1260
gccttgtcct tgaggtgagc cccatgtcca tctgggccac tgtcaggacc acctttggga 1320
gtgtcatcct tacaaaccac agcatgcccg gctcctccca gaaccagtcc cagcctggga 1380
ggatcaaggc ctggatcccg ggccgttatc catctggagg ctgcaggtcc cttggggtaa 1440
cagggaccac agacccctca ccactcacag attcctcaca ctggggaaat aaagccattt 1500
cagaggaaaa aaaaaaaaaa aaaa 1524

<210> 110
<211> 3410
<212> DNA
<213> Homo sapien

<400> 110
gggaaccagc ctgcacgcgc tggctccggg tgacagccgc gcgcctcggc caggatctga 60
gtgatgagac gtgtccccac tgaggtgccc cacagcagca ggtgttgagc atgggctgag 120
aagctggacc ggcaccaaag ggctggcaga aatgggcgcc tggctgattc ctaggcagtt 180
ggcggcagca aggaggagag gccgcagctt ctggagcaga gccgagacga agcagttctg 240
gagtgcctga acggccccct gagccctacc cgcctggccc actatggtcc agaggctgtg 300
ggtgagccgc ctgctgcggc accggaaagc ccagctcttg ctggtcaacc tgctaacctt 360
tggcctggag gtgtgtttgg ccgcaggcat cacctatgtg ccgcctctgc tgctggaagt 420
gggggtagag gagaagttca tgaccatggt gctgggcatt ggtccagtgc tgggcctggt 480
ctgtgtcccg ctcctaggct cagccagtga ccactggcgt ggacgctatg gccgccgccg 540
gcccttcatc tgggcactgt ccttgggcat cctgctgagc ctctttctca tcccaagggc 600
cggctggcta gcagggctgc tgtgcccgga tcccaggccc ctggagctgg cactgctcat 660
cctgggcgtg gggctgctgg acttctgtgg ccaggtgtgc ttcactccac tggaggccct 720
gctctctgac ctcttccggg acccggacca ctgtcgccag gcctactctg tctatgcctt 780
catgatcagt cttgggggct gcctgggcta cctcctgcct gccattgact gggacaccag 840
tgccctggcc ccctacctgg gcacccagga ggagtgcctc tttggcctgc tcaccctcat 900
cttcctcacc tgcgtagcag ccacactgct ggtggctgag gaggcagcgc tgggccccac 960
cgagccagca gaagggctgt cggccccctc cttgtcgccc cactgctgtc catgccgggc 1020
ccgcttggct ttccggaacc tgggcgccct gcttccccgg ctgcaccagc tgtgctgccg 1080
catgccccgc accctgcgcc ggctcttcgt ggctgagctg tgcagctgga tggcactcat 1140
gaccttcacg ctgttttaca cggatttcgt gggcgagggg ctgtaccagg gcgtgcccag 1200
agctgagccg ggcaccgagg cccggagaca ctatgatgaa ggcgttcgga tgggcagcct 1260
ggggctgttc ctgcagtgcg ccatctccct ggtcttctct ctggtcatgg accggctggt 1320
gcagcgattc ggcactcgag cagtctattt ggccagtgtg gcagctttcc ctgtggctgc 1380
cggtgccaca tgcctgtccc acagtgtggc cgtggtgaca gcttcagccg ccctcaccgg 1440
gttcaccttc tcagccctgc agatcctgcc ctacacactg gcctccctct accaccggga 1500
gaagcaggtg ttcctgccca aataccgagg ggacactgga ggtgctagca gtgaggacag 1560
cctgatgacc agcttcctgc caggccctaa gcctggagct cccttcccta atggacacgt 1620
gggtgctgga ggcagtggcc tgctcccacc tccacccgcg ctctgcgggg cctctgcctg 1680
tgatgtctcc gtacgtgtgg tggtgggtga gcccaccgag gccagggtgg ttccgggccg 1740
gggcatctgc ctggacctcg ccatcctgga tagtgccttc ctgctgtccc aggtggcccc 1800
atccctgttt atgggctcca ttgtccagct cagccagtct gtcactgcct atatggtgtc 1860
tgccgcaggc ctgggtctgg tcgccattta ctttgctaca caggtagtat ttgacaagag 1920
cgacttggcc aaatactcag cgtagaaaac ttccagcaca ttggggtgga gggcctgcct 1980
cactgggtcc cagctccccg ctcctgttag ccccatgggg ctgccgggct ggccgccagt 2040
ttctgttgct gccaaagtaa tgtggctctc tgctgccacc ctgtgctgct gaggtgcgta 2100
gctgcacagc tgggggctgg ggcgtccctc tcctctctcc ccagtctcta gggctgcctg 2160
actggaggcc ttccaagggg gtttcagtct ggacttatac agggaggcca gaagggctcc 2220
atgcactgga atgcggggac tctgcaggtg gattacccag gctcagggtt aacagctagc 2280
ctcctagttg agacacacct agagaagggt ttttgggagc tgaataaact cagtcacctg 2340
gtttcccatc tctaagcccc ttaacctgca gcttcgttta atgtagctct tgcatgggag 2400
tttctaggat gaaacactcc tccatgggat ttgaacatat gacttatttg taggggaaga 2460
gtcctgaggg gcaacacaca agaaccaggt cccctcagcc cacagcactg tctttttgct 2520
gatccacccc cctcttacct tttatcagga tgtggcctgt tggtccttct gttgccatca 2580
cagagacaca ggcatttaaa tatttaactt atttatttaa caaagtagaa gggaatccat 2640
tgctagcttt tctgtgttgg tgtctaatat ttgggtaggg tgggggatcc ccaacaatca 2700
ggtcccctga gatagctggt cattgggctg atcattgcca gaatcttctt ctcctggggt 2760
ctggcccccc aaaatgccta acccaggacc ttggaaattc tactcatccc aaatgataat 2820

tccaaatgct gttacccaag gttagggtgt tgaaggaagg tagagggtgg ggcttcaggt 2880
ctcaacggct tccctaacca cccctcttct cttggcccag cctggttccc cccacttcca 2940
ctcccctcta ctctctctag gactgggctg atgaaggcac tgcccaaaat ttcccctacc 3000
cccaactttc ccctaccccc aactttcccc accagctcca caaccctgtt tggagctact 3060
gcaggaccag aagcacaaag tgcggtttcc caagcctttg tccatctcag cccccagagt 3120
atatctgtgc ttggggaatc tcacacagaa actcaggagc accccctgcc tgagctaagg 3180
gaggtcttat ctctcagggg gggtttaagt gccgtttgca ataatgtcgt cttatttatt 3240
tagcggggtg aatattttat actgtaagtg agcaatcaga gtataatgtt tatggtgaca 3300
aaattaaagg ctttcttata tgtttaaaaa aaaaaaaaaa aaaaaaaaaa aaaaaaaaaa 3360
aaaaaaaara aaaaaaaaaa aaaaaaaaaa aaaaaaataa aaaaaaaaaa 3410

<210> 111
<211> 1289
<212> DNA
<213> Homo sapien

<400> 111
agccaggcgt ccctctgcct gcccactcag tggcaacacc cgggagctgt tttgtccttt 60
gtggagcctc agcagttccc tctttcagaa ctcactgcca agagccctga acaggagcca 120
ccatgcagtg cttcagcttc attaagacca tgatgatcct cttcaatttg ctcatctttc 180
tgtgtggtgc agccctgttg gcagtgggca tctgggtgtc aatcgatggg gcatcctttc 240
tgaagatctt cgggccactg tcgtccagtg ccatgcagtt tgtcaacgtg ggctacttcc 300
tcatcgcagc cggcgttgtg gtctttgctc ttggtttcct gggctgctat ggtgctaaga 360
ctgagagcaa gtgtgccctc gtgacgttct tcttcatcct cctcctcatc ttcattgctg 420
aggttgcagc tgctgtggtc gccttggtgt acaccacaat ggctgagcac ttcctgacgt 480
tgctggtagt gcctgccatc aagaaagatt atggttccca ggaagacttc actcaagtgt 540
ggaacaccac catgaaaggg ctcaagtgct gtggcttcac caactatacg gattttgagg 600
actcacccta cttcaaagag aacagtgcct ttcccccatt ctgttgcaat gacaacgtca 660
ccaacacagc caatgaaacc tgcaccaagc aaaaggctca cgaccaaaaa gtagagggtt 720
gcttcaatca gcttttgtat gacatccgaa ctaatgcagt caccgtgggt ggtgtggcag 780
ctggaattgg gggcctcgag ctggctgcca tgattgtgtc catgtatctg tactgcaatc 840
tacaataagt ccacttctgc ctctgccact actgctgcca catgggaact gtgaagaggc 900
accctggcaa gcagcagtga ttgggggagg ggacaggatc taacaatgtc acttgggcca 960
gaatggacct gccctttctg ctccagactt ggggctagat agggaccact ccttttagcg 1020
atgcctgact ttccttccat tggtgggtgg atgggtgggg ggcattccag agcctctaag 1080
gtagccagtt ctgttgccca ttcccccagt ctattaaacc cttgatatgc cccctaggcc 1140
tagtggtgat cccagtgctc tactggggga tgagagaaag gcattttata gcctgggcat 1200
aagtgaaatc agcagagcct ctgggtggat gtgtagaagg cacttcaaaa tgcataaacc 1260
tgttacaatg ttaaaaaaaa aaaaaaaaa 1289

<210> 112
<211> 315
<212> PRT
<213> Homo sapien

<400> 112
Met Val Phe Thr Val Arg Leu Leu His Ile Phe Thr Val Asn Lys Gln
1               5                   10                  15
Leu Gly Pro Lys Ile Val Ile Val Ser Lys Met Met Lys Asp Val Phe
            20                  25                  30
Phe Phe Leu Phe Phe Leu Gly Val Trp Leu Val Ala Tyr Gly Val Ala
        35                  40                  45
Thr Glu Gly Leu Leu Arg Pro Arg Asp Ser Asp Phe Pro Ser Ile Leu
    50                  55                  60
Arg Arg Val Phe Tyr Arg Pro Tyr Leu Gln Ile Phe Gly Gln Ile Pro
65                  70                  75                  80
Gln Glu Asp Met Asp Val Ala Leu Met Glu His Ser Asn Cys Ser Ser
                85                  90                  95
Glu Pro Gly Phe Trp Ala His Pro Pro Gly Ala Gln Ala Gly Thr Cys
            100                 105                 110
Val Ser Gln Tyr Ala Asn Trp Leu Val Val Leu Leu Leu Val Ile Phe
        115                 120                 125

Leu Leu Val Ala Asn Ile Leu Leu Val Asn Leu Leu Ile Ala Met Phe
    130                 135                 140
Ser Tyr Thr Phe Gly Lys Val Gln Gly Asn Ser Asp Leu Tyr Trp Lys
145                 150                 155                 160
Ala Gln Arg Tyr Arg Leu Ile Arg Glu Phe His Ser Arg Pro Ala Leu
                165                 170                 175
Ala Pro Pro Phe Ile Val Ile Ser His Leu Arg Leu Leu Leu Arg Gln
            180                 185                 190
Leu Cys Arg Arg Pro Arg Ser Pro Gln Pro Ser Ser Pro Ala Leu Glu
        195                 200                 205
His Phe Arg Val Tyr Leu Ser Lys Glu Ala Glu Arg Lys Leu Leu Thr
    210                 215                 220
Trp Glu Ser Val His Lys Glu Asn Phe Leu Leu Ala Arg Ala Arg Asp
225                 230                 235                 240
Lys Arg Glu Ser Asp Ser Glu Arg Leu Lys Arg Thr Ser Gln Lys Val
                245                 250                 255
Asp Leu Ala Leu Lys Gln Leu Gly His Ile Arg Glu Tyr Glu Gln Arg
            260                 265                 270
Leu Lys Val Leu Glu Arg Glu Val Gln Gln Cys Ser Arg Val Leu Gly
        275                 280                 285
Trp Val Ala Glu Ala Leu Ser Arg Ser Ala Leu Leu Pro Pro Gly Gly
    290                 295                 300
Pro Pro Pro Pro Asp Leu Pro Gly Ser Lys Asp
305                 310                 315

<210> 113
<211> 553
<212> PRT
<213> Homo sapien

<400> 113
   Met Val Gln Arg Leu Trp Val Ser Arg Leu Leu Arg His Arg Lys Ala
    1               5                  10                  15
   Gln Leu Leu Leu Val Asn Leu Leu Thr Phe Gly Leu Glu Val Cys Leu
               20                  25                  30
   Ala Ala Gly Ile Thr Tyr Val Pro Pro Leu Leu Leu Glu Val Gly Val
           35                  40                  45
   Glu Glu Lys Phe Met Thr Met Val Leu Gly Ile Gly Pro Val Leu Gly
       50                  55                  60
   Leu Val Cys Val Pro Leu Leu Gly Ser Ala Ser Asp His Trp Arg Gly
   65                  70                  75                  80
   Arg Tyr Gly Arg Arg Arg Pro Phe Ile Trp Ala Leu Ser Leu Gly Ile
                   85                  90                  95
   Leu Leu Ser Leu Phe Leu Ile Pro Arg Ala Gly Trp Leu Ala Gly Leu
               100                 105                 110
   Leu Cys Pro Asp Pro Arg Pro Leu Glu Leu Ala Leu Leu Ile Leu Gly
           115                 120                 125
   Val Gly Leu Leu Asp Phe Cys Gly Gln Val Cys Phe Thr Pro Leu Glu
       130                 135                 140
   Ala Leu Leu Ser Asp Leu Phe Arg Asp Pro Asp His Cys Arg Gln Ala
   145                 150                 155                 160
   Tyr Ser Val Tyr Ala Phe Met Ile Ser Leu Gly Gly Cys Leu Gly Tyr
                   165                 170                 175
   Leu Leu Pro Ala Ile Asp Trp Asp Thr Ser Ala Leu Ala Pro Tyr Leu
               180                 185                 190
   Gly Thr Gln Glu Glu Cys Leu Phe Gly Leu Leu Thr Leu Ile Phe Leu
           195                 200                 205
   Thr Cys Val Ala Ala Thr Leu Leu Val Ala Glu Glu Ala Ala Leu Gly
       210                 215                 220
   Pro Thr Glu Pro Ala Glu Gly Leu Ser Ala Pro Ser Leu Ser Pro His
   225                 230                 235                 240
   Cys Cys Pro Cys Arg Ala Arg Leu Ala Phe Arg Asn Leu Gly Ala Leu
   245 250 255
   Leu Pro Arg Leu His Gln Leu Cys Cys Arg Met Pro Arg Thr Leu Arg
   260 265 270
   Arg Leu Phe Val Ala Glu Leu Cys Ser Trp Met Ala Leu Met Thr Phe
   275 280 285
   Thr Leu Phe Tyr Thr Asp Phe Val Gly Glu Gly Leu Tyr Gln Gly Val
   290 295 300
   Pro Arg Ala Glu Pro Gly Thr Glu Ala Arg Arg His Tyr Asp Glu Gly
   305 310 315 320
   Val Arg Met Gly Ser Leu Gly Leu Phe Leu Gln Cys Ala Ile Ser Leu
   325 330 335
   Val Phe Ser Leu Val Met Asp Arg Leu Val Gln Arg Phe Gly Thr Arg
   340 345 350
   Ala Val Tyr Leu Ala Ser Val Ala Ala Phe Pro Val Ala Ala Gly Ala
   355 360 365
   Thr Cys Leu Ser His Ser Val Ala Val Val Thr Ala Ser Ala Ala Leu
   370 375 380
   Thr Gly Phe Thr Phe Ser Ala Leu Gln Ile Leu Pro Tyr Thr Leu Ala
   385 390 395 400
   Ser Leu Tyr His Arg Glu Lys Gln Val Phe Leu Pro Lys Tyr Arg Gly
   405 410 415
   Asp Thr Gly Gly Ala Ser Ser Glu Asp Ser Leu Met Thr Ser Phe Leu
   420 425 430
   Pro Gly Pro Lys Pro Gly Ala Pro Phe Pro Asn Gly His Val Gly Ala
   435 440 445
   Gly Gly Ser Gly Leu Leu Pro Pro Pro Pro Ala Leu Cys Gly Ala Ser
   450 455 460
   Ala Cys Asp Val Ser Val Arg Val Val Val Gly Glu Pro Thr Glu Ala
   465 470 475 480
   Arg Val Val Pro Gly Arg Gly Ile Cys Leu Asp Leu Ala Ile Leu Asp
   485 490 495
   Ser Ala Phe Leu Leu Ser Gln Val Ala Pro Ser Leu Phe Met Gly Ser
   500 505 510
   Ile Val Gln Leu Ser Gln Ser Val Thr Ala Tyr Met Val Ser Ala Ala
   515 520 525
   Gly Leu Gly Leu Val Ala Ile Tyr Phe Ala Thr Gln Val Val Phe Asp
   530 535 540
   Lys Ser Asp Leu Ala Lys Tyr Ser Ala
   545 550

<210> 114
<211> 241
<212> PRT
<213> Homo sapien

<400> 114
Met Gln Cys Phe Ser Phe Ile Lys Thr Met Met Ile Leu Phe Asn Leu
1               5                   10                  15
Leu Ile Phe Leu Cys Gly Ala Ala Leu Leu Ala Val Gly Ile Trp Val
            20                  25                  30
Ser Ile Asp Gly Ala Ser Phe Leu Lys Ile Phe Gly Pro Leu Ser Ser
        35                  40                  45
Ser Ala Met Gln Phe Val Asn Val Gly Tyr Phe Leu Ile Ala Ala Gly
    50                  55                  60
Val Val Val Phe Ala Leu Gly Phe Leu Gly Cys Tyr Gly Ala Lys Thr
65                  70                  75                  80
Glu Ser Lys Cys Ala Leu Val Thr Phe Phe Phe Ile Leu Leu Leu Ile
                85                  90                  95
Phe Ile Ala Glu Val Ala Ala Ala Val Val Ala Leu Val Tyr Thr Thr
            100                 105                 110
Met Ala Glu His Phe Leu Thr Leu Leu Val Val Pro Ala Ile Lys Lys
        115                 120                 125

Asp Tyr Gly Ser Gln Glu Asp Phe Thr Gln Val Trp Asn Thr Thr Met
    130                 135                 140
Lys Gly Leu Lys Cys Cys Gly Phe Thr Asn Tyr Thr Asp Phe Glu Asp
145                 150                 155                 160
Ser Pro Tyr Phe Lys Glu Asn Ser Ala Phe Pro Pro Phe Cys Cys Asn
                165                 170                 175
Asp Asn Val Thr Asn Thr Ala Asn Glu Thr Cys Thr Lys Gln Lys Ala
            180                 185                 190
His Asp Gln Lys Val Glu Gly Cys Phe Asn Gln Leu Leu Tyr Asp Ile
        195                 200                 205
Arg Thr Asn Ala Val Thr Val Gly Gly Val Ala Ala Gly Ile Gly Gly
    210                 215                 220
Leu Glu Leu Ala Ala Met Ile Val Ser Met Tyr Leu Tyr Cys Asn Leu
225                 230                 235                 240
Gln

<210> 115
<211> 366
<212> DNA
<213> Homo sapien

<400> 115
gctctttctc tcccctcctc tgaatttaat tctttcaact tgcaatttgc aaggattaca      60
catttcactg tgatgtatat tgtgttgcaa aaaaaaaaaa gtgtctttgt ttaaaattac     120
ttggtttgtg aatccatctt gctttttccc cattggaact agtcattaac ccatctctga     180
actggtagaa aaacatctga agagctagtc tatcagcatc tgacaggtga attggatggt     240
tctcagaacc atttcaccca gacagcctgt ttctatcctg tttaataaat tagtttgggt     300
tctctacatg cataacaaac cctgctccaa tctgtcacat aaaagtctgt gacttgaagt     360
ttagtc                                                                366

<210> 116
<211> 282
<212> DNA
<213> Homo sapien

<220>
<221> misc_feature
<222> (1)...(282)
<223> n = A,T,C or G

<400> 116
acaaagatga accatttcct atattatagc aaaattaaaa tctacccgta ttctaatatt      60
gagaaatgag atnaaacaca atnttataaa gtctacttag agaagatcaa gtgacctcaa     120
agactttact attttcatat tttaagacac atgatttatc ctattttagt aacctggttc     180
atacgttaaa caaaggataa tgtgaacagc agagaggatt tgttggcaga aaatctatgt     240
tcaatctnga actatctana tcacagacat ttctattcct tt                        282

<210> 117
<211> 305
<212> DNA
<213> Homo sapien

<220>
<221> misc_feature
<222> (1)...(305)
<223> n = A,T,C or G

<400> 117
acacatgtcg cttcactgcc ttcttagatg cttctggtca acatanagga acagggacca      60
tatttatcct ccctcctgaa acaattgcaa aataanacaa aatatatgaa acaattgcaa     120
aataaggcaa aatatatgaa acaacaggtc tcgagatatt ggaaatcagt caatgaagga     180

tactgatccc tgatcactgt cctaatgcag gatgtgggaa acagatgagg tcacctctgt 240
gactgcccca gcttactgcc tgtagagagt ttctangctg cagttcagac agggagaaat 300
tgggt 305

<210> 118
<211> 71
<212> DNA
<213> Homo sapien

<220>
<221> misc_feature
<222> (1)...(71)
<223> n = A,T,C or G

<400> 118
accaaggtgt ntgaatctct gacgtgggga tctctgattc ccgcacaatc tgagtggaaa 60
aantcctggg t 71

<210> 119
<211> 212
<212> DNA
<213> Homo sapien

<220>
<221> misc_feature
<222> (1)...(212)
<223> n = A,T,C or G

<400> 119
actccggttg gtgtcagcag cacgtggcat tgaacatngc aatgtggagc ccaaaccaca 60
gaaaatgggg tgaaattggc caactttcta tnaacttatg ttggcaantt tgccaccaac 120
agtaagctgg cccttctaat aaaagaaaat tgaaaggttt ctcactaanc ggaattaant 180
aatggantca aganactccc aggcctcagc gt 212

<210> 120
<211> 90
<212> DNA
<213> Homo sapien

<220>
<221> misc_feature
<222> (1)...(90)
<223> n = A,T,C or G

<400> 120
actcgttgca natcaggggc cccccagagt caccgttgca ggagtccttc tggtcttgcc 60
ctccgccggc gcagaacatg ctggggtggt 90

<210> 121
<211> 218
<212> DNA
<213> Homo sapien

<220>
<221> misc_feature
<222> (1)...(218)
<223> n = A,T,C or G

<400> 121
tgtancgtga anacgacaga nagggttgtc aaaaatggag aanccttgaa gtcattttga 60
gaataagatt tgctaaaaga tttggggcta aaacatggtt attgggagac atttctgaag 120
atatncangt aaattangga atgaattcat ggttcttttg ggaattcctt tacgatngcc 180

agcatanact tcatgtgggg atancagcta cccttgta 218

<210> 122
<211> 171
<212> DNA
<213> Homo sapien

<400> 122
taggggtgta tgcaactgta aggacaaaaa ttgagactca actggcttaa ccaataaagg 60
catttgttag ctcatggaac aggaagtcgg atggtggggc atcttcagtg ctgcatgagt 120
caccaccccg gcggggtcat ctgtgccaca ggtccctgtt gacagtgcgg t 171

<210> 123
<211> 76
<212> DNA
<213> Homo sapien

<220>
<221> misc_feature
<222> (1)...(76)
<223> n = A,T,C or G

<400> 123
tgtagcgtga agacnacaga atggtgtgtg ctgtgctatc caggaacaca tttattatca 60
ttatcaanta ttgtgt 76

<210> 124
<211> 131
<212> DNA
<213> Homo sapien

<400> 124
acctttcccc aaggccaatg tcctgtgtgc taactggccg gctgcaggac agctgcaatt 60
caatgtgctg ggtcatatgg aggggaggag actctaaaat agccaatttt attctcttgg 120
ttaagatttg t 131

<210> 125
<211> 432
<212> DNA
<213> Homo sapien

<400> 125
actttatcta ctggctatga aatagatggt ggaaaattgc gttaccaact ataccactgg 60
cttgaaaaag aggtgatagc tcttcagagg acttgtgact tttgctcaga tgctgaagaa 120
ctacagtctg catttggcag aaatgaagat gaatttggat taaatgagga tgctgaagat 180
ttgcctcacc aaacaaaagt gaaacaactg agagaaaatt ttcaggaaaa aagacagtgg 240
ctcttgaagt atcagtcact tttgagaatg tttcttagtt actgcatact tcatggatcc 300
catggtgggg gtcttgcatc tgtaagaatg gaattgattt tgcttttgca agaatctcag 360
caggaaacat cagaaccact attttctagc cctctgtcag agcaaacctc agtgcctctc 420
ctctttgctt gt 432

<210> 126
<211> 112
<212> DNA
<213> Homo sapien

<400> 126
acacaacttg aatagtaaaa tagaaactga gctgaaattt ctaattcact ttctaaccat 60
agtaagaatg atatttcccc ccagggatca ccaaatattt ataaaaattt gt 112

<210> 127
<211> 54

<212> DNA
<213> Homo sapien

<400> 127
accacgaaac cacaaacaag atggaagcat caatccactt gccaagcaca gcag 54

<210> 128
<211> 323
<212> DNA
<213> Homo sapien

<400> 128
acctcattag taattgtttt gttgtttcat ttttttctaa tgtctcccct ctaccagctc 60
acctgagata acagaatgaa aatggaagga cagccagatt tctcctttgc tctctgctca 120
ttctctctga agtctaggtt acccattttg gggacccatt ataggcaata aacacagttc 180
ccaaagcatt tggacagttt cttgttgtgt tttagaatgg ttttcctttt tcttagcctt 240
ttcctgcaaa aggctcactc agtcccttgc ttgctcagtg gactgggctc cccagggcct 300
aggctgcctt cttttccatg tcc 323

<210> 129
<211> 192
<212> DNA
<213> Homo sapien

<220>
<221> misc_feature
<222> (1)...(192)
<223> n = A,T,C or G

<400> 129
acatacatgt gtgtatattt ttaaatatca cttttgtatc actctgactt tttagcatac 60
tgaaaacaca ctaacataat ttntgtgaac catgatcaga tacaacccaa atcattcatc 120
tagcacattc atctgtgata naaagatagg tgagtttcat ttccttcacg ttggccaatg 180
gataaacaaa gt 192

<210> 130
<211> 362
<212> DNA
<213> Homo sapien

<220>
<221> misc_feature
<222> (1)...(362)
<223> n = A,T,C or G

<400> 130
ccctttttta tggaatgagt agactgtatg tttgaanatt tanccacaac ctctttgaca 60
tataatgacg caacaaaaag gtgctgttta gtcctatggt tcagtttatg cccctgacaa 120
gtttccattg tgttttgccg atcttctggc taatcgtggt atcctccatg ttattagtaa 180
ttctgtattc cattttgtta acgcctggta gatgtaacct gctangaggc taactttata 240
cttatttaaa agctcttatt ttgtggtcat taaaatggca atttatgtgc agcactttat 300
tgcagcagga agcacgtgtg ggttggttgt aaagctcttt gctaatctta aaaagtaatg 360
gg 362

<210> 131
<211> 332
<212> DNA
<213> Homo sapien

<220>
<221> misc_feature
<222> (1)...(332)

<223> n = A,T,C or G

<400> 131
ctttttgaaa gatcgtgtcc actcctgtgg acatcttgtt ttaatggagt ttcccatgca 60
gtangactgg tatggttgca gctgtccaga taaaaacatt tgaagagctc caaaatgaga 120
gttctcccag gttcgccctg ctgctccaag tctcagcagc agcctctttt aggaggcatc 180
ttctgaacta gattaaggca gcttgtaaat ctgatgtgat ttggtttatt atccaactaa 240
cttccatctg ttatcactgg agaaagccca gactccccan gacnggtacg gattgtgggc 300
atanaaggat tgggtgaagc tggcgttgtg gt 332

<210> 132
<211> 322
<212> DNA
<213> Homo sapien

<220>
<221> misc_feature
<222> (1)...(322)
<223> n = A,T,C or G

<400> 132
acttttgcca ttttgtatat ataaacaatc ttgggacatt ctcctgaaaa ctaggtgtcc 60
agtggctaag agaactcgat ttcaagcaat tctgaaagga aaaccagcat gacacagaat 120
ctcaaattcc caaacagggg ctctgtggga aaaatgaggg aggacctttg tatctcgggt 180
tttagcaagt taaaatgaan atgacaggaa aggcttattt atcaacaaag agaagagttg 240
ggatgcttct aaaaaaaact ttggtagaga aaataggaat gctnaatcct agggaagcct 300
gtaacaatct acaattggtc ca 322

<210> 133
<211> 278
<212> DNA
<213> Homo sapien

<220>
<221> misc_feature
<222> (1)...(278)
<223> n = A,T,C or G

<400> 133
acaagccttc acaagtttaa ctaaattggg attaatcttt ctgtanttat ctgcataatt 60
cttgtttttc tttccatctg gctcctgggt tgacaatttg tggaaacaac tctattgcta 120
ctatttaaaa aaaatcacaa atctttccct ttaagctatg ttnaattcaa actattcctg 180
ctattcctgt tttgtcaaag aaattatatt tttcaaaata tgtntatttg tttgatgggt 240
cccacgaaac actaataaaa accacagaga ccagcctg 278

<210> 134
<211> 121
<212> DNA
<213> Homo sapien

<220>
<221> misc_feature
<222> (1)...(121)
<223> n = A,T,C or G

<400> 134
gtttanaaaa cttgtttagc tccatagagg aaagaatgtt aaactttgta ttttaaaaca 60
tgattctctg aggttaaact tggttttcaa atgttatttt tacttgtatt ttgcttttgg 120
t 121

<210> 135
<211> 350

<212> DNA
<213> Homo sapien

<220>
<221> misc_feature
<222> (1)...(350)
<223> n = A,T,C or G

<400> 135
acttanaacc atgcctagca catcagaatc cctcaaagaa catcagtata atcctatacc 60
atancaagtg gtgactggtt aagcgtgcga caaaggtcag ctggcacatt acttgtgtgc 120
aaacttgata cttttgttct aagtaggaac tagtatacag tncctaggan tggtactcca 180
gggtgccccc caactcctgc agccgctcct ctgtgccagn ccctgnaagg aactttcgct 240
ccacctcaat caagccctgg gccatgctac ctgcaattgg ctgaacaaac gtttgctgag 300
ttcccaagga tgcaaagcct ggtgctcaac tcctggggcg tcaactcagt 350

<210> 136
<211> 399
<212> DNA
<213> Homo sapien

<220>
<221> misc_feature
<222> (1)...(399)
<223> n = A,T,C or G

<400> 136
tgtaccgtga agacgacaga agttgcatgg cagggacagg gcagggccga ggccagggtt 60
gctgtgattg tatccgaata ntcctcgtga gaaaagataa tgagatgacg tgagcagcct 120
gcagacttgt gtctgccttc aanaagccag acaggaaggc cctgcctgcc ttggctctga 180
cctggcggcc agccagccag ccacaggtgg gcttcttcct tttgtggtga caacnccaag 240
aaaactgcag aggcccaggg tcaggtgtna gtgggtangt gaccataaaa caccaggtgc 300
tcccaggaac ccgggcaaag gccatcccca cctacagcca gcatgcccac tggcgtgatg 360
ggtgcagang gatgaagcag ccagntgttc tgctgtggt 399

<210> 137
<211> 165
<212> DNA
<213> Homo sapien

<220>
<221> misc_feature
<222> (1)...(165)
<223> n = A,T,C or G

<400> 137
actggtgtgg tngggggtga tgctggtggt anaagttgan gtgacttcan gatggtgtgt 60
ggaggaagtg tgtgaacgta gggatgtaga ngttttggcc gtgctaaatg agcttcggga 120
ttggctggtc ccactggtgg tcactgtcat tggtggggtt cctgt 165

<210> 138
<211> 338
<212> DNA
<213> Homo sapien

<220>
<221> misc_feature
<222> (1)...(338)
<223> n = A,T,C or G

<400> 138
actcactgga atgccacatt cacaacagaa tcagaggtct gtgaaaacat taatggctcc 60

ttaacttctc cagtaagaat cagggacttg aaatggaaac gttaacagcc acatgcccaa 120
tgctgggcag tctcccatgc cttccacagt gaaagggctt gagaaaaatc acatccaatg 180
tcatgtgttt ccagccacac caaaaggtgc ttggggtgga gggctggggg catananggt 240
cangcctcag gaagcctcaa gttccattca gctttgccac tgtacattcc ccatntttaa 300
aaaaactgat gccttttttt tttttttttg taaaattc 338

<210> 139
<211> 382
<212> DNA
<213> Homo sapien

<400> 139
gggaatcttg gtttttggca tctggtttgc ctatagccga ggccactttg acagaacaaa 60
gaaagggact tcgagtaaga aggtgattta cagccagcct agtgcccgaa gtgaaggaga 120
attcaaacag acctcgtcat tcctggtgtg agcctggtcg gctcaccgcc tatcatctgc 180
atttgcctta ctcaggtgct accggactct ggcccctgat gtctgtagtt tcacaggatg 240
ccttatttgt cttctacacc ccacagggcc ccctacttct tcggatgtgt ttttaataat 300
gtcagctatg tgccccatcc tccttcatgc cctccctccc tttcctacca ctgctgagtg 360
gcctggaact tgtttaaagt gt 382

<210> 140
<211> 200
<212> DNA
<213> Homo sapien

<220>
<221> misc_feature
<222> (1)...(200)
<223> n = A,T,C or G

<400> 140
accaaanctt ctttctgttg tgttngattt tactataggg gtttngcttn ttctaaanat 60
acttttcatt taacancttt tgttaagtgt caggctgcac tttgctccat anaattattg 120
ttttcacatt tcaacttgta tgtgtttgtc tcttanagca ttggtgaaat cacatatttt 180
atattcagca taaaggagaa 200

<210> 141
<211> 335
<212> DNA
<213> Homo sapien

<220>
<221> misc_feature
<222> (1)...(335)
<223> n = A,T,C or G

<400> 141
actttatttt caaaacactc atatgttgca aaaaacacat agaaaaataa agtttggtgg 60
gggtgctgac taaacttcaa gtcacagact tttatgtgac agattggagc agggtttgtt 120
atgcatgtag agaacccaaa ctaatttatt aaacaggata gaaacaggct gtctgggtga 180
aatggttctg agaaccatcc aattcacctg tcagatgctg atanactagc tcttcagatg 240
tttttctacc agttcagaga tnggttaatg actanttcca atggggaaaa agcaagatgg 300
attcacaaac caagtaattt taaacaaaga cactt 335

<210> 142
<211> 459
<212> DNA
<213> Homo sapien

<220>
<221> misc_feature
<222> (1)...(459)

<223> n = A,T,C or G

<400> 142
accaggttaa tattgccaca tatatccttt ccaattgcgg gctaaacaga cgtgtattta 60
gggttgttta aagacaaccc agcttaatat caagagaaat tgtgaccttt catggagtat 120
ctgatggaga aaacactgag ttttgacaaa tcttatttta ttcagatagc agtctgatca 180
cacatggtcc aacaacactc aaataataaa tcaaatatna tcagatgtta aagattggtc 240
ttcaaacatc atagccaatg atgccccgct tgcctataat ctctccgaca taaaaccaca 300
tcaacacctc agtggccacc aaaccattca gcacagcttc cttaactgtg agctgtttga 360
agctaccagt ctgagcacta ttgactatnt ttttcangct ctgaatagct ctagggatct 420
cagcangggt gggaggaacc agctcaacct tggcgtant 459

<210> 143
<211> 140
<212> DNA
<213> Homo sapien

<400> 143
acatttcctt ccaccaagtc aggactcctg gcttctgtgg gagttcttat cacctgaggg 60
aaatccaaac agtctctcct agaaaggaat agtgtcacca accccaccca tctccctgag 120
accatccgac ttccctgtgt 140

<210> 144
<211> 164
<212> DNA
<213> Homo sapien

<220>
<221> misc_feature
<222> (1)...(164)
<223> n = A,T,C or G

<400> 144
acttcagtaa caacatacaa taacaacatt aagtgtatat tgccatcttt gtcattttct 60
atctatacca ctctcccttc tgaaaacaan aatcactanc caatcactta tacaaatttg 120
aggcaattaa tccatatttg ttttcaataa ggaaaaaaag atgt 164

<210> 145
<211> 303
<212> DNA
<213> Homo sapien

<220>
<221> misc_feature
<222> (1)...(303)
<223> n = A,T,C or G

<400> 145
acgtagacca tccaactttg tatttgtaat ggcaaacatc cagnagcaat tcctaaacaa 60
actggagggt atttataccc aattatccca ttcattaaca tgccctcctc ctcaggctat 120
gcaggacagc tatcataagt cggcccaggc atccagatac taccatttgt ataaacttca 180
gtaggggagt ccatccaagt gacaggtcta atcaaaggag gaaatggaac ataagcccag 240
tagtaaaatn ttgcttagct gaaacagcca caaaagactt accgccgtgg tgattaccat 300
caa 303

<210> 146
<211> 327
<212> DNA
<213> Homo sapien

<220>
<221> misc_feature

<222> (1)...(327)
<223> n = A,T,C or G

<400> 146
actgcagctc aattagaagt ggtctctgac tttcatcanc ttctccctgg gctccatgac 60
actggcctgg agtgactcat tgctctggtt ggttgagaga gctcctttgc caacaggcct 120
ccaagtcagg gctgggattt gtttcctttc cacattctag caacaatatg ctggccactt 180
cctgaacagg gagggtggga ggagccagca tggaacaagc tgccactttc taaagtagcc 240
agacttgccc ctgggcctgt cacacctact gatgaccttc tgtgcctgca ggatggaatg 300
taggggtgag ctgtgtgact ctatggt 327

<210> 147
<211> 173
<212> DNA
<213> Homo sapien

<220>
<221> misc_feature
<222> (1)...(173)
<223> n = A,T,C or G

<400> 147
acattgtttt tttgagataa agcattgana gagctctcct taacgtgaca caatggaagg 60
actggaacac atacccacat ctttgttctg agggataatt ttctgataaa gtcttgctgt 120
atattcaagc acatatgtta tatattattc agttccatgt ttatagccta gtt 173

<210> 148
<211> 477
<212> DNA
<213> Homo sapien

<220>
<221> misc_feature
<222> (1)...(477)
<223> n = A,T,C or G

<400> 148
acaaccactt tatctcatcg aatttttaac ccaaactcac tcactgtgcc tttctatcct 60
atgggatata ttatttgatg ctccatttca tcacacatat atgaataata cactcatact 120
gccctactac ctgctgcaat aatcacattc ccttcctgtc ctgaccctga agccattggg 180
gtggtcctag tggccatcag tccangcctg caccttgagc ccttgagctc cattgctcac 240
nccancccac ctcaccgacc ccatcctctt acacagctac ctccttgctc tctaacccca 300
tagattatnt ccaaattcag tcaattaagt tactattaac actctacccg acatgtccag 360
caccactggt aagccttctc cagccaacac acacacacac acacncacac acacacatat 420
ccaggcacag gctacctcat cttcacaatc acccctttaa ttaccatgct atggtgg 477

<210> 149
<211> 207
<212> DNA
<213> Homo sapien

<400> 149
acagttgtat tataatatca agaaataaac ttgcaatgag agcatttaag agggaagaac 60
taacgtattt tagagagcca aggaaggttt ctgtggggag tgggatgtaa ggtggggcct 120
gatgataaat aagagtcagc caggtaagtg ggtggtgtgg tatgggcaca gtgaagaaca 180
tttcaggcag agggaacagc agtgaaa 207

<210> 150
<211> 111
<212> DNA
<213> Homo sapien

<220>
<221> misc_feature
<222> (1)...(111)
<223> n = A,T,C or G

<400> 150
accttgattt cattgctgct ctgatggaaa cccaactatc taatttagct aaaacatggg 60
cacttaaatg tggtcagtgt ttggacttgt taactantgg catctttggg t 111

<210> 151
<211> 196
<212> DNA
<213> Homo sapien

<400> 151
agcgcggcag gtcatattga acattccaga tacctatcat tactcgatgc tgttgataac 60
agcaagatgg ctttgaactc agggtcacca ccagctattg gaccttacta tgaaaaccat 120
ggataccaac cggaaaaccc ctatcccgca cagcccactg tggtccccac tgtctacgag 180
gtgcatccgg ctcagt 196

<210> 152
<211> 132
<212> DNA
<213> Homo sapien

<400> 152
acagcacttt cacatgtaag aagggagaaa ttcctaaatg taggagaaag ataacagaac 60
cttccccttt tcatctagtg gtggaaacct gatgctttat gttgacagga atagaaccag 120
gagggagttt gt 132

<210> 153
<211> 285
<212> DNA
<213> Homo sapien

<220>
<221> misc_feature
<222> (1)...(285)
<223> n = A,T,C or G

<400> 153
acaanaccca nganaggcca ctggccgtgg tgtcatggcc tccaaacatg aaagtgtcag 60
cttctgctct tatgtcctca tctgacaact ctttaccatt tttatcctcg ctcagcagga 120
gcacatcaat aaagtccaaa gtcttggact tggccttggc ttggaggaag tcatcaacac 180
cctggctagt gagggtgcgg cgccgctcct ggatgacggc atctgtgaag tcgtgcacca 240
gtctgcaggc cctgtggaag cgccgtccac acggagtnag gaatt 285

<210> 154
<211> 333
<212> DNA
<213> Homo sapien

<400> 154
accacagtcc tgttgggcca gggcttcatg accctttctg tgaaaagcca tattatcacc 60
accccaaatt tttccttaaa tatctttaac tgaaggggtc agcctcttga ctgcaaagac 120
cctaagccgg ttacacagct aactcccact ggccctgatt tgtgaaattg ctgctgcctg 180
attggcacag gagtcgaagg tgttcagctc ccctcctccg tggaacgaga ctctgatttg 240
agtttcacaa attctcgggc cacctcgtca ttgctcctct gaaataaaat ccggagaatg 300
gtcaggcctg tctcatccat atggatcttc cgg 333

<210> 155
<211> 308

<212> DNA
<213> Homo sapien

<220>
<221> misc_feature
<222> (1)...(308)
<223> n = A,T,C or G

<400> 155
actggaaata ataaaaccca catcacagtg ttgtgtcaaa gatcatcagg gcatggatgg 60
gaaagtgctt tgggaactgt aaagtgccta acacatgatc gatgattttt gttataatat 120
ttgaatcacg gtgcatacaa actctcctgc ctgctcctcc tgggccccag ccccagcccc 180
atcacagctc actgctctgt tcatccaggc ccagcatgta gtggctgatt cttcttggct 240
gcttttagcc tccanaagtt tctctgaagc caaccaaacc tctangtgta aggcatgctg 300
gccctggt 308

<210> 156
<211> 295
<212> DNA
<213> Homo sapien

<400> 156
accttgctcg gtgcttggaa catattagga actcaaaata tgagatgata acagtgccta 60
ttattgatta ctgagagaac tgttagacat ttagttgaag attttctaca caggaactga 120
gaataggaga ttatgtttgg ccctcatatt ctctcctatc ctccttgcct cattctatgt 180
ctaatatatt ctcaatcaaa taaggttagc ataatcagga aatcgaccaa ataccaatat 240
aaaaccagat gtctatcctt aagattttca aatagaaaac aaattaacag actat 295

<210> 157
<211> 126
<212> DNA
<213> Homo sapien

<400> 157
acaagtttaa atagtgctgt cactgtgcat gtgctgaaat gtgaaatcca ccacatttct 60
gaagagcaaa acaaattctg tcatgtaatc tctatcttgg gtcgtgggta tatctgtccc 120
cttagt 126

<210> 158
<211> 442
<212> DNA
<213> Homo sapien

<220>
<221> misc_feature
<222> (1)...(442)
<223> n = A,T,C or G

<400> 158
acccactggt cttggaaaca cccatcctta atacgatgat ttttctgtcg tgtgaaaatg 60
aanccagcag gctgccccta gtcagtcctt ccttccagag aaaaagagat ttgagaaagt 120
gcctgggtaa ttcaccatta atttcctccc ccaaactctc tgagtcttcc cttaatattt 180
ctggtggttc tgaccaaagc aggtcatggt ttgttgagca tttgggatcc cagtgaagta 240
natgtttgta gccttgcata cttagccctt cccacgcaca aacggagtgg cagagtggtg 300
ccaaccctgt tttcccagtc cacgtagaca gattcacagt gcggaattct ggaagctgga 360
nacagacggg ctctttgcag agccgggact ctgagangga catgagggcc tctgcctctg 420
tgttcattct ctgatgtcct gt 442

<210> 159
<211> 498
<212> DNA
<213> Homo sapien

<220>
<221> misc_feature
<222> (1)...(498)
<223> n = A,T,C or G

<400> 159
acttccaggt aacgttgttg tttccgttga gcctgaactg atgggtgacg ttgtaggttc 60
tccaacaaga actgaggttg cagagcgggt agggaagagt gctgttccag ttgcacctgg 120
gctgctgtgg actgttgttg attcctcact acggcccaag gttgtggaac tggcanaaag 180
gtgtgttgtt gganttgagc tcgggcggct gtggtaggtt gtgggctctt caacaggggc 240
tgctgtggtg ccgggangtg aangtgttgt gtcacttgag cttggccagc tctggaaagt 300
antanattct tcctgaaggc cagcgcttgt ggagctggca ngggtcantg ttgtgtgtaa 360
cgaaccagtg ctgctgtggg tgggtgtana tcctccacaa agcctgaagt tatggtgtcn 420
tcaggtaana atgtggtttc agtgtccctg ggcngctgtg gaaggttgta nattgtcacc 480
aagggaataa gctgtggt 498

<210> 160
<211> 380
<212> DNA
<213> Homo sapien

<220>
<221> misc_feature
<222> (1)...(380)
<223> n = A,T,C or G

<400> 160
acctgcatcc agcttccctg ccaaactcac aaggagacat caacctctag acagggaaac 60
agcttcagga tacttccagg agacagagcc accagcagca aaacaaatat tcccatgcct 120
ggagcatggc atagaggaag ctganaaatg tggggtctga ggaagccatt tgagtctggc 180
cactagacat ctcatcagcc acttgtgtga agagatgccc catgacccca gatgcctctc 240
ccacccttac ctccatctca cacacttgag ctttccactc tgtataattc taacatcctg 300
gagaaaaatg gcagtttgac cgaacctgtt cacaacggta gaggctgatt tctaacgaaa 360
cttgtagaat gaagcctgga 380

<210> 161
<211> 114
<212> DNA
<213> Homo sapien

<400> 161
actccacatc ccctctgagc aggcggttgt cgttcaaggt gtatttggcc ttgcctgtca 60
cactgtccac tggcccctta tccacttggt gcttaatccc tcgaaagagc atgt 114

<210> 162
<211> 177
<212> DNA
<213> Homo sapien

<400> 162
actttctgaa tcgaatcaaa tgatacttag tgtagtttta atatcctcat atatatcaaa 60
gttttactac tctgataatt ttgtaaacca ggtaaccaga acatccagtc atacagcttt 120
tggtgatata taacttggca ataacccagt ctggtgatac ataaaactac tcactgt 177

<210> 163
<211> 137
<212> DNA
<213> Homo sapien

<220>
<221> misc_feature

<222> (1)...(137)
<223> n = A,T,C or G

<400> 163
catttataca gacaggcgtg aagacattca cgacaaaaac gcgaaattct atcccgtgac 60
canagaaggc agctacggct actcctacat cctggcgtgg gtggccttcg cctgcacctt 120
catcagcggc atgatgt 137

<210> 164
<211> 469
<212> DNA
<213> Homo sapien

<220>
<221> misc_feature
<222> (1)...(469)
<223> n = A,T,C or G

<400> 164
cttatcacaa tgaatgttct cctgggcagc gttgtgatct ttgccacctt cgtgacttta 60
tgcaatgcat catgctattt catacctaat gagggagttc caggagattc aaccaggaaa 120
tgcatggatc tcaaaggaaa caaacaccca ataaactcgg agtggcagac tgacaactgt 180
gagacatgca cttgctacga aacagaaatt tcatgttgca cccttgtttc tacacctgtg 240
ggttatgaca aagacaactg ccaaagaatc ttcaagaagg aggactgcaa gtatatcgtg 300
gtggagaaga aggacccaaa aaagacctgt tctgtcagtg aatggataat ctaatgtgct 360
tctagtaggc acagggctcc caggccaggc ctcattctcc tctggcctct aatagtcaat 420
gattgtgtag ccatgcctat cagtaaaaag atntttgagc aaacacttt 469

<210> 165
<211> 195
<212> DNA
<213> Homo sapien

<220>
<221> misc_feature
<222> (1)...(195)
<223> n = A,T,C or G

<400> 165
acagtttttt atanatatcg acattgccgg cacttgtgtt cagtttcata aagctggtgg 60
atccgctgtc atccactatt ccttggctag agtaaaaatt attcttatag cccatgtccc 120
tgcaggccgc ccgcccgtag ttctcgttcc agtcgtcttg gcacacaggg tgccaggact 180
tcctctgaga tgagt 195

<210> 166
<211> 383
<212> DNA
<213> Homo sapien

<220>
<221> misc_feature
<222> (1)...(383)
<223> n = A,T,C or G

<400> 166
acatcttagt agtgtggcac atcagggggc catcagggtc acagtcactc atagcctcgc 60
cgaggtcgga gtccacacca ccggtgtagg tgtgctcaat cttgggcttg gcgcccacct 120
ttggagaagg gatatgctgc acacacatgt ccacaaagcc tgtgaactcg ccaaagaatt 180
tttgcagacc agcctgagca aggggcggat gttcagcttc agctcctcct tcgtcaggtg 240
gatgccaacc tcgtctangg tccgtgggaa gctggtgtcc acntcaccta caacctgggc 300
gangatctta taaagaggct ccnagataaa ctccacgaaa cttctctggg agctgctagt 360
nggggccttt ttggtgaact ttc 383

<210> 167
<211> 247
<212> DNA
<213> Homo sapien

<220>
<221> misc_feature
<222> (1)...(247)
<223> n = A,T,C or G

<400> 167
acagagccag accttggcca taaatgaanc agagattaag actaaacccc aagtcganat 60
tggagcagaa actggagcaa gaagtgggcc tggggctgaa gtagagacca aggccactgc 120
tatanccata cacagagcca actctcaggc caaggcnatg gttggggcag anccagagac 180
tcaatctgan tccaaagtgg tggctggaac actggtcatg acanaggcag tgactctgac 240
tgangtc 247

<210> 168
<211> 273
<212> DNA
<213> Homo sapien

<220>
<221> misc_feature
<222> (1)...(273)
<223> n = A,T,C or G

<400> 168
acttctaagt tttctagaag tggaaggatt gtantcatcc tgaaaatggg tttacttcaa 60
aatccctcan ccttgttctt cacnactgtc tatactgana gtgtcatgtt tccacaaagg 120
gctgacacct gagcctgnat tttcactcat ccctgagaag ccctttccag tagggtgggc 180
aattcccaac ttccttgcca caagcttccc aggctttctc ccctggaaaa ctccagcttg 240
agtcccagat acactcatgg gctgccctgg gca 273

<210> 169
<211> 431
<212> DNA
<213> Homo sapien

<220>
<221> misc_feature
<222> (1)...(431)
<223> n = A,T,C or G

<400> 169
acagccttgg cttccccaaa ctccacagtc tcagtgcaga aagatcatct tccagcagtc 60
agctcagacc agggtcaaag gatgtgacat caacagtttc tggtttcaga acaggttcta 120
ctactgtcaa atgacccccc atacttcctc aaaggctgtg gtaagttttg cacaggtgag 180
ggcagcagaa agggggtant tactgatgga caccatcttc tctgtatact ccacactgac 240
cttgccatgg gcaaaggccc ctaccacaaa aacaatagga tcactgctgg gcaccagctc 300
acgcacatca ctgacaaccg ggatggaaaa agaantgcca actttcatac atccaactgg 360
aaagtgatct gatactggat tcttaattac cttcaaaagc ttctgggggc catcagctgc 420
tcgaacactg a 431

<210> 170
<211> 266
<212> DNA
<213> Homo sapien

<220>
<221> misc_feature

<222> (1)...(266)
<223> n = A,T,C or G

<400> 170
acctgtgggc tgggctgtta tgcctgtgcc ggctgctgaa agggagttca gaggtggagc 60
tcaaggagct ctgcaggcat tttgccaanc ctctccanag canagggagc aacctacact 120
ccccgctaga aagacaccag attggagtcc tgggaggggg agttggggtg ggcatttgat 180
gtatacttgt cacctgaatg aangagccag agaggaanga gacgaanatg anattggcct 240
tcaaagctag gggtctggca ggtgga 266

<210> 171
<211> 1248
<212> DNA
<213> Homo sapien

<220>
<221> misc_feature
<222> (1)...(1248)
<223> n = A,T,C or G

<400> 171
   ggcagccaaa tcataaacgg cgaggactgc agcccgcact cgcagccctg gcaggcggca 60
   ctggtcatgg aaaacgaatt gttctgctcg ggcgtcctgg tgcatccgca gtgggtgctg 120
   tcagccgcac actgtttcca gaagtgagtg cagagctcct acaccatcgg gctgggcctg 180
   cacagtcttg aggccgacca agagccaggg agccagatgg tggaggccag cctctccgta 240
   cggcacccag agtacaacag acccttgctc gctaacgacc tcatgctcat caagttggac 300
   gaatccgtgt ccgagtctga caccatccgg agcatcagca ttgcttcgca gtgccctacc 360
   gcggggaact cttgcctcgt ttctggctgg ggtctgctgg cgaacggcag aatgcctacc 420
   gtgctgcagt gcgtgaacgt gtcggtggtg tctgaggagg tctgcagtaa gctctatgac 480
   ccgctgtacc accccagcat gttctgcgcc ggcggagggc aagaccagaa ggactcctgc 540
   aacggtgact ctggggggcc cctgatctgc aacgggtact tgcagggcct tgtgtctttc 600
   ggaaaagccc cgtgtggcca agttggcgtg ccaggtgtct acaccaacct ctgcaaattc 660
   actgagtgga tagagaaaac cgtccaggcc agttaactct ggggactggg aacccatgaa 720
   attgaccccc aaatacatcc tgcggaagga attcaggaat atctgttccc agcccctcct 780
   ccctcaggcc caggagtcca ggcccccagc ccctcctccc tcaaaccaag ggtacagatc 840
   cccagcccct cctccctcag acccaggagt ccagaccccc cagcccctcc tccctcagac 900
   ccaggagtcc agcccctcct ccctcagacc caggagtcca gaccccccag cccctcctcc 960
   ctcagaccca ggggtccagg cccccaaccc ctcctccctc agactcagag gtccaagccc 1020
   ccaacccntc attccccaga cccagaggtc caggtcccag cccctcntcc ctcagaccca 1080
   gcggtccaat gccacctaga ctntccctgt acacagtgcc cccttgtggc acgttgaccc 1140
   aaccttacca gttggttttt catttttngt ccctttcccc tagatccaga aataaagttt 1200
   aagagaagng caaaaaaaaa aaaaaaaaaa aaaaaaaaaa aaaaaaaa 1248

<210> 172
<211> 159
<212> PRT
<213> Homo sapien

<220>
<221> VARIANT
<222> (1)...(159)
<223> Xaa = Any Amino Acid

<400> 172
Met Val Glu Ala Ser Leu Ser Val Arg His Pro Glu Tyr Asn Arg Pro
1               5                   10                  15
Leu Leu Ala Asn Asp Leu Met Leu Ile Lys Leu Asp Glu Ser Val Ser
            20                  25                  30
Glu Ser Asp Thr Ile Arg Ser Ile Ser Ile Ala Ser Gln Cys Pro Thr
        35                  40                  45
Ala Gly Asn Ser Cys Leu Val Ser Gly Trp Gly Leu Leu Ala Asn Gly
    50                  55                  60

Arg Met Pro Thr Val Leu Gln Cys Val Asn Val Ser Val Val Ser Glu
65                  70                  75                  80
Glu Val Cys Ser Lys Leu Tyr Asp Pro Leu Tyr His Pro Ser Met Phe
                85                  90                  95
Cys Ala Gly Gly Gly Gln Xaa Gln Xaa Asp Ser Cys Asn Gly Asp Ser
            100                 105                 110
Gly Gly Pro Leu Ile Cys Asn Gly Tyr Leu Gln Gly Leu Val Ser Phe
        115                 120                 125
Gly Lys Ala Pro Cys Gly Gln Val Gly Val Pro Gly Val Tyr Thr Asn
    130                 135                 140
Leu Cys Lys Phe Thr Glu Trp Ile Glu Lys Thr Val Gln Ala Ser
145                 150                 155

<210> 173
<211> 1265
<212> DNA
<213> Homo sapien

<220>
<221> misc_feature
<222> (1)...(1265)
<223> n = A,T,C or G

<400> 173
ggcagcccgc actcgcagcc ctggcaggcg gcactggtca tggaaaacga attgttctgc      60
tcgggcgtcc tggtgcatcc gcagtgggtg ctgtcagccg cacactgttt ccagaactcc     120
tacaccatcg ggctgggcct gcacagtctt gaggccgacc aagagccagg gagccagatg     180
gtggaggcca gcctctccgt acggcaccca gagtacaaca gacccttgct cgctaacgac     240
ctcatgctca tcaagttgga cgaatccgtg tccgagtctg acaccatccg gagcatcagc     300
attgcttcgc agtgccctac cgcggggaac tcttgcctcg tttctggctg gggtctgctg     360
gcgaacggtg agctcacggg tgtgtgtctg ccctcttcaa ggaggtcctc tgcccagtcg     420
cgggggctga cccagagctc tgcgtcccag gcagaatgcc taccgtgctg cagtgcgtga     480
acgtgtcggt ggtgtctgag gaggtctgca gtaagctcta tgacccgctg taccacccca     540
gcatgttctg cgccggcgga gggcaagacc agaaggactc ctgcaacggt gactctgggg     600
ggcccctgat ctgcaacggg tacttgcagg gccttgtgtc tttcggaaaa gccccgtgtg     660
gccaagttgg cgtgccaggt gtctacacca acctctgcaa attcactgag tggatagaga     720
aaaccgtcca ggccagttaa ctctggggac tgggaaccca tgaaattgac ccccaaatac     780
atcctgcgga aggaattcag gaatatctgt tcccagcccc tcctccctca ggcccaggag     840
tccaggcccc cagcccctcc tccctcaaac caagggtaca gatccccagc ccctcctccc     900
tcagacccag gagtccagac cccccagccc ctcctccctc agacccagga gtccagcccc     960
tcctccntca gacccaggag tccagacccc ccagcccctc ctccctcaga cccaggggtt    1020
gaggccccca acccctcctc cttcagagtc agaggtccaa gcccccaacc cctcgttccc    1080
cagacccaga ggtnnaggtc ccagcccctc ttccntcaga cccagnggtc caatgccacc    1140
tagattttcc ctgnacacag tgcccccttg tggnangttg acccaacctt accagttggt    1200
ttttcatttt tngtcccttt cccctagatc cagaaataaa gtttaagaga ngngcaaaaa    1260
aaaaa                                                                1265

<210> 174
<211> 1459
<212> DNA
<213> Homo sapien

<220>
<221> misc_feature
<222> (1)...(1459)
<223> n = A,T,C or G

<400> 174
   ggtcagccgc acactgtttc cagaagtgag tgcagagctc ctacaccatc gggctgggcc      60
   tgcacagtct tgaggccgac caagagccag ggagccagat ggtggaggcc agcctctccg     120
   tacggcaccc agagtacaac agacccttgc tcgctaacga cctcatgctc atcaagttgg     180
   acgaatccgt gtccgagtct gacaccatcc ggagcatcag cattgcttcg cagtgcccta     240
   ccgcggggaa ctcttgcctc gtttctggct ggggtctgct ggcgaacggt gagctcacgg 300
   gtgtgtgtct gccctcttca aggaggtcct ctgcccagtc gcgggggctg acccagagct 360
   ctgcgtccca ggcagaatgc ctaccgtgct gcagtgcgtg aacgtgtcgg tggtgtctga 420
   ngaggtctgc antaagctct atgacccgct gtaccacccc ancatgttct gcgccggcgg 480
   agggcaagac cagaaggact cctgcaacgt gagagagggg aaaggggagg gcaggcgact 540
   cagggaaggg tggagaaggg ggagacagag acacacaggg ccgcatggcg agatgcagag 600
   atggagagac acacagggag acagtgacaa ctagagagag aaactgagag aaacagagaa 660
   ataaacacag gaataaagag aagcaaagga agagagaaac agaaacagac atggggaggc 720
   agaaacacac acacatagaa atgcagttga ccttccaaca gcatggggcc tgagggcggt 780
   gacctccacc caatagaaaa tcctcttata acttttgact ccccaaaaac ctgactagaa 840
   atagcctact gttgacgggg agccttacca ataacataaa tagtcgattt atgcatacgt 900
   tttatgcatt catgatatac ctttgttgga attttttgat atttctaagc tacacagttc 960
   gtctgtgaat ttttttaaat tgttgcaact ctcctaaaat ttttctgatg tgtttattga 1020
   aaaaatccaa gtataagtgg acttgtgcat tcaaaccagg gttgttcaag ggtcaactgt 1080
   gtacccagag ggaaacagtg acacagattc atagaggtga aacacgaaga gaaacaggaa 1140
   aaatcaagac tctacaaaga ggctgggcag ggtggctcat gcctgtaatc ccagcacttt 1200
   gggaggcgag gcaggcagat cacttgaggt aaggagttca agaccagcct ggccaaaatg 1260
   gtgaaatcct gtctgtacta aaaatacaaa agttagctgg atatggtggc aggcgcctgt 1320
   aatcccagct acttgggagg ctgaggcagg agaattgctt gaatatggga ggcagaggtt 1380
   gaagtgagtt gagatcacac cactatactc cagctggggc aacagagtaa gactctgtct 1440
   caaaaaaaaa aaaaaaaaa 1459

<210> 175
<211> 1167
<212> DNA
<213> Homo sapien

<220>
<221> misc_feature
<222> (1)...(1167)
<223> n = A,T,C or G

<400> 175
gcgcagccct ggcaggcggc actggtcatg gaaaacgaat tgttctgctc gggcgtcctg 60
gtgcatccgc agtgggtgct gtcagccgca cactgtttcc agaactccta caccatcggg 120
ctgggcctgc acagtcttga ggccgaccaa gagccaggga gccagatggt ggaggccagc 180
ctctccgtac ggcacccaga gtacaacaga ctcttgctcg ctaacgacct catgctcatc 240
aagttggacg aatccgtgtc cgagtctgac accatccgga gcatcagcat tgcttcgcag 300
tgccctaccg cggggaactc ttgcctcgtn tctggctggg gtctgctggc gaacggcaga 360
atgcctaccg tgctgcactg cgtgaacgtg tcggtggtgt ctgaggangt ctgcagtaag 420
ctctatgacc cgctgtacca ccccagcatg ttctgcgccg gcggagggca agaccagaag 480
gactcctgca acggtgactc tggggggccc ctgatctgca acgggtactt gcagggcctt 540
gtgtctttcg gaaaagcccc gtgtggccaa cttggcgtgc caggtgtcta caccaacctc 600
tgcaaattca ctgagtggat agagaaaacc gtccagncca gttaactctg gggactggga 660
acccatgaaa ttgaccccca aatacatcct gcggaangaa ttcaggaata tctgttccca 720
gcccctcctc cctcaggccc aggagtccag gcccccagcc cctcctccct caaaccaagg 780
gtacagatcc ccagcccctc ctccctcaga cccaggagtc cagacccccc agcccctcnt 840
ccntcagacc caggagtcca gcccctcctc cntcagacgc aggagtccag accccccagc 900
ccntcntccg tcagacccag gggtgcaggc ccccaacccc tcntccntca gagtcagagg 960
tccaagcccc caacccctcg ttccccagac ccagaggtnc aggtcccagc ccctcctccc 1020
tcagacccag cggtccaatg ccacctagan tntccctgta cacagtgccc ccttgtggca 1080
ngttgaccca accttaccag ttggtttttc attttttgtc cctttcccct agatccagaa 1140
ataaagtnta agagaagcgc aaaaaaa 1167

<210> 176
<211> 205
<212> PRT
<213> Homo sapien

<220>
<221> VARIANT
<222> (1)...(205)

<223> Xaa = Any Amino Acid

<400> 176
Met Glu Asn Glu Leu Phe Cys Ser Gly Val Leu Val His Pro Gln Trp
1               5                   10                  15
Val Leu Ser Ala Ala His Cys Phe Gln Asn Ser Tyr Thr Ile Gly Leu
            20                  25                  30
Gly Leu His Ser Leu Glu Ala Asp Gln Glu Pro Gly Ser Gln Met Val
        35                  40                  45
Glu Ala Ser Leu Ser Val Arg His Pro Glu Tyr Asn Arg Leu Leu Leu
    50                  55                  60
Ala Asn Asp Leu Met Leu Ile Lys Leu Asp Glu Ser Val Ser Glu Ser
65                  70                  75                  80
Asp Thr Ile Arg Ser Ile Ser Ile Ala Ser Gln Cys Pro Thr Ala Gly
                85                  90                  95
Asn Ser Cys Leu Val Ser Gly Trp Gly Leu Leu Ala Asn Gly Arg Met
            100                 105                 110
Pro Thr Val Leu His Cys Val Asn Val Ser Val Val Ser Glu Xaa Val
        115                 120                 125
Cys Ser Lys Leu Tyr Asp Pro Leu Tyr His Pro Ser Met Phe Cys Ala
    130                 135                 140
Gly Gly Gly Gln Asp Gln Lys Asp Ser Cys Asn Gly Asp Ser Gly Gly
145                 150                 155                 160
Pro Leu Ile Cys Asn Gly Tyr Leu Gln Gly Leu Val Ser Phe Gly Lys
                165                 170                 175
Ala Pro Cys Gly Gln Leu Gly Val Pro Gly Val Tyr Thr Asn Leu Cys
            180                 185                 190
Lys Phe Thr Glu Trp Ile Glu Lys Thr Val Gln Xaa Ser
        195                 200                 205

<210> 177
<211> 1119
<212> DNA
<213> Homo sapien

<400> 177
gcgcactcgc agccctggca ggcggcactg gtcatggaaa acgaattgtt ctgctcgggc      60
gtcctggtgc atccgcagtg ggtgctgtca gccgcacact gtttccagaa ctcctacacc     120
atcgggctgg gcctgcacag tcttgaggcc gaccaagagc cagggagcca gatggtggag     180
gccagcctct ccgtacggca cccagagtac aacagaccct tgctcgctaa cgacctcatg     240
ctcatcaagt tggacgaatc cgtgtccgag tctgacacca tccggagcat cagcattgct     300
tcgcagtgcc ctaccgcggg gaactcttgc ctcgtttctg gctggggtct gctggcgaac     360
gatgctgtga ttgccatcca gtcccagact gtgggaggct gggagtgtga gaagctttcc     420
caaccctggc agggttgtac catttcggca acttccagtg caaggacgtc ctgctgcatc     480
ctcactgggt gctcactact gctcactgca tcacccggaa cactgtgatc aactagccag     540
caccatagtt ctccgaagtc agactatcat gattactgtg ttgactgtgc tgtctattgt     600
actaaccatg ccgatgttta ggtgaaatta gcgtcacttg gcctcaacca tcttggtatc     660
cagttatcct cactgaattg agatttcctg cttcagtgtc agccattccc acataatttc     720
tgacctacag aggtgaggga tcatatagct cttcaaggat gctggtactc ccctcacaaa     780
ttcatttctc ctgttgtagt gaaaggtgcg ccctctggag cctcccaggg tgggtgtgca     840
ggtcacaatg atgaatgtat gatcgtgttc ccattaccca aagcctttaa atccctcatg     900
ctcagtacac cagggcaggt ctagcatttc ttcatttagt gtatgctgtc cattcatgca     960
accacctcag gactcctgga ttctctgcct agttgagctc ctgcatgctg cctccttggg    1020
gaggtgaggg agagggccca tggttcaatg ggatctgtgc agttgtaaca cattaggtgc    1080
ttaataaaca gaagctgtga tgttaaaaaa aaaaaaaaa                           1119

<210> 178
<211> 164
<212> PRT
<213> Homo sapien

<220>

<221> VARIANT
<222> (1)...(164)
<223> Xaa = Any Amino Acid

<400> 178
Met Glu Asn Glu Leu Phe Cys Ser Gly Val Leu Val His Pro Gln Trp
 1               5                  10                  15
Val Leu Ser Ala Ala His Cys Phe Gln Asn Ser Tyr Thr Ile Gly Leu
            20                  25                  30
Gly Leu His Ser Leu Glu Ala Asp Gln Glu Pro Gly Ser Gln Met Val
        35                  40                  45
Glu Ala Ser Leu Ser Val Arg His Pro Glu Tyr Asn Arg Pro Leu Leu
    50                  55                  60
Ala Asn Asp Leu Met Leu Ile Lys Leu Asp Glu Ser Val Ser Glu Ser
65                  70                  75                  80
Asp Thr Ile Arg Ser Ile Ser Ile Ala Ser Gln Cys Pro Thr Ala Gly
                85                  90                  95
Asn Ser Cys Leu Val Ser Gly Trp Gly Leu Leu Ala Asn Asp Ala Val
            100                 105                 110
Ile Ala Ile Gln Ser Xaa Thr Val Gly Gly Trp Glu Cys Glu Lys Leu
        115                 120                 125
Ser Gln Pro Trp Gln Gly Cys Thr Ile Ser Ala Thr Ser Ser Ala Arg
    130                 135                 140
Thr Ser Cys Cys Ile Leu Thr Gly Cys Ser Leu Leu Leu Thr Ala Ser
145                 150                 155                 160
Pro Gly Thr Leu

<210> 179
<211> 250
<212> DNA
<213> Homo sapien

<400> 179
ctggagtgcc ttggtgtttc aagcccctgc aggaagcaga atgcaccttc tgaggcacct      60
ccagctgccc ccggccgggg gatgcgaggc tcggagcacc cttgcccggc tgtgattgct     120
gccaggcact gttcatctca gcttttctgt ccctttgctc ccggcaagcg cttctgctga     180
aagttcatat ctggagcctg atgtcttaac gaataaaggt cccatgctcc acccgaaaaa     240
aaaaaaaaaa                                                            250

<210> 180
<211> 202
<212> DNA
<213> Homo sapien

<400> 180
actagtccag tgtggtggaa ttccattgtg ttgggcccaa cacaatggct acctttaaca      60
tcacccagac cccgcccctg cccgtgcccc acgctgctgc taacgacagt atgatgctta     120
ctctgctact cggaaactat ttttatgtaa ttaatgtatg ctttcttgtt tataaatgcc     180
tgatttaaaa aaaaaaaaaa aa                                              202

<210> 181
<211> 558
<212> DNA
<213> Homo sapien

<220>
<221> misc_feature
<222> (1)...(558)
<223> n = A,T,C or G

<400> 181

tccytttgkt naggtttkkg agacamccck agacctwaan ctgtgtcaca gacttcyngg 60
aatgtttagg cagtgctagt aatttcytcg taatgattct gttattactt tcctnattct 120
ttattcctct ttcttctgaa gattaatgaa gttgaaaatt gaggtggata aatacaaaaa 180
ggtagtgtga tagtataagt atctaagtgc agatgaaagt gtgttatata tatccattca 240
aaattatgca agttagtaat tactcagggt taactaaatt actttaatat gctgttgaac 300
ctactctgtt ccttggctag aaaaaattat aaacaggact ttgttagttt gggaagccaa 360
attgataata ttctatgttc taaaagttgg gctatacata aattattaag aaatatggaw 420
ttttattccc aggaatatgg kgttcatttt atgaatatta cscrggatag awgtwtgagt 480
aaaaycagtt ttggtwaata ygtwaatatg tcmtaaataa acaakgcttt gacttatttc 540
caaaaaaaaa aaaaaaaa 558

<210> 182
<211> 479
<212> DNA
<213> Homo sapien

<220>
<221> misc_feature
<222> (1)...(479)
<223> n = A,T,C or G

<400> 182
acagggwttk grggatgcta agsccccrga rwtygtttga tccaaccctg gcttwttttc 60
agaggggaaa atggggccta gaagttacag mscatytagy tggtgcgmtg gcacccctgg 120
cstcacacag astcccgagt agctgggact acaggcacac agtcactgaa gcaggccctg 180
ttwgcaattc acgttgccac ctccaactta aacattcttc atatgtgatg tccttagtca 240
ctaaggttaa actttcccac ccagaaaagg caacttagat aaaatcttag agtactttca 300
tactmttcta agtcctcttc cagcctcact kkgagtcctm cytgggggtt gataggaant 360
ntctcttggc tttctcaata aartctctat ycatctcatg tttaatttgg tacgcatara 420
awtgstgara aaattaaaat gttctggtty mactttaaaa araaaaaaaa aaaaaaaaa 479

<210> 183
<211> 384
<212> DNA
<213> Homo sapien

<400> 183
aggcgggagc agaagctaaa gccaaagccc aagaagagtg gcagtgccag cactggtgcc 60
agtaccagta ccaataacag tgccagtgcc agtgccagca ccagtggtgg cttcagtgct 120
ggtgccagcc tgaccgccac tctcacattt gggctcttcg ctggccttgg tggagctggt 180
gccagcacca gtggcagctc tggtgcctgt ggtttctcct acaagtgaga ttttagatat 240
tgttaatcct gccagtcttt ctcttcaagc cagggtgcat cctcagaaac ctactcaaca 300
cagcactcta ggcagccact atcaatcaat tgaagttgac actctgcatt aratctattt 360
gccatttcaa aaaaaaaaaa aaaa 384

<210> 184
<211> 496
<212> DNA
<213> Homo sapien

<220>
<221> misc_feature
<222> (1)...(496)
<223> n = A,T,C or G

<400> 184
accgaattgg gaccgctggc ttataagcga tcatgtyynt ccrgtatkac ctcaacgagc 60
agggagatcg agtctatacg ctgaagaaat ttgacccgat gggacaacag acctgctcag 120
cccatcctgc tcggttctcc ccagatgaca aatactctsg acaccgaatc accatcaaga 180
aacgcttcaa ggtgctcatg acccagcaac cgcgccctgt cctctgaggg tcccttaaac 240
tgatgtcttt tctgccacct gttacccctc ggagactccg taaccaaact cttcggactg 300
tgagccctga tgcctttttg ccagccatac tctttggcat ccagtctctc gtggcgattg 360

attatgcttg tgtgaggcaa tcatggtggc atcacccata aagggaacac atttgacttt 420
tttttctcat attttaaatt actacmagaw tattwmagaw waaatgawtt gaaaaactst 480
taaaaaaaaa aaaaaa 496

<210> 185
<211> 384
<212> DNA
<213> Homo sapien

<400> 185
gctggtagcc tatggcgkgg cccacggagg ggctcctgag gccacggrac agtgacttcc 60
caagtatcyt gcgcsgcgtc ttctaccgtc cctacctgca gatcttcggg cagattcccc 120
aggaggacat ggacgtggcc ctcatggagc acagcaactg ytcgtcggag cccggcttct 180
gggcacaccc tcctggggcc caggcgggca cctgcgtctc ccagtatgcc aactggctgg 240
tggtgctgct cctcgtcatc ttcctgctcg tggccaacat cctgctggtc aacttgctca 300
ttgccatgtt cagttacaca ttcggcaaag tacagggcaa cagcgatctc tactgggaag 360
gcgcagcgtt accgcctcat ccgg 384

<210> 186
<211> 577
<212> DNA
<213> Homo sapien

<220>
<221> misc_feature
<222> (1)...(577)
<223> n = A,T,C or G

<400> 186
gagttagctc ctccacaacc ttgatgaggt cgtctgcagt ggcctctcgc ttcataccgc 60
tnccatcgtc atactgtagg tttgccacca cytcctggca tcttggggcg gcntaatatt 120
ccaggaaact ctcaatcaag tcaccgtcga tgaaacctgt gggctggttc tgtcttccgc 180
tcggtgtgaa aggatctccc agaaggagtg ctcgatcttc cccacacttt tgatgacttt 240
attgagtcga ttctgcatgt ccagcaggag gttgtaccag ctctctgaca gtgaggtcac 300
cagccctatc atgccgttga mcgtgccgaa garcaccgag ccttgtgtgg gggkkgaagt 360
ctcacccaga ttctgcatta ccagagagcc gtggcaaaag acattgacaa actcgcccag 420
gtggaaaaag amcamctcct ggargtgctn gccgctcctc gtcmgttggt ggcagcgctw 480
tccttttgac acacaaacaa gttaaaggca ttttcagccc ccagaaantt gtcatcatcc 540
aagatntcgc acagcactna tccagttggg attaaat 577

<210> 187
<211> 534
<212> DNA
<213> Homo sapien

<220>
<221> misc_feature
<222> (1)...(534)
<223> n = A,T,C or G

<400> 187
aacatcttcc tgtataatgc tgtgtaatat cgatccgatn ttgtctgstg agaatycatw 60
actkggaaaa gmaacattaa agcctggaca ctggtattaa aattcacaat atgcaacact 120
ttaaacagtg tgtcaatctg ctcccyynac tttgtcatca ccagtctggg aakaagggta 180
tgccctattc acacctgtta aaagggcgct aagcattttt gattcaacat cttttttttt 240
gacacaagtc cgaaaaaagc aaaagtaaac agttatyaat ttgttagcca attcactttc 300
ttcatgggac agagccatyt gatttaaaaa gcaaattgca taatattgag cttygggagc 360
tgatatttga gcggaagagt agcctttcta cttcaccaga cacaactccc tttcatattg 420
ggatgttnac naaagtwatg tctctwacag atgggatgct tttgtggcaa ttctgttctg 480
aggatctccc agtttattta ccacttgcac aagaaggcgt tttcttcctc aggc 534

<210> 188

<211> 761
<212> DNA
<213> Homo sapien

<220>
<221> misc_feature
<222> (1)...(761)
<223> n = A,T,C or G

<400> 188
agaaaccagt atctctnaaa acaacctctc ataccttgtg gacctaattt tgtgtgcgtg 60
tgtgtgtgcg cgcatattat atagacaggc acatcttttt tacttttgta aaagcttatg 120
cctctttggt atctatatct gtgaaagttt taatgatctg ccataatgtc ttggggacct 180
ttgtcttctg tgtaaatggt actagagaaa acacctatnt tatgagtcaa tctagttngt 240
tttattcgac atgaaggaaa tttccagatn acaacactna caaactctcc ctkgackarg 300
ggggacaaag aaaagcaaaa ctgamcataa raaacaatwa cctggtgaga arttgcataa 360
acagaaatwr ggtagtatat tgaarnacag catcattaaa rmgttwtktt wttctccctt 420
gcaaaaaaca tgtacngact tcccgttgag taatgccaag ttgttttttt tatnataaaa 480
cttgcccttc attacatgtt tnaaagtggt gtggtgggcc aaaatattga aatgatggaa 540
ctgactgata aagctgtaca aataagcagt gtgcctaaca agcaacacag taatgttgac 600
atgcttaatt cacaaatgct aatttcatta taaatgtttg ctaaaataca ctttgaacta 660
tttttctgtn ttcccagagc tgagatntta gattttatgt agtatnaagt gaaaaantac 720
gaaaataata acattgaaga aaaananaaa aaanaaaaaa a 761

<210> 189
<211> 482
<212> DNA
<213> Homo sapien

<220>
<221> misc_feature
<222> (1)...(482)
<223> n = A,T,C or G

<400> 189
tttttttttt tttgccgatn ctactatttt attgcaggan gtgggggtgt atgcaccgca 60
caccggggct atnagaagca agaaggaagg agggagggca cagccccttg ctgagcaaca 120
aagccgcctg ctgccttctc tgtctgtctc ctggtgcagg cacatgggga gaccttcccc 180
aaggcagggg ccaccagtcc aggggtggga atacaggggg tgggangtgt gcataagaag 240
tgataggcac aggccacccg gtacagaccc ctcggctcct gacaggtnga tttcgaccag 300
gtcattgtgc cctgcccagg cacagcgtan atctggaaaa gacagaatgc tttccttttc 360
aaatttggct ngtcatngaa ngggcanttt tccaanttng gctnggtctt ggtacncttg 420
gttcggccca gctccncgtc caaaaantat tcacccnnct ccnaattgct tgcnggnccc 480
cc 482

<210> 190
<211> 471
<212> DNA
<213> Homo sapien

<220>
<221> misc_feature
<222> (1)...(471)
<223> n = A,T,C or G

<400> 190
tttttttttt ttttaaaaca gtttttcaca acaaaattta ttagaagaat agtggttttg 60
aaaactctcg catccagtga gaactaccat acaccacatt acagctngga atgtnctcca 120
aatgtctggt caaatgatac aatggaacca ttcaatctta cacatgcacg aaagaacaag 180
cgcttttgac atacaatgca caaaaaaaaa aggggggggg gaccacatgg attaaaattt 240
taagtactca tcacatacat taagacacag ttctagtcca gtcnaaaatc agaactgcnt 300
tgaaaaattt catgtatgca atccaaccaa agaacttnat tggtgatcat gantnctcta 360

ctacatcnac cttgatcatt gccaggaacn aaaagttnaa ancacncngt acaaaaanaa 420
tctgtaattn anttcaacct ccgtacngaa aaatnttnnt tatacactcc c 471

<210> 191
<211> 402
<212> DNA
<213> Homo sapien

<220>
<221> misc_feature
<222> (1)...(402)
<223> n = A,T,C or G

<400> 191
gagggattga aggtctgttc tastgtcggm ctgttcagcc accaactcta acaagttgct 60
gtcttccact cactgtctgt aagcttttta acccagacwg tatcttcata aatagaacaa 120
attcttcacc agtcacatct tctaggacct ttttggattc agttagtata agctcttcca 180
cttcctttgt taagacttca tctggtaaag tcttaagttt tgtagaaagg aattyaattg 240
ctcgttctct aacaatgtcc tctccttgaa gtatttggct gaacaaccca cctaaagtcc 300
ctttgtgcat ccattttaaa tatacttaat agggcattgk tncactaggt taaattctgc 360
aagagtcatc tgtctgcaaa agttgcgtta gtatatctgc ca 402

<210> 192
<211> 601
<212> DNA
<213> Homo sapien

<220>
<221> misc_feature
<222> (1)...(601)
<223> n = A,T,C or G

<400> 192
gagctcggat ccaataatct ttgtctgagg gcagcacaca tatncagtgc catggnaact 60
ggtctacccc acatgggagc agcatgccgt agntatataa ggtcattccc tgagtcagac 120
atgcytyttt gaytaccgtg tgccaagtgc tggtgattct yaacacacyt ccatcccgyt 180
cttttgtgga aaaactggca cttktctgga actagcarga catcacttac aaattcaccc 240
acgagacact tgaaaggtgt aacaaagcga ytcttgcatt gctttttgtc cctccggcac 300
cagttgtcaa tactaacccg ctggtttgcc tccatcacat ttgtgatctg tagctctgga 360
tacatctcct gacagtactg aagaacttct tcttttgttt caaaagcarc tcttggtgcc 420
tgttggatca ggttcccatt tcccagtcyg aatgttcaca tggcatattt wacttcccac 480
aaaacattgc gatttgaggc tcagcaacag caaatcctgt tccggcattg gctgcaagag 540
cctcgatgta gccggccagc gccaaggcag gcgccgtgag ccccaccagc agcagaagca 600
g 601

<210> 193
<211> 608
<212> DNA
<213> Homo sapien

<220>
<221> misc_feature
<222> (1)...(608)
<223> n = A,T,C or G

<400> 193
atacagccca natcccacca cgaagatgcg cttgttgact gagaacctga tgcggtcact 60
ggtcccgctg tagccccagc gactctccac ctgctggaag cggttgatgc tgcactcytt 120
cccaacgcag gcagmagcgg gsccggtcaa tgaactccay tcgtggcttg gggtkgacgg 180
tkaagtgcag gaagaggctg accacctcgc ggtccaccag gatgcccgac tgtgcgggac 240
ctgcagcgaa actcctcgat ggtcatgagc gggaagcgaa tgaggcccag ggccttgccc 300
agaaccttcc gcctgttctc tggcgtcacc tgcagctgct gccgctgaca ctcggcctcg 360

gaccagcgga caaacggcrt tgaacagccg cacctcacgg atgcccagtg tgtcgcgctc 420
caggammgsc accagcgtgt ccaggtcaat gtcggtgaag ccctccgcgg gtratggcgt 480
ctgcagtgtt tttgtcgatg ttctccaggc acaggctggc cagctgcggt tcatcgaaga 540
gtcgcgcctg cgtgagcagc atgaaggcgt tgtcggctcg cagttcttct tcaggaactc 600
cacgcaat 608

<210> 194
<211> 392
<212> DNA
<213> Homo sapien

<220>
<221> misc_feature
<222> (1)...(392)
<223> n = A,T,C or G

<400> 194
gaacggctgg accttgcctc gcattgtgct tgctggcagg gaataccttg gcaagcagyt 60
ccagtccgag cagccccaga ccgctgccgc ccgaagctaa gcctgcctct ggccttcccc 120
tccgcctcaa tgcagaacca gtagtgggag cactgtgttt agagttaaga gtgaacactg 180
tttgatttta cttgggaatt tcctctgtta tatagctttt cccaatgcta atttccaaac 240
aacaacaaca aaataacatg tttgcctgtt aagttgtata aaagtaggtg attctgtatt 300
taaagaaaat attactgtta catatactgc ttgcaatttc tgtatttatt gktnctstgg 360
aaataaatat agttattaaa ggttgtcant cc 392

<210> 195
<211> 502
<212> DNA
<213> Homo sapien

<220>
<221> misc_feature
<222> (1)...(502)
<223> n = A,T,C or G

<400> 195
ccsttkgagg ggtkaggkyc cagttyccga gtggaagaaa caggccagga gaagtgcgtg 60
ccgagctgag gcagatgttc ccacagtgac ccccagagcc stgggstata gtytctgacc 120
cctcncaagg aaagaccacs ttctggggac atgggctgga gggcaggacc tagaggcacc 180
aagggaaggc cccattccgg ggstgttccc cgaggaggaa gggaaggggc tctgtgtgcc 240
ccccasgagg aagaggccct gagtcctggg atcagacacc ccttcacgtg tatccccaca 300
caaatgcaag ctcaccaagg tcccctctca gtccccttcc stacaccctg amcggccact 360
gscscacacc cacccagagc acgccacccg ccatggggar tgtgctcaag gartcgcngg 420
gcarcgtgga catctngtcc cagaaggggg cagaatctcc aatagangga ctgarcmstt 480
gctnanaaaa aaaaanaaaa aa 502

<210> 196
<211> 665
<212> DNA
<213> Homo sapien

<220>
<221> misc_feature
<222> (1)...(665)
<223> n = A,T,C or G

<400> 196
ggttacttgg tttcattgcc accacttagt ggatgtcatt tagaaccatt ttgtctgctc 60
cctctggaag ccttgcgcag agcggacttt gtaattgttg gagaataact gctgaatttt 120
wagctgtttk gagttgatts gcaccactgc acccacaact tcaatatgaa aacyawttga 180
actwatttat tatcttgtga aaagtataac aatgaaaatt ttgttcatac tgtattkatc 240
aagtatgatg aaaagcaawa gatatatatt cttttattat gttaaattat gattgccatt 300

attaatcggc aaaatgtgga gtgtatgttc ttttcacagt aatatatgcc ttttgtaact 360
tcacttggtt attttattgt aaatgartta caaaattctt aatttaagar aatggtatgt 420
watatttatt tcattaattt ctttcctkgt ttacgtwaat tttgaaaaga wtgcatgatt 480
tcttgacaga aatcgatctt gatgctgtgg aagtagtttg acccacatcc ctatgagttt 540
ttcttagaat gtataaaggt tgtagcccat cnaacttcaa agaaaaaaat gaccacatac 600
tttgcaatca ggctgaaatg tggcatgctn ttctaattcc aactttataa actagcaaan 660
aagtg 665

<210> 197
<211> 492
<212> DNA
<213> Homo sapien

<220>
<221> misc_feature
<222> (1)...(492)
<223> n = A,T,C or G

<400> 197
ttttnttttt ttttttttgc aggaaggatt ccatttattg tggatgcatt ttcacaatat 60
atgtttattg gagcgatcca ttatcagtga aaagtatcaa gtgtttataa natttttagg 120
aaggcagatt cacagaacat gctngtcngc ttgcagtttt acctcgtana gatnacagag 180
aattatagtc naaccagtaa acnaggaatt tacttttcaa aagattaaat ccaaactgaa 240
caaaattcta ccctgaaact tactccatcc aaatattgga ataanagtca gcagtgatac 300
attctcttct gaactttaga ttttctagaa aaatatgtaa tagtgatcag gaagagctct 360
tgttcaaaag tacaacnaag caatgttccc ttaccatagg ccttaattca aactttgatc 420
catttcactc ccatcacggg agtcaatgct acctgggaca cttgtatttt gttcatnctg 480
ancntggctt aa 492

<210> 198
<211> 478
<212> DNA
<213> Homo sapien

<220>
<221> misc_feature
<222> (1)...(478)
<223> n = A,T,C or G

<400> 198
tttnttttgn atttcantct gtannaanta ttttcattat gtttattana aaaatatnaa 60
tgtntccacn acaaatcatn ttacntnagt aagaggccan ctacattgta caacatacac 120
tgagtatatt ttgaaaagga caagtttaaa gtanacncat attgccganc atancacatt 180
tatacatggc ttgattgata tttagcacag canaaactga gtgagttacc agaaanaaat 240
natatatgtc aatcngattt aagatacaaa acagatccta tggtacatan catcntgtag 300
gagttgtggc tttatgttta ctgaaagtca atgcagttcc tgtacaaaga gatggccgta 360
agcattctag tacctctact ccatggttaa gaatcgtaca cttatgttta catatgtnca 420
gggtaagaat tgtgttaagt naanttatgg agaggtccan gagaaaaatt tgatncaa 478

<210> 199
<211> 482
<212> DNA
<213> Homo sapien

<220>
<221> misc_feature
<222> (1)...(482)
<223> n = A,T,C or G

<400> 199
agtgacttgt cctccaacaa aaccccttga tcaagtttgt ggcactgaca atcagaccta 60
tgctagttcc tgtcatctat tcgctactaa atgcagactg gaggggacca aaaaggggca 120

tcaactccag ctggattatt ttggagcctg caaatctatt cctacttgta cggactttga 180
agtgattcag tttcctctac ggatgagaga ctggctcaag aatatcctca tgcagcttta 240
tgaagccnac tctgaacacg ctggttatct nagatgagaa ncagagaaat aaagtcnaga 300
aaatttacct ggangaaaag aggctttngg ctggggacca tcccattgaa ccttctctta 360
anggacttta agaanaaact accacatgtn tgtngtatcc tggtgccngg ccgtttantg 420
aacntngacn ncacccttnt ggaatanant cttgacngcn tcctgaactt gctcctctgc 480
ga 482

<210> 200
<211> 270
<212> DNA
<213> Homo sapien

<220>
<221> misc_feature
<222> (1)...(270)
<223> n = A,T,C or G

<400> 200
cggccgcaag tgcaactcca gctggggccg tgcggacgaa gattctgcca gcagttggtc 60
cgactgcgac gacggcggcg gcgacagtcg caggtgcagc gcgggcgcct ggggtcttgc 120
aaggctgagc tgacgccgca gaggtcgtgt cacgtcccac gaccttgacg ccgtcgggga 180
cagccggaac agagcccggt gaangcggga ggcctcgggg agcccctcgg gaagggcggc 240
ccgagagata cgcaggtgca ggtggccgcc 270

<210> 201
<211> 419
<212> DNA
<213> Homo sapien

<220>
<221> misc_feature
<222> (1)...(419)
<223> n = A,T,C or G

<400> 201
tttttttttt ttttggaatc tactgcgagc acagcaggtc agcaacaagt ttattttgca 60
gctagcaagg taacagggta gggcatggtt acatgttcag gtcaacttcc tttgtcgtgg 120
ttgattggtt tgtctttatg ggggcggggt ggggtagggg aaancgaagc anaantaaca 180
tggagtgggt gcaccctccc tgtagaacct ggttacnaaa gcttggggca gttcacctgg 240
tctgtgaccg tcattttctt gacatcaatg ttattagaag tcaggatatc ttttagagag 300
tccactgtnt ctggagggag attagggttt cttgccaana tccaancaaa atccacntga 360
aaaagttgga tgatncangt acngaatacc ganggcatan ttctcatant cggtggcca 419

<210> 202
<211> 509
<212> DNA
<213> Homo sapien

<220>
<221> misc_feature
<222> (1)...(509)
<223> n = A,T,C or G

<400> 202
tttntttttt tttttttttt tttttttttt tttttttttt tttttttttt tttttttttt 60
tggcacttaa tccattttta tttcaaaatg tctacaaant ttnaatncnc cattatacng 120
gtnattttnc aaaatctaaa nnttattcaa atntnagcca aantccttac ncaaatnnaa 180
tacncncaaa aatcaaaaat atacntntct ttcagcaaac ttngttacat aaattaaaaa 240
aatatatacg gctggtgttt tcaaagtaca attatcttaa cactgcaaac atntttnnaa 300
ggaactaaaa taaaaaaaaa cactnccgca aaggttaaag ggaacaacaa attcntttta 360
caacancnnc nattataaaa atcatatctc aaatcttagg ggaatatata cttcacacng 420

ggatcttaac ttttactnca ctttgtttat ttttttanaa ccattgtntt gggcccaaca 480
caatggnaat nccnccncnc tggactagt 509

<210> 203
<211> 583
<212> DNA
<213> Homo sapien

<220>
<221> misc_feature
<222> (1)...(583)
<223> n = A,T,C or G

<400> 203
tttttttttt ttttttttga cccccctctt ataaaaaaca agttaccatt ttattttact 60
tacacatatt tattttataa ttggtattag atattcaaaa ggcagctttt aaaatcaaac 120
taaatggaaa ctgccttaga tacataattc ttaggaatta gcttaaaatc tgcctaaagt 180
gaaaatcttc tctagctctt ttgactgtaa atttttgact cttgtaaaac atccaaattc 240
atttttcttg tctttaaaat tatctaatct ttccattttt tccctattcc aagtcaattt 300
gcttctctag cctcatttcc tagctcttat ctactattag taagtggctt ttttcctaaa 360
agggaaaaca ggaagagana atggcacaca aaacaaacat tttatattca tatttctacc 420
tacgttaata aaatagcatt ttgtgaagcc agctcaaaag aaggcttaga tccttttatg 480
tccattttag tcactaaacg atatcnaaag tgccagaatg caaaaggttt gtgaacattt 540
attcaaaagc taatataaga tatttcacat actcatcttt ctg 583

<210> 204
<211> 589
<212> DNA
<213> Homo sapien

<220>
<221> misc_feature
<222> (1)...(589)
<223> n = A,T,C or G

<400> 204
ttttttttnt tttttttttt ttttttnctc ttcttttttt ttganaatga ggatcgagtt 60
tttcactctc tagatagggc atgaagaaaa ctcatctttc cagctttaaa ataacaatca 120
aatctcttat gctatatcat attttaagtt aaactaatga gtcactggct tatcttctcc 180
tgaaggaaat ctgttcattc ttctcattca tatagttata tcaagtacta ccttgcatat 240
tgagaggttt ttcttctcta tttacacata tatttccatg tgaatttgta tcaaaccttt 300
attttcatgc aaactagaaa ataatgtntt cttttgcata agagaagaga acaatatnag 360
cattacaaaa ctgctcaaat tgtttgttaa gnttatccat tataattagt tnggcaggag 420
ctaatacaaa tcacatttac ngacnagcaa taataaaact gaagtaccag ttaaatatcc 480
aaaataatta aaggaacatt tttagcctgg gtataattag ctaattcact ttacaagcat 540
ttattnagaa tgaattcaca tgttattatt ccntagccca acacaatgg 589

<210> 205
<211> 545
<212> DNA
<213> Homo sapien

<220>
<221> misc_feature
<222> (1)...(545)
<223> n = A,T,C or G

<400> 205
tttttntttt ttttttcagt aataatcaga acaatattta tttttatatt taaaattcat 60
agaaaagtgc cttacattta ataaaagttt gtttctcaaa gtgatcagag gaattagata 120
tngtcttgaa caccaatatt aatttgagga aaatacacca aaatacatta agtaaattat 180
ttaagatcat agagcttgta agtgaaaaga taaaatttga cctcagaaac tctgagcatt 240

aaaaatccac tattagcaaa taaattacta tggacttctt gctttaattt tgtgatgaat 300
atggggtgtc actggtaaac caacacattc tgaaggatac attacttagt gatagattct 360
tatgtacttt gctanatnac gtggatatga gttgacaagt ttctctttct tcaatctttt 420
aaggggcnga ngaaatgagg aagaaaagaa aaggattacg catactgttc tttctatngg 480
aaggattaga tatgtttcct ttgccaatat taaaaaaata ataatgttta ctactagtga 540
aaccc 545

<210> 206
<211> 487
<212> DNA
<213> Homo sapien

<220>
<221> misc_feature
<222> (1)...(487)
<223> n = A,T,C or G

<400> 206
tttttttttt ttttttagtc aagtttctna tttttattat aattaaagtc ttggtcattt 60
catttattag ctctgcaact tacatattta aattaaagaa acgttnttag acaactgtna 120
caatttataa atgtaaggtg ccattattga gtanatatat tcctccaaga gtggatgtgt 180
cccttctccc accaactaat gaancagcaa cattagttta attttattag tagatnatac 240
actgctgcaa acgctaattc tcttctccat ccccatgtng atattgtgta tatgtgtgag 300
ttggtnagaa tgcatcanca atctnacaat caacagcaag atgaagctag gcntggcttt 360
tcggtgaaaa tagactgtgt ctgtctgaat caaatgatct gacctatcct cggtggcaag 420
aactcttcga accgcttcct caaaggcngc tgccacattt gtggcntctn ttgcacttgt 480
ttcaaaa 487

<210> 207
<211> 332
<212> DNA
<213> Homo sapien

<220>
<221> misc_feature
<222> (1)...(332)
<223> n = A,T,C or G

<400> 207
tgaattggct aaaagactgc atttttanaa ctagcaactc ttatttcttt cctttaaaaa 60
tacatagcat taaatcccaa atcctattta aagacctgac agcttgagaa ggtcactact 120
gcatttatag gaccttctgg tggttctgct gttacntttg aantctgaca atccttgana 180
atctttgcat gcagaggagg taaaaggtat tggattttca cagaggaana acacagcgca 240
gaaatgaagg ggccaggctt actgagcttg tccactggag ggctcatggg tgggacatgg 300
aaaagaaggc agcctaggcc ctggggagcc ca 332

<210> 208
<211> 524
<212> DNA
<213> Homo sapien

<220>
<221> misc_feature
<222> (1)...(524)
<223> n = A,T,C or G

<400> 208
agggcgtggt gcggagggcg ttactgtttt gtctcagtaa caataaatac aaaaagactg 60
gttgtgttcc ggccccatcc aaccacgaag ttgatttctc ttgtgtgcag agtgactgat 120
tttaaaggac atggagcttg tcacaatgtc acaatgtcac agtgtgaagg gcacactcac 180
tcccgcgtga ttcacattta gcaaccaaca atagctcatg agtccatact tgtaaatact 240
tttggcagaa tacttnttga aacttgcaga tgataactaa gatccaagat atttcccaaa 300

gtaaatagaa gtgggtcata atattaatta cctgttcaca tcagcttcca tttacaagtc 360
atgagcccag acactgacat caaactaagc ccacttagac tcctcaccac cagtctgtcc 420
tgtcatcaga caggaggctg tcaccttgac caaattctca ccagtcaatc atctatccaa 480
aaaccattac ctgatccact tccggtaatg caccaccttg gtga 524

<210> 209
<211> 159
<212> DNA
<213> Homo sapien

<400> 209
gggtgaggaa atccagagtt gccatggaga aaattccagt gtcagcattc ttgctccttg 60
tggccctctc ctacactctg gccagagata ccacagtcaa acctggagcc aaaaaggaca 120
caaaggactc tcgacccaaa ctgccccaga ccctctcca 159

<210> 210
<211> 256
<212> DNA
<213> Homo sapien

<220>
<221> misc_feature
<222> (1)...(256)
<223> n = A,T,C or G

<400> 210
actccctggc agacaaaggc agaggagaga gctctgttag ttctgtgttg ttgaactgcc 60
actgaatttc tttccacttg gactattaca tgccanttga gggactaatg gaaaaacgta 120
tggggagatt ttanccaatt tangtntgta aatggggaga ctggggcagg cgggagagat 180
ttgcagggtg naaatgggan ggctggtttg ttanatgaac agggacatag gaggtaggca 240
ccaggatgct aaatca 256

<210> 211
<211> 264
<212> DNA
<213> Homo sapien

<220>
<221> misc_feature
<222> (1)...(264)
<223> n = A,T,C or G

<400> 211
acattgtttt tttgagataa agcattgaga gagctctcct taacgtgaca caatggaagg 60
actggaacac atacccacat ctttgttctg agggataatt ttctgataaa gtcttgctgt 120
atattcaagc acatatgtta tatattattc agttccatgt ttatagccta gttaaggaga 180
ggggagatac attcngaaag aggactgaaa gaaatactca agtnggaaaa cagaaaaaga 240
aaaaaaggag caaatgagaa gcct 264

<210> 212
<211> 328
<212> DNA
<213> Homo sapien

<220>
<221> misc_feature
<222> (1)...(328)
<223> n = A,T,C or G

<400> 212
acccaaaaat ccaatgctga atatttggct tcattattcc canattcttt gattgtcaaa 60
ggatttaatg ttgtctcagc ttgggcactt cagttaggac ctaaggatgc cagccggcag 120

gtttatatat gcagcaacaa tattcaagcg cgacaacagg ttattgaact tgcccgccag 180
ttnaatttca ttcccattga cttgggatcc ttatcatcag ccagagagat tgaaaattta 240
cccctacnac tctttactct ctgganaggg ccagtggtgg tagctataag cttggccaca 300
tttttttttc ctttattcct ttgtcaga 328

<210> 213
<211> 250
<212> DNA
<213> Homo sapien

<220>
<221> misc_feature
<222> (1)...(250)
<223> n = A,T,C or G

<400> 213
acttatgagc agagcgacat atccnagtgt agactgaata aaactgaatt ctctccagtt 60
taaagcattg ctcactgaag ggatagaagt gactgccagg agggaaagta agccaaggct 120
cattatgcca aagganatat acatttcaat tctccaaact tcttcctcat tccaagagtt 180
ttcaatattt gcatgaacct gctgataanc catgttaana aacaaatatc tctctnacct 240
tctcatcggt 250

<210> 214
<211> 444
<212> DNA
<213> Homo sapien

<220>
<221> misc_feature
<222> (1)...(444)
<223> n = A,T,C or G

<400> 214
acccagaatc caatgctgaa tatttggctt cattattccc agattctttg attgtcaaag 60
gatttaatgt tgtctcagct tgggcacttc agttaggacc taaggatgcc agccggcagg 120
tttatatatg cagcaacaat attcaagcgc gacaacaggt tattgaactt gcccgccagt 180
tgaatttcat tcccattgac ttgggatcct tatcatcagc canagagatt gaaaatttac 240
ccctacgact ctttactctc tggagagggc cagtggtggt agctataagc ttggccacat 300
ttttttttcc tttattcctt tgtcagagat gcgattcatc catatgctan aaaccaacag 360
agtgactttt acaaaattcc tataganatt gtgaataaaa ccttacctat agttgccatt 420
actttgctct ccctaatata cctc 444

<210> 215
<211> 366
<212> DNA
<213> Homo sapien

<220>
<221> misc_feature
<222> (1)...(366)
<223> n = A,T,C or G

<400> 215
acttatgagc agagcgacat atccaagtgt anactgaata aaactgaatt ctctccagtt 60
taaagcattg ctcactgaag ggatagaagt gactgccagg agggaaagta agccaaggct 120
cattatgcca aagganatat acatttcaat tctccaaact tcttcctcat tccaagagtt 180
ttcaatattt gcatgaacct gctgataagc catgttgaga aacaaatatc tctctgacct 240
tctcatcggt aagcagaggc tgtaggcaac atggaccata gcgaanaaaa aacttagtaa 300
tccaagctgt tttctacact gtaaccaggt ttccaaccaa ggtggaaatc tcctatactt 360
ggtgcc 366

<210> 216

<211> 260
<212> DNA
<213> Homo sapien

<220>
<221> misc_feature
<222> (1)...(260)
<223> n = A,T,C or G

<400> 216
ctgtataaac agaactccac tgcangaggg agggccgggc caggagaatc tccgcttgtc 60
caagacaggg gcctaaggag ggtctccaca ctgctnntaa gggctnttnc atttttttat 120
taataaaaag tnnaaaaggc ctcttctcaa cttttttccc ttnggctgga aaatttaaaa 180
atcaaaaatt tcctnaagtt ntcaagctat catatatact ntatcctgaa aaagcaacat 240
aattcttcct tccctccttt 260

<210> 217
<211> 262
<212> DNA
<213> Homo sapien

<220>
<221> misc_feature
<222> (1)...(262)
<223> n = A,T,C or G

<400> 217
acctacgtgg gtaagtttan aaatgttata atttcaggaa naggaacgca tataattgta 60
tcttgcctat aattttctat tttaataagg aaatagcaaa ttggggtggg gggaatgtag 120
ggcattctac agtttgagca aaatgcaatt aaatgtggaa ggacagcact gaaaaatttt 180
atgaataatc tgtatgatta tatgtctcta gagtagattt ataattagcc acttacccta 240
atatccttca tgcttgtaaa gt 262

<210> 218
<211> 205
<212> DNA
<213> Homo sapien

<220>
<221> misc_feature
<222> (1)...(205)
<223> n = A,T,C or G

<400> 218
accaaggtgg tgcattaccg gaantggatc aangacacca tcgtggccaa cccctgagca 60
cccctatcaa ctcccttttg tagtaaactt ggaaccttgg aaatgaccag gccaagactc 120
aggcctcccc agttctactg acctttgtcc ttangtntna ngtccagggt tgctaggaaa 180
anaaatcagc agacacaggt gtaaa 205

<210> 219
<211> 114
<212> DNA
<213> Homo sapien

<400> 219
tactgttttg tctcagtaac aataaataca aaaagactgg ttgtgttccg gccccatcca 60
accacgaagt tgatttctct tgtgtgcaga gtgactgatt ttaaaggaca tgga 114

<210> 220
<211> 93
<212> DNA
<213> Homo sapien

<400> 220
actagccagc acaaaaggca gggtagcctg aattgctttc tgctctttac atttctttta 60
aaataagcat ttagtgctca gtccctactg agt 93

<210> 221
<211> 167
<212> DNA
<213> Homo sapien

<220>
<221> misc_feature
<222> (1)...(167)
<223> n = A,T,C or G

<400> 221
actangtgca ggtgcgcaca aatatttgtc gatattccct tcatcttgga ttccatgagg 60
tcttttgccc agcctgtggc tctactgtag taagtttctg ctgatgagga gccagnatgc 120
cccccactac cttccctgac gctccccana aatcacccaa cctctgt 167

<210> 222
<211> 351
<212> DNA
<213> Homo sapien

<400> 222
agggcgtggt gcggagggcg gtactgacct cattagtagg aggatgcatt ctggcacccc 60
gttcttcacc tgtcccccaa tccttaaaag gccatactgc ataaagtcaa caacagataa 120
atgtttgctg aattaaagga tggatgaaaa aaattaataa tgaatttttg cataatccaa 180
ttttctcttt tatatttcta gaagaagttt ctttgagcct attagatccc gggaatcttt 240
taggtgagca tgattagaga gcttgtaggt tgcttttaca tatatctggc atatttgagt 300
ctcgtatcaa aacaatagat tggtaaaggt ggtattattg tattgataag t 351

<210> 223
<211> 383
<212> DNA
<213> Homo sapien

<220>
<221> misc_feature
<222> (1)...(383)
<223> n = A,T,C or G

<400> 223
aaaacaaaca aacaaaaaaa acaattcttc attcagaaaa attatcttag ggactgatat 60
tggtaattat ggtcaattta atwrtrttkt ggggcatttc cttacattgt cttgacaaga 120
ttaaaatgtc tgtgccaaaa ttttgtattt tatttggaga cttcttatca aaagtaatgc 180
tgccaaagga agtctaagga attagtagtg ttcccmtcac ttgtttggag tgtgctattc 240
taaaagattt tgatttcctg gaatgacaat tatattttaa ctttggtggg ggaaanagtt 300
ataggaccac agtcttcact tctgatactt gtaaattaat cttttattgc acttgttttg 360
accattaagc tatatgttta aaa 383

<210> 224
<211> 320
<212> DNA
<213> Homo sapien

<400> 224
cccctgaagg cttcttgtta gaaaatagta cagttacaac caataggaac aacaaaaaga 60
aaaagtttgt gacattgtag tagggagtgt gtacccctta ctccccatca aaaaaaaaat 120
ggatacatgg ttaaaggata raagggcaat attttatcat atgttctaaa agagaaggaa 180
gagaaaatac tactttctcr aaatggaagc ccttaaaggt gctttgatac tgaaggacac 240

aaatgtggcc gtccatcctc ctttaragtt gcatgacttg gacacggtaa ctgttgcagt 300
tttaractcm gcattgtgac 320

<210> 225
<211> 1214
<212> DNA
<213> Homo sapien

<400> 225
gaggactgca gcccgcactc gcagccctgg caggcggcac tggtcatgga aaacgaattg 60
ttctgctcgg gcgtcctggt gcatccgcag tgggtgctgt cagccgcaca ctgtttccag 120
aactcctaca ccatcgggct gggcctgcac agtcttgagg ccgaccaaga gccagggagc 180
cagatggtgg aggccagcct ctccgtacgg cacccagagt acaacagacc cttgctcgct 240
aacgacctca tgctcatcaa gttggacgaa tccgtgtccg agtctgacac catccggagc 300
atcagcattg cttcgcagtg ccctaccgcg gggaactctt gcctcgtttc tggctggggt 360
ctgctggcga acggcagaat gcctaccgtg ctgcagtgcg tgaacgtgtc ggtggtgtct 420
gaggaggtct gcagtaagct ctatgacccg ctgtaccacc ccagcatgtt ctgcgccggc 480
ggagggcaag accagaagga ctcctgcaac ggtgactctg gggggcccct gatctgcaac 540
gggtacttgc agggccttgt gtctttcgga aaagccccgt gtggccaagt tggcgtgcca 600
ggtgtctaca ccaacctctg caaattcact gagtggatag agaaaaccgt ccaggccagt 660
taactctggg gactgggaac ccatgaaatt gacccccaaa tacatcctgc ggaaggaatt 720
caggaatatc tgttcccagc ccctcctccc tcaggcccag gagtccaggc ccccagcccc 780
tcctccctca aaccaagggt acagatcccc agcccctcct ccctcagacc caggagtcca 840
gaccccccag cccctcctcc ctcagaccca ggagtccagc ccctcctccc tcagacccag 900
gagtccagac cccccagccc ctcctccctc agacccaggg gtccaggccc ccaacccctc 960
ctccctcaga ctcagaggtc caagccccca acccctcctt ccccagaccc agaggtccag 1020
gtcccagccc ctcctccctc agacccagcg gtccaatgcc acctagactc tccctgtaca 1080
cagtgccccc ttgtggcacg ttgacccaac cttaccagtt ggtttttcat tttttgtccc 1140
tttcccctag atccagaaat aaagtctaag agaagcgcaa aaaaaaaaaa aaaaaaaaaa 1200
aaaaaaaaaa aaaa 1214

<210> 226
<211> 119
<212> DNA
<213> Homo sapien

<400> 226
acccagtatg tgcagggaga cggaacccca tgtgacagcc cactccacca gggttcccaa 60
agaacctggc ccagtcataa tcattcatcc tgacagtggc aataatcacg ataaccagt 119

<210> 227
<211> 818
<212> DNA
<213> Homo sapien

<400> 227
acaattcata gggacgacca atgaggacag ggaatgaacc cggctctccc ccagccctga 60
tttttgctac atatggggtc ccttttcatt ctttgcaaaa acactgggtt ttctgagaac 120
acggacggtt cttagcacaa tttgtgaaat ctgtgtaraa ccgggctttg caggggagat 180
aattttcctc ctctggagga aaggtggtga ttgacaggca gggagacagt gacaaggcta 240
gagaaagcca cgctcggcct tctctgaacc aggatggaac ggcagacccc tgaaaacgaa 300
gcttgtcccc ttccaatcag ccacttctga gaacccccat ctaacttcct actggaaaag 360
agggcctcct caggagcagt ccaagagttt tcaaagataa cgtgacaact accatctaga 420
ggaaagggtg caccctcagc agagaagccg agagcttaac tctggtcgtt tccagagaca 480
acctgctggc tgtcttggga tgcgcccagc ctttgagagg ccactacccc atgaacttct 540
gccatccact ggacatgaag ctgaggacac tgggcttcaa cactgagttg tcatgagagg 600
gacaggctct gccctcaagc cggctgaggg cagcaaccac tctcctcccc tttctcacgc 660
aaagccattc ccacaaatcc agaccatacc atgaagcaac gagacccaaa cagtttggct 720
caagaggata tgaggactgt ctcagcctgg ctttgggctg acaccatgca cacacacaag 780
gtccacttct aggttttcag cctagatggg agtcgtgt 818

<210> 228

<211> 744
<212> DNA
<213> Homo sapien

<400> 228
actggagaca ctgttgaact tgatcaagac ccagaccacc ccaggtctcc ttcgtgggat 60
gtcatgacgt ttgacatacc tttggaacga gcctcctcct tggaagatgg aagaccgtgt 120
tcgtggccga cctggcctct cctggcctgt ttcttaagat gcggagtcac atttcaatgg 180
taggaaaagt ggcttcgtaa aatagaagag cagtcactgt ggaactacca aatggcgaga 240
tgctcggtgc acattggggt gctttgggat aaaagattta tgagccaact attctctggc 300
accagattct aggccagttt gttccactga agcttttccc acagcagtcc acctctgcag 360
gctggcagct gaatggcttg ccggtggctc tgtggcaaga tcacactgag atcgatgggt 420
gagaaggcta ggatgcttgt ctagtgttct tagctgtcac gttggctcct tccaggttgg 480
ccagacggtg ttggccactc ccttctaaaa cacaggcgcc ctcctggtga cagtgacccg 540
ccgtggtatg ccttggccca ttccagcagt cccagttatg catttcaagt ttggggtttg 600
ttcttttcgt taatgttcct ctgtgttgtc agctgtcttc atttcctggg ctaagcagca 660
ttgggagatg tggaccagag atccactcct taagaaccag tggcgaaaga cactttcttt 720
cttcactctg aagtagctgg tggt 744

<210> 229
<211> 300
<212> DNA
<213> Homo sapien

<400> 229
cgagtctggg ttttgtctat aaagtttgat ccctcctttt ctcatccaaa tcatgtgaac 60
cattacacat cgaaataaaa gaaaggtggc agacttgccc aacgccaggc tgacatgtgc 120
tgcagggttg ttgtttttta attattattg ttagaaacgt cacccacagt ccctgttaat 180
ttgtatgtga cagccaactc tgagaaggtc ctatttttcc acctgcagag gatccagtct 240
cactaggctc ctccttgccc tcacactgga gtctccgcca gtgtgggtgc ccactgacat 300

<210> 230
<211> 301
<212> DNA
<213> Homo sapien

<400> 230
cagcagaaca aatacaaata tgaagagtgc aaagatctca taaaatctat gctgaggaat 60
gagcgacagt tcaaggagga gaagcttgca gagcagctca agcaagctga ggagctcagg 120
caatataaag tcctggttca cactcaggaa cgagagctga cccagttaag ggagaagttg 180
cgggaaggga gagatgcctc cctctcattg aatgagcatc tccaggccct cctcactccg 240
gatgaaccgg acaagtccca ggggcaggac ctccaagaaa cagacctcgg ccgcgaccac 300
g 301

<210> 231
<211> 301
<212> DNA
<213> Homo sapien

<400> 231
gcaagcacgc tggcaaatct ctgtcaggtc agctccagag aagccattag tcattttagc 60
caggaactcc aagtccacat ccttggcaac tggggacttg cgcaggttag ccttgaggat 120
ggcaacacgg gacttctcat caggaagtgg gatgtagatg agctgatcaa gacggccagg 180
tctgaggatg gcaggatcaa tgatgtcagg ccggttggta ccgccaatga tgaacacatt 240
tttttttgtg gacatgccat ccatttctgt caggatctgg ttgatgactc ggtcagcagc 300
c 301

<210> 232
<211> 301
<212> DNA
<213> Homo sapien

<400> 232
agtaggtatt tcgtgagaag ttcaacacca aaactggaac atagttctcc ttcaagtgtt 60
ggcgacagcg gggcttcctg attctggaat ataactttgt gtaaattaac agccacctat 120
agaagagtcc atctgctgtg aaggagagac agagaactct gggttccgtc gtcctgtcca 180
cgtgctgtac caagtgctgg tgccagcctg ttacctgttc tcactgaaaa tctggctaat 240
gctcttgtgt atcacttctg attctgacaa tcaatcaatc aatggcctag agcactgact 300
g 301

<210> 233
<211> 301
<212> DNA
<213> Homo sapien

<400> 233
   atgactgact tcccagtaag gctctctaag ggtaagtagg gaggatccac aggatttgag 60
   atgctaaggc cccagagatc gtttgatcca accctcttat tttcagaggg gaaaatgggg 120
   cctagaagtt acagagcatc tagctggtgc gctggcaccc ctggcctcac acagactccc 180
   gagtagctgg gactacaggc acacagtcac tgaagcaggc cctgttagca attctatgcg 240
   tacaaattaa catgagatga gtagagactt tattgagaaa gcaagagaaa atcctatcaa 300
   c 301

<210> 234
<211> 301
<212> DNA
<213> Homo sapien

<400> 234
aggtcctaca catcgagact catccatgat tgatatgaat ttaaaaatta caagcaaaga 60
cattttattc atcatgatgc tttcttttgt ttcttctttt cgttttcttc tttttctttt 120
tcaatttcag caacatactt ctcaatttct tcaggattta aaatcttgag ggattgatct 180
cgcctcatga cagcaagttc aatgtttttg ccacctgact gaaccacttc caggagtgcc 240
ttgatcacca gcttaatggt cagatcatct gcttcaatgg cttcgtcagt atagttcttc 300
t 301

<210> 235
<211> 283
<212> DNA
<213> Homo sapien

<400> 235
tggggctgtg catcaggcgg gtttgagaaa tattcaattc tcagcagaag ccagaatttg 60
aattccctca tcttttaggg aatcatttac caggtttgga gaggattcag acagctcagg 120
tgctttcact aatgtctctg aacttctgtc cctctttgtt catggatagt ccaataaata 180
atgttatctt tgaactgatg ctcataggag agaatataag aactctgagt gatatcaaca 240
ttagggattc aaagaaatat tagatttaag ctcacactgg tca 283

<210> 236
<211> 301
<212> DNA
<213> Homo sapien

<400> 236
aggtcctcca ccaactgcct gaagcacggt taaaattggg aagaagtata gtgcagcata 60
aatactttta aatcgatcag atttccctaa cccacatgca atcttcttca ccagaagagg 120
tcggagcagc atcattaata ccaagcagaa tgcgtaatag ataaatacaa tggtatatag 180
tgggtagacg gcttcatgag tacagtgtac tgtggtatcg taatctggac ttgggttgta 240
aagcatcgtg taccagtcag aaagcatcaa tactcgacat gaacgaatat aaagaacacc 300
a 301

<210> 237
<211> 301
<212> DNA

<213> Homo sapien

<400> 237
cagtggtagt ggtggtggac gtggcgttgg tcgtggtgcc ttttttggtg cccgtcacaa 60
actcaatttt tgttcgctcc tttttggcct tttccaattt gtccatctca attttctggg 120
ccttggctaa tgcctcatag taggagtcct cagaccagcc atggggatca aacatatcct 180
ttgggtagtt ggtgccaagc tcgtcaatgg cacagaatgg atcagcttct cgtaaatcta 240
gggttccgaa attctttctt cctttggata atgtagttca tatccattcc ctcctttatc 300
t 301

<210> 238
<211> 301
<212> DNA
<213> Homo sapien

<400> 238
gggcaggttt tttttttttt ttttttgatg gtgcagaccc ttgctttatt tgtctgactt 60
gttcacagtt cagccccctg ctcagaaaac caacgggcca gctaaggaga ggaggaggca 120
ccttgagact tccggagtcg aggctctcca gggttcccca gcccatcaat cattttctgc 180
accccctgcc tgggaagcag ctccctgggg ggtgggaatg ggtgactaga agggatttca 240
gtgtgggacc cagggtctgt tcttcacagt aggaggtgga agggatgact aatttcttta 300
t 301

<210> 239
<211> 239
<212> DNA
<213> Homo sapien

<400> 239
ataagcagct agggaattct ttatttagta atgtcctaac ataaaagttc acataactgc 60
ttctgtcaaa ccatgatact gagctttgtg acaacccaga aataactaag agaaggcaaa 120
cataatacct tagagatcaa gaaacattta cacagttcaa ctgtttaaaa atagctcaac 180
attcagccag tgagtagagt gtgaatgcca gcatacacag tatacaggtc cttcaggga 239

<210> 240
<211> 300
<212> DNA
<213> Homo sapien

<400> 240
ggtcctaatg aagcagcagc ttccacattt taacgcaggt ttacggtgat actgtccttt 60
gggatctgcc ctccagtgga accttttaag gaagaagtgg gcccaagcta agttccacat 120
gctgggtgag ccagatgact tctgttccct ggtcactttc ttcaatgggg cgaatggggg 180
ctgccaggtt tttaaaatca tgcttcatct tgaagcacac ggtcacttca ccctcctcac 240
gctgtgggtg tactttgatg aaaataccca ctttgttggc ctttctgaag ctataatgtc 300

<210> 241
<211> 301
<212> DNA
<213> Homo sapien

<400> 241
gaggtctggt gctgaggtct ctgggctagg aagaggagtt ctgtggagct ggaagccaga 60
cctctttgga ggaaactcca gcagctatgt tggtgtctct gagggaatgc aacaaggctg 120
ctcctccatg tattggaaaa ctgcaaactg gactcaactg gaaggaagtg ctgctgccag 180
tgtgaagaac cagcctgagg tgacagaaac ggaagcaaac aggaacagcc agtcttttct 240
tcctcctcct gtcatacggt ctctctcaag catcctttgt tgtcaggggc ctaaagggga 300
g 301

<210> 242
<211> 301
<212> DNA

<213> Homo sapien

<400> 242
ccgaggtcct gggatgcaac caatcactct gtttcacgtg acttttatca ccatacaatt 60
tgtggcattt cctcattttc tacattgtag aatcaagagt gtaaataaat gtatatcgat 120
gtcttcaaga atatatcatt cctttttcac tagaacccat tcaaaatata agtcaagaat 180
cttaatatca acaaatatat caagcaaact ggaaggcaga ataactacca taatttagta 240
taagtaccca aagttttata aatcaaaagc cctaatgata accattttta gaattcaatc 300
a 301

<210> 243
<211> 301
<212> DNA
<213> Homo sapien

<400> 243
aggtaagtcc cagtttgaag ctcaaaagat ctggtatgag cataggctca tcgacgacat 60
ggtggcccaa gctatgaaat cagagggagg cttcatctgg gcctgtaaaa actatgatgg 120
tgacgtgcag tcggactctg tggcccaagg gtatggctct ctcggcatga tgaccagcgt 180
gctggtttgt ccagatggca agacagtaga agcagaggct gcccacggga ctgtaacccg 240
tcactaccgc atgttccaga aaggacagga gacgtccacc aatcccattg cttccatttt 300
t 301

<210> 244
<211> 300
<212> DNA
<213> Homo sapien

<400> 244
gctggtttgc aagaatgaaa tgaatgattc tacagctagg acttaacctt gaaatggaaa 60
gtcatgcaat cccatttgca ggatctgtct gtgcacatgc ctctgtagag agcagcattc 120
ccaggacctt tggaaacagt tgacactgta aggtgcttgc tccccaagac acatcctaaa 180
aggtgttgta atggtgaaaa cgtcttcctt ctttattgcc ccttcttatt tatgtgaaca 240
actgtttgtc ttttgtgtat cttttttaaa ctgtaaagtt caattgtgaa aatgaatatc 300

<210> 245
<211> 301
<212> DNA
<213> Homo sapien

<400> 245
gtctgagtat ttaaaatgtt attgaaatta tccccaacca atgttagaaa agaaagaggt 60
tatatactta gataaaaaat gaggtgaatt actatccatt gaaatcatgc tcttagaatt 120
aaggccagga gatattgtca ttaatgtara cttcaggaca ctagagtata gcagccctat 180
gttttcaaag agcagagatg caattaaata ttgtttagca tcaaaaaggc cactcaatac 240
agctaataaa atgaaagacc taatttctaa agcaattctt tataatttac aaagttttaa 300
g 301

<210> 246
<211> 301
<212> DNA
<213> Homo sapien

<400> 246
ggtctgtcct acaatgcctg cttcttgaaa gaagtcggca ctttctagaa tagctaaata 60
acctgggctt attttaaaga actatttgta gctcagattg gttttcctat ggctaaaata 120
agtgcttctt gtgaaaatta aataaaacag ttaattcaaa gccttgatat atgttaccac 180
taacaatcat actaaatata ttttgaagta caaagtttga catgctctaa agtgacaacc 240
caaatgtgtc ttacaaaaca cgttcctaac aaggtatgct ttacactacc aatgcagaaa 300
c 301

<210> 247

<211> 301
<212> DNA
<213> Homo sapien

<400> 247
aggtcctttg gcagggctca tggatcagag ctcaaactgg agggaaaggc atttcgggta 60
gcctaagagg gcgactggcg gcagcacaac caaggaaggc aaggttgttt cccccacgct 120
gtgtcctgtg ttcaggtgcg acacacaatc ctcatgggaa caggatcacc catgcgctgc 180
ccttgatgat caaggttggg gcttaagtgg attaagggag gcaagttctg ggttccttgc 240
cttttcaaac catgaagtca ggctctgtat ccctcctttt cctaactgat attctaacta 300
a 301

<210> 248
<211> 301
<212> DNA
<213> Homo sapien

<400> 248
aggtccttgg agatgccatt tcagccgaag gactcttctw ttcggaagta caccctcact 60
attaggaaga ttcttagggg taatttttct gaggaaggag aactagccaa cttaagaatt 120
acaggaagaa agtggtttgg aagacagcca aagaaataaa agcagattaa attgtatcag 180
gtacattcca gcctgttggc aactccataa aaacatttca gattttaatc ccgaatttag 240
ctaatgagac tggatttttg ttttttatgt tgtgtgtcgc agagctaaaa actcagttcc 300
c 301

<210> 249
<211> 301
<212> DNA
<213> Homo sapien

<400> 249
gtccagagga agcacctggt gctgaactag gcttgccctg ctgtgaactt gcacttggag 60
ccctgacgct gctgttctcc ccgaaaaacc cgaccgacct ccgcgatctc cgtcccgccc 120
ccagggagac acagcagtga ctcagagctg gtcgcacact gtgcctccct cctcaccgcc 180
catcgtaatg aattattttg aaaattaatt ccaccatcct ttcagattct ggatggaaag 240
actgaatctt tgactcagaa ttgtttgctg aaaagaatga tgtgactttc ttagtcattt 300
a 301

<210> 250
<211> 301
<212> DNA
<213> Homo sapien

<400> 250
ggtctgtgac aaggacttgc aggctgtggg aggcaagtga cccttaacac tacacttctc 60
cttatcttta ttggcttgat aaacataatt atttctaaca ctagcttatt tccagttgcc 120
cataagcaca tcagtacttt tctctggctg gaatagtaaa ctaaagtatg gtacatctac 180
ctaaaagact actatgtgga ataatacata ctaatgaagt attacatgat ttaaagacta 240
caataaaacc aaacatgctt ataacattaa gaaaaacaat aaagatacat gattgaaacc 300
a 301

<210> 251
<211> 301
<212> DNA
<213> Homo sapien

<400> 251
gccgaggtcc tacatttggc ccagtttccc cctgcatcct ctccagggcc cctgcctcat 60
agacaacctc atagagcata ggagaactgg ttgccctggg ggcaggggga ctgtctggat 120
ggcaggggtc ctcaaaaatg ccactgtcac tgccaggaaa tgcttctgag cagtacacct 180
cattgggatc aatgaaaagc ttcaagaaat cttcaggctc actctcttga aggcccggaa 240
cctctggagg ggggcagtgg aatcccagct ccaggacgga tcctgtcgaa aagatatcct 300

c                                                                 301

<210> 252
<211> 301
<212> DNA
<213> Homo sapien

<400> 252
gcaaccaatc actctgtttc acgtgacttt tatcaccata caatttgtgg catttcctca      60
ttttctacat tgtagaatca agagtgtaaa taaatgtata tcgatgtctt caagaatata     120
tcattccttt ttcactagga acccattcaa aatataagtc aagaatctta atatcaacaa     180
atatatcaag caaactggaa ggcagaataa ctaccataat ttagtataag tacccaaagt     240
tttataaatc aaaagcccta atgataacca tttttagaat tcaatcatca ctgtagaatc     300
a                                                                 301

<210> 253
<211> 301
<212> DNA
<213> Homo sapien

<400> 253
ttccctaaga agatgttatt ttgttgggtt ttgttccccc tccatctcga ttctcgtacc      60
caactaaaaa aaaaaaataa agaaaaaatg tgctgcgttc tgaaaaataa ctccttagct     120
tggtctgatt gttttcagac cttaaaatat aaacttgttt cacaagcttt aatccatgtg     180
gatttttttt cttagagaac cacaaaacat aaaaggagca agtcggactg aatacctgtt     240
tccatagtgc ccacagggta ttcctcacat tttctccata ggaaaatgct ttttcccaag     300
g                                                                 301

<210> 254
<211> 301
<212> DNA
<213> Homo sapien

<400> 254
cgctgcgcct ttcccttggg ggaggggcaa ggccagaggg ggtccaagtg cagcacgagg      60
aacttgacca attcccttga agcgggtggg ttaaaccctg taaatgggaa caaaatcccc     120
ccaaatctct tcatcttacc ctggtggact cctgactgta gaattttttg gttgaaacaa     180
gaaaaaaata aagctttgga cttttcaagg ttgcttaaca ggtactgaaa gactggcctc     240
acttaaactg agccaggaaa agctgcagat ttattaatgg gtgtgttagt gtgcagtgcc     300
t                                                                 301

<210> 255
<211> 302
<212> DNA
<213> Homo sapien

<400> 255
agcttttttt tttttttttt tttttttttt ttcattaaaa aatagtgctc tttattataa      60
attactgaaa tgtttctttt ctgaatataa atataaatat gtgcaaagtt tgacttggat     120
tgggattttg ttgagttctt caagcatctc ctaataccct caagggcctg agtagggggg     180
aggaaaaagg actggaggtg gaatctttat aaaaaacaag agtgattgag gcagattgta     240
aacattatta aaaaacaaga aacaaacaaa aaaatagaga aaaaaaccac cccaacacac     300
aa                                                                302

<210> 256
<211> 301
<212> DNA
<213> Homo sapien

<220>
<221> misc_feature
<222> (1)...(301)

<223> n = A,T,C or G

<400> 256
gttccagaaa acattgaagg tggcttccca aagtctaact agggataccc cctctagcct 60
aggaccctcc tccccacacc tcaatccacc aaaccatcca taatgcaccc agataggccc 120
acccccaaaa gcctggacac cttgagcaca cagttatgac caggacagac tcatctctat 180
aggcaaatag ctgctggcaa actggcatta cctggtttgt ggggatgggg gggcaagtgt 240
gtggcctctc ggcctggtta gcaagaacat tcagggtagg cctaagttan tcgtgttagt 300
t 301

<210> 257
<211> 301
<212> DNA
<213> Homo sapien

<400> 257
gttgtggagg aactctggct tgctcattaa gtcctactga ttttcactat cccctgaatt 60
tccccactta tttttgtctt tcactatcgc aggccttaga agaggtctac ctgcctccag 120
tcttacctag tccagtctac cccctggagt tagaatggcc atcctgaagt gaaaagtaat 180
gtcacattac tcccttcagt gatttcttgt agaagtgcca atccctgaat gccaccaaga 240
tcttaatctt cacatcttta atcttatctc tttgactcct ctttacaccg gagaaggctc 300
c 301

<210> 258
<211> 301
<212> DNA
<213> Homo sapien

<220>
<221> misc_feature
<222> (1)...(301)
<223> n = A,T,C or G

<400> 258
cagcagtagt agatgccgta tgccagcacg cccagcactc ccaggatcag caccagcacc 60
aggggcccag ccaccaggcg cagaagcaag ataaacagta ggctcaagac cagagccacc 120
cccagggcaa caagaatcca ataccaggac tgggcaaaat cttcaaagat cttaacactg 180
atgtctcggg cattgaggct gtcaataana cgctgatccc ctgctgtatg gtggtgtcat 240
tggtgatccc tgggagcgcc ggtggagtaa cgttggtcca tggaaagcag cgcccacaac 300
t 301

<210> 259
<211> 301
<212> DNA
<213> Homo sapien

<220>
<221> misc_feature
<222> (1)...(301)
<223> n = A,T,C or G

<400> 259
tcatatatgc aaacaaatgc agactangcc tcaggcagag actaaaggac atctcttggg 60
gtgtcctgaa gtgatttgga cccctgaggg cagacaccta agtaggaatc ccagtgggaa 120
gcaaagccat aaggaagccc aggattcctt gtgatcagga agtgggccag gaaggtctgt 180
tccagctcac atctcatctg catgcagcac ggaccggatg cgcccactgg gtcttggctt 240
ccctcccatc ttctcaagca gtgtccttgt tgagccattt gcatccttgg ctccaggtgg 300
c 301

<210> 260
<211> 301
<212> DNA

<213> Homo sapien

<400> 260
ttttttttct ccctaaggaa aaagaaggaa caagtctcat aaaaccaaat aagcaatggt 60
aaggtgtctt aacttgaaaa agattaggag tcactggttt acaagttata attgaatgaa 120
agaactgtaa cagccacagt tggccatttc atgccaatgg cagcaaacaa caggattaac 180
tagggcaaaa taaataagtg tgtggaagcc ctgataagtg cttaataaac agactgattc 240
actgagacat cagtacctgc ccgggcggcc gctcgagccg aattctgcag atatccatca 300
c 301

<210> 261
<211> 301
<212> DNA
<213> Homo sapien

<400> 261
aaatattcga gcaaatcctg taactaatgt gtctccataa aaggctttga actcagtgaa 60
tctgcttcca tccacgattc tagcaatgac ctctcggaca tcaaagctcc tcttaaggtt 120
agcaccaact attccataca attcatcagc aggaaataaa ggctcttcag aaggttcaat 180
ggtgacatcc aatttcttct gataatttag attcctcaca accttcctag ttaagtgaag 240
ggcatgatga tcatccaaag cccagtggtc acttactcca gactttctgc aatgaagatc 300
a 301

<210> 262
<211> 301
<212> DNA
<213> Homo sapien

<400> 262
gaggagagcc tgttacagca tttgtaagca cagaatactc caggagtatt tgtaattgtc 60
tgtgagcttc ttgccgcaag tctctcagaa atttaaaaag atgcaaatcc ctgagtcacc 120
cctagacttc ctaaaccaga tcctctgggg ctggaacctg gcactctgca tttgtaatga 180
gggctttctg gtgcacacct aattttgtgc atctttgccc taaatcctgg attagtgccc 240
catcattacc cccacattat aatgggatag attcagagca gatactctcc agcaaagaat 300
c 301

<210> 263
<211> 301
<212> DNA
<213> Homo sapien

<220>
<221> misc_feature
<222> (1)...(301)
<223> n = A,T,C or G

<400> 263
tttagcttgt ggtaaatgac tcacaaaact gattttaaaa tcaagttaat gtgaattttg 60
aaaattacta cttaatccta attcacaata acaatggcat taaggtttga cttgagttgg 120
ttcttagtat tatttatggt aaataggctc ttaccacttg caaataactg gccacatcat 180
taatgactga cttcccagta aggctctcta aggggtaagt angaggatcc acaggatttg 240
agatgctaag gccccagaga tcgtttgatc caaccctctt attttcagag gggaaaatgg 300
g 301

<210> 264
<211> 301
<212> DNA
<213> Homo sapien

<400> 264
aaagacgtta aaccactcta ctaccacttg tggaactctc aaagggtaaa tgacaaascc 60
aatgaatgac tctaaaaaca atatttacat ttaatggttt gtagacaata aaaaaacaag 120

gtggatagat ctagaattgt aacattttaa gaaaaccata scatttgaca gatgagaaag 180
ctcaattata gatgcaaagt tataactaaa ctactatagt agtaaagaaa tacatttcac 240
acccttcata taaattcact atcttggctt gaggcactcc ataaaatgta tcacgtgcat 300
a 301

<210> 265
<211> 301
<212> DNA
<213> Homo sapien

<400> 265
tgcccaagtt atgtgtaagt gtatccgcac ccagaggtaa aactacactg tcatctttgt 60
cttcttgtga cgcagtattt cttctctggg gagaagccgg gaagtcttct cctggctcta 120
catattcttg gaagtctcta atcaactttt gttccatttg tttcatttct tcaggaggga 180
ttttcagttt gtcaacatgt tctctaacaa cacttgccca tttctgtaaa gaatccaaag 240
cagtccaagg ctttgacatg tcaacaacca gcataactag agtatccttc agagatacgg 300
c 301

<210> 266
<211> 301
<212> DNA
<213> Homo sapien

<400> 266
taccgtctgc ccttcctccc atccaggcca tctgcgaatc tacatgggtc ctcctattcg 60
acaccagatc actctttcct ctacccacag gcttgctatg agcaagagac acaacctcct 120
ctcttctgtg ttccagcttc ttttcctgtt cttcccaccc cttaagttct attcctgggg 180
atagagacac caatacccat aacctctctc ctaagcctcc ttataaccca gggtgcacag 240
cacagactcc tgacaactgg taaggccaat gaactgggag ctcacagctg gctgtgcctg 300
a 301

<210> 267
<211> 301
<212> DNA
<213> Homo sapien

<400> 267
aaagagcaca ggccagctca gcctgccctg gccatctaga ctcagcctgg ctccatgggg 60
gttctcagtg ctgagtccat ccaggaaaag ctcacctaga ccttctgagg ctgaatcttc 120
atcctcacag gcagcttctg agagcctgat attcctagcc ttgatggtct ggagtaaagc 180
ctcattctga ttcctctcct tcttttcttt caagttggct ttcctcacat ccctctgttc 240
aattcgcttc agcttgtctg ctttagccct catttccaga agcttcttct ctttggcatc 300
t 301

<210> 268
<211> 301
<212> DNA
<213> Homo sapien

<400> 268
aatgtctcac tcaactactt cccagcctac cgtggcctaa ttctgggagt tttcttctta 60
gatcttggga gagctggttc ttctaaggag aaggaggaag gacagatgta actttggatc 120
tcgaagagga agtctaatgg aagtaattag tcaacggtcc ttgtttagac tcttggaata 180
tgctgggtgg ctcagtgagc ccttttggag aaagcaagta ttattcttaa ggagtaacca 240
cttcccattg ttctactttc taccatcatc aattgtatat tatgtattct ttggagaact 300
a 301

<210> 269
<211> 301
<212> DNA
<213> Homo sapien

<400> 269
taacaatata cactagctat ctttttaact gtccatcatt agcaccaatg aagattcaat 60
aaaattacct ttattcacac atctcaaaac aattctgcaa attcttagtg aagtttaact 120
atagtcacag accttaaata ttcacattgt tttctatgtc tactgaaaat aagttcacta 180
cttttctgga tattctttac aaaatcttat taaaattcct ggtattatca cccccaatta 240
tacagtagca caaccacctt atgtagtttt tacatgatag ctctgtagaa gtttcacatc 300
t 301

<210> 270
<211> 301
<212> DNA
<213> Homo sapien

<400> 270
cattgaagag cttttgcgaa acatcagaac acaagtgctt ataaaattaa ttaagcctta 60
cacaagaata catattcctt ttatttctaa ggagttaaac atagatgtag ctgatgtgga 120
gagcttgctg gtgcagtgca tattggataa cactattcat ggccgaattg atcaagtcaa 180
ccaactcctt gaactggatc atcagaagaa gggtggtgca cgatatactg cactagataa 240
tggaccaacc aactaaattc tctcaccagg ctgtatcagt aaactggctt aacagaaaac 300
a 301

<210> 271
<211> 301
<212> DNA
<213> Homo sapien

<220>
<221> misc_feature
<222> (1)...(301)
<223> n = A,T,C or G

<400> 271
aaaaggttct cataagatta acaatttaaa taaatatttg atagaacatt ctttctcatt 60
tttatagctc atctttaggg ttgatattca gttcatgctt cccttgctgt tcttgatcca 120
gaattgcaat cacttcatca gcctgtattc gctccaattc tctataaagt gggtccaagg 180
tgaaccacag agccacagca cacctctttc ccttggtgac tgccttcacc ccatganggt 240
tctctcctcc agatganaac tgatcatgcg cccacatttt gggttttata gaagcagtca 300
c 301

<210> 272
<211> 301
<212> DNA
<213> Homo sapien

<400> 272
taaattgcta agccacagat aacaccaatc aaatggaaca aatcactgtc ttcaaatgtc 60
ttatcagaaa accaaatgag cctggaatct tcataatacc taaacatgcc gtatttagga 120
tccaataatt ccctcatgat gagcaagaaa aattctttgc gcacccctcc tgcatccaca 180
gcatcttctc caacaaatat aaccttgagt ggcttcttgt aatctatgtt ctttgttttc 240
ctaaggactt ccattgcatc tcctacaata ttttctctac gcaccactag aattaagcag 300
g 301

<210> 273
<211> 301
<212> DNA
<213> Homo sapien

<220>
<221> misc_feature
<222> (1)...(301)
<223> n = A,T,C or G

<400> 273
acatgtgtgt atgtgtatct ttgggaaaan aanaagacat cttgtttayt atttttttgg 60
agagangctg ggacatggat aatcacwtaa tttgctayta tyactttaat ctgactygaa 120
gaaccgtcta aaaataaaat ttaccatgtc dtatattcct tatagtatgc ttatttcacc 180
ttytttctgt ccagagagag tatcagtgac ananatttma gggtgaamac atgmattggt 240
gggacttnty tttacngagm accctgcccg sgcgccctcg makcngantt ccgcsananc 300
t 301

<210> 274
<211> 301
<212> DNA
<213> Homo sapien

<220>
<221> misc_feature
<222> (1)...(301)
<223> n = A,T,C or G

<400> 274
cttatatact ctttctcaga ggcaaaagag gagatgggta atgtagacaa ttctttgagg 60
aacagtaaat gattattaga gagaangaat ggaccaagga gacagaaatt aacttgtaaa 120
tgattctctt tggaatctga atgagatcaa gaggccagct ttagcttgtg gaaaagtcca 180
tctaggtatg gttgcattct cgtcttcttt tctgcagtag ataatgaggt aaccgaaggc 240
aattgtgctt cttttgataa gaagctttct tggtcatatc aggaaattcc aganaaagtc 300
c 301

<210> 275
<211> 301
<212> DNA
<213> Homo sapien

<220>
<221> misc_feature
<222> (1)...(301)
<223> n = A,T,C or G

<400> 275
tcggtgtcag cagcacgtgg cattgaacat tgcaatgtgg agcccaaacc acagaaaatg 60
gggtgaaatt ggccaacttt ctattaactt atgttggcaa ttttgccacc aacagtaagc 120
tggcccttct aataaaagaa aattgaaagg tttctcacta aacggaatta agtagtggag 180
tcaagagact cccaggcctc agcgtacctg cccgggcggc cgctcgaagc cgaattctgc 240
agatatccat cacactggcg gncgctcgan catgcatcta gaaggnccaa ttcgccctat 300
a 301

<210> 276
<211> 301
<212> DNA
<213> Homo sapien

<400> 276
tgtacacata ctcaataaat aaatgactgc attgtggtat tattactata ctgattatat 60
ttatcatgtg acttctaatt agaaaatgta tccaaaagca aaacagcaga tatacaaaat 120
taaagagaca gaagatagac attaacagat aaggcaactt atacattgag aatccaaatc 180
caatacattt aaacatttgg gaaatgaggg ggacaaatgg aagccagatc aaatttgtgt 240
aaaactattc agtatgtttc ccttgcttca tgtctgagaa ggctctcctt caatggggat 300
g 301

<210> 277
<211> 301
<212> DNA
<213> Homo sapien

<220>
<221> misc_feature
<222> (1)...(301)
<223> n = A,T,C or G

<400> 277
tttgttgatg tcagtatttt attacttgcg ttatgagtgc tcacctggga aattctaaag 60
atacagagga cttggaggaa gcagagcaac tgaatttaat ttaaaagaag gaaaacattg 120
gaatcatggc actcctgata ctttcccaaa tcaacactct caatgcccca ccctcgtcct 180
caccatagtg gggagactaa agtggccacg gatttgcctt angtgtgcag tgcgttctga 240
gttcnctgtc gattacatct gaccagtctc ctttttccga agtccntccg ttcaatcttg 300
c 301

<210> 278
<211> 301
<212> DNA
<213> Homo sapien

<220>
<221> misc_feature
<222> (1)...(301)
<223> n = A,T,C or G

<400> 278
taccactaca ctccagcctg ggcaacagag caagacctgt ctcaaagcat aaaatggaat 60
aacatatcaa atgaaacagg gaaaatgaag ctgacaattt atggaagcca gggcttgtca 120
cagtctctac tgttattatg cattacctgg gaatttatat aagcccttaa taataatgcc 180
aatgaacatc tcatgtgtgc tcacaatgtt ctggcactat tataagtgct tcacaggttt 240
tatgtgttct tcgtaacttt atggantagg tactcggccg cgaacacgct aagccgaatt 300
c 301

<210> 279
<211> 301
<212> DNA
<213> Homo sapien

<220>
<221> misc_feature
<222> (1)...(301)
<223> n = A,T,C or G

<400> 279
aaagcaggaa tgacaaagct tgcttttctg gtatgttcta ggtgtattgt gacttttact 60
gttatattaa ttgccaatat aagtaaatat agattatata tgtatagtgt ttcacaaagc 120
ttagaccttt accttccagc caccccacag tgcttgatat ttcagagtca gtcattggtt 180
atacatgtgt agttccaaag cacataagct agaanaanaa atatttctag ggagcactac 240
catctgtttt cacatgaaat gccacacaca tagaactcca acatcaattt cattgcacag 300
a 301

<210> 280
<211> 301
<212> DNA
<213> Homo sapien

<400> 280
ggtactggag ttttcctccc ctgtgaaaac gtaactactg ttgggagtga attgaggatg 60
tagaaaggtg gtggaaccaa attgtggtca atggaaatag gagaatatgg ttctcactct 120
tgagaaaaaa acctaagatt agcccaggta gttgcctgta acttcagttt ttctgcctgg 180
gtttgatata gtttagggtt ggggttagat taagatctaa attacatcag gacaaagaga 240
cagactatta actccacagt taattaagga ggtatgttcc atgtttattt gttaaagcag 300
t 301

<210> 281
<211> 301
<212> DNA
<213> Homo sapien

<400> 281
aggtacaaga aggggaatgg gaaagagctg ctgctgtggc attgttcaac ttggatattc 60
gccgagcaat ccaaatcctg aatgaagggg catcttctga aaaaggagat ctgaatctca 120
atgtggtagc aatggcttta tcgggttata cggatgagaa gaactccctt tggagagaaa 180
tgtgtagcac actgcgatta cagctaaata acccgtattt gtgtgtcatg tttgcatttc 240
tgacaagtga aacaggatct tacgatggag ttttgtatga aaacaaagtt gcagtacctc 300
g 301

<210> 282
<211> 301
<212> DNA
<213> Homo sapien

<400> 282
caggtactac agaattaaaa tactgacaag caagtagttt cttggcgtgc acgaattgca 60
tccagaaccc aaaaattaag aaattcaaaa agacattttg tgggcacctg ctagcacaga 120
agcgcagaag caaagcccag gcagaaccat gctaacctta cagctcagcc tgcacagaag 180
cgcagaagca aagcccaggc agaaccatgc taaccttaca gctcagcctg cacagaagcg 240
cagaagcaaa gcccaggcag aacatgctaa ccttacagct cagcctgcac agaagcacag 300
a 301

<210> 283
<211> 301
<212> DNA
<213> Homo sapien

<400> 283
atctgtatac ggcagacaaa ctttatarag tgtagagagg tgagcgaaag gatgcaaaag 60
cactttgagg gctttataat aatatgctgc ttgaaaaaaa aaatgtgtag ttgatactca 120
gtgcatctcc agacatagta aggggttgct ctgaccaatc aggtgatcat tttttctatc 180
acttcccagg ttttatgcaa aaattttgtt aaattctata atggtgatat gcatctttta 240
ggaaacatat acatttttaa aaatctattt tatgtaagaa ctgacagacg aatttgcttt 300
g 301

<210> 284
<211> 301
<212> DNA
<213> Homo sapien

<400> 284
caggtacaaa acgctattaa gtggcttaga atttgaacat ttgtggtctt tatttacttt 60
gcttcgtgtg tgggcaaagc aacatcttcc ctaaatatat attaccaaga aaagcaagaa 120
gcagattagg tttttgacaa aacaaacagg ccaaaagggg gctgacctgg agcagagcat 180
ggtgagaggc aaggcatgag agggcaagtt tgttgtggac agatctgtgc ctactttatt 240
actggagtaa aagaaaacaa agttcattga tgtcgaagga tatatacagt gttagaaatt 300
a 301

<210> 285
<211> 301
<212> DNA
<213> Homo sapien

<220>
<221> misc_feature
<222> (1)...(301)
<223> n = A,T,C or G

<400> 285
acatcaccat gatcggatcc cccacccatt atacgttgta tgtttacata aatactcttc 60
aatgatcatt agtgttttaa aaaaaatact gaaaactcct tctgcatccc aatctctaac 120
caggaaagca aatgctattt acagacctgc aagccctccc tcaaacnaaa ctatttctgg 180
attaaatatg tctgacttct tttgaggtca cacgactagg caaatgctat ttacgatctg 240
caaaagctgt ttgaagagtc aaagccccca tgtgaacacg atttctggac cctgtaacag 300
t 301

<210> 286
<211> 301
<212> DNA
<213> Homo sapien

<400> 286
taccactgca ttccagcctg ggtgacagag tgagactccg tctccaaaaa aaactttgct 60
tgtatattat ttttgcctta cagtggatca ttctagtagg aaaggacagt aagatttttt 120
atcaaaatgt gtcatgccag taagagatgt tatattcttt tctcatttct tccccaccca 180
aaaataagct accatatagc ttataagtct caaatttttg ccttttacta aaatgtgatt 240
gtttctgttc attgtgtatg cttcatcacc tatattaggc aaattccatt ttttcccttg 300
t 301

<210> 287
<211> 301
<212> DNA
<213> Homo sapien

<400> 287
tacagatctg ggaactaaat attaaaaatg agtgtggctg gatatatgga gaatgttggg 60
cccagaagga acgtagagat cagatattac aacagctttg ttttgagggt tagaaatatg 120
aaatgatttg gttatgaacg cacagtttag gcagcagggc cagaatcctg accctctgcc 180
ccgtggttat ctcctcccca gcttggctgc ctcatgttat cacagtattc cattttgttt 240
gttgcatgtc ttgtgaagcc atcaagattt tctcgtctgt tttcctctca ttggtaatgc 300
t 301

<210> 288
<211> 301
<212> DNA
<213> Homo sapien

<400> 288
gtacacctaa ctgcaaggac agctgaggaa tgtaatgggc agccgctttt aaagaagtag 60
agtcaatagg aagacaaatt ccagttccag ctcagtctgg gtatctgcaa agctgcaaaa 120
gatctttaaa gacaatttca agagaatatt tccttaaagt tggcaatttg gagatcatac 180
aaaagcatct gcttttgtga tttaatttag ctcatctggc cactggaaga atccaaacag 240
tctgccttaa ttttggatga atgcatgatg gaaattcaat aatttagaaa gttaaaaaaa 300
a 301

<210> 289
<211> 301
<212> DNA
<213> Homo sapien

<220>
<221> misc_feature
<222> (1)...(301)
<223> n = A,T,C or G

<400> 289
ggtacactgt ttccatgtta tgtttctaca cattgctacc tcagtgctcc tggaaactta 60
gcttttgatg tctccaagta gtccaccttc atttaactct ttgaaactgt atcatctttg 120
ccaagtaaga gtggtggcct atttcagctg ctttgacaaa atgactggct cctgacttaa 180
cgttctataa atgaatgtgc tgaagcaaag tgcccatggt ggcggcgaan aagagaaaga 240

tgtgttttgt tttggactct ctgtggtccc ttccaatgct gtgggtttcc aaccagngga 300
a 301

<210> 290
<211> 301
<212> DNA
<213> Homo sapien

<220>
<221> misc_feature
<222> (1)...(301)
<223> n = A,T,C or G

<400> 290
acactgagct cttcttgata aatatacaga atgcttggca tatacaagat tctatactac 60
tgactgatct gttcatttct ctcacagctc ttacccccaa aagcttttcc accctaagtg 120
ttctgacctc cttttctaat cacagtaggg atagaggcag anccacctac aatgaacatg 180
gagttctatc aagaggcaga aacagcacag aatcccagtt ttaccattcg ctagcagtgc 240
tgccttgaac aaaaacattt ctccatgtct cattttcttc atgcctcaag taacagtgag 300
a 301

<210> 291
<211> 301
<212> DNA
<213> Homo sapien

<400> 291
caggtaccaa tttcttctat cctagaaaca tttcatttta tgttgttgaa acataacaac 60
tatatcagct agattttttt tctatgcttt acctgctatg gaaaatttga cacattctgc 120
tttactcttt tgtttatagg tgaatcacaa aatgtatttt tatgtattct gtagttcaat 180
agccatggct gtttacttca tttaatttat ttagcataaa gacattatga aaaggcctaa 240
acatgagctt cacttcccca ctaactaatt agcatctgtt atttcttaac cgtaatgcct 300
a 301

<210> 292
<211> 301
<212> DNA
<213> Homo sapien

<220>
<221> misc_feature
<222> (1)...(301)
<223> n = A,T,C or G

<400> 292
accttttagt agtaatgtct aataataaat aagaaatcaa ttttataagg tccatatagc 60
tgtattaaat aatttttaag tttaaaagat aaaataccat cattttaaat gttggtattc 120
aaaaccaaag natataaccg aaaggaaaaa cagatgagac ataaaatgat ttgcnagatg 180
ggaaatatag tasttyatga atgttnatta aattccagtt ataatagtgg ctacacactc 240
tcactacaca cacagacccc acagtcctat atgccacaaa cacatttcca taacttgaaa 300
a 301

<210> 293
<211> 301
<212> DNA
<213> Homo sapien

<400> 293
ggtaccaagt gctggtgcca gcctgttacc tgttctcact gaaaagtctg gctaatgctc 60
ttgtgtagtc acttctgatt ctgacaatca atcaatcaat ggcctagagc actgactgtt 120
aacacaaacg tcactagcaa agtagcaaca gctttaagtc taaatacaaa gctgttctgt 180
gtgagaattt tttaaaaggc tacttgtata ataacccttg tcatttttaa tgtacctcgg 240

ccgcgaccac gctaagccga attctgcaga tatccatcac actggcggcc gctcgagcat 300
g 301

<210> 294
<211> 301
<212> DNA
<213> Homo sapien

<220>
<221> misc_feature
<222> (1)...(301)
<223> n = A,T,C or G

<400> 294
tgacccataa caatatacac tagctatctt tttaactgtc catcattagc accaatgaag 60
attcaataaa attaccttta ttcacacatc tcaaaacaat tctgcaaatt cttagtgaag 120
tttaactata gtcacaganc ttaaatattc acattgtttt ctatgtctac tgaaaataag 180
ttcactactt ttctgggata ttctttacaa aatcttatta aaattcctgg tattatcacc 240
cccaattata cagtagcaca accaccttat gtagttttta catgatagct ctgtagaggt 300
t 301

<210> 295
<211> 305
<212> DNA
<213> Homo sapien

<400> 295
gtactctttc tctcccctcc tctgaattta attctttcaa cttgcaattt gcaaggatta 60
cacatttcac tgtgatgtat attgtgttgc aaaaaaaaaa gtgtctttgt ttaaaattac 120
ttggtttgtg aatccatctt gctttttccc cattggaact agtcattaac ccatctctga 180
actggtagaa aaacrtctga agagctagtc tatcagcatc tgacaggtga attggatggt 240
tctcagaacc atttcaccca gacagcctgt ttctatcctg tttaataaat tagtttgggt 300
tctct 305

<210> 296
<211> 301
<212> DNA
<213> Homo sapien

<400> 296
aggtactatg ggaagctgct aaaataatat ttgatagtaa aagtatgtaa tgtgctatct 60
cacctagtag taaactaaaa ataaactgaa actttatgga atctgaagtt attttccttg 120
attaaataga attaataaac caatatgagg aaacatgaaa ccatgcaatc tactatcaac 180
tttgaaaaag tgattgaacg aaccacttag ctttcagatg atgaacactg ataagtcatt 240
tgtcattact ataaatttta aaatctgtta ataagatggc ctatagggag gaaaaagggg 300
c 301

<210> 297
<211> 300
<212> DNA
<213> Homo sapien

<220>
<221> misc_feature
<222> (1)...(300)
<223> n = A,T,C or G

<400> 297
actgagtttt aactggacgc caagcaggca aggctggaag gttttgctct ctttgtgcta 60
aaggttttga aaaccttgaa ggagaatcat tttgacaaga agtacttaag agtctagaga 120
acaaagangt gaaccagctg aaagctctcg ggggaanctt acatgtgttg ttaggcctgt 180
tccatcattg ggagtgcact ggccatccct caaaatttgt ctgggctggc ctgagtggtc 240

accgcacctc ggccgcgacc acgctaagcc gaattctgca gatatccatc acactggcgg 300

<210> 298
<211> 301
<212> DNA
<213> Homo sapien

<220>
<221> misc_feature
<222> (1)...(301)
<223> n = A,T,C or G

<400> 298
tatggggttt gtcacccaaa agctgatgct gagaaaggcc tccctggggc ccctcccgcg 60
ggcatctgag agacctggtg ttccagtgtt tctggaaatg ggtcccagtg ccgccggctg 120
tgaagctctc agatcaatca cgggaagggc ctggcggtgg tggccacctg gaaccaccct 180
gtcctgtctg tttacatttc actaycaggt tttctctggg cattacnatt tgttccccta 240
caacagtgac ctgtgcattc tgctgtggcc tgctgtgtct gcaggtggct ctcagcgagg 300
t 301

<210> 299
<211> 301
<212> DNA
<213> Homo sapien

<400> 299
gttttgagac ggagtttcac tcttgttgcc cagactggac tgcaatggca gggtctctgc 60
tcactgcacc ctctgcctcc caggttcgag caattctcct gcctcagcct cccaggtagc 120
tgggattgca ggctcacgcc accataccca gctaattttt ttgtattttt agtagagacg 180
gagtttcgcc atgttggcca gctggtctca aactcctgac ctcaagcgac ctgcctgcct 240
cggcctccca aagtgctgga attataggca tgagtcaaca cgcccagcct aaagatattt 300
t 301

<210> 300
<211> 301
<212> DNA
<213> Homo sapien

<400> 300
attcagtttt atttgctgcc ccagtatctg taaccaggag tgccacaaaa tcttgccaga 60
tatgtcccac acccactggg aaaggctccc acctggctac ttcctctatc agctgggtca 120
gctgcattcc acaaggttct cagcctaatg agtttcacta cctgccagtc tcaaaactta 180
gtaaagcaag accatgacat tcccccacgg aaatcagagt ttgccccacc gtcttgttac 240
tataaagcct gcctctaaca gtccttgctt cttcacacca atcccgagcg catcccccat 300
g 301

<210> 301
<211> 301
<212> DNA
<213> Homo sapien

<400> 301
ttaaattttt gagaggataa aaaggacaaa taatctagaa atgtgtcttc ttcagtctgc 60
agaggacccc aggtctccaa gcaaccacat ggtcaagggc atgaataatt aaaagttggt 120
gggaactcac aaagaccctc agagctgaga cacccacaac agtgggagct cacaaagacc 180
ctcagagctg agacacccac aacagtggga gctcacaaag accctcagag ctgagacacc 240
cacaacagca cctcgttcag ctgccacatg tgtgaataag gatgcaatgt ccagaagtgt 300
t 301

<210> 302
<211> 301
<212> DNA

<213> Homo sapien

<400> 302
aggtacacat ttagcttgtg gtaaatgact cacaaaactg attttaaaat caagttaatg 60
tgaattttga aaattactac ttaatcctaa ttcacaataa caatggcatt aaggtttgac 120
ttgagttggt tcttagtatt atttatggta aataggctct taccacttgc aaataactgg 180
ccacatcatt aatgactgac ttcccagtaa ggctctctaa ggggtaagta ggaggatcca 240
caggatttga gatgctaagg ccccagagat cgtttgatcc aaccctctta ttttcagagg 300
g 301

<210> 303
<211> 301
<212> DNA
<213> Homo sapien

<400> 303
aggtaccaac tgtggaaata ggtagaggat catttttctt ttccatatca actaagttgt 60
atattgtttt ttgacagttt aacacatctt cttctgtcag agattctttc acaatagcac 120
tggctaatgg aactaccgct tgcatgttaa aaatggtggt ttgtgaaatg atcataggcc 180
agtaacgggt atgtttttct aactgatctt ttgctcgttc caaaggaccc tcaagacttc 240
catcgatttt atatctgggg tctagaaaag gagttaatct gttttccctc ataaattcac 300
c 301

<210> 304
<211> 301
<212> DNA
<213> Homo sapien

<400> 304
acatggatgt tattttgcag actgtcaacc tgaatttgta tttgcttgac attgcctaat 60
tattagtttc agtttcagct tacccacttt ttgtctgcaa catgcaraas agacagtgcc 120
ctttttagtg tatcatatca ggaatcatct cacattggtt tgtgccatta ctggtgcagt 180
gactttcagc cacttgggta aggtggagtt ggccatatgt ctccactgca aaattactga 240
ttttcctttt gtaattaata agtgtgtgtg tgaagattct ttgagatgag gtatatatct 300
c 301

<210> 305
<211> 301
<212> DNA
<213> Homo sapien

<220>
<221> misc_feature
<222> (1)...(301)
<223> n = A,T,C or G

<400> 305
gangtacagc gtggtcaagg taacaagaag aaaaaaatgt gagtggcatc ctgggatgag 60
cagggggaca gacctggaca gacacgttgt catttgctgc tgtgggtagg aaaatgggcg 120
taaaggagga gaaacagata caaaatctcc aactcagtat taaggtattc tcatgcctag 180
aatattggta gaaacaagaa tacattcata tggcaaataa ctaaccatgg tggaacaaaa 240
ttctgggatt taagttggat accaangaaa ttgtattaaa agagctgttc atggaataag 300
a 301

<210> 306
<211> 8
<212> PRT
<213> Homo sapien

<400> 306
Val Leu Gly Trp Val Ala Glu Leu
1               5

<210> 307
<211> 637
<212> DNA
<213> Homo sapien

<400> 307
acagggratg aagggaaagg gagaggatga ggaagccccc ctggggattt ggtttggtcc 60
ttgtgatcag gtggtctatg gggcttatcc ctacaaagaa gaatccagaa ataggggcac 120
attgaggaat gatacttgag cccaaagagc attcaatcat tgttttattt gccttmtttt 180
cacaccattg gtgagggagg gattaccacc ctggggttat gaagatggtt gaacacccca 240
cacatagcac cggagatatg agatcaacag tttcttagcc atagagattc acagcccaga 300
gcaggaggac gcttgcacac catgcaggat gacatggggg atgcgctcgg gattggtgtg 360
aagaagcaag gactgttaga ggcaggcttt atagtaacaa gacggtgggg caaactctga 420
tttccgtggg ggaatgtcat ggtcttgctt tactaagttt tgagactggc aggtagtgaa 480
actcattagg ctgagaacct tgtggaatgc acttgaccca sctgatagag gaagtagcca 540
ggtgggagcc tttcccagtg ggtgtgggac atatctggca agattttgtg gcactcctgg 600
ttacagatac tggggcagca aataaaactg aatcttg 637

<210> 308
<211> 647
<212> DNA
<213> Homo sapien

<220>
<221> misc_feature
<222> (1)...(647)
<223> n = A,T,C or G

<400> 308
acgattttca ttatcatgta aatcgggtca ctcaaggggc caaccacagc tgggagccac 60
tgctcagggg aaggttcata tgggactttc tactgcccaa ggttctatac aggatataaa 120
ggngcctcac agtatagatc tggtagcaaa gaagaagaaa caaacactga tctctttctg 180
ccacccctct gaccctttgg aactcctctg accctttaga acaagcctac ctaatatctg 240
ctagagaaaa gaccaacaac ggcctcaaag gatctcttac catgaaggtc tcagctaatt 300
cttggctaag atgtgggttc cacattaggt tctgaatatg gggggaaggg tcaatttgct 360
cattttgtgt gtggataaag tcaggatgcc caggggccag agcagggggc tgcttgcttt 420
gggaacaatg gctgagcata taaccatagg ttatggggaa caaaacaaca tcaaagtcac 480
tgtatcaatt gccatgaaga cttgagggac ctgaatctac cgattcatct taaggcagca 540
ggaccagttt gagtggcaac aatgcagcag cagaatcaat ggaaacaaca gaatgattgc 600
aatgtccttt tttttctcct gcttctgact tgataaaagg ggaccgt 647

<210> 309
<211> 460
<212> DNA
<213> Homo sapien

<400> 309
actttatagt ttaggctgga cattggaaaa aaaaaaaagc cagaacaaca tgtgatagat 60
aatatgattg gctgcacact tccagactga tgaatgatga acgtgatgga ctattgtatg 120
gagcacatct tcagcaagag ggggaaatac tcatcatttt tggccagcag ttgtttgatc 180
accaaacatc atgccagaat actcagcaaa ccttcttagc tcttgagaag tcaaagtccg 240
ggggaattta ttcctggcaa ttttaattgg actccttatg tgagagcagc ggctacccag 300
ctggggtggt ggagcgaacc cgtcactagt ggacatgcag tggcagagct cctggtaacc 360
acctagagga atacacaggc acatgtgtga tgccaagcgt gacacctgta gcactcaaat 420
ttgtcttgtt tttgtctttc ggtgtgtaag attcttaagt 460

<210> 310
<211> 539
<212> DNA
<213> Homo sapien

<400> 310
acgggactta tcaaataaag ataggaaaag aagaaaactc aaatattata ggcagaaatg 60
ctaaaggttt taaaatatgt caggattgga agaaggcatg gataaagaac aaagttcagt 120
taggaaagag aaacacagaa ggaagagaca caataaaagt cattatgtat tctgtgagaa 180
gtcagacagt aagatttgtg ggaaatgggt tggtttgttg tatggtatgt attttagcaa 240
taatctttat ggcagagaaa gctaaaatcc tttagcttgc gtgaatgatc acttgctgaa 300
ttcctcaagg taggcatgat gaaggagggt ttagaggaga cacagacaca atgaactgac 360
ctagatagaa agccttagta tactcagcta ggaatagtga ttctgagggc acactgtgac 420
atgattatgt cattacatgt atggtagtga tggggatgat aggaaggaag aacttatggc 480
atattttcac ccccacaaaa gtcagttaaa tattgggaca ctaaccatcc aggtcaaga 539

<210> 311
<211> 526
<212> DNA
<213> Homo sapien

<220>
<221> misc_feature
<222> (1)...(526)
<223> n = A,T,C or G

<400> 311
caaatttgag ccaatgacat agaattttac aaatcaagaa gcttattctg gggccatttc 60
ttttgacgtt ttctctaaac tactaaagag gcattaatga tccataaatt atattatcta 120
catttacagc atttaaaatg tgttcagcat gaaatattag ctacagggga agctaaataa 180
attaaacatg gaataaagat ttgtccttaa atataatcta caagaagact ttgatatttg 240
tttttcacaa gtgaagcatt cttataaagt gtcataacct ttttggggaa actatgggaa 300
aaaatgggga aactctgaag ggttttaagt atcttacctg aagctacaga ctccataacc 360
tctctttaca gggagctcct gcagccccta cagaaatgag tggctgagat tcttgattgc 420
acagcaagag cttctcatct aaaccctttc cctttttagt atctgtgtat caagtataaa 480
agttctataa actgtagtnt acttatttta atccccaaag cacagt 526

<210> 312
<211> 500
<212> DNA
<213> Homo sapien

<220>
<221> misc_feature
<222> (1)...(500)
<223> n = A,T,C or G

<400> 312
cctctctctc cccaccccct gactctagag aactgggttt tctcccagta ctccagcaat 60
tcatttctga aagcagttga gccactttat tccaaagtac actgcagatg ttcaaactct 120
ccatttctct ttcccttcca cctgccagtt ttgctgactc tcaacttgtc atgagtgtaa 180
gcattaagga cattatgctt cttcgattct gaagacaggc cctgctcatg gatgactctg 240
gcttcttagg aaaatatttt tcttccaaaa tcagtaggaa atctaaactt atcccctctt 300
tgcagatgtc tagcagcttc agacatttgg ttaagaaccc atgggaaaaa aaaaaatcct 360
tgctaatgtg gtttcctttg taaaccanga ttcttatttg nctggtatag aatatcagct 420
ctgaacgtgt ggtaaagatt tttgtgtttg aatataggag aaatcagttt gctgaaaagt 480
tagtcttaat tatctattgg 500

<210> 313
<211> 718
<212> DNA
<213> Homo sapien

<220>
<221> misc_feature
<222> (1)...(718)
<223> n = A,T,C or G

<400> 313
ggagatttgt gtggtttgca gccgagggag accaggaaga tctgcatggt gggaaggacc 60
tgatgataca gaggtgagaa ataagaaagg ctgctgactt taccatctga ggccacacat 120
ctgctgaaat ggagataatt aacatcacta gaaacagcaa gatgacaata taatgtctaa 180
gtagtgacat gtttttgcac atttccagcc cttttaaata tccacacaca caggaagcac 240
aaaaggaagc acagagatcc ctgggagaaa tgcccggccg ccatcttggg tcatcgatga 300
gcctcgccct gtgcctgntc ccgcttgtga gggaaggaca ttagaaaatg aattgatgtg 360
ttccttaaag gatggcagga aaacagatcc tgttgtggat atttatttga acgggattac 420
agatttgaaa tgaagtcaca aagtgagcat taccaatgag aggaaaacag acgagaaaat 480
cttgatggtt cacaagacat gcaacaaaca aaatggaata ctgtgatgac acgagcagcc 540
aactggggag gagataccac ggggcagagg tcaggattct ggccctgctg cctaactgtg 600
cgttatacca atcatttcta tttctaccct caaacaagct gtngaatatc tgacttacgg 660
ttcttntggc ccacattttc atnatccacc ccntcntttt aannttantc caaantgt 718

<210> 314
<211> 358
<212> DNA
<213> Homo sapien

<400> 314
gtttatttac attacagaaa aaacatcaag acaatgtata ctatttcaaa tatatccata 60
cataatcaaa tatagctgta gtacatgttt tcattggtgt agattaccac aaatgcaagg 120
caacatgtgt agatctcttg tcttattctt ttgtctataa tactgtattg tgtagtccaa 180
gctctcggta gtccagccac tgtgaaacat gctcccttta gattaacctc gtggacgctc 240
ttgttgtatt gctgaactgt agtgccctgt attttgcttc tgtctgtgaa ttctgttgct 300
tctggggcat ttccttgtga tgcagaggac caccacacag atgacagcaa tctgaatt 358

<210> 315
<211> 341
<212> DNA
<213> Homo sapien

<400> 315
taccacctcc ccgctggcac tgatgagccg catcaccatg gtcaccagca ccatgaaggc 60
ataggtgatg atgaggacat ggaatgggcc cccaaggatg gtctgtccaa agaagcgagt 120
gacccccatt ctgaagatgt ctggaacctc taccagcagg atgatgatag ccccaatgac 180
agtcaccagc tccccgacca gccggatatc gtccttaggg gtcatgtagg cttcctgaag 240
tagcttctgc tgtaagaggg tgttgtcccg ggggctcgtg cggttattgg tcctgggctt 300
gagggggcgg tagatgcagc acatggtgaa gcagatgatg t 341

<210> 316
<211> 151
<212> DNA
<213> Homo sapien

<400> 316
agactgggca agactcttac gccccacact gcaatttggt cttgttgccg tatccattta 60
tgtgggcctt tctcgagttt ctgattataa acaccactgg agcgatgtgt tgactggact 120
cattcaggga gctctggttg caatattagt t 151

<210> 317
<211> 151
<212> DNA
<213> Homo sapien

<400> 317
agaactagtg gatcctaatg aaatacctga aacatatatt ggcatttatc aatggctcaa 60
atcttcattt atctctggcc ttaaccctgg ctcctgaggc tgcggccagc agatcccagg 120
ccagggctct gttcttgcca cacctgcttg a 151

<210> 318

<211> 151
<212> DNA
<213> Homo sapien

<400> 318
actggtggga ggcgctgttt agttggctgt tttcagaggg gtctttcgga gggacctcct 60
gctgcaggct ggagtgtctt tattcctggc gggagaccgc acattccact gctgaggctg 120
tgggggcggt ttatcaggca gtgataaaca t 151

<210> 319
<211> 151
<212> DNA
<213> Homo sapien

<400> 319
aactagtgga tccagagcta taggtacagt gtgatctcag ctttgcaaac acattttcta 60
catagatagt actaggtatt aatagatatg taaagaaaga aatcacacca ttaataatgg 120
taagattggg tttatgtgat tttagtgggt a 151

<210> 320
<211> 150
<212> DNA
<213> Homo sapien

<400> 320
aactagtgga tccactagtc cagtgtggtg gaattccatt gtgttggggt tctagatcgc 60
gagcggctgc cctttttttt tttttttttg gggggaattt tttttttttt aatagttatt 120
gagtgttcta cagcttacag taaataccat 150

<210> 321
<211> 151
<212> DNA
<213> Homo sapien

<400> 321
agcaactttg tttttcatcc aggttatttt aggcttagga tttcctctca cactgcagtt 60
tagggtggca ttgtaaccag ctatggcata ggtgttaacc aaaggctgag taaacatggg 120
tgcctctgag aaatcaaagt cttcatacac t 151

<210> 322
<211> 151
<212> DNA
<213> Homo sapien

<220>
<221> misc_feature
<222> (1)...(151)
<223> n = A,T,C or G

<400> 322
atccagcatc ttctcctgtt tcttgccttc ctttttcttc ttcttasatt ctgcttgagg 60
tttgggcttg gtcagtttgc cacagggctt ggagatggtg acagtcttct ggcattcggc 120
attgtgcagg gctcgcttca nacttccagt t 151

<210> 323
<211> 151
<212> DNA
<213> Homo sapien

<220>
<221> misc_feature
<222> (1)...(151)

<223> n = A,T,C or G

<400> 323
tgaggacttg tkttcttttt ctttattttt aatcctctta ckttgtaaat atattgccta 60
nagactcant tactacccag tttgtggttt twtgggagaa atgtaactgg acagttagct 120
gttcaatyaa aaagacactt ancccatgtg g 151

<210> 324
<211> 461
<212> DNA
<213> Homo sapien

<220>
<221> misc_feature
<222> (1)...(461)
<223> n = A,T,C or G

<400> 324
acctgtgtgg aatttcagct ttcctcatgc aaaaggattt tgtatccccg gcctacttga 60
agaagtggtc agctaaagga atccaggttg ttggttggac tgttaatacc tttgatgaaa 120
agagttacta cgaatcccat cttggttcca gctatatcac tgacagcatg gtagaagact 180
gcgaacctca cttctagact ttcacggtgg gacgaaacgg gttcagaaac tgccaggggc 240
ctcatacagg gatatcaaaa taccctttgt gctacccagg ccctggggaa tcaggtgact 300
cacacaaatg caatagttgg tcactgcatt tttacctgaa ccaaagctaa acccggtgtt 360
gccaccatgc accatggcat gccagagttc aacactgttg ctcttgaaaa ttgggtctga 420
aaaaacgcac aagagcccct gccctgccct agctgangca c 461

<210> 325
<211> 400
<212> DNA
<213> Homo sapien

<400> 325
acactgtttc catgttatgt ttctacacat tgctacctca gtgctcctgg aaacttagct 60
tttgatgtct ccaagtagtc caccttcatt taactctttg aaactgtatc atctttgcca 120
agtaagagtg gtggcctatt tcagctgctt tgacaaaatg actggctcct gacttaacgt 180
tctataaatg aatgtgctga agcaaagtgc ccatggtggc ggcgaagaag agaaagatgt 240
gttttgtttt ggactctctg tggtcccttc caatgctgtg ggtttccaac caggggaagg 300
gtcccttttg cattgccaag tgccataacc atgagcacta cgctaccatg gttctgcctc 360
ctggccaagc aggctggttt gcaagaatga aatgaatgat 400

<210> 326
<211> 1215
<212> DNA
<213> Homo sapien

<400> 326
   ggaggactgc agcccgcact cgcagccctg gcaggcggca ctggtcatgg aaaacgaatt 60
   gttctgctcg ggcgtcctgg tgcatccgca gtgggtgctg tcagccgcac actgtttcca 120
   gaactcctac accatcgggc tgggcctgca cagtcttgag gccgaccaag agccagggag 180
   ccagatggtg gaggccagcc tctccgtacg gcacccagag tacaacagac ccttgctcgc 240
   taacgacctc atgctcatca agttggacga atccgtgtcc gagtctgaca ccatccggag 300
   catcagcatt gcttcgcagt gccctaccgc ggggaactct tgcctcgttt ctggctgggg 360
   tctgctggcg aacggcagaa tgcctaccgt gctgcagtgc gtgaacgtgt cggtggtgtc 420
   tgaggaggtc tgcagtaagc tctatgaccc gctgtaccac cccagcatgt tctgcgccgg 480
   cggagggcaa gaccagaagg actcctgcaa cggtgactct ggggggcccc tgatctgcaa 540
   cgggtacttg cagggccttg tgtctttcgg aaaagccccg tgtggccaag ttggcgtgcc 600
   aggtgtctac accaacctct gcaaattcac tgagtggata gagaaaaccg tccaggccag 660
   ttaactctgg ggactgggaa cccatgaaat tgacccccaa atacatcctg cggaaggaat 720
   tcaggaatat ctgttcccag cccctcctcc ctcaggccca ggagtccagg cccccagccc 780
   ctcctccctc aaaccaaggg tacagatccc cagcccctcc tccctcagac ccaggagtcc 840
   agacccccca gcccctcctc cctcagaccc aggagtccag cccctcctcc ctcagaccca 900
   ggagtccaga ccccccagcc cctcctccct cagacccagg ggtccaggcc cccaacccct 960
   cctccctcag actcagaggt ccaagccccc aacccctcct tccccagacc cagaggtcca 1020
   ggtcccagcc cctcctccct cagacccagc ggtccaatgc cacctagact ctccctgtac 1080
   acagtgcccc cttgtggcac gttgacccaa ccttaccagt tggtttttca ttttttgtcc 1140
   ctttccccta gatccagaaa taaagtctaa gagaagcgca aaaaaaaaaa aaaaaaaaaa 1200
   aaaaaaaaaa aaaaa 1215

<210> 327
<211> 220
<212> PRT
<213> Homo sapien

<400> 327
Glu Asp Cys Ser Pro His Ser Gln Pro Trp Gln Ala Ala Leu Val Met
1               5                   10                  15
Glu Asn Glu Leu Phe Cys Ser Gly Val Leu Val His Pro Gln Trp Val
            20                  25                  30
Leu Ser Ala Ala His Cys Phe Gln Asn Ser Tyr Thr Ile Gly Leu Gly
        35                  40                  45
Leu His Ser Leu Glu Ala Asp Gln Glu Pro Gly Ser Gln Met Val Glu
    50                  55                  60
Ala Ser Leu Ser Val Arg His Pro Glu Tyr Asn Arg Pro Leu Leu Ala
65                  70                  75                  80
Asn Asp Leu Met Leu Ile Lys Leu Asp Glu Ser Val Ser Glu Ser Asp
                85                  90                  95
Thr Ile Arg Ser Ile Ser Ile Ala Ser Gln Cys Pro Thr Ala Gly Asn
            100                 105                 110
Ser Cys Leu Val Ser Gly Trp Gly Leu Leu Ala Asn Gly Arg Met Pro
        115                 120                 125
Thr Val Leu Gln Cys Val Asn Val Ser Val Val Ser Glu Glu Val Cys
    130                 135                 140
Ser Lys Leu Tyr Asp Pro Leu Tyr His Pro Ser Met Phe Cys Ala Gly
145                 150                 155                 160
Gly Gly Gln Asp Gln Lys Asp Ser Cys Asn Gly Asp Ser Gly Gly Pro
                165                 170                 175
Leu Ile Cys Asn Gly Tyr Leu Gln Gly Leu Val Ser Phe Gly Lys Ala
            180                 185                 190
Pro Cys Gly Gln Val Gly Val Pro Gly Val Tyr Thr Asn Leu Cys Lys
        195                 200                 205
Phe Thr Glu Trp Ile Glu Lys Thr Val Gln Ala Ser
    210                 215                 220

<210> 328
<211> 234
<212> DNA
<213> Homo sapien

<400> 328
cgctcgtctc tggtagctgc agccaaatca taaacggcga ggactgcagc ccgcactcgc 60
agccctggca ggcggcactg gtcatggaaa acgaattgtt ctgctcgggc gtcctggtgc 120
atccgcagtg ggtgctgtca gccacacact gtttccagaa ctcctacacc atcgggctgg 180
gcctgcacag tcttgaggcc gaccaagagc cagggagcca gatggtggag gcca 234

<210> 329
<211> 77
<212> PRT
<213> Homo sapien

<400> 329
Leu Val Ser Gly Ser Cys Ser Gln Ile Ile Asn Gly Glu Asp Cys Ser
1               5                   10                  15
Pro His Ser Gln Pro Trp Gln Ala Ala Leu Val Met Glu Asn Glu Leu

                20                  25                  30
Phe Cys Ser Gly Val Leu Val His Pro Gln Trp Val Leu Ser Ala Thr
        35                  40                  45
His Cys Phe Gln Asn Ser Tyr Thr Ile Gly Leu Gly Leu His Ser Leu
    50                  55                  60
Glu Ala Asp Gln Glu Pro Gly Ser Gln Met Val Glu Ala
65                  70                  75

<210> 330
<211> 70
<212> DNA
<213> Homo sapien

<400> 330
cccaacacaa tggcccgatc ccatccctga ctccgccctc aggatcgctc gtctctggta      60
gctgcagcca                                                              70

<210> 331
<211> 22
<212> PRT
<213> Homo sapien

<400> 331
Gln His Asn Gly Pro Ile Pro Ser Leu Thr Pro Pro Ser Gly Ser Leu
 1               5                  10                  15
Val Ser Gly Ser Cys Ser
            20

<210> 332
<211> 2507
<212> DNA
<213> Homo sapien

<400> 332
tggtgccgct gcagccggca gagatggttg agctcatgtt cccgctgttg ctcctccttc      60
tgcccttcct tctgtatatg gctgcgcccc aaatcaggaa aatgctgtcc agtggggtgt     120
gtacatcaac tgttcagctt cctgggaaag tagttgtggt cacaggagct aatacaggta     180
tcgggaagga gacagccaaa gagctggctc agagaggagc tcgagtatat ttagcttgcc     240
gggatgtgga aaagggggaa ttggtggcca aagagatcca gaccacgaca gggaaccagc     300
aggtgttggt gcggaaactg gacctgtctg atactaagtc tattcgagct tttgctaagg     360
gcttcttagc tgaggaaaag cacctccacg ttttgatcaa caatgcagga gtgatgatgt     420
gtccgtactc gaagacagca gatggctttg agatgcacat aggagtcaac cacttgggtc     480
acttcctcct aacccatctg ctgctagaga aactaaagga atcagcccca tcaaggatag     540
taaatgtgtc ttccctcgca catcacctgg gaaggatcca cttccataac ctgcagggcg     600
agaaattcta caatgcaggc ctggcctact gtcacagcaa gctagccaac atcctcttca     660
cccaggaact ggcccggaga ctaaaaggct ctggcgttac gacgtattct gtacaccctg     720
gcacagtcca atctgaactg gttcggcact catctttcat gagatggatg tggtggcttt     780
tctccttttt catcaagact cctcagcagg gagcccagac cagcctgcac tgtgccttaa     840
cagaaggtct tgagattcta agtgggaatc atttcagtga ctgtcatgtg gcatgggtct     900
ctgcccaagc tcgtaatgag actatagcaa ggcggctgtg ggacgtcagt tgtgacctgc     960
tgggcctccc aatagactaa caggcagtgc cagttggacc caagagaaga ctgcagcaga    1020
ctacacagta cttcttgtca aaatgattct ccttcaaggt tttcaaaacc tttagcacaa    1080
agagagcaaa accttccagc cttgcctgct tggtgtccag ttaaaactca gtgtactgcc    1140
agattcgtct aaatgtctgt catgtccaga tttactttgc ttctgttact gccagagtta    1200
ctagagatat cataatagga taagaagacc ctcatatgac ctgcacagct cattttcctt    1260
ctgaaagaaa ctactaccta ggagaatcta agctatagca gggatgattt atgcaaattt    1320
gaactagctt ctttgttcac aattcagttc ctcccaacca accagtcttc acttcaagag    1380
ggccacactg caacctcagc ttaacatgaa taacaaagac tggctcagga gcagggcttg    1440
cccaggcatg gtggatcacc ggaggtcagt agttcaagac cagcctggcc aacatggtga    1500
aaccccacct ctactaaaaa ttgtgtatat ctttgtgtgt cttcctgttt atgtgtgcca    1560
agggagtatt ttcacaaagt tcaaaacagc cacaataatc agagatggag caaaccagtg    1620
ccatccagtc tttatgcaaa tgaaatgctg caaagggaag cagattctgt atatgttggt    1680

aactacccac caagagcaca tgggtagcag ggaagaagta aaaaaagaga aggagaatac 1740
tggaagataa tgcacaaaat gaagggacta gttaaggatt aactagccct ttaaggatta 1800
actagttaag gattaatagc aaaagayatt aaatatgcta acatagctat ggaggaattg 1860
agggcaagca cccaggactg atgaggtctt aacaaaaacc agtgtggcaa aaaaaaaaaa 1920
aaaaaaaaaa aaaaatccta aaaacaaaca aacaaaaaaa acaattcttc attcagaaaa 1980
attatcttag ggactgatat tggtaattat ggtcaattta ataatatttt ggggcatttc 2040
cttacattgt cttgacaaga ttaaaatgtc tgtgccaaaa ttttgtattt tatttggaga 2100
cttcttatca aaagtaatgc tgccaaagga agtctaagga attagtagtg ttcccatcac 2160
ttgtttggag tgtgctattc taaaagattt tgatttcctg gaatgacaat tatattttaa 2220
ctttggtggg ggaaagagtt ataggaccac agtcttcact tctgatactt gtaaattaat 2280
cttttattgc acttgttttg accattaagc tatatgttta gaaatggtca ttttacggaa 2340
aaattagaaa aattctgata atagtgcaga ataaatgaat taatgtttta cttaatttat 2400
attgaactgt caatgacaaa taaaaattct ttttgattat tttttgtttt catttaccag 2460
aataaaaacg taagaattaa aagtttgatt acaaaaaaaa aaaaaaa 2507

<210> 333
<211> 3030
<212> DNA
<213> Homo sapien

<400> 333
gcaggcgact tgcgagctgg gagcgattta aaacgctttg gattcccccg gcctgggtgg 60
ggagagcgag ctgggtgccc cctagattcc ccgcccccgc acctcatgag ccgaccctcg 120
gctccatgga gcccggcaat tatgccacct tggatggagc caaggatatc gaaggcttgc 180
tgggagcggg aggggggcgg aatctggtcg cccactcccc tctgaccagc cacccagcgg 240
cgcctacgct gatgcctgct gtcaactatg cccccttgga tctgccaggc tcggcggagc 300
cgccaaagca atgccaccca tgccctgggg tgccccaggg gacgtcccca gctcccgtgc 360
cttatggtta ctttggaggc gggtactact cctgccgagt gtcccggagc tcgctgaaac 420
cctgtgccca ggcagccacc ctggccgcgt accccgcgga gactcccacg gccggggaag 480
agtaccccag ycgccccact gagtttgcct tctatccggg atatccggga acctaccagc 540
ctatggccag ttacctggac gtgtctgtgg tgcagactct gggtgctcct ggagaaccgc 600
gacatgactc cctgttgcct gtggacagtt accagtcttg ggctctcgct ggtggctgga 660
acagccagat gtgttgccag ggagaacaga acccaccagg tcccttttgg aaggcagcat 720
ttgcagactc cagcgggcag caccctcctg acgcctgcgc ctttcgtcgc ggccgcaaga 780
aacgcattcc gtacagcaag gggcagttgc gggagctgga gcgggagtat gcggctaaca 840
agttcatcac caaggacaag aggcgcaaga tctcggcagc caccagcctc tcggagcgcc 900
agattaccat ctggtttcag aaccgccggg tcaaagagaa gaaggttctc gccaaggtga 960
agaacagcgc taccccttaa gagatctcct tgcctgggtg ggaggagcga aagtgggggt 1020
gtcctgggga gaccaggaac ctgccaagcc caggctgggg ccaaggactc tgctgagagg 1080
cccctagaga caacaccctt cccaggccac tggctgctgg actgttcctc aggagcggcc 1140
tgggtaccca gtatgtgcag ggagacggaa ccccatgtga cagcccactc caccagggtt 1200
cccaaagaac ctggcccagt cataatcatt catcctgaca gtggcaataa tcacgataac 1260
cagtactagc tgccatgatc gttagcctca tattttctat ctagagctct gtagagcact 1320
ttagaaaccg ctttcatgaa ttgagctaat tatgaataaa tttggaaggc gatccctttg 1380
cagggaagct ttctctcaga cccccttcca ttacacctct caccctggta acagcaggaa 1440
gactgaggag aggggaacgg gcagattcgt tgtgtggctg tgatgtccgt ttagcatttt 1500
tctcagctga cagctgggta ggtggacaat tgtagaggct gtctcttcct ccctccttgt 1560
ccaccccata gggtgtaccc actggtcttg gaagcaccca tccttaatac gatgattttt 1620
ctgtcgtgtg aaaatgaagc cagcaggctg cccctagtca gtccttcctt ccagagaaaa 1680
agagatttga gaaagtgcct gggtaattca ccattaattt cctcccccaa actctctgag 1740
tcttccctta atatttctgg tggttctgac caaagcaggt catggtttgt tgagcatttg 1800
ggatcccagt gaagtagatg tttgtagcct tgcatactta gcccttccca ggcacaaacg 1860
gagtggcaga gtggtgccaa ccctgttttc ccagtccacg tagacagatt cacagtgcgg 1920
aattctggaa gctggagaca gacgggctct ttgcagagcc gggactctga gagggacatg 1980
agggcctctg cctctgtgtt cattctctga tgtcctgtac ctgggctcag tgcccggtgg 2040
gactcatctc ctggccgcgc agcaaagcca gcgggttcgt gctggtcctt cctgcacctt 2100
aggctggggg tggggggcct gccggcgcat tctccacgat tgagcgcaca ggcctgaagt 2160
ctggacaacc cgcagaaccg aagctccgag cagcgggtcg gtggcgagta gtggggtcgg 2220
tggcgagcag ttggtggtgg gccgcggccg ccactacctc gaggacattt ccctcccgga 2280
gccagctctc ctagaaaccc cgcggcggcc gccgcagcca agtgtttatg gcccgcggtc 2340
gggtgggatc ctagccctgt ctcctctcct gggaaggagt gagggtggga cgtgacttag 2400
acacctacaa atctatttac caaagaggag cccgggactg agggaaaagg ccaaagagtg 2460

tgagtgcatg cggactgggg gttcagggga agaggacgag gaggaggaag atgaggtcga 2520
tttcctgatt taaaaaatcg tccaagcccc gtggtccagc ttaaggtcct cggttacatg 2580
cgccgctcag agcaggtcac tttctgcctt ccacgtcctc cttcaaggaa gccccatgtg 2640
ggtagctttc aatatcgcag gttcttactc ctctgcctct ataagctcaa acccaccaac 2700
gatcgggcaa gtaaaccccc tccctcgccg acttcggaac tggcgagagt tcagcgcaga 2760
tgggcctgtg gggagggggc aagatagatg aggggagcgg gcatggtgcg gggtgacccc 2820
ttggagagag gaaaaaggcc acaagagggg ctgccaccgc cactaacgga gatggccctg 2880
gtagagacct ttgggggtct ggaacctctg gactccccat gctctaactc ccacactctg 2940
ctatcagaaa cttaaacttg aggattttct ctgtttttca ctcgcaataa aytcagagca 3000
aacaaaaaaa aaaaaaaaaa aaaactcgag 3030

<210> 334
<211> 2417
<212> DNA
<213> Homo sapien

<400> 334
   ggcggccgct ctagagctag tgggatcccc cgggctgcac gaattcggca cgagtgagtt 60
   ggagttttac ctgtattgtt ttaatttcaa caagcctgag gactagccac aaatgtaccc 120
   agtttacaaa tgaggaaaca ggtgcaaaaa ggttgttacc tgtcaaaggt cgtatgtggc 180
   agagccaaga tttgagccca gttatgtctg atgaacttag cctatgctct ttaaacttct 240
   gaatgctgac cattgaggat atctaaactt agatcaattg cattttccct ccaagactat 300
   ttacttatca atacaataat accaccttta ccaatctatt gttttgatac gagactcaaa 360
   tatgccagat atatgtaaaa gcaacctaca agctctctaa tcatgctcac ctaaaagatt 420
   cccgggatct aataggctca aagaaacttc ttctagaaat ataaaagaga aaattggatt 480
   atgcaaaaat tcattattaa tttttttcat ccatccttta attcagcaaa catttatctg 540
   ttgttgactt tatgcagtat ggccttttaa ggattggggg acaggtgaag aacggggtgc 600
   cagaatgcat cctcctacta atgaggtcag tacacatttg cattttaaaa tgccctgtcc 660
   agctgggcat ggtggatcat gcctgtaatc tcaacattgg aaggccaagg caggaggatt 720
   gcttcagccc aggagttcaa gaccagcctg ggcaacatag aaagacccca tctctcaatc 780
   aatcaatcaa tgccctgtct ttgaaaataa aactctttaa gaaaggttta atgggcaggg 840
   tgtggtagct catgcctata atacagcact ttgggaggct gaggcaggag gatcacttta 900
   gcccagaagt tcaagaccag cctgggcaac aagtgacacc tcatctcaat tttttaataa 960
   aatgaataca tacataagga aagataaaaa gaaaagttta atgaaagaat acagtataaa 1020
   acaaatctct tggacctaaa agtatttttg ttcaagccaa atattgtgaa tcacctctct 1080
   gtgttgagga tacagaatat ctaagcccag gaaactgagc agaaagttca tgtactaact 1140
   aatcaacccg aggcaaggca aaaatgagac taactaatca atccgaggca aggggcaaat 1200
   tagacggaac ctgactctgg tctattaagc gacaactttc cctctgttgt attttctttt 1260
   tattcaatgt aaaaggataa aaactctcta aaactaaaaa caatgtttgt caggagttac 1320
   aaaccatgac caactaatta tggggaatca taaaatatga ctgtatgaga tcttgatggt 1380
   ttacaaagtg tacccactgt taatcacttt aaacattaat gaacttaaaa atgaatttac 1440
   ggagattgga atgtttcttt cctgttgtat tagttggctc aggctgccat aacaaaatac 1500
   cacagactgg gaggcttaag taacagaaat tcatttctca cagttctggg ggctggaagt 1560
   ccacgatcaa ggtgcaggaa aggcaggctt cattctgagg cccctctctt ggctcacatg 1620
   tggccaccct cccactgcgt gctcacatga cctctttgtg ctcctggaaa gagggtgtgg 1680
   gggacagagg gaaagagaag gagagggaac tctctggtgt ctcgtctttc aaggacccta 1740
   acctgggcca ctttggccca ggcactgtgg ggtggggggt tgtggctgct ctgctctgag 1800
   tggccaagat aaagcaacag aaaaatgtcc aaagctgtgc agcaaagaca agccaccgaa 1860
   cagggatctg ctcatcagtg tggggacctc caagtcggcc accctggagg caagccccca 1920
   cagagcccat gcaaggtggc agcagcagaa gaagggaatt gtccctgtcc ttggcacatt 1980
   cctcaccgac ctggtgatgc tggacactgc gatgaatggt aatgtggatg agaatatgat 2040
   ggactcccag aaaaggagac ccagctgctc aggtggctgc aaatcattac agccttcatc 2100
   ctggggagga actggggccc tggttctggg tcagagagca gcccagtgag ggtgagagct 2160
   acagcctgtc ctgccagctg gatccccagt cccggtcaac cagtaatcaa ggctgagcag 2220
   atcaggcttc ccggagctgg tcttgggaag ccagccctgg ggtgagttgg ctcctgctgt 2280
   ggtactgaga caatattgtc ataaattcaa tgcgcccttg tatccctttt tcttttttat 2340
   ctgtctacat ctataatcac tatgcatact agtctttgtt agtgtttcta ttcmacttaa 2400
   tagagatatg ttatact 2417

<210> 335
<211> 2984
<212> DNA

<213> Homo sapien

<400> 335
atccctcctt ccccactctc ctttccagaa ggcacttggg gtcttatctg ttggactctg 60
aaaacacttc aggcgccctt ccaaggcttc cccaaacccc taagcagccg cagaagcgct 120
cccgagctgc cttctcccac actcaggtga tcgagttgga gaggaagttc agccatcaga 180
agtacctgtc ggcccctgaa cgggcccacc tggccaagaa cctcaagctc acggagaccc 240
aagtgaagat atggttccag aacagacgct ataagactaa gcgaaagcag ctctcctcgg 300
agctgggaga cttggagaag cactcctctt tgccggccct gaaagaggag gccttctccc 360
gggcctccct ggtctccgtg tataacagct atccttacta cccatacctg tactgcgtgg 420
gcagctggag cccagctttt tggtaatgcc agctcaggtg acaaccatta tgatcaaaaa 480
ctgccttccc cagggtgtct ctatgaaaag cacaaggggc caaggtcagg gagcaagagg 540
tgtgcacacc aaagctattg gagatttgcg tggaaatctc asattcttca ctggtgagac 600
aatgaaacaa cagagacagt gaaagtttta atacctaagt cattccccca gtgcatactg 660
taggtcattt tttttgcttc tggctacctg tttgaagggg agagagggaa aatcaagtgg 720
tattttccag cactttgtat gattttggat gagctgtaca cccaaggatt ctgttctgca 780
actccatcct cctgtgtcac tgaatatcaa ctctgaaaga gcaaacctaa caggagaaag 840
gacaaccagg atgaggatgt caccaactga attaaactta agtccagaag cctcctgttg 900
gccttggaat atggccaagg ctctctctgt ccctgtaaaa gagaggggca aatagagagt 960
ctccaagaga acgccctcat gctcagcaca tatttgcatg ggagggggag atgggtggga 1020
ggagatgaaa atatcagctt ttcttattcc tttttattcc ttttaaaatg gtatgccaac 1080
ttaagtattt acagggtggc ccaaatagaa caagatgcac tcgctgtgat tttaagacaa 1140
gctgtataaa cagaactcca ctgcaagagg gggggccggg ccaggagaat ctccgcttgt 1200
ccaagacagg ggcctaagga gggtctccac actgctgcta ggggctgttg cattttttta 1260
ttagtagaaa gtggaaaggc ctcttctcaa cttttttccc ttgggctgga gaatttagaa 1320
tcagaagttt cctggagttt tcaggctatc atatatactg tatcctgaaa ggcaacataa 1380
ttcttccttc cctcctttta aaattttgtg ttcctttttg cagcaattac tcactaaagg 1440
gcttcatttt agtccagatt tttagtctgg ctgcacctaa cttatgcctc gcttatttag 1500
cccgagatct ggtctttttt tttttttttt tttttccgtc tccccaaagc tttatctgtc 1560
ttgacttttt aaaaaagttt gggggcagat tctgaattgg ctaaaagaca tgcattttta 1620
aaactagcaa ctcttatttc tttcctttaa aaatacatag cattaaatcc caaatcctat 1680
ttaaagacct gacagcttga gaaggtcact actgcattta taggaccttc tggtggttct 1740
gctgttacgt ttgaagtctg acaatccttg agaatctttg catgcagagg aggtaagagg 1800
tattggattt tcacagagga agaacacagc gcagaatgaa gggccaggct tactgagctg 1860
tccagtggag ggctcatggg tgggacatgg aaaagaaggc agcctaggcc ctggggagcc 1920
cagtccactg agcaagcaag ggactgagtg agccttttgc aggaaaaggc taagaaaaag 1980
gaaaaccatt ctaaaacaca acaagaaact gtccaaatgc tttgggaact gtgtttattg 2040
cctataatgg gtccccaaaa tgggtaacct agacttcaga gagaatgagc agagagcaaa 2100
ggagaaatct ggctgtcctt ccattttcat tctgttatct caggtgagct ggtagagggg 2160
agacattaga aaaaaatgaa acaacaaaac aattactaat gaggtacgct gaggcctggg 2220
agtctcttga ctccactact taattccgtt tagtgagaaa cctttcaatt ttcttttatt 2280
agaagggcca gcttactgtt ggtggcaaaa ttgccaacat aagttaatag aaagttggcc 2340
aatttcaccc cattttctgt ggtttgggct ccacattgca atgttcaatg ccacgtgctg 2400
ctgacaccga ccggagtact agccagcaca aaaggcaggg tagcctgaat tgctttctgc 2460
tctttacatt tcttttaaaa taagcattta gtgctcagtc cctactgagt actctttctc 2520
tcccctcctc tgaatttaat tctttcaact tgcaatttgc aaggattaca catttcactg 2580
tgatgtatat tgtgttgcaa aaaaaaaaaa aagtgtcttt gtttaaaatt acttggtttg 2640
tgaatccatc ttgctttttc cccattggaa ctagtcatta acccatctct gaactggtag 2700
aaaaacatct gaagagctag tctatcagca tctgacaggt gaattggatg gttctcagaa 2760
ccatttcacc cagacagcct gtttctatcc tgtttaataa attagtttgg gttctctaca 2820
tgcataacaa accctgctcc aatctgtcac ataaaagtct gtgacttgaa gtttagtcag 2880
cacccccacc aaactttatt tttctatgtg ttttttgcaa catatgagtg ttttgaaaat 2940
aaagtaccca tgtctttatt agaaaaaaaa aaaaaaaaaa aaaa 2984

<210> 336
<211> 147
<212> PRT
<213> Homo sapien

<400> 336
Pro Ser Phe Pro Thr Leu Leu Ser Arg Arg His Leu Gly Ser Tyr Leu
1               5                   10                  15

Leu Asp Ser Glu Asn Thr Ser Gly Ala Leu Pro Arg Leu Pro Gln Thr
            20                  25                  30
Pro Lys Gln Pro Gln Lys Arg Ser Arg Ala Ala Phe Ser His Thr Gln
        35                  40                  45
Val Ile Glu Leu Glu Arg Lys Phe Ser His Gln Lys Tyr Leu Ser Ala
    50                  55                  60
Pro Glu Arg Ala His Leu Ala Lys Asn Leu Lys Leu Thr Glu Thr Gln
65                  70                  75                  80
Val Lys Ile Trp Phe Gln Asn Arg Arg Tyr Lys Thr Lys Arg Lys Gln
                85                  90                  95
Leu Ser Ser Glu Leu Gly Asp Leu Glu Lys His Ser Ser Leu Pro Ala
            100                 105                 110
Leu Lys Glu Glu Ala Phe Ser Arg Ala Ser Leu Val Ser Val Tyr Asn
        115                 120                 125
Ser Tyr Pro Tyr Tyr Pro Tyr Leu Tyr Cys Val Gly Ser Trp Ser Pro
    130                 135                 140
Ala Phe Trp
145

<210> 337
<211> 9
<212> PRT
<213> Homo sapien

<400> 337
Ala Leu Thr Gly Phe Thr Phe Ser Ala
 1               5

<210> 338
<211> 9
<212> PRT
<213> Homo sapien

<400> 338
Leu Leu Ala Asn Asp Leu Met Leu Ile
 1               5

<210> 339
<211> 318
<212> PRT
<213> Homo sapien

<400> 339
Met Val Glu Leu Met Phe Pro Leu Leu Leu Leu Leu Leu Pro Phe Leu
 1               5                  10                  15
Leu Tyr Met Ala Ala Pro Gln Ile Arg Lys Met Leu Ser Ser Gly Val
            20                  25                  30
Cys Thr Ser Thr Val Gln Leu Pro Gly Lys Val Val Val Val Thr Gly
        35                  40                  45
Ala Asn Thr Gly Ile Gly Lys Glu Thr Ala Lys Glu Leu Ala Gln Arg
    50                  55                  60
Gly Ala Arg Val Tyr Leu Ala Cys Arg Asp Val Glu Lys Gly Glu Leu
65                  70                  75                  80
Val Ala Lys Glu Ile Gln Thr Thr Thr Gly Asn Gln Gln Val Leu Val
                85                  90                  95
Arg Lys Leu Asp Leu Ser Asp Thr Lys Ser Ile Arg Ala Phe Ala Lys
            100                 105                 110
Gly Phe Leu Ala Glu Glu Lys His Leu His Val Leu Ile Asn Asn Ala
        115                 120                 125
Gly Val Met Met Cys Pro Tyr Ser Lys Thr Ala Asp Gly Phe Glu Met
    130                 135                 140

His Ile Gly Val Asn His Leu Gly His Phe Leu Leu Thr His Leu Leu
145                 150                 155                 160
Leu Glu Lys Leu Lys Glu Ser Ala Pro Ser Arg Ile Val Asn Val Ser
                165                 170                 175
Ser Leu Ala His His Leu Gly Arg Ile His Phe His Asn Leu Gln Gly
            180                 185                 190
Glu Lys Phe Tyr Asn Ala Gly Leu Ala Tyr Cys His Ser Lys Leu Ala
        195                 200                 205
Asn Ile Leu Phe Thr Gln Glu Leu Ala Arg Arg Leu Lys Gly Ser Gly
    210                 215                 220
Val Thr Thr Tyr Ser Val His Pro Gly Thr Val Gln Ser Glu Leu Val
225                 230                 235                 240
Arg His Ser Ser Phe Met Arg Trp Met Trp Trp Leu Phe Ser Phe Phe
                245                 250                 255
Ile Lys Thr Pro Gln Gln Gly Ala Gln Thr Ser Leu His Cys Ala Leu
            260                 265                 270
Thr Glu Gly Leu Glu Ile Leu Ser Gly Asn His Phe Ser Asp Cys His
        275                 280                 285
Val Ala Trp Val Ser Ala Gln Ala Arg Asn Glu Thr Ile Ala Arg Arg
    290                 295                 300
Leu Trp Asp Val Ser Cys Asp Leu Leu Gly Leu Pro Ile Asp
305                 310                 315

<210> 340
<211> 483
<212> DNA
<213> Homo sapien

<400> 340
gccgaggtct gccttcacac ggaggacacg agactgcttc ctcaagggct cctgcctgcc      60
tggacactgg tgggaggcgc tgtttagttg gctgttttca gaggggtctt tcggagggac     120
ctcctgctgc aggctggagt gtctttattc ctggcgggag accgcacatt ccactgctga     180
ggttgtgggg gcggtttatc aggcagtgat aaacataaga tgtcatttcc ttgactccgg     240
ccttcaattt tctctttggc tgacgacgga gtccgtggtg tcccgatgta actgacccct     300
gctccaaacg tgacatcact gatgctcttc tcgggggtgc tgatggcccg cttggtcacg     360
tgctcaatct cgccattcga ctcttgctcc aaactgtatg aagacacctg actgcacgtt     420
ttttctgggc ttccagaatt taaagtgaaa ggcagcactc ctaagctccg actccgatgc     480
ctg                                                                   483

<210> 341
<211> 344
<212> DNA
<213> Homo sapien

<400> 341
ctgctgctga gtcacagatt tcattataaa tagcctccct aaggaaaata cactgaatgc      60
tatttttact aaccattcta tttttataga aatagctgag agtttctaaa ccaactctct     120
gctgccttac aagtattaaa tattttactt ctttccataa agagtagctc aaaatatgca     180
attaatttaa taatttctga tgatggtttt atctgcagta atatgtatat catctattag     240
aatttactta atgaaaaact gaagagaaca aaatttgtaa ccactagcac ttaagtactc     300
ctgattctta acattgtctt taatgaccac aagacaacca acag                      344

<210> 342
<211> 592
<212> DNA
<213> Homo sapien

<400> 342
acagcaaaaa agaaactgag aagcccaaty tgctttcttg ttaacatcca cttatccaac      60
caatgtggaa acttcttata cttggttcca ttatgaagtt ggacaattgc tgctatcaca     120
cctggcaggt aaaccaatgc caagagagtg atggaaacca ttggcaagac tttgttgatg     180
accaggattg gaattttata aaaatattgt tgatgggaag ttgctaaagg gtgaattact     240

tccctcagaa gagtgtaaag aaaagtcaga gatgctataa tagcagctat tttaattggc 300
aagtgccact gtggaaagag ttcctgtgtg tgctgaagtt ctgaagggca gtcaaattca 360
tcagcatggg ctgtttggtg caaatgcaaa agcacaggtc tttttagcat gctggtctct 420
cccgtgtcct tatgcaaata atcgtcttct tctaaatttc tcctaggctt cattttccaa 480
agttcttctt ggtttgtgat gtcttttctg ctttccatta attctataaa atagtatggc 540
ttcagccacc cactcttcgc cttagcttga ccgtgagtct cggctgccgc tg 592

<210> 343
<211> 382
<212> DNA
<213> Homo sapien

<400> 343
ttcttgacct cctcctcctt caagctcaaa caccacctcc cttattcagg accggcactt 60
cttaatgttt gtggctttct ctccagcctc tcttaggagg ggtaatggtg gagttggcat 120
cttgtaactc tcctttctcc tttcttcccc tttctctgcc cgcctttccc atcctgctgt 180
agacttcttg attgtcagtc tgtgtcacat ccagtgattg ttttggtttc tgttcccttt 240
ctgactgccc aaggggctca gaaccccagc aatcccttcc tttcactacc ttcttttttg 300
ggggtagttg gaagggactg aaattgtggg gggaaggtag gaggcacatc aataaagagg 360
aaaccaccaa gctgaaaaaa aa 382

<210> 344
<211> 536
<212> DNA
<213> Homo sapien

<400> 344
ctgggcctga agctgtaggg taaatcagag gcaggcttct gagtgatgag agtcctgaga 60
caataggcca cataaacttg gctggatgga acctcacaat aaggtggtca cctcttgttt 120
gtttaggggg atgccaagga taaggccagc tcagttatat gaagagaagc agaacaaaca 180
agtctttcag agaaatggat gcaatcagag tgggatcccg gtcacatcaa ggtcacactc 240
caccttcatg tgcctgaatg gttgccaggt cagaaaaatc caccccttac gagtgcggct 300
tcgaccctat atcccccgcc cgcgtccctt tctccataaa attcttctta gtagctatta 360
ccttcttatt atttgatcta gaaattgccc tccttttacc cctaccatga gccctacaaa 420
caactaacct gccactaata gttatgtcat ccctcttatt aatcatcatc ctagccctaa 480
gtctggccta tgagtgacta caaaaaggat tagactgagc cgaataacaa aaaaaa 536

<210> 345
<211> 251
<212> DNA
<213> Homo sapien

<400> 345
accttttgag gtctctctca ccacctccac agccaccgtc accgtgggat gtgctggatg 60
tgaatgaagc ccccatcttt gtgcctcctg aaaagagagt ggaagtgtcc gaggactttg 120
gcgtgggcca ggaaatcaca tcctacactg cccaggagcc agacacattt atggaacaga 180
aaataacata tcggatttgg agagacactg ccaactggct ggagattaat ccggacactg 240
gtgccatttc c 251

<210> 346
<211> 282
<212> DNA
<213> Homo sapien

<220>
<221> misc_feature
<222> (1)...(282)
<223> n = A,T,C or G

<400> 346
cgcgtctctg acactgtgat catgacaggg gttcaaacag aaagtgcctg ggccctcctt 60
ctaagtcttg ttaccaaaaa aaggaaaaag aaaagatctt ctcagttaca aattctggga 120

agggagacta tacctggctc ttgccctaag tgagaggtct tccctcccgc accaaaaaat 180
agaaaggctt tctatttcac tggcccaggt agggggaagg agagtaactt tgagtctgtg 240
ggtctcattt cccaaggtgc cttcaatgct catnaaaacc aa 282

<210> 347
<211> 201
<212> DNA
<213> Homo sapien

<220>
<221> misc_feature
<222> (1)...(201)
<223> n = A,T,C or G

<400> 347
   acacacataa tattataaaa tgccatctaa ttggaaggag ctttctatca ttgcaagtca 60
   taaatataac ttttaaaana ntactancag cttttaccta ngctcctaaa tgcttgtaaa 120
   tctgagactg actggaccca cccagaccca gggcaaagat acatgttacc atatcatctt 180
   tataaagaat ttttttttgt c 201

<210> 348
<211> 251
<212> DNA
<213> Homo sapien

<400> 348
ctgttaatca caacatttgt gcatcacttg tgccaagtga gaaaatgttc taaaatcaca 60
agagagaaca gtgccagaat gaaactgacc ctaagtccca ggtgcccctg ggcaggcaga 120
aggagacact cccagcatgg aggagggttt atcttttcat cctaggtcag gtctacaatg 180
ggggaaggtt ttattataga actcccaaca gcccacctca ctcctgccac ccacccgatg 240
gccctgcctc c 251

<210> 349
<211> 251
<212> DNA
<213> Homo sapien

<400> 349
taaaaatcaa gccatttaat tgtatctttg aaggtaaaca atatatggga gctggatcac 60
aacccctgag gatgccagag ctatgggtcc agaacatggt gtggtattat caacagagtt 120
cagaagggtc tgaactctac gtgttaccag agaacataat gcaattcatg cattccactt 180
agcaattttg taaaatacca gaaacagacc ccaagagtct ttcaagatga ggaaaattca 240
actcctggtt t 251

<210> 350
<211> 908
<212> DNA
<213> Homo sapien

<400> 350
ctggacactt tgcgagggct tttgctggct gctgctgctg cccgtcatgc tactcatcgt 60
agcccgcccg gtgaagctcg ctgctttccc tacctcctta agtgactgcc aaacgcccac 120
cggctggaat tgctctggtt atgatgacag agaaaatgat ctcttcctct gtgacaccaa 180
cacctgtaaa tttgatgggg aatgtttaag aattggagac actgtgactt gcgtctgtca 240
gttcaagtgc aacaatgact atgtgcctgt gtgtggctcc aatggggaga gctaccagaa 300
tgagtgttac ctgcgacagg ctgcatgcaa acagcagagt gagatacttg tggtgtcaga 360
aggatcatgt gccacagtcc atgaaggctc tggagaaact agtcaaaagg agacatccac 420
ctgtgatatt tgccagtttg gtgcagaatg tgacgaagat gccgaggatg tctggtgtgt 480
gtgtaatatt gactgttctc aaaccaactt caatcccctc tgcgcttctg atgggaaatc 540
ttatgataat gcatgccaaa tcaaagaagc atcgtgtcag aaacaggaga aaattgaagt 600
catgtctttg ggtcgatgtc aagataacac aactacaact actaagtctg aagatgggca 660
ttatgcaaga acagattatg cagagaatgc taacaaatta gaagaaagtg ccagagaaca 720

ccacatacct tgtccggaac attacaatgg cttctgcatg catgggaagt gtgagcattc 780
tatcaatatg caggagccat cttgcaggtg tgatgctggt tatactggac aacactgtga 840
aaaaaaggac tacagtgttc tatacgttgt tcccggtcct gtacgatttc agtatgtctt 900
aatcgcag 908

<210> 351
<211> 472
<212> DNA
<213> Homo sapien

<400> 351
ccagttattt gcaagtggta agagcctatt taccataaat aatactaaga accaactcaa 60
gtcaaacctt aatgccattg ttattgtgaa ttaggattaa gtagtaattt tcaaaattca 120
cattaacttg attttaaaat cagwtttgyg agtcatttac cacaagctaa atgtgtacac 180
tatgataaaa acaaccattg tattcctgtt tttctaaaca gtcctaattt ctaacactgt 240
atatatcctt cgacatcaat gaactttgtt ttcttttact ccagtaataa agtaggcaca 300
gatctgtcca caacaaactt gccctctcat gccttgcctc tcaccatgct ctgctccagg 360
tcagccccct tttggcctgt ttgttttgtc aaaaacctaa tctgcttctt gcttttcttg 420
gtaatatata tttagggaag atgttgcttt gcccacacac gaagcaaagt aa 472

<210> 352
<211> 251
<212> DNA
<213> Homo sapien

<400> 352
ctcaaagcta atctctcggg aatcaaacca gaaaagggca aggatcttag gcatggtgga 60
tgtggataag gccaggtcaa tggctgcaag catgcagaga aagaggtaca tcggagcgtg 120
caggctgcgt tccgtcctta cgatgaagac cacgatgcag tttccaaaca ttgccactac 180
atacatggaa aggaggggga agccaaccca gaaatgggct ttctctaatc ctgggatacc 240
aataagcaca a 251

<210> 353
<211> 436
<212> DNA
<213> Homo sapien

<400> 353
tttttttttt tttttttttt tttttacaa caatgcagtc atttatttat tgagtatgtg 60
cacattatgg tattattact atactgatta tatttatcat gtgacttcta attaraaaat 120
gtatccaaaa gcaaaacagc agatatacaa aattaaagag acagaagata gacattaaca 180
gataaggcaa cttatacatt gacaatccaa atccaataca tttaaacatt tgggaaatga 240
gggggacaaa tggaagccar atcaaatttg tgtaaaacta ttcagtatgt ttcccttgct 300
tcatgtctga raaggctctc ccttcaatgg ggatgacaaa ctccaaatgc cacacaaatg 360
ttaacagaat actagattca cactggaacg gggtaaaga agaaattatt ttctataaaa 420
gggctcctaa tgtagt 436

<210> 354
<211> 854
<212> DNA
<213> Homo sapien

<400> 354
ccttttctag ttcaccagtt ttctgcaagg atgctggtta gggagtgtct gcaggaggag 60
caagtctgaa accaaatcta ggaaacatag gaaacgagcc aggcacaggg ctggtgggcc 120
atcagggacc accctttggg ttgatatttt gcttaatctg catcttttga gtaagatcat 180
ctggcagtag aagctgttct ccaggtacat ttctctagct catgtacaaa aacatcctga 240
aggactttgt caggtgcctt gctaaaagcc agatgcgttc ggcacttcct tggtctgagg 300
ttaattgcac acctacaggc actgggctca tgctttcaag tattttgtcc tcactttagg 360
gtgagtgaaa gatccccatt ataggagcac ttgggagaga tcatataaaa gctgactctt 420
gagtacatgc agtaatgggg tagatgtgtg tggtgtgtct tcattcctgc aagggtgctt 480
gttagggagt gtttccagga ggaacaagtc tgaaaccaat catgaaataa atggtaggtg 540

tgaactggaa aactaattca aaagagagat cgtgatatca gtgtggttga tacaccttgg 600
caatatggaa ggctctaatt tgcccatatt tgaaataata attcagcttt ttgtaataca 660
aaataacaaa ggattgagaa tcatggtgtc taatgtataa aagacccagg aaacataaat 720
atatcaactg cataaatgta aaatgcatgt gacccaagaa ggccccaaag tggcagacaa 780
cattgtaccc attttccctt ccaaaatgtg agcggcgggc ctgctgcttt caaggctgtc 840
acacgggatg tcag 854

<210> 355
<211> 676
<212> DNA
<213> Homo sapien

<400> 355
gaaattaagt atgagctaaa ttccctgtta aaacctctag gggtgacaga tctcttcaac 60
caggtcaaag ctgatctttc tggaatgtca ccaaccaagg gcctatattt atcaaaagcc 120
atccacaagt catacctgga tgtcagcgaa gagggcacgg aggcagcagc agccactggg 180
gacagcatcg ctgtaaaaag cctaccaatg agagctcagt tcaaggcgaa ccaccccttc 240
ctgttcttta taaggcacac tcataccaac acgatcctat tctgtggcaa gcttgcctct 300
ccctaatcag atggggttga gtaaggctca gagttgcaga tgaggtgcag agacaatcct 360
gtgactttcc cacggccaaa aagctgttca cacctcacgc acctctgtgc ctcagtttgc 420
tcatctgcaa aataggtcta ggatttcttc caaccatttc atgagttgtg aagctaaggc 480
tttgttaatc atggaaaaag gtagacttat gcagaaagcc tttctggctt tcttatctgt 540
ggtgtctcat ttgagtgctg tccagtgaca tgatcaagtc aatgagtaaa attttaaggg 600
attagatttt cttgacttgt atgtatctgt gagatcttga ataagtgacc tgacatctct 660
gcttaaagaa aaccag 676

<210> 356
<211> 574
<212> DNA
<213> Homo sapien

<400> 356
tttttttttt tttttcagga aaacattctc ttactttatt tgcatctcag caaaggttct 60
catgtggcac ctgactggca tcaaaccaaa gttcgtaggc caacaaagat gggccactca 120
caagcttccc atttgtagat ctcagtgcct atgagtatct gacacctgtt cctctcttca 180
gtctcttagg gaggcttaaa tctgtctcag gtgtgctaag agtgccagcc caaggkggtc 240
aaaagtccac aaaactgcag tctttgctgg gatagtaagc caagcagtgc ctggacagca 300
gagttctttt cttgggcaac agataaccag acaggactct aatcgtgctc ttattcaaca 360
ttcttctgtc tctgcctaga ctggaataaa aagccaatct ctctcgtggc acagggaagg 420
agatacaagc tcgtttacat gtgatagatc taacaaaggc atctaccgaa gtctggtctg 480
gatagacggc acagggagct cttaggtcag cgctgctggt tggaggacat tcctgagtcc 540
agctttgcag cctttgtgca acagtacttt ccca 574

<210> 357
<211> 393
<212> DNA
<213> Homo sapien

<400> 357
tttttttttt tttttttttt tttttttttt tacagaatat aratgcttta tcactgkact 60
taatatggkg kcttgttcac tatacttaaa aatgcaccac tcataaatat ttaattcagc 120
aagccacaac caaracttga ttttatcaac aaaaacccct aaatataaac ggsaaaaaag 180
atagatataa ttattccagt ttttttaaaa cttaaaarat attccattgc cgaattaara 240
araarataag tgttatatgg aaagaagggc attcaagcac actaaaraaa cctgaggkaa 300
gcataatctg tacaaaatta aactgtcctt tttggcattt taacaaattt gcaacgktct 360
tttttttctt tttctgtttt tttttttttt tac 393

<210> 358
<211> 630
<212> DNA
<213> Homo sapien

<400> 358
acagggtaaa caggaggatc cttgctctca cggagcttac attctagcag gaggacaata 60
ttaatgttta taggaaaatg atgagtttat gacaaaggaa gtagatagtg ttttacaaga 120
gcatagagta gggaagctaa tccagcacag ggaggtcaca gagacatccc taaggaagtg 180
gagtttaaac tgagagaagc aagtgcttaa actgaaggat gtgttgaaga agaagggaga 240
gtagaacaat ttgggcagag ggaaccttat agaccctaag gtgggaaggt tcaaagaact 300
gaaagagagc tagaacagct ggagccgttc tccggtgtaa agaggagtca aagagataag 360
attaaagatg tgaagattaa gatcttggtg gcattcaggg attggcactt ctacaagaaa 420
tcactgaagg gagtaatgtg acattacttt tcacttcagg atggccattc taactccagg 480
gggtagactg gactaggtaa gactggaggc aggtagacct cttctaaggc ctgcgatagt 540
gaaagacaaa aataagtggg gaaattcagg ggatagtgaa aatcagtagg acttaatgag 600
caagccagag gttcctccac aacaaccagt 630

<210> 359
<211> 620
<212> DNA
<213> Homo sapien

<400> 359
acagcattcc aaaatataca tctagagact aarrgtaaat gctctatagt gaagaagtaa 60
taattaaaaa atgctactaa tatagaaaat ttataatcag aaaaataaat attcagggag 120
ctcaccagaa gaataaagtg ctctgccagt tattaaagga ttactgctgg tgaattaaat 180
atggcattcc ccaagggaaa tagagagatt cttctggatt atgttcaata tttatttcac 240
aggattaact gttttaggaa cagatataaa gcttcgccac ggaagagatg gacaaagcac 300
aaagacaaca tgatacctta ggaagcaaca ctaccctttc aggcataaaa tttggagaaa 360
tgcaacatta tgcttcatga ataatatgta gaaagaaggt ctgatgaaaa tgacatcctt 420
aatgtaagat aactttataa gaattctggg tcaaataaaa ttctttgaag aaaacatcca 480
aatgtcattg acttatcaaa tactatcttg gcatataacc tatgaaggca aaactaaaca 540
aacaaaaagc tcacaccaaa caaaaccatc aacttatttt gtattctata acatacgaga 600
ctgtaaagat gtgacagtgt 620

<210> 360
<211> 431
<212> DNA
<213> Homo sapien

<400> 360
aaaaaaaaaa agccagaaca acatgtgata gataatatga ttggctgcac acttccagac 60
tgatgaatga tgaacgtgat ggactattgt atggagcaca tcttcagcaa gagggggaaa 120
tactcatcat ttttggccag cagttgtttg atcaccaaac atcatgccag aatactcagc 180
aaaccttctt agctcttgag aagtcaaagt ccgggggaat ttattcctgg caattttaat 240
tggactcctt atgtgagagc agcggctacc cagctggggt ggtggagcga acccgtcact 300
agtggacatg cagtggcaga gctcctggta accacctaga ggaatacaca ggcacatgtg 360
tgatgccaag cgtgacacct gtagcactca aatttgtctt gtttttgtct ttcggtgtgt 420
agattcttag t 431

<210> 361
<211> 351
<212> DNA
<213> Homo sapien

<400> 361
acactgattt ccgatcaaaa gaatcatcat ctttaccttg acttttcagg gaattactga 60
actttcttct cagaagatag ggcacagcca ttgccttggc ctcacttgaa gggtctgcat 120
ttgggtcctc tggtctcttg ccaagtttcc cagccactcg agggagaaat atcgggaggt 180
ttgacttcct ccggggcttt cccgagggct tcaccgtgag ccctgcggcc ctcagggctg 240
caatcctgga ttcaatgtct gaaacctcgc tctctgcctg ctggacttct gaggccgtca 300
ctgccactct gtcctccagc tctgacagct cctcatctgt ggtcctgttg t 351

<210> 362
<211> 463
<212> DNA

<213> Homo sapien

<400> 362
acttcatcag gccataatgg gtgcctcccg tgagaatcca agcacctttg gactgcgcga 60
tgtagatgag ccggctgaag atcttgcgca tgcgcggctt cagggcgaag ttcttggcgc 120
ccccggtcac agaaatgacc aggttgggtg ttttcaggtg ccagtgctgg gtcagcagct 180
cgtaaaggat ttccgcgtcc gtgtcgcagg acagacgtat atacttccct ttcttcccca 240
gtgtctcaaa ctgaatatcc ccaaaggcgt cggtaggaaa ttccttggtg tgtttcttgt 300
agttccattt ctcactttgg ttgatctggg tgccttccat gtgctggctc tgggcatagc 360
cacacttgca cacattctcc ctgataagca cgatggtgtg gacaggaagg aaggatttca 420
ttgagcctgc ttatggaaac tggtattgtt agcttaaata gac 463

<210> 363
<211> 653
<212> DNA
<213> Homo sapien

<220>
<221> misc_feature
<222> (1)...(653)
<223> n = A,T,C or G

<400> 363
acccccgagt ncctgnctgg catactgnga acgaccaacg acacacccaa gctcggcctc 60
ctcttggnga ttctgggtga catcttcatg aatggcaacc gtgccagwga ggctgtcctc 120
tgggaggcac tacgcaagat gggactgcgt cctggggtga gacatcctct ccttggagat 180
ctaacgaaac ttctcaccta tgagttgtaa agcagaaata cctgnactac agacgagtgc 240
ccaacagcaa ccccccggaa gtatgagttc ctctrgggcc tccgttccta ccatgagasc 300
tagcaagatg naagtgttga gantcattgc agaggttcag aaaagagacc cntcgtgact 360
ggtctgcaca gttcatggag gctgcagatg aggccttgga tgctctggat gctgctgcag 420
ctgaggccga agcccgggct gaagcaagaa cccgcatggg aattggagat gaggctgtgt 480
ntgggccctg gagctgggat gacattgagt ttgagctgct gacctgggat gaggaaggag 540
attttggaga tccntggtcc agaattccat ttaccttctg ggccagatac caccagaatg 600
cccgctccag attccctcag acctttgccg gtcccattat tggtcstggt ggt 653

<210> 364
<211> 401
<212> DNA
<213> Homo sapien

<400> 364
actagaggaa agacgttaaa ccactctact accacttgtg gaactctcaa agggtaaatg 60
acaaagccaa tgaatgactc taaaaacaat atttacattt aatggtttgt agacaataaa 120
aaaacaaggt ggatagatct agaattgtaa cattttaaga aaaccatagc atttgacaga 180
tgagaaagct caattataga tgcaaagtta taactaaact actatagtag taaagaaata 240
catttcacac ccttcatata aattcactat cttggcttga ggcactccat aaaatgtatc 300
acgtgcatag taaatcttta tatttgctat ggcgttgcac tagaggactt ggactgcaac 360
aagtggatgc gcggaaaatg aaatcttctt caatagccca g 401

<210> 365
<211> 356
<212> DNA
<213> Homo sapien

<400> 365
ccagtgtcat atttgggctt aaaatttcaa gaagggcact tcaaatggct ttgcatttgc 60
atgtttcagt gctagagcgt aggaatagac cctggcgtcc actgtgagat gttcttcagc 120
taccagagca tcaagtctct gcagcaggtc attcttgggt aaagaaatga cttccacaaa 180
ctctccatcc cctggctttg gcttcggcct tgcgttttcg gcatcatctc cgttaatggt 240
gactgtcacg atgtgtatag tacagtttga caagcctggg tccatacaga ccgctggaga 300
acattcggca atgtcccctt tgtagccagt ttcttcttcg agctcccgga gagcag 356

<210> 366
<211> 1851
<212> DNA
<213> Homo sapien

<400> 366
tcatcaccat tgccagcagc ggcaccgtta gtcaggtttt ctgggaatcc cacatgagta 60
cttccgtgtt cttcattctt cttcaatagc cataaatctt ctagctctgg ctggctgttt 120
tcacttcctt taagcctttg tgactcttcc tctgatgtca gctttaagtc ttgttctgga 180
ttgctgtttt cagaagagat ttttaacatc tgtttttctt tgtagtcaga aagtaactgg 240
caaattacat gatgatgact agaaacagca tactctctgg ccgtctttcc agatcttgag 300
aagatacatc aacattttgc tcaagtagag ggctgactat acttgctgat ccacaacata 360
cagcaagtat gagagcagtt cttccatatc tatccagcgc atttaaattc gcttttttct 420
tgattaaaaa tttcaccact tgctgttttt gctcatgtat accaagtagc agtggtgtga 480
ggccatgctt gttttttgat tcgatatcag caccgtataa gagcagtgct ttggccatta 540
atttatcttc attgtagaca gcatagtgta gagtggtatt tccatactca tctggaatat 600
ttggatcagt gccatgttcc agcaacatta acgcacattc atcttcctgg cattgtacgg 660
cctttgtcag agctgtcctc tttttgttgt caaggacatt aagttgacat cgtctgtcca 720
gcacgagttt tactacttct gaattcccat tggcagaggc cagatgtaga gcagtcctct 780
tttgcttgtc cctcttgttc acatccgtgt ccctgagcat gacgatgaga tcctttctgg 840
ggactttacc ccaccaggca gctctgtgga gcttgtccag atcttctcca tggacgtggt 900
acctgggatc catgaaggcg ctgtcatcgt agtctcccca agcgaccacg ttgctcttgc 960
cgctcccctg cagcagggga agcagtggca gcaccacttg cacctcttgc tcccaagcgt 1020
cttcacagag gagtcgttgt ggtctccaga agtgcccacg ttgctcttgc cgctccccct 1080
gtccatccag ggaggaagaa atgcaggaaa tgaaagatgc atgcacgatg gtatactcct 1140
cagccatcaa acttctggac agcaggtcac ttccagcaag gtggagaaag ctgtccaccc 1200
acagaggatg agatccagaa accacaatat ccattcacaa acaaacactt ttcagccaga 1260
cacaggtact gaaatcatgt catctgcggc aacatggtgg aacctaccca atcacacatc 1320
aagagatgaa gacactgcag tatatctgca caacgtaata ctcttcatcc ataacaaaat 1380
aatataattt tcctctggag ccatatggat gaactatgaa ggaagaactc cccgaagaag 1440
ccagtcgcag agaagccaca ctgaagctct gtcctcagcc atcagcgcca cggacaggar 1500
tgtgtttctt ccccagtgat gcagcctcaa gttatcccga agctgccgca gcacacggtg 1560
gctcctgaga aacaccccag ctcttccggt ctaacacagg caagtcaata aatgtgataa 1620
tcacataaac agaattaaaa gcaaagtcac ataagcatct caacagacac agaaaaggca 1680
tttgacaaaa tccagcatcc ttgtatttat tgttgcagtt ctcagaggaa atgcttctaa 1740
cttttcccca tttagtatta tgttggctgt gggcttgtca taggtggttt ttattacttt 1800
aaggtatgtc ccttctatgc ctgttttgct gagggtttta attctcgtgc c 1851

<210> 367
<211> 668
<212> DNA
<213> Homo sapien

<400> 367
   cttgagcttc caaataygga agactggccc ttacacasgt caatgttaaa atgaatgcat 60
   ttcagtattt tgaagataaa attrgtagat ctataccttg ttttttgatt cgatatcagc 120
   accrtataag agcagtgctt tggccattaa tttatctttc attrtagaca gcrtagtgya 180
   gagtggtatt tccatactca tctggaatat ttggatcagt gccatgttcc agcaacatta 240
   acgcacattc atcttcctgg cattgtacgg cctgtcagta ttagacccaa aaacaaatta 300
   catatcttag gaattcaaaa taacattcca cagctttcac caactagtta tatttaaagg 360
   agaaaactca tttttatgcc atgtattgaa atcaaaccca cctcatgctg atatagttgg 420
   ctactgcata cctttatcag agctgtcctc tttttgttgt caaggacatt aagttgacat 480
   cgtctgtcca gcaggagttt tactacttct gaattcccat tggcagaggc cagatgtaga 540
   gcagtcctat gagagtgaga agacttttta ggaaattgta gtgcactagc tacagccata 600
   gcaatgattc atgtaactgc aaacactgaa tagcctgcta ttactctgcc ttcaaaaaaa 660
   aaaaaaaa 668

<210> 368
<211> 1512
<212> DNA
<213> Homo sapien

<400> 368
gggtcgccca gggggsgcgt gggctttcct cgggtgggtg tgggttttcc ctgggtgggg 60
tgggctgggc trgaatcccc tgctggggtt ggcaggtttt ggctgggatt gacttttytc 120
ttcaaacaga ttggaaaccc ggagttacct gctagttggt gaaactggtt ggtagacgcg 180
atctgttggc tactactggc ttctcctggc tgttaaaagc agatggtggt tgaggttgat 240
tccatgccgg ctgcttcttc tgtgaagaag ccatttggtc tcaggagcaa gatgggcaag 300
tggtgctgcc gttgcttccc ctgctgcagg gagagcggca agagcaacgt gggcacttct 360
ggagaccacg acgactctgc tatgaagaca ctcaggagca agatgggcaa gtggtgccgc 420
cactgcttcc cctgctgcag ggggagtggc aagagcaacg tgggcgcttc tggagaccac 480
gacgaytctg ctatgaagac actcaggaac aagatgggca agtggtgctg ccactgcttc 540
ccctgctgca gggggagcrg caagagcaag gtgggcgctt ggggagacta cgatgacagt 600
gccttcatgg agcccaggta ccacgtccgt ggagaagatc tggacaagct ccacagagct 660
gcctggtggg gtaaagtccc cagaaaggat ctcatcgtca tgctcaggga cactgacgtg 720
aacaagaagg acaagcaaaa gaggactgct ctacatctgg cctctgccaa tgggaattca 780
gaagtagtaa aactcstgct ggacagacga tgtcaactta atgtccttga caacaaaaag 840
aggacagctc tgayaaaggc cgtacaatgc caggaagatg aatgtgcgtt aatgttgctg 900
gaacatggca ctgatccaaa tattccagat gagtatggaa ataccactct rcactaygct 960
rtctayaatg aagataaatt aatggccaaa gcactgctct tatayggtgc tgatatcgaa 1020
tcaaaaaaca aggtatagat ctactaattt tatcttcaaa atactgaaat gcattcattt 1080
taacattgac gtgtgtaagg gccagtcttc cgtatttgga agctcaagca taacttgaat 1140
gaaaatattt tgaaatgacc taattatctm agactttatt ttaaatattg ttattttcaa 1200
agaagcatta gagggtacag tttttttttt ttaaatgcac ttctggtaaa tacttttgtt 1260
gaaaacactg aatttgtaaa aggtaatact tactattttt caatttttcc ctcctaggat 1320
ttttttcccc taatgaatgt aagatggcaa aatttgccct gaaataggtt ttacatgaaa 1380
actccaagaa aagttaaaca tgtttcagtg aatagagatc ctgctccttt ggcaagttcc 1440
taaaaaacag taatagatac gaggtgatgc gcctgtcagt ggcaaggttt aagatatttc 1500
tgatctcgtg cc 1512

<210> 369
<211> 1853
<212> DNA
<213> Homo sapien

<400> 369
gggtcgccca gggggsgcgt gggctttcct cgggtgggtg tgggttttcc ctgggtgggg 60
tgggctgggc trgaatcccc tgctggggtt ggcaggtttt ggctgggatt gacttttytc 120
ttcaaacaga ttggaaaccc ggagttacct gctagttggt gaaactggtt ggtagacgcg 180
atctgttggc tactactggc ttctcctggc tgttaaaagc agatggtggt tgaggttgat 240
tccatgccgg ctgcttcttc tgtgaagaag ccatttggtc tcaggagcaa gatgggcaag 300
tggtgctgcc gttgcttccc ctgctgcagg gagagcggca agagcaacgt gggcacttct 360
ggagaccacg acgactctgc tatgaagaca ctcaggagca agatgggcaa gtggtgccgc 420
cactgcttcc cctgctgcag ggggagtggc aagagcaacg tgggcgcttc tggagaccac 480
gacgaytctg ctatgaagac actcaggaac aagatgggca agtggtgctg ccactgcttc 540
ccctgctgca gggggagcrg caagagcaag gtgggcgctt ggggagacta cgatgacagy 600
gccttcatgg akcccaggta ccacgtccrt ggagaagatc tggacaagct ccacagagct 660
gcctggtggg gtaaagtccc cagaaaggat ctcatcgtca tgctcaggga cackgaygtg 720
aacaagargg acaagcaaaa gaggactgct ctacatctgg cctctgccaa tgggaattca 780
gaagtagtaa aactcstgct ggacagacga tgtcaactta atgtccttga caacaaaaag 840
aggacagctc tgayaaaggc cgtacaatgc caggaagatg aatgtgcgtt aatgttgctg 900
gaacatggca ctgatccaaa tattccagat gagtatggaa ataccactct rcactaygct 960
rtctayaatg aagataaatt aatggccaaa gcactgctct tatayggtgc tgatatcgaa 1020
tcaaaaaaca agcatggcct cacaccactg ytacttggtr tacatgagca aaaacagcaa 1080
gtsgtgaaat ttttaatyaa gaaaaaagcg aatttaaaat gcrctggata gatatggaag 1140
ractgctctc atacttgctg tatgttgtgg atcagcaagt atagtcagcc ytctacttga 1200
gcaaaatrtt gatgtatctt ctcaagatct ggaaagacgg ccagagagta tgctgtttct 1260
agtcatcatc atgtaatttg ccagttactt tctgactaca aagaaaaaca gatgttaaaa 1320
atctcttctg aaaacagcaa tccagaacaa gacttaaagc tgacatcaga ggaagagtca 1380
caaaggctta aaggaagtga aaacagccag ccagaggcat ggaaactttt aaatttaaac 1440
ttttggttta atgttttttt tttttgcctt aataatatta gatagtccca aatgaaatwa 1500
cctatgagac taggctttga gaatcaatag attctttttt taagaatctt ttggctagga 1560
gcggtgtctc acgcctgtaa ttccagcacc ttgagaggct gaggtgggca gatcacgaga 1620
tcaggagatc gagaccatcc tggctaacac ggtgaaaccc catctctact aaaaatacaa 1680

aaacttagct gggtgtggtg gcgggtgcct gtagtcccag ctactcagga rgctgaggca 1740
ggagaatggc atgaacccgg gaggtggagg ttgcagtgag ccgagatccg ccactacact 1800
ccagcctggg tgacagagca agactctgtc tcaaaaaaaa aaaaaaaaaa aaa 1853

<210> 370
<211> 2184
<212> DNA
<213> Homo sapien

<400> 370
ggcacgagaa ttaaaaccct cagcaaaaca ggcatagaag ggacatacct taaagtaata 60
aaaaccacct atgacaagcc cacagccaac ataatactaa atggggaaaa gttagaagca 120
tttcctctga gaactgcaac aataaataca aggatgctgg attttgtcaa atgccttttc 180
tgtgtctgtt gagatgctta tgtgactttg cttttaattc tgtttatgtg attatcacat 240
ttattgactt gcctgtgtta gaccggaaga gctggggtgt ttctcaggag ccaccgtgtg 300
ctgcggcagc ttcgggataa cttgaggctg catcactggg gaagaaacac aytcctgtcc 360
gtggcgctga tggctgagga cagagcttca gtgtggcttc tctgcgactg gcttcttcgg 420
ggagttcttc cttcatagtt catccatatg gctccagagg aaaattatat tattttgtta 480
tggatgaaga gtattacgtt gtgcagatat actgcagtgt cttcatctct tgatgtgtga 540
ttgggtaggt tccaccatgt tgccgcagat gacatgattt cagtacctgt gtctggctga 600
aaagtgtttg tttgtgaatg gatattgtgg tttctggatc tcatcctctg tgggtggaca 660
gctttctcca ccttgctgga agtgacctgc tgtccagaag tttgatggct gaggagtata 720
ccatcgtgca tgcatctttc atttcctgca tttcttcctc cctggatgga cagggggagc 780
ggcaagagca acgtgggcac ttctggagac cacaacgact cctctgtgaa gacgcttggg 840
agcaagaggt gcaagtggtg ctgccactgc ttcccctgct gcaggggagc ggcaagagca 900
acgtggtcgc ttggggagac tacgatgaca gcgccttcat ggatcccagg taccacgtcc 960
atggagaaga tctggacaag ctccacagag ctgcctggtg gggtaaagtc cccagaaagg 1020
atctcatcgt catgctcagg gacacggatg tgaacaagag ggacaagcaa aagaggactg 1080
ctctacatct ggcctctgcc aatgggaatt cagaagtagt aaaactcgtg ctggacagac 1140
gatgtcaact taatgtcctt gacaacaaaa agaggacagc tctgacaaag gccgtacaat 1200
gccaggaaga tgaatgtgcg ttaatgttgc tggaacatgg cactgatcca aatattccag 1260
atgagtatgg aaataccact ctacactatg ctgtctacaa tgaagataaa ttaatggcca 1320
aagcactgct cttatacggt gctgatatcg aatcaaaaaa caagcatggc ctcacaccac 1380
tgctacttgg tatacatgag caaaaacagc aagtggtgaa atttttaatc aagaaaaaag 1440
cgaatttaaa tgcgctggat agatatggaa gaactgctct catacttgct gtatgttgtg 1500
gatcagcaag tatagtcagc cctctacttg agcaaaatgt tgatgtatct tctcaagatc 1560
tggaaagacg gccagagagt atgctgtttc tagtcatcat catgtaattt gccagttact 1620
ttctgactac aaagaaaaac agatgttaaa aatctcttct gaaaacagca atccagaaca 1680
agacttaaag ctgacatcag aggaagagtc acaaaggctt aaaggaagtg aaaacagcca 1740
gccagaggca tggaaacttt taaatttaaa cttttggttt aatgtttttt ttttttgcct 1800
taataatatt agatagtccc aaatgaaatw acctatgaga ctaggctttg agaatcaata 1860
gattcttttt ttaagaatct tttggctagg agcggtgtct cacgcctgta attccagcac 1920
cttgagaggc tgaggtgggc agatcacgag atcaggagat cgagaccatc ctggctaaca 1980
cggtgaaacc ccatctctac taaaaataca aaaacttagc tgggtgtggt ggcgggtgcc 2040
tgtagtccca gctactcagg argctgaggc aggagaatgg catgaacccg ggaggtggag 2100
gttgcagtga gccgagatcc gccactacac tccagcctgg gtgacagagc aagactctgt 2160
ctcaaaaaaa aaaaaaaaaa aaaa 2184

<210> 371
<211> 1855
<212> DNA
<213> Homo sapien

<220>
<221> misc_feature
<222> (1)...(1855)
<223> n = A,T,C or G

<400> 371
tgcacgcatc ggccagtgtc tgtgccacgt acactgacgc cccctgagat gtgcacgccg 60
cacgcgcacg ttgcacgcgc ggcagcggct tggctggctt gtaacggctt gcacgcgcac 120
gccgcccccg cataaccgtc agactggcct gtaacggctt gcaggcgcac gccgcacgcg 180

cgtaacggct tggctgccct gtaacggctt gcacgtgcat gctgcacgcg cgttaacggc 240
ttggctggca tgtagccgct tggcttggct ttgcattytt tgctkggctk ggcgttgkty 300
tcttggattg acgcttcctc cttggatkga cgtttcctcc ttggatkgac gtttcytyty 360
tcgcgttcct ttgctggact tgacctttty tctgctgggt ttggcattcc tttggggtgg 420
gctgggtgtt ttctccgggg gggktkgccc ttcctggggt gggcgtgggk cgcccccagg 480
gggcgtgggc tttccccggg tgggtgtggg ttttcctggg gtggggtggg ctgtgctggg 540
atccccctgc tggggttggc agggattgac ttttttcttc aaacagattg gaaacccgga 600
gtaacntgct agttggtgaa actggttggt agacgcgatc tgctggtact actgtttctc 660
ctggctgtta aaagcagatg gtggctgagg ttgattcaat gccggctgct tcttctgtga 720
agaagccatt tggtctcagg agcaagatgg gcaagtggtg cgccactgct tcccctgctg 780
cagggggagc ggcaagagca acgtgggcac ttctggagac cacaacgact cctctgtgaa 840
gacgcttggg agcaagaggt gcaagtggtg ctgcccactg cttcccctgc tgcaggggag 900
cggcaagagc aacgtggkcg cttggggaga ctacgatgac agcgccttca tggakcccag 960
gtaccacgtc crtggagaag atctggacaa gctccacaga gctgcctggt ggggtaaagt 1020
ccccagaaag gatctcatcg tcatgctcag ggacactgay gtgaacaaga rggacaagca 1080
aaagaggact gctctacatc tggcctctgc caatgggaat tcagaagtag taaaactcgt 1140
gctggacaga cgatgtcaac ttaatgtcct tgacaacaaa aagaggacag ctctgacaaa 1200
ggccgtacaa tgccaggaag atgaatgtgc gttaatgttg ctggaacatg gcactgatcc 1260
aaatattcca gatgagtatg gaaataccac tctacactat gctgtctaca atgaagataa 1320
attaatggcc aaagcactgc tcttatacgg tgctgatatc gaatcaaaaa acaaggtata 1380
gatctactaa ttttatcttc aaaatactga aatgcattca ttttaacatt gacgtgtgta 1440
agggccagtc ttccgtattt ggaagctcaa gcataacttg aatgaaaata ttttgaaatg 1500
acctaattat ctaagacttt attttaaata ttgttatttt caaagaagca ttagagggta 1560
cagttttttt tttttaaatg cacttctggt aaatactttt gttgaaaaca ctgaatttgt 1620
aaaaggtaat acttactatt tttcaatttt tccctcctag gatttttttc ccctaatgaa 1680
tgtaagatgg caaaatttgc cctgaaatag gttttacatg aaaactccaa gaaaagttaa 1740
acatgtttca gtgaatagag atcctgctcc tttggcaagt tcctaaaaaa cagtaataga 1800
tacgaggtga tgcgcctgtc agtggcaagg tttaagatat ttctgatctc gtgcc 1855

<210> 372
<211> 1059
<212> DNA
<213> Homo sapien

<400> 372
   gcaacgtggg cacttctgga gaccacaacg actcctctgt gaagacgctt gggagcaaga 60
   ggtgcaagtg gtgctgccca ctgcttcccc tgctgcaggg gagcggcaag agcaacgtgg 120
   gcgcttgrgg agactmcgat gacagygcct tcatggagcc caggtaccac gtccgtggag 180
   aagatctgga caagctccac agagctgccc tggtggggta aagtccccag aaaggatctc 240
   atcgtcatgc tcagggacac tgaygtgaac aagarggaca agcaaaagag gactgctcta 300
   catctggcct ctgccaatgg gaattcagaa gtagtaaaac tcstgctgga cagacgatgt 360
   caacttaatg tccttgacaa caaaaagagg acagctctga yaaaggccgt acaatgccag 420
   gaagatgaat gtgcgttaat gttgctggaa catggcactg atccaaatat tccagatgag 480
   tatggaaata ccactctrca ctaygctrtc tayaatgaag ataaattaat ggccaaagca 540
   ctgctcttat ayggtgctga tatcgaatca aaaaacaagg tatagatcta ctaattttat 600
   cttcaaaata ctgaaatgca ttcattttaa cattgacgtg tgtaagggcc agtcttccgt 660
   atttggaagc tcaagcataa cttgaatgaa aatattttga aatgacctaa ttatctaaga 720
   ctttatttta aatattgtta ttttcaaaga agcattagag ggtacagttt ttttttttta 780
   aatgcacttc tggtaaatac ttttgttgaa aacactgaat ttgtaaaagg taatacttac 840
   tatttttcaa tttttccctc ctaggatttt tttcccctaa tgaatgtaag atggcaaaat 900
   ttgccctgaa ataggtttta catgaaaact ccaagaaaag ttaaacatgt ttcagtgaat 960
   agagatcctg ctcctttggc aagttcctaa aaaacagtaa tagatacgag gtgatgcgcc 1020
   tgtcagtggc aaggtttaag atatttctga tctcgtgcc 1059

<210> 373
<211> 1155
<212> DNA
<213> Homo sapien

<400> 373
atggtggttg aggttgattc catgccggct gcctcttctg tgaagaagcc atttggtctc 60
aggagcaaga tgggcaagtg gtgctgccgt tgcttcccct gctgcaggga gagcggcaag 120

agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaaa tgtctcaaga 1140
accagaaata aataa 1155

<210> 374
<211> 2000
<212> DNA
<213> Homo sapien

<400> 374
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaca agacttaaag 1140
ctgacatcag aggaagagtc acaaaggttc aaaggcagtg aaaatagcca gccagagaaa 1200
atgtctcaag aaccagaaat aaataaggat ggtgatagag aggttgaaga agaaatgaag 1260
aagcatgaaa gtaataatgt gggattacta gaaaacctga ctaatggtgt cactgctggc 1320
aatggtgata atggattaat tcctcaaagg aagagcagaa cacctgaaaa tcagcaattt 1380
cctgacaacg aaagtgaaga gtatcacaga atttgcgaat tagtttctga ctacaaagaa 1440
aaacagatgc caaaatactc ttctgaaaac agcaacccag aacaagactt aaagctgaca 1500
tcagaggaag agtcacaaag gcttgagggc agtgaaaatg gccagccaga gctagaaaat 1560
tttatggcta tcgaagaaat gaagaagcac ggaagtactc atgtcggatt cccagaaaac 1620
ctgactaatg gtgccactgc tggcaatggt gatgatggat taattcctcc aaggaagagc 1680
agaacacctg aaagccagca atttcctgac actgagaatg aagagtatca cagtgacgaa 1740
caaaatgata ctcagaagca attttgtgaa gaacagaaca ctggaatatt acacgatgag 1800
attctgattc atgaagaaaa gcagatagaa gtggttgaaa aaatgaattc tgagctttct 1860
cttagttgta agaaagaaaa agacatcttg catgaaaata gtacgttgcg ggaagaaatt 1920
gccatgctaa gactggagct agacacaatg aaacatcaga gccagctaaa aaaaaaaaaa 1980
aaaaaaaaaa aaaaaaaaaa 2000

<210> 375
<211> 2040

<212> DNA
<213> Homo sapien

<400> 375
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaca agacttaaag 1140
ctgacatcag aggaagagtc acaaaggttc aaaggcagtg aaaatagcca gccagagaaa 1200
atgtctcaag aaccagaaat aaataaggat ggtgatagag aggttgaaga agaaatgaag 1260
aagcatgaaa gtaataatgt gggattacta gaaaacctga ctaatggtgt cactgctggc 1320
aatggtgata atggattaat tcctcaaagg aagagcagaa cacctgaaaa tcagcaattt 1380
cctgacaacg aaagtgaaga gtatcacaga atttgcgaat tagtttctga ctacaaagaa 1440
aaacagatgc caaaatactc ttctgaaaac agcaacccag aacaagactt aaagctgaca 1500
tcagaggaag agtcacaaag gcttgagggc agtgaaaatg gccagccaga gaaaagatct 1560
caagaaccag aaataaataa ggatggtgat agagagctag aaaattttat ggctatcgaa 1620
gaaatgaaga agcacggaag tactcatgtc ggattcccag aaaacctgac taatggtgcc 1680
actgctggca atggtgatga tggattaatt cctccaagga agagcagaac acctgaaagc 1740
cagcaatttc ctgacactga gaatgaagag tatcacagtg acgaacaaaa tgatactcag 1800
aagcaatttt gtgaagaaca gaacactgga atattacacg atgagattct gattcatgaa 1860
gaaaagcaga tagaagtggt tgaaaaaatg aattctgagc tttctcttag ttgtaagaaa 1920
gaaaaagaca tcttgcatga aaatagtacg ttgcgggaag aaattgccat gctaagactg 1980
gagctagaca caatgaaaca tcagagccag ctaaaaaaaa aaaaaaaaaa aaaaaaaaaa 2040

<210> 376
<211> 329
<212> PRT
<213> Homo sapien

<400> 376
Met Asp Ile Val Val Ser Gly Ser His Pro Leu Trp Val Asp Ser Phe
1               5                   10                  15
Leu His Leu Ala Gly Ser Asp Leu Leu Ser Arg Ser Leu Met Ala Glu
            20                  25                  30
Glu Tyr Thr Ile Val His Ala Ser Phe Ile Ser Cys Ile Ser Ser Ser
        35                  40                  45
Leu Asp Gly Gln Gly Glu Arg Gln Glu Gln Arg Gly His Phe Trp Arg
    50                  55                  60
Pro Gln Arg Leu Leu Cys Glu Asp Ala Trp Glu Gln Glu Val Gln Val
65                  70                  75                  80
Val Leu Pro Leu Leu Pro Leu Leu Gln Gly Ser Gly Lys Ser Asn Val
                85                  90                  95
Val Ala Trp Gly Asp Tyr Asp Asp Ser Ala Phe Met Asp Pro Arg Tyr
            100                 105                 110
His Val His Gly Glu Asp Leu Asp Lys Leu His Arg Ala Ala Trp Trp
        115                 120                 125
Gly Lys Val Pro Arg Lys Asp Leu Ile Val Met Leu Arg Asp Thr Asp

130                 135                 140
Val Asn Lys Arg Asp Lys Gln Lys Arg Thr Ala Leu His Leu Ala Ser
145                 150                 155                 160
Ala Asn Gly Asn Ser Glu Val Val Lys Leu Val Leu Asp Arg Arg Cys
                165                 170                 175
Gln Leu Asn Val Leu Asp Asn Lys Lys Arg Thr Ala Leu Thr Lys Ala
            180                 185                 190
Val Gln Cys Gln Glu Asp Glu Cys Ala Leu Met Leu Leu Glu His Gly
        195                 200                 205
Thr Asp Pro Asn Ile Pro Asp Glu Tyr Gly Asn Thr Thr Leu His Tyr
    210                 215                 220
Ala Val Tyr Asn Glu Asp Lys Leu Met Ala Lys Ala Leu Leu Leu Tyr
225                 230                 235                 240
Gly Ala Asp Ile Glu Ser Lys Asn Lys His Gly Leu Thr Pro Leu Leu
                245                 250                 255
Leu Gly Ile His Glu Gln Lys Gln Gln Val Val Lys Phe Leu Ile Lys
            260                 265                 270
Lys Lys Ala Asn Leu Asn Ala Leu Asp Arg Tyr Gly Arg Thr Ala Leu
        275                 280                 285
Ile Leu Ala Val Cys Cys Gly Ser Ala Ser Ile Val Ser Pro Leu Leu
    290                 295                 300
Glu Gln Asn Val Asp Val Ser Ser Gln Asp Leu Glu Arg Arg Pro Glu
305                 310                 315                 320
Ser Met Leu Phe Leu Val Ile Ile Met
                325

<210> 377
<211> 148
<212> PRT
<213> Homo sapien

<220>
<221> VARIANT
<222> (1)...(148)
<223> Xaa = Any Amino Acid

<400> 377
Met Thr Xaa Pro Ser Trp Ser Pro Gly Thr Thr Ser Val Glu Lys Ile
 1               5                  10                  15
Trp Thr Ser Ser Thr Glu Leu Pro Trp Trp Gly Lys Val Pro Arg Lys
            20                  25                  30
Asp Leu Ile Val Met Leu Arg Asp Thr Asp Val Asn Lys Xaa Asp Lys
        35                  40                  45
Gln Lys Arg Thr Ala Leu His Leu Ala Ser Ala Asn Gly Asn Ser Glu
    50                  55                  60
Val Val Lys Leu Xaa Leu Asp Arg Arg Cys Gln Leu Asn Val Leu Asp
65                  70                  75                  80
Asn Lys Lys Arg Thr Ala Leu Xaa Lys Ala Val Gln Cys Gln Glu Asp
                85                  90                  95
Glu Cys Ala Leu Met Leu Leu Glu His Gly Thr Asp Pro Asn Ile Pro
            100                 105                 110
Asp Glu Tyr Gly Asn Thr Thr Leu His Tyr Ala Xaa Tyr Asn Glu Asp
        115                 120                 125
Lys Leu Met Ala Lys Ala Leu Leu Leu Tyr Gly Ala Asp Ile Glu Ser
    130                 135                 140
Lys Asn Lys Val
145

<210> 378
<211> 1719
<212> PRT
<213> Homo sapien

<400> 378

<210> 379
<211> 656
<212> PRT
<213> Homo sapien

<400> 379
Met Val Val Glu Val Asp Ser Met Pro Ala Ala Ser Ser Val Lys Lys
 1               5                  10                  15
Pro Phe Gly Leu Arg Ser Lys Met Gly Lys Trp Cys Cys Arg Cys Phe
            20                  25                  30
Pro Cys Cys Arg Glu Ser Gly Lys Ser Asn Val Gly Thr Ser Gly Asp
        35                  40                  45
His Asp Asp Ser Ala Met Lys Thr Leu Arg Ser Lys Met Gly Lys Trp
    50                  55                  60
Cys Arg His Cys Phe Pro Cys Cys Arg Gly Ser Gly Lys Ser Asn Val
65                  70                  75                  80
Gly Ala Ser Gly Asp His Asp Asp Ser Ala Met Lys Thr Leu Arg Asn
                85                  90                  95
Lys Met Gly Lys Trp Cys Cys His Cys Phe Pro Cys Cys Arg Gly Ser
            100                 105                 110
Gly Lys Ser Lys Val Gly Ala Trp Gly Asp Tyr Asp Asp Ser Ala Phe
        115                 120                 125
Met Glu Pro Arg Tyr His Val Arg Gly Glu Asp Leu Asp Lys Leu His
    130                 135                 140
Arg Ala Ala Trp Trp Gly Lys Val Pro Arg Lys Asp Leu Ile Val Met
145                 150                 155                 160
Leu Arg Asp Thr Asp Val Asn Lys Lys Asp Lys Gln Lys Arg Thr Ala
                165                 170                 175
Leu His Leu Ala Ser Ala Asn Gly Asn Ser Glu Val Val Lys Leu Leu

            180                 185                 190
Leu Asp Arg Arg Cys Gln Leu Asn Val Leu Asp Asn Lys Lys Arg Thr
        195                 200                 205
Ala Leu Ile Lys Ala Val Gln Cys Gln Glu Asp Glu Cys Ala Leu Met
    210                 215                 220
Leu Leu Glu His Gly Thr Asp Pro Asn Ile Pro Asp Glu Tyr Gly Asn
225                 230                 235                 240
Thr Thr Leu His Tyr Ala Ile Tyr Asn Glu Asp Lys Leu Met Ala Lys
                245                 250                 255
Ala Leu Leu Leu Tyr Gly Ala Asp Ile Glu Ser Lys Asn Lys His Gly
            260                 265                 270
Leu Thr Pro Leu Leu Leu Gly Val His Glu Gln Lys Gln Gln Val Val
        275                 280                 285
Lys Phe Leu Ile Lys Lys Lys Ala Asn Leu Asn Ala Leu Asp Arg Tyr
    290                 295                 300
Gly Arg Thr Ala Leu Ile Leu Ala Val Cys Cys Gly Ser Ala Ser Ile
305                 310                 315                 320
Val Ser Leu Leu Leu Glu Gln Asn Ile Asp Val Ser Ser Gln Asp Leu
                325                 330                 335
Ser Gly Gln Thr Ala Arg Glu Tyr Ala Val Ser Ser His His His Val
            340                 345                 350
Ile Cys Gln Leu Leu Ser Asp Tyr Lys Glu Lys Gln Met Leu Lys Ile
        355                 360                 365
Ser Ser Glu Asn Ser Asn Pro Glu Gln Asp Leu Lys Leu Thr Ser Glu
    370                 375                 380
Glu Glu Ser Gln Arg Phe Lys Gly Ser Glu Asn Ser Gln Pro Glu Lys
385                 390                 395                 400
Met Ser Gln Glu Pro Glu Ile Asn Lys Asp Gly Asp Arg Glu Val Glu
                405                 410                 415
Glu Glu Met Lys Lys His Glu Ser Asn Asn Val Gly Leu Leu Glu Asn
            420                 425                 430
Leu Thr Asn Gly Val Thr Ala Gly Asn Gly Asp Asn Gly Leu Ile Pro
        435                 440                 445
Gln Arg Lys Ser Arg Thr Pro Glu Asn Gln Gln Phe Pro Asp Asn Glu
    450                 455                 460
Ser Glu Glu Tyr His Arg Ile Cys Glu Leu Val Ser Asp Tyr Lys Glu
465                 470                 475                 480
Lys Gln Met Pro Lys Tyr Ser Ser Glu Asn Ser Asn Pro Glu Gln Asp
                485                 490                 495
Leu Lys Leu Thr Ser Glu Glu Glu Ser Gln Arg Leu Glu Gly Ser Glu
            500                 505                 510
Asn Gly Gln Pro Glu Leu Glu Asn Phe Met Ala Ile Glu Glu Met Lys
        515                 520                 525
Lys His Gly Ser Thr His Val Gly Phe Pro Glu Asn Leu Thr Asn Gly
    530                 535                 540
Ala Thr Ala Gly Asn Gly Asp Asp Gly Leu Ile Pro Pro Arg Lys Ser
545                 550                 555                 560
Arg Thr Pro Glu Ser Gln Gln Phe Pro Asp Thr Glu Asn Glu Glu Tyr
                565                 570                 575
His Ser Asp Glu Gln Asn Asp Thr Gln Lys Gln Phe Cys Glu Glu Gln
            580                 585                 590
Asn Thr Gly Ile Leu His Asp Glu Ile Leu Ile His Glu Glu Lys Gln
        595                 600                 605
Ile Glu Val Val Glu Lys Met Asn Ser Glu Leu Ser Leu Ser Cys Lys
    610                 615                 620
Lys Glu Lys Asp Ile Leu His Glu Asn Ser Thr Leu Arg Glu Glu Ile
625                 630                 635                 640
Ala Met Leu Arg Leu Glu Leu Asp Thr Met Lys His Gln Ser Gln Leu
                645                 650                 655

<210> 380
<211> 671

<212> PRT
<213> Homo sapien

<400> 380
Met Val Val Glu Val Asp Ser Met Pro Ala Ala Ser Ser Val Lys Lys
1               5                   10                  15
Pro Phe Gly Leu Arg Ser Lys Met Gly Lys Trp Cys Cys Arg Cys Phe
            20                  25                  30
Pro Cys Cys Arg Glu Ser Gly Lys Ser Asn Val Gly Thr Ser Gly Asp
        35                  40                  45
His Asp Asp Ser Ala Met Lys Thr Leu Arg Ser Lys Met Gly Lys Trp
    50                  55                  60
Cys Arg His Cys Phe Pro Cys Cys Arg Gly Ser Gly Lys Ser Asn Val
65                  70                  75                  80
Gly Ala Ser Gly Asp His Asp Asp Ser Ala Met Lys Thr Leu Arg Asn
                85                  90                  95
Lys Met Gly Lys Trp Cys Cys His Cys Phe Pro Cys Cys Arg Gly Ser
            100                 105                 110
Gly Lys Ser Lys Val Gly Ala Trp Gly Asp Tyr Asp Asp Ser Ala Phe
        115                 120                 125
Met Glu Pro Arg Tyr His Val Arg Gly Glu Asp Leu Asp Lys Leu His
    130                 135                 140
Arg Ala Ala Trp Trp Gly Lys Val Pro Arg Lys Asp Leu Ile Val Met
145                 150                 155                 160
Leu Arg Asp Thr Asp Val Asn Lys Lys Asp Lys Gln Lys Arg Thr Ala
                165                 170                 175
Leu His Leu Ala Ser Ala Asn Gly Asn Ser Glu Val Val Lys Leu Leu
            180                 185                 190
Leu Asp Arg Arg Cys Gln Leu Asn Val Leu Asp Asn Lys Lys Arg Thr
        195                 200                 205
Ala Leu Ile Lys Ala Val Gln Cys Gln Glu Asp Glu Cys Ala Leu Met
    210                 215                 220
Leu Leu Glu His Gly Thr Asp Pro Asn Ile Pro Asp Glu Tyr Gly Asn
225                 230                 235                 240
Thr Thr Leu His Tyr Ala Ile Tyr Asn Glu Asp Lys Leu Met Ala Lys
                245                 250                 255
Ala Leu Leu Leu Tyr Gly Ala Asp Ile Glu Ser Lys Asn Lys His Gly
            260                 265                 270
Leu Thr Pro Leu Leu Leu Gly Val His Glu Gln Lys Gln Gln Val Val
        275                 280                 285
Lys Phe Leu Ile Lys Lys Lys Ala Asn Leu Asn Ala Leu Asp Arg Tyr
    290                 295                 300
Gly Arg Thr Ala Leu Ile Leu Ala Val Cys Cys Gly Ser Ala Ser Ile
305                 310                 315                 320
Val Ser Leu Leu Leu Glu Gln Asn Ile Asp Val Ser Ser Gln Asp Leu
                325                 330                 335
Ser Gly Gln Thr Ala Arg Glu Tyr Ala Val Ser Ser His His His Val
            340                 345                 350
Ile Cys Gln Leu Leu Ser Asp Tyr Lys Glu Lys Gln Met Leu Lys Ile
        355                 360                 365
Ser Ser Glu Asn Ser Asn Pro Glu Gln Asp Leu Lys Leu Thr Ser Glu
    370                 375                 380
Glu Glu Ser Gln Arg Phe Lys Gly Ser Glu Asn Ser Gln Pro Glu Lys
385                 390                 395                 400
Met Ser Gln Glu Pro Glu Ile Asn Lys Asp Gly Asp Arg Glu Val Glu
                405                 410                 415
Glu Glu Met Lys Lys His Glu Ser Asn Asn Val Gly Leu Leu Glu Asn
            420                 425                 430
Leu Thr Asn Gly Val Thr Ala Gly Asn Gly Asp Asn Gly Leu Ile Pro
        435                 440                 445
Gln Arg Lys Ser Arg Thr Pro Glu Asn Gln Gln Phe Pro Asp Asn Glu
    450                 455                 460

Ser Glu Glu Tyr His Arg Ile Cys Glu Leu Val Ser Asp Tyr Lys Glu
465                 470                 475                 480
Lys Gln Met Pro Lys Tyr Ser Ser Glu Asn Ser Asn Pro Glu Gln Asp
                485                 490                 495
Leu Lys Leu Thr Ser Glu Glu Glu Ser Gln Arg Leu Glu Gly Ser Glu
            500                 505                 510
Asn Gly Gln Pro Glu Lys Arg Ser Gln Glu Pro Glu Ile Asn Lys Asp
        515                 520                 525
Gly Asp Arg Glu Leu Glu Asn Phe Met Ala Ile Glu Glu Met Lys Lys
    530                 535                 540
His Gly Ser Thr His Val Gly Phe Pro Glu Asn Leu Thr Asn Gly Ala
545                 550                 555                 560
Thr Ala Gly Asn Gly Asp Asp Gly Leu Ile Pro Pro Arg Lys Ser Arg
                565                 570                 575
Thr Pro Glu Ser Gln Gln Phe Pro Asp Thr Glu Asn Glu Glu Tyr His
            580                 585                 590
Ser Asp Glu Gln Asn Asp Thr Gln Lys Gln Phe Cys Glu Glu Gln Asn
        595                 600                 605
Thr Gly Ile Leu His Asp Glu Ile Leu Ile His Glu Glu Lys Gln Ile
    610                 615                 620
Glu Val Val Glu Lys Met Asn Ser Glu Leu Ser Leu Ser Cys Lys Lys
625                 630                 635                 640
Glu Lys Asp Ile Leu His Glu Asn Ser Thr Leu Arg Glu Glu Ile Ala
                645                 650                 655
Met Leu Arg Leu Glu Leu Asp Thr Met Lys His Gln Ser Gln Leu
            660                 665                 670

<210> 381
<211> 251
<212> DNA
<213> Homo sapien

<400> 381
ggagaagcgt ctgctggggc aggaaggggt ttccctgccc tctcacctgt ccctcaccaa      60
ggtaacatgc ttcccctaag ggtatcccaa cccaggggcc tcaccatgac ctctgagggg     120
ccaatatccc aggagaagca ttggggagtt gggggcaggt gaaggaccca ggactcacac     180
atcctgggcc tccaaggcag aggagagggt cctcaagaag gtcaggagga aaatccgtaa     240
caagcagtca g                                                          251

<210> 382
<211> 3279
<212> DNA
<213> Homo sapiens

<400> 382
cttcctgcag cccccatgct ggtgaggggc acgggcagga acagtggacc caacatggaa 60
atgctggagg gtgtcaggaa gtgatcgggc tctggggcag ggaggagggg tggggagtgt 120
cactgggagg ggacatcctg cagaaggtag gagtgagcaa acacccgctg caggggaggg 180
gagagccctg cggcacctgg gggagcagag ggagcagcac ctgcccaggc ctgggaggag 240
gggcctggag ggcgtgagga ggagcgaggg ggctgcatgg ctggagtgag ggatcagggg 300
cagggcgcga gatggcctca cacagggaag agagggcccc tcctgcaggg cctcacctgg 360
gccacaggag gacactgctt ttcctctgag gagtcaggag ctgtggatgg tgctggacag 420
aagaaggaca gggcctggct caggtgtcca gaggctgtcg ctggcttccc tttgggatca 480
gactgcaggg agggagggcg gcagggttgt ggggggagtg acgatgagga tgacctgggg 540
gtggctccag gccttgcccc tgcctgggcc ctcacccagc ctccctcaca gtctcctggc 600
cctcagtctc tcccctccac tccatcctcc atctggcctc agtgggtcat tctgatcact 660
gaactgacca tacccagccc tgcccacggc cctccatggc tccccaatgc cctggagagg 720
ggacatctag tcagagagta gtcctgaaga ggtggcctct gcgatgtgcc tgtgggggca 780
gcatcctgca gatggtcccg gccctcatcc tgctgacctg tctgcaggga ctgtcctcct 840
ggaccttgcc ccttgtgcag gagctggacc ctgaagtccc ctccccatag gccaagactg 900
gagccttgtt ccctctgttg gactccctgc ccatattctt gtgggagtgg gttctggaga 960
catttctgtc tgttcctgag agctgggaat tgctctcagt catctgcctg cgcggttctg 1020

agagatggag ttgcctaggc agttattggg gccaatcttt ctcactgtgt ctctcctcct 1080
ttacccttag ggtgattctg ggggtccact tgtctgtaat ggtgtgcttc aaggtatcac 1140
atcatggggc cctgagccat gtgccctgcc tgaaaagcct gctgtgtaca ccaaggtggt 1200
gcattaccgg aagtggatca aggacaccat cgcagccaac ccctgagtgc ccctgtccca 1260
cccctacctc tagtaaattt aagtccacct cacgttctgg catcacttgg cctttctgga 1320
tgctggacac ctgaagcttg gaactcacct ggccgaagct cgagcctcct gagtcctact 1380
gacctgtgct ttctggtgtg gagtccaggg ctgctaggaa aaggaatggg cagacacagg 1440
tgtatgccaa tgtttctgaa atgggtataa tttcgtcctc tccttcggaa cactggctgt 1500
ctctgaagac ttctcgctca gtttcagtga ggacacacac aaagacgtgg gtgaccatgt 1560
tgtttgtggg gtgcagagat gggaggggtg gggcccaccc tggaagagtg gacagtgaca 1620
caaggtggac actctctaca gatcactgag gataagctgg agccacaatg catgaggcac 1680
acacacagca aggttgacgc tgtaaacata gcccacgctg tcctgggggc actgggaagc 1740
ctagataagg ccgtgagcag aaagaagggg aggatcctcc tatgttgttg aaggagggac 1800
tagggggaga aactgaaagc tgattaatta caggaggttt gttcaggtcc cccaaaccac 1860
cgtcagattt gatgatttcc tagcaggact tacagaaata aagagctatc atgctgtggt 1920
ttattatggt ttgttacatt gataggatac atactgaaat cagcaaacaa aacagatgta 1980
tagattagag tgtggagaaa acagaggaaa acttgcagtt acgaagactg gcaacttggc 2040
tttactaagt tttcagactg gcaggaagtc aaacctatta ggctgaggac cttgtggagt 2100
gtagctgatc cagctgatag aggaactagc caggtggggg cctttccctt tggatggggg 2160
gcatatccga cagttattct ctccaagtgg agacttacgg acagcatata attctccctg 2220
caaggatgta tgataatatg tacaaagtaa ttccaactga ggaagctcac ctgatcctta 2280
gtgtccaggg tttttactgg gggtctgtag gacgagtatg gagtacttga ataattgacc 2340
tgaagtcctc agacctgagg ttccctagag ttcaaacaga tacagcatgg tccagagtcc 2400
cagatgtaca aaaacaggga ttcatcacaa atcccatctt tagcatgaag ggtctggcat 2460
ggcccaaggc cccaagtata tcaaggcact tgggcagaac atgccaagga atcaaatgtc 2520
atctcccagg agttattcaa gggtgagccc tttacttggg atgtacaggc tttgagcagt 2580
gcagggctgc tgagtcaacc ttttattgta caggggatga gggaaaggga gaggatgagg 2640
aagcccccct ggggatttgg tttggtcttg tgatcaggtg gtctatgggg ctatccctac 2700
aaagaagaat ccagaaatag gggcacattg aggaatgata ctgagcccaa agagcattca 2760
atcattgttt tatttgcctt cttttcacac cattggtgag ggagggatta ccaccctggg 2820
gttatgaaga tggttgaaca ccccacacat agcaccggag atatgagatc aacagtttct 2880
tagccataga gattcacagc ccagagcagg aggacgctgc acaccatgca ggatgacatg 2940
ggggatgcgc tcgggattgg tgtgaagaag caaggactgt tagaggcagg ctttatagta 3000
acaagacggt ggggcaaact ctgatttccg tgggggaatg tcatggtctt gctttactaa 3060
gttttgagac tggcaggtag tgaaactcat taggctgaga accttgtgga atgcagctga 3120
cccagctgat agaggaagta gccaggtggg agcctttccc agtgggtgtg ggacatatct 3180
ggcaagattt tgtggcactc ctggttacag atactggggc agcaaataaa actgaatctt 3240
gttttcagac cttaaaaaaa aaaaaaaaaa aaaagtttt 3279

<210> 383
<211> 155
<212> PRT
<213> Homo sapiens

<400> 383

<210> 384
<211> 557
<212> DNA
<213> Homo sapiens

<400> 384
ggatcctcta gagcggccgc ctactactac taaattcgcg gccgcgtcga cgaagaagag 60
aaagatgtgt tttgttttgg actctctgtg gtcccttcca atgctgtggg tttccaacca 120
ggggaagggt cccttttgca ttgccaagtg ccataaccat gagcactact ctaccatggt 180
tctgcctcct ggccaagcag gctggtttgc aagaatgaaa tgaatgattc tacagctagg 240
acttaacctt gaaatggaaa gtcttgcaat cccatttgca ggatccgtct gtgcacatgc 300
ctctgtagag agcagcattc ccagggacct tggaaacagt tggcactgta aggtgcttgc 360
tccccaagac acatcctaaa aggtgttgta atggtgaaaa cgtcttcctt ctttattgcc 420
ccttcttatt tatgtgaaca actgtttgtc tttttttgta tctttttaa actgtaaagt 480
tcaattgtga aaatgaatat catgcaaata aattatgcga tttttttttc aaagtaaaaa 540
aaaaaaaaaa aaaaaaa                                                557

<210> 385
<211> 337
<212> DNA
<213> Homo sapiens

<400> 385
ttcccaggtg atgtgcgagg gaagacacat ttactatcct tgatggggct gattccttta 60
gtttctctag cagcagatgg gttaggagga agtgacccaa gtggttgact cctatgtgca 120
tctcaaagcc atctgctgtc ttcgagtacg gacacatcat cactcctgca ttgttgatca 180
aaacgtggag gtgcttttcc tcagctaaga agcccttagc aaaagctcga atagacttag 240
tatcagacag gtccagtttc cgcaccaaca cctgctggtt ccctgtcgtg gtctggatct 300
ctttggccac caattccccc ttttccacat cccggca                          337

<210> 386
<211> 300
<212> DNA
<213> Homo sapiens

<400> 386
gggcccgcta ccggcccagg ccccgcctcg cgagtcctcc tccccgggtg cctgcccgca 60
gcccgctcgg cccagagggt gggcgcgggg ctgcctctac cggctggcgg ctgtaactca 120
gcgaccttgg cccgaaggct ctagcaagga cccaccgacc ccagccgcgg cggcggcggc 180
gcggactttg cccggtgtgt ggggcggagc ggactgcgtg tccgcggacg ggcagcgaag 240
atgttagcct tcgctgccag gaccgtggac cgatcccagg gctgtggtgt aacctcagcc 300

<210> 387
<211> 537
<212> DNA
<213> Homo sapiens

<400> 387

gggccgagtc gggcaccaag ggactctttg caggcttcct tcctcggatc atcaaggctg 60
ccccctcctg tgccatcatg atcagcacct atgagttcgg caaaagcttc ttccagaggc 120
tgaaccagga ccggcttctg ggcggctgaa aggggcaagg aggcaaggac cccgtctctc 180
ccacggatgg ggagagggca ggaggagacc cagccaagtg ccttttcctc agcactgagg 240
gagggggctt gtttcccttc cctcccggcg acaagctcca gggcagggct gtccctctgg 300
gcggcccagc acttcctcag acacaacttc ttcctgctgc tccagtcgtg gggatcatca 360
cttacccacc ccccaagttc aagaccaaat cttccagctg cccccttcgt gtttccctgt 420
gtttgctgta gctgggcatg tctccaggaa ccaagaagcc ctcagcctgg tgtagtctcc 480
ctgacccttg ttaattcctt aagtctaaag atgatgaact tcaaaaaaaa aaaaaaa 537

<210> 388
<211> 520
<212> DNA
<213> Homo sapiens

<400> 388
aggataattt ttaaaccaat caaatgaaaa aaacaaacaa acaaaaaagg aaatgtcatg 60
tgaggttaaa ccagtttgca ttcccctaat gtggaaaaag taagaggact actcagcact 120
gtttgaagat tgcctcttct acagcttctg agaattgtgt tatttcactt gccaagtgaa 180
ggaccccctc cccaacatgc cccagcccac ccctaagcat ggtcccttgt caccaggcaa 240
ccaggaaact gctacttgtg gacctcacca gagaccagga gggtttggtt agctcacagg 300
acttccccca ccccagaaga ttagcatccc atactagact catactcaac tcaactaggc 360
tcatactcaa ttgatggtta ttagacaatt ccatttcttt ctggttatta taaacagaaa 420
atctttcctc ttctcattac cagtaaaggc tcttggtatc tttctgttgg aatgatttct 480
atgaacttgt cttattttaa tggtgggttt tttttctggt 520

<210> 389
<211> 365
<212> DNA
<213> Homo sapiens

<400> 389
cgttgcccca gtttgacaga aggaaaggcg gagcttattc aaagtctaga gggagtggag 60
gagttaaggc tggatttcag atctgcctgg ttccagccgc agtgtgccct ctgctccccc 120
aacgactttc caaataatct caccagcgcc ttccagctca ggcgtcctag aagcgtcttg 180
aagcctatgg ccagctgtct ttgtgttccc tctcacccgc ctgtcctcac agctgagact 240
cccaggaaac cttcagacta ccttcctctg ccttcagcaa ggggcgttgc ccacattctc 300
tgagggtcag tggaagaacc tagactccca ttgctagagg tagaaagggg aagggtgctg 360
gggag 365

<210> 390
<211> 221
<212> DNA
<213> Homo sapiens

<220>
<221> misc_feature
<222> (1)...(221)
<223> n = A,T,C or G

<400> 390
tgcctctcca tcctggcccc gacttctctg tcaggaaagt ggggatggac cccatctgca 60
tacacggntt ctcatgggtg tggaacatct ctgcttgcgg tttcaggaag gcctctggct 120
gctctangag tctgancnga ntcgttgccc cantntgaca naaggaaagg cggagcttat 180
tcaaagtcta gagggagtgg aggagttaag gctggatttc a 221

<210> 391
<211> 325
<212> DNA
<213> Homo sapiens

<220>

<221> misc_feature
<222> (1)...(325)
<223> n = A,T,C or G

<400> 391
tggagcaggt cccgaggcct ccctagagcc tggggccgac tctgtgncga tgcangcttt 60
ctctcgcgcc cagcctggag ctgctcctgg catctaccaa caatcagncg aggcgagcag 120
tagccagggc actgctgcca acagccagtc cnnataccat catgtnaccc ggtgngctct 180
naanttngat ntccanagcc ctacccatcn tagttctgct ctcccaccgg ntaccagccc 240
cactgcccag gaatcctaca gccagtaccc tgtcccgacg tctctaccta ccagtacgat 300
gagacctccg gctactacta tgacc 325

<210> 392
<211> 277
<212> DNA
<213> Homo sapiens

<220>
<221> misc_feature
<222> (1)...(277)
<223> n = A,T,C or G

<400> 392
atattgttta actccttcct ttatatcttt taacattttc atggngaaag gttcacatct 60
agtctcactt nggcnagngn ctcctacttg agtctcttcc ccggcctgnn ccagtngnaa 120
antaccanga accgncatgn cttaanaacn ncctggtttn tgggttnntc aatgactgca 180
tgcagtgcac caccctgtcc actacgtgat gctgtaggat taaagtctca cagtgggcgg 240
ctgaggatac agcgccgcgt cctgtgttgc tggggaa 277

<210> 393
<211> 566
<212> DNA
<213> Homo sapiens

<400> 393
actagtccag tgtggtggaa ttcgcggccg cgtcgacgga caggtcagct gtctggctca 60
gtgatctaca ttctgaagtt gtctgaaaat gtcttcatga ttaaattcag cctaaacgtt 120
ttgccgggaa cactgcagag acaatgctgt gagtttccaa ccttagccca tctgcgggca 180
gagaaggtct agtttgtcca tcagcattat catgatatca ggactggtta cttggttaag 240
gaggggtcta ggagatctgt cccttttaga gacaccttac ttataatgaa gtatttggga 300
gggtggtttt caaaagtaga aatgtcctgt attccgatga tcatcctgta aacattttat 360
catttattaa tcatccctgc ctgtgtctat tattatattc atatctctac gctggaaact 420
ttctgcctca atgtttactg tgcctttgtt tttgctagtt tgtgttgttg aaaaaaaaaa 480
cattctctgc ctgagtttta atttttgtcc aaagttattt taatctatac aattaaaagc 540
ttttgcctat caaaaaaaaa aaaaaa 566

<210> 394
<211> 384
<212> DNA
<213> Homo sapiens

<220>
<221> misc_feature
<222> (1)...(384)
<223> n = A,T,C or G

<400> 394
gaacatacat gtcccggcac ctgagctgca gtctgacatc atcgccatca cgggcctcgc 60
tgcaaattng gaccgggcca aggctggact gctggagcgt gtgaaggagc tacaggccna 120
gcaggaggac cgggctttaa ggagttttaa gctgagtgtc actgtagacc ccaaatacca 180
tcccaagatt atcgggagaa agggggcagt aattacccaa atccggttgg agcatgacgt 240
gaacatccag tttcctgata aggacgatgg gaaccagccc caggaccaaa ttaccatcac 300

agggtacgaa aagaacacag aagctgccag ggatgctata ctgagaattg tgggtgaact 360
tgagcagatg gtttctgagg acgt 384

<210> 395
<211> 399
<212> DNA
<213> Homo sapiens

<400> 395
ggcaaaactg tgtgacctca ataagacctc gcagatccaa ggtcaagtat cagaagtgac 60
tctgaccttg gactccaaga cctacatcaa cagcctggct atattagatg atgagccagt 120
tatcagaggt ttcatcattg cggaaattgt ggagtctaag gaaatcatgg cctctgaagt 180
attcacgtct ttccagtacc ctgagttctc tatagagttg cctaacacag gcagaattgg 240
ccagctactt gtctgcaatt gtatcttcaa gaataccctg gccatccctt tgactgacgt 300
caagttctct ttggaaagcc tgggcatctc ctcactacag acctctgacc atgggacggt 360
gcagcctggt gagaccatcc aatcccaaat aaaatgcac 399

<210> 396
<211> 403
<212> DNA
<213> Homo sapiens

<220>
<221> misc_feature
<222> (1)...(403)
<223> n = A,T,C or G

<400> 396
tggagttntc agtgcaaaca agccataaag cttcagtagc aaattactgt ctcacagaaa 60
gacattttca acttctgctc cagctgctga taaaacaaat catgtgttta gcttgactcc 120
agacaaggac aacctgttcc ttcataactc tctagagaaa aaaaggagtt gttagtagat 180
actaaaaaaa gtggatgaat aatctggata tttttcctaa aaagattcct tgaaacacat 240
taggaaaatg gagggcctta tgatcagaat gctagaatta gtccattgtg ctgaagcagg 300
gtttagggga gggagtgagg gataaaagaa ggaaaaaaag aagagtgaga aaacctattt 360
atcaaagcag gtgctatcac tcaatgttag gccctgctct ttt 403

<210> 397
<211> 100
<212> DNA
<213> Homo sapiens

<220>
<221> misc_feature
<222> (1)...(100)
<223> n = A,T,C or G

<400> 397
actagtncag tgtggtggaa ttcgcggccg cgtcgaccta naanccatct ctatagcaaa 60
tccatccccg ctcctggttg gtnacagaat gactgacaaa 100

<210> 398
<211> 278
<212> DNA
<213> Homo sapiens

<220>
<221> misc_feature
<222> (1)...(278)
<223> n = A,T,C or G

<400> 398
gcggccgcgt cgacagcagt tccgccagcg ctcgcccctg ggtggggatg tgctgcacgc 60

ccacctggac atctggaagt cagcggcctg gatgaaagag cggacttcac ctggggcgat 120
tcactactgt gcctcgacca gtgaggagag ctggaccgac agcgaggtgg actcatcatg 180
ctccgggcag cccatccacc tgtggcagtt cctcaaggag ttgctactca agccccacag 240
ctatggccgc ttcattangt ggctcaacaa ggagaagg 278

<210> 399
<211> 298
<212> DNA
<213> Homo sapiens

<220>
<221> misc_feature
<222> (1)...(298)
<223> n = A,T,C or G

<400> 399
acggaggtgg aggaagcgnc cctgggatcg anaggatggg tcctgncatt gaccncctcn 60
ggggtgccng catggagcgc atgggcgcgg gcctgggcca cggcatggat cgcgtgggct 120
ccgagatcga gcgcatgggc ctggtcatgg accgcatggg ctccgtggag cgcatgggct 180
ccggcattga gcgcatgggc ccgctgggcc tcgaccacat ggcctccanc attgancgca 240
tgggccagac catggagcgc attggctctg gcgtggagcn catgggtgcc ggcatggg 298

<210> 400
<211> 548
<212> DNA
<213> Homo sapiens

<400> 400
acatcaacta cttcctcatt ttaaggtatg gcagttccct tcatcccctt ttcctgcctt 60
gtacatgtac atgtatgaaa tttccttctc ttaccgaact ctctccacac atcacaaggt 120
caaagaacca cacgcttaga agggtaagag ggcaccctat gaaatgaaat ggtgatttct 180
tgagtctctt ttttccacgt ttaaggggcc atggcaggac ttagagttgc gagttaagac 240
tgcagagggc tagagaatta tttcatacag gctttgaggc cacccatgtc acttatcccg 300
tataccctct caccatcccc ttgtctactc tgatgccccc aagatgcaac tgggcagcta 360
gttggcccca taattctggg cctttgttgt ttgttttaat tacttgggca tcccaggaag 420
ctttccagtg atctcctacc atgggccccc ctcctgggat caagcccctc ccaggccctg 480
tccccagccc ctcctgcccc agcccacccg cttgccttgg tgctcagccc tcccattggg 540
agcaggtt 548

<210> 401
<211> 355
<212> DNA
<213> Homo sapiens

<220>
<221> misc_feature
<222> (1)...(355)
<223> n = A,T,C or G

<400> 401
actgtttcca tgttatgttt ctacacattg ctacctcagt gctcctggaa acttagcttt 60
tgatgtctcc aagtagtcca ccttcattta actctttgaa actgtatcat ctttgccaag 120
taagagtggt ggcctatttc agctgctttg acaaaatgac tggctcctga cttaacgttc 180
tataaatgaa tgtgctgaag caaagtgccc atggtggcgg cgaagaagan aaagatgtgt 240
tttgtttggg actctctgtg gtcccttcca atgctgnggg tttccaacca ggggaagggt 300
cccttttgca ttgccaagtg ccataaccat gagcactact ctaccatggn tctgc 355

<210> 402
<211> 407
<212> DNA
<213> Homo sapiens

<220>
<221> misc_feature
<222> (1)...(407)
<223> n = A,T,C or G

<400> 402
atggggcaag ctggataaag aaccaagacc cactggagta tgctgtcttc aagaaaccca 60
tctcacatgc ggtggcatac ataggctcaa aataaaggaa tggagaaaaa tatttcaagc 120
aaatggaaaa cagaaaaaag caggtgttgc actcctactt tctgacaaaa cagactatgc 180
gaataaagat aaaaaagaga aggacattac aaaggtggtc ctgacctttg ataaatctca 240
ttgcttgata ccaacctggg ctgttttaat tgcccaaacc aaaaggataa tttgctgagg 300
ttgtggagct tctcccctgc agagagtccc tgatctccca aaatttggtt gagatgtaag 360
gntgattttg ctgacaactc cttttctgaa gttttactca tttccaa 407

<210> 403
<211> 303
<212> DNA
<213> Homo sapiens

<220>
<221> misc_feature
<222> (1)...(303)
<223> n = A,T,C or G

<400> 403
cagtatttat agccnaactg aaaagctagt agcaggcaag tctcaaatcc aggcaccaaa 60
tcctaagcaa gagccatggc atggtgaaaa tgcaaaagga gagtctggcc aatctacaaa 120
tagagaacaa gacctactca gtcatgaaca aaaaggcaga caccaacatg gatctcatgg 180
gggattggat attgtaatta tagagcagga agatgacagt gatcgtcatt tggcacaaca 240
tcttaacaac gaccgaaacc cattatttac ataaacctcc attcggtaac catgttgaaa 300
gga 303

<210> 404
<211> 225
<212> DNA
<213> Homo sapiens

<400> 404
aagtgtaact tttaaaaatt tagtggattt tgaaaattct tagaggaaag taaaggaaaa 60
attgttaatg cactcattta cctttacatg gtgaaagttc tctcttgatc ctacaaacag 120
acattttcca ctcgtgtttc catagttgtt aagtgtatca gatgtgttgg gcatgtgaat 180
ctccaagtgc ctgtgtaata aataaagtat ctttatttca ttcat 225

<210> 405
<211> 334
<212> DNA
<213> Homo sapiens

<220>
<221> misc_feature
<222> (1)...(334)
<223> n = A,T,C or G

<400> 405
gagctgttat actgtgagtt ctactaggaa atcatcaaat ctgagggttg tctggaggac 60
ttcaatacac ctccccccat agtgaatcag cttccagggg gtccagtccc tctccttact 120
tcatccccat cccatgccaa aggaagaccc tccctccttg gctcacagcc ttctctaggc 180
ttcccagtgc ctccaggaca gagtgggtta tgttttcagc tccatccttg ctgtgagtgt 240
ctggtgcggt tgtgcctcca gcttctgctc agtgcttcat ggacagtgtc cagcccatgt 300
cactctccac tctctcanng tggatcccac ccct 334

<210> 406

<211> 216
<212> DNA
<213> Homo sapiens

<220>
<221> misc_feature
<222> (1)...(216)
<223> n = A,T,C or G

<400> 406
tttcatacct aatgagggag ttganatnac atnnaaccag gaaatgcatg gatctcaang 60
gaaacaaaca cccaataaac tcggagtggc agactgacaa ctgtgagaca tgcacttgct 120
acnaaacaca aatttnatgt tgcacccttg tttctacacc tgtgggttat gacaaagaca 180
actgccaaag aatnttcaag aaggaggact gccant 216

<210> 407
<211> 413
<212> DNA
<213> Homo sapiens

<400> 407
gctgacttgc tagtatcatc tgcattcatt gaagcacaag aacttcatgc cttgactcat 60
gtaaatgcaa taggattaaa aaataaattt gatatcacat ggaaacagac aaaaaatatt 120
gtacaacatt gcacccagtg tcagattcta cacctggcca ctcaggaagc aagagttaat 180
cccagaggtc tatgtcctaa tgtgttatgg caaatggatg tcatgcacgt accttcattt 240
ggaaaattgt catttgtcca tgtgacagtt gatacttatt cacatttcat atgggcaacc 300
tgccagacag gagaaagtct tcccatgtta aaagacattt attatcttgt tttcctgtca 360
tgggagttcc agaaaaagtt aaaacagaca atgggccagg ttctgtagta aag 413

<210> 408
<211> 183
<212> DNA
<213> Homo sapiens

<220>
<221> misc_feature
<222> (1)...(183)
<223> n = A,T,C or G

<400> 408
ggagctngcc ctcaattcct ccatntctat gttancatat ttaatgtctt ttgnnattaa 60
tncttaacta gttaatcctt aaagggctan ntaatcctta actagtccct ccattgtgag 120
cattatcctt ccagtattcn ccttctnttt tatttactcc ttcctggcta cccatgtact 180
ntt 183

<210> 409
<211> 250
<212> DNA
<213> Homo sapiens

<220>
<221> misc_feature
<222> (1)...(250)
<223> n = A,T,C or G

<400> 409
cccacgcatg ataagctctt tatttctgta agtcctgcta ggaaatcatc aaatctgacg 60
gtggtttggg ggacctgaac aaacctcctg taattaatca gctttcagtt tctcccccta 120
gtccctcctt caacaacata ggaggatcct ccccttcttt ctgctcacgg ccttatctag 180
gcttcccagt gcccccagga cagcgtgggc tatgtttaca gcgcntcctt gctggggggg 240
ggccntatgc 250

<210> 410
<211> 306
<212> DNA
<213> Homo sapiens

<220>
<221> misc_feature
<222> (1)...(306)
<223> n = A,T,C or G

<400> 410
ggctggtttg caagaatgaa atgaatgatt ctacagctag gacttaacct tgaaatggaa 60
agtcttgcaa tcccatttgc aggatccgtc tgtgcacatg cctctgtaga gagcagcatt 120
cccagggacc ttggaaacag ttggcactgt aaggtgcttg ctccccaaga cacatcctaa 180
aaggtgttgt aatggtgaaa accgcttcct tctttattgc cccttcttat ttatgtgaac 240
nactggttgg ctttttttgn atctttttta aactggaaag ttcaattgng aaaatgaata 300
tcntgc 306

<210> 411
<211> 261
<212> DNA
<213> Homo sapiens

<220>
<221> misc_feature
<222> (1)...(261)
<223> n = A,T,C or G

<400> 411
agagatattn cttaggtnaa agttcataga gttcccatga actatatgac tggccacaca 60
ggatcttttg tatttaagga ttctgagatt ttgcttgagc aggattagat aaggctgttc 120
tttaaatgtc tgaaatggaa cagatttcaa aaaaaaaccc cacaatctag ggtgggaaca 180
aggaaggaaa gatgtgaata ggctgatggg caaaaaacca atttacccat cagttccagc 240
cttctctcaa ggngaggcaa a 261

<210> 412
<211> 241
<212> DNA
<213> Homo sapiens

<220>
<221> misc_feature
<222> (1)...(241)
<223> n = A,T,C or G

<400> 412
gttcaatgtt acctgacatt tctacaacac cccactcacc gatgtattcg ttgcccagtg 60
ggaacatacc agcctgaatt tggaaaaaat aattgtgttt cttgcccagg aaatactacg 120
actgactttg atggctccac aaacataacc cagtgtaaaa acagaagatg tggaggggag 180
ctgggagatt tcactgggta cattgaattc ccaaactacc cangcaatta cccagccaac 240
a 241

<210> 413
<211> 231
<212> DNA
<213> Homo sapiens

<220>
<221> misc_feature
<222> (1)...(231)
<223> n = A,T,C or G

<400> 413
aactcttaca atccaagtga ctcatctgtg tgcttgaatc ctttccactg tctcatctcc 60
ctcatccaag tttctagtac cttctctttg ttgtgaagga taatcaaact gaacaacaaa 120
aagtttactc tcctcatttg gaacctaaaa actctcttct tcctgggtct gagggctcca 180
agaatccttg aatcanttct cagatcattg gggacaccan atcaggaacc t 231

<210> 414
<211> 234
<212> DNA
<213> Homo sapiens

<400> 414
actgtccatg aagcactgag cagaagctgg aggcacaacg caccagacac tcacagcaag 60
gatggagctg aaaacataac ccactctgtc ctggaggcac tgggaagcct agagaaggct 120
gtgagccaag gagggagggt cttcctttgg catgggatgg ggatgaagta aggagaggga 180
ctggaccccc tggaagctga ttcactatgg ggggaggtgt attgaagtcc tcca 234

<210> 415
<211> 217
<212> DNA
<213> Homo sapiens

<220>
<221> misc_feature
<222> (1)...(217)
<223> n = A,T,C or G

<400> 415
gcataggatt aagactgagt atcttttcta cattctttta actttctaag gggcacttct 60
caaaacacag accaggtagc aaatctccac tgctctaagg ntctcaccac cactttctca 120
cacctagcaa tagtagaatt cagtcctact tctgaggcca gaagaatggt tcagaaaaat 180
antggattat aaaaaataac aattaagaaa aataatc 217

<210> 416
<211> 213
<212> DNA
<213> Homo sapiens

<220>
<221> misc_feature
<222> (1)...(213)
<223> n = A,T,C or G

<400> 416
atgcatatnt aaagganact gcctcgcttt tagaagacat ctggnctgct ctctgcatga 60
ggcacagcag taaagctctt tgattcccag aatcaagaac tctccccttc agactattac 120
cgaatgcaag gtggttaatt gaaggccact aattgatgct caaatagaag gatattgact 180
atattggaac agatggagtc tctactacaa aag 213

<210> 417
<211> 303
<212> DNA
<213> Homo sapiens

<220>
<221> misc_feature
<222> (1)...(303)
<223> n = A,T,C or G

<400> 417
nagtcttcag gcccatcagg gaagttcaca ctggagagaa gtcatacata tgtactgtat 60
gtgggaaagg ctttactctg agttcaaatc ttcaagccca tcagagagtc cacactggag 120

agaagccata caaatgcaat gagtgtggga agagcttcag gagggattcc cattatcaag 180
ttcatctagt ggtccacaca ggagagaaac cctataaatg tgagatatgt gggaagggct 240
tcantcaaag ttcgtatctt caaatccatc ngaaggncca cagtatanan aaacctttta 300
agt 303

<210> 418
<211> 328
<212> DNA
<213> Homo sapiens

<220>
<221> misc_feature
<222> (1)...(328)
<223> n = A,T,C or G

<400> 418
tttttggcgg tggtggggca gggacgggac angagtctca ctctgttgcc caggctggag 60
tgcacaggca tgatctcggc tcactacaac ccctgcctcc catgtccaag cgattcttgt 120
gcctcagcct tccctgtagc tagaattaca ggcacatgcc accacaccca gctagttttt 180
gtatttttag tagagacagg gtttcaccat gttggccagg ctggtctcaa actcctnacc 240
tcagnggtca ggctggtctc aaactcctga cctcaagtga tctgcccacc tcagcctccc 300
aaagtgctan gattacaggc cgtgagcc 328

<210> 419
<211> 389
<212> DNA
<213> Homo sapiens

<220>
<221> misc_feature
<222> (1)...(389)
<223> n = A,T,C or G

<400> 419
cctcctcaag acggcctgtg gtccgcctcc cggcaaccaa gaagcctgca gtgccatatg 60
acccctgagc catggactgg agcctgaaag gcagcgtaca ccctgctcct gatcttgctg 120
cttgtttcct ctctgtggct ccattcatag cacagttgtt gcactgaggc ttgtgcaggc 180
cgagcaaggc caagctggct caaagagcaa ccagtcaact ctgccacggt gtgccaggca 240
ccggttctcc agccaccaac ctcactcgct cccgcaaatg gcacatcagt tcttctaccc 300
taaaggtagg accaaagggc atctgctttt ctgaagtcct ctgctctatc agccatcacg 360
tggcagccac tcnggctgtg tcgacgcgg 389

<210> 420
<211> 408
<212> DNA
<213> Homo sapiens

<400> 420
gttcctccta actcctgcca gaaacagctc tcctcaacat gagagctgca cccctcctcc 60
tggccagggc agcaagcctt agccttggct tcttgtttct gctttttttc tggctagacc 120
gaagtgtact agccaaggag ttgaagtttg tgactttggt gtttcggcat ggagaccgaa 180
gtcccattga cacctttccc actgacccca taaaggaatc ctcatggcca caaggatttg 240
gccaactcac ccagctgggc atggagcagc attatgaact tggagagtat ataagaaaga 300
gatatagaaa attcttgaat gagtcctata aacatgaaca ggtttatatt cgaagcacag 360
acgttgaccg gactttgatg aagtgctatg acaaacctgg caagcccg 408

<210> 421
<211> 352
<212> DNA
<213> Homo sapiens

<220>

<221> misc_feature
<222> (1)...(352)
<223> n = A,T,C or G

<400> 421
gctcaaaaat ctttttactg atnggcatgg ctacacaatc attgactatt acggaggcca 60
gaggagaatg aggcctggcc tgggagccct gtgcctacta naagcacatt agattatcca 120
ttcactgaca gaacaggtct tttttgggtc cttcttctcc accacnatat acttgcagtc 180
ctccttcttg aagattcttt ggcagttgtc tttgtcataa cccacaggtg tagaaacaag 240
ggtgcaacat gaaatttctg tttcgtagca agtgcatgtc tcacaagttg gcangtctgc 300
cactccgagt ttattgggtg tttgtttcct ttgagatcca tgcatttcct gg 352

<210> 422
<211> 337
<212> DNA
<213> Homo sapiens

<400> 422
atgccaccat gctggcaatg cagcgggcgg tcgaaggcct gcatatccag cccaagctgg 60
cgatgatcga cggcaaccgt tgcccgaagt tgccgatgcc agccgaagcg gtggtcaagg 120
gcgatagcaa ggtgccggcg atcgcggcgg cgtcaatcct ggccaaggtc agccgtgatc 180
gtgaaatggc agctgtcgaa ttgatctacc cgggttatgg catcggcggg cataagggct 240
atccgacacc ggtgcacctg gaagccttgc agcggctggg gccgacgccg attcaccgac 300
gcttcttccg ccggtacggc tggcctatga aaattat 337

<210> 423
<211> 310
<212> DNA
<213> Homo sapiens

<220>
<221> misc_feature
<222> (1)...(310)
<223> n = A,T,C or G

<400> 423
gctcaaaaat ctttttactg atatggcatg gctacacaat cattgactat tagaggccag 60
aggagaatga ggcctggcct gggagccctg tgcctactan aagcncatta gattatccat 120
tcactgacag aacaggtctt ttttgggtcc ttcttctcca ccacgatata cttgcagtcc 180
tccttcttga agattctttg gcagttgtct ttgtcataac ccacaggtgt anaaacaagg 240
gtgcaacatg aaatttctgt ttcgtagcaa gtgcatgtct cacagttgtc aagtctgccc 300
tccgagttta 310

<210> 424
<211> 370
<212> DNA
<213> Homo sapiens

<220>
<221> misc_feature
<222> (1)...(370)
<223> n = A,T,C or G

<400> 424
gctcaaaaat ctttttactg ataggcatgg ctacacaatc attgactatt agaggccaga 60
ggagaatgag gcctggcctg ggagccctgt gcctactaga agcacattag attatccatt 120
cactgacaga acaggtcttt tttgggtcct tcttctccac cacgatatac ttgcagtcct 180
ccttcttgaa gattctttgg cagttgtctt tgtcataacc cacaggtgta gaaacatcct 240
ggttgaatct cctggaactc cctcattagg tatgaaatag catgatgcat tgcataaagt 300
cacgaaggtg gcaaagatca caacgctgcc cagganaaca ttcattgtga taagcaggac 360
tccgtcgacg 370

<210> 425
<211> 216
<212> DNA
<213> Homo sapiens

<220>
<221> misc_feature
<222> (1)...(216)
<223> n = A,T,C or G

<400> 425
aattgctatn ntttattttg ccactcaaaa taattaccaa aaaaaaaaaa tnttaaatga 60
taacaacnca acatcaaggn aaananaaca ggaatggntg actntgcata aatnggccga 120
anattatcca ttatnttaag ggttgacttc aggntacagc acacagacaa acatgcccag 180
gaggntntca ggaccgctcg atgtnttntg aggagg 216

<210> 426
<211> 596
<212> DNA
<213> Homo sapiens

<400> 426
cttccagtga ggataaccct gttgccccgg gccgaggttc tccattaggc tctgattgat 60
tggcagtcag tgatggaagg gtgttctgat cattccgact gccccaaggg tcgctggcca 120
gctctctgtt ttgctgagtt ggcagtagga cctaatttgt taattaagag tagatggtga 180
gctgtccttg tattttgatt aacctaatgg ccttcccagc acgactcgga ttcagctgga 240
gacatcacgg caacttttaa tgaaatgatt tgaagggcca ttaagaggca cttcccgtta 300
ttaggcagtt catctgcact gataacttct tggcagctga gctggtcgga gctgtggccc 360
aaacgcacac ttggcttttg gttttgagat acaactctta atcttttagt catgcttgag 420
ggtggatggc cttttcagct ttaacccaat ttgcactgcc ttggaagtgt agccaggaga 480
atacactcat atactcgtgg gcttagaggc cacagcagat gtcattggtc tactgcctga 540
gtcccgctgg tcccatccca ggaccttcca tcggcgagta cctgggagcc cgtgct 596

<210> 427
<211> 107
<212> DNA
<213> Homo sapiens

<220>
<221> misc_feature
<222> (1)...(107)
<223> n = A,T,C or G

<400> 427
gaagaattca agttaggttt attcaaaggg cttacngaga atcctanacc caggncccag 60
cccgggagca gccttanaga gctcctgttt gactgcccgg ctcagng 107

<210> 428
<211> 38
<212> DNA
<213> Homo sapiens

<220>
<221> misc_feature
<222> (1)...(38)
<223> n = A,T,C or G

<400> 428
gaacttccna anaangactt tattcactat tttacatt 38

<210> 429
<211> 544

<212> DNA
<213> Homo sapiens

<400> 429
ctttgctgga cggaataaaa gtggacgcaa gcatgacctc ctgatgaggg cgctgcattt 60
attgaagagc ggctgcagcc ctgcggttca gattaaaatc cgagaattgt atagacgccg 120
atatccacga actcttgaag gactttctga tttatccaca atcaaatcat cggttttcag 180
tttggatggt ggctcatcac ctgtagaacc tgacttggcc gtggctggaa tccactcgtt 240
gccttccact tcagttacac ctcactcacc atcctctcct gttggttctg tgctgcttca 300
agatactaag cccacatttg agatgcagca gccatctccc ccaattcctc ctgtccatcc 360
tgatgtgcag ttaaaaaatc tgccctttta tgatgtcctt gatgttctca tcaagcccac 420
gagtttagtt caaagcagta ttcagcgatt tcaagagaag ttttttattt ttgctttgac 480
acctcaacaa gttagagaga tatgcatatc cagggatttt ttgccaggtg gtaggagaga 540
ttat 544

<210> 430
<211> 507
<212> DNA
<213> Homo sapiens

<220>
<221> misc_feature
<222> (1)...(507)
<223> n = A,T,C or G

<400> 430
   cttatcncaa tggggctccc aaacttggct gtgcagtgga aactccgggg gaattttgaa 60
   gaacactgac acccatcttc caccccgaca ctctgattta attgggctgc agtgagaaca 120
   gagcatcaat ttaaaaagct gcccagaatg ttntcctggg cagcgttgtg atctttgccn 180
   ccttcgtgac tttatgcaat gcatcatgct atttcatacc taatgaggga gttccaggag 240
   attcaaccag gatgtttcta cncctgtggg ttatgacaaa gacaactgcc aaagaatntt 300
   caagaaggag gactgcaagt atatcgtggt ggagaagaag gacccaaaaa agacctgttc 360
   tgtcagtgaa tggataatct aatgtgcttc tagtaggcac agggctccca ggccaggcct 420
   cattctcctc tggcctctaa tagtcaatga ttgtgtagcc atgcctatca gtaaaaagat 480
   ttttgagcaa aaaaaaaaaa aaaaaaa 507

<210> 431
<211> 392
<212> DNA
<213> Homo sapiens

<220>
<221> misc_feature
<222> (1)...(392)
<223> n = A,T,C or G

<400> 431
gaaaattcag aatggataaa aacaaatgaa gtacaaaata tttcagattt acatagcgat 60
aaacaagaaa gcacttatca ggaggactta caaatggaag tacactctan aaccatcatc 120
tatcatggct aaatgtgaga ttagcacagc tgtattattt gtacattgca aacacctaga 180
aagagatggg aaacaaaatc ccaggagttt tgtgtgtgga gtcctgggtt ttccaacaga 240
catcattcca gcattctgag attagggnga ttggggatca ttctggagtt ggaatgttca 300
acaaaagtga tgttgttagg taaaatgtac aacttctgga tctatgcaga cattgaaggt 360
gcaatgagtc tggcttttac tctgctgttt ct 392

<210> 432
<211> 387
<212> DNA
<213> Homo sapiens

<220>
<221> misc_feature

<222> (1)...(387)
<223> n = A,T,C or G

<400> 432
ggtatccnta cataatcaaa tatagctgta gtacatgttt tcattggngt agattaccac 60
aaatgcaagg caacatgtgt agatctcttg tcttattctt ttgtctataa tactgtattg 120
ngtagtccaa gctctcggna gtccagccac tgngaaacat gctcccttta gattaacctc 180
gtggacnctn ttgttgnatt gtctgaactg tagngccctg tattttgctt ctgtctgnga 240
attctgttgc ttctggggca tttccttgng atgcagagga ccaccacaca gatgacagca 300
atctgaattg ntccaatcac agctgcgatt aagacatact gaaatcgtac aggaccggga 360
acaacgtata gaacactgga gtccttt 387

<210> 433
<211> 281
<212> DNA
<213> Homo sapiens

<220>
<221> misc_feature
<222> (1)...(281)
<223> n = A,T,C or G

<400> 433
ttcaactagc anagaanact gcttcagggn gtgtaaaatg aaaggcttcc acgcagttat 60
ctgattaaag aacactaaga gagggacaag gctagaagcc gcaggatgtc tacactatag 120
caggcnctat ttgggttggc tggaggagct gtggaaaaca tggagagatt ggcgctggag 180
atcgccgtgg ctattcctcn ttgntattac accagngagg ntctctgtnt gcccactggt 240
tnnaaaaccg ntatacaata atgatagaat aggacacaca t 281

<210> 434
<211> 484
<212> DNA
<213> Homo sapiens

<400> 434
ttttaaaata agcatttagt gctcagtccc tactgagtac tctttctctc ccctcctctg 60
aatttaattc tttcaacttg caatttgcaa ggattacaca tttcactgtg atgtatattg 120
tgttgcaaaa aaaaaaaagt gtctttgttt aaaattactt ggtttgtgaa tccatcttgc 180
tttttcccca ttggaactag tcattaaccc atctctgaac tggtagaaaa acatctgaag 240
agctagtcta tcagcatctg acaggtgaat tggatggttc tcagaaccat ttcacccaga 300
cagcctgttt ctatcctgtt taataaatta gtttgggttc tctacatgca taacaaaccc 360
tgctccaatc tgtcacataa aagtctgtga cttgaagttt agtcagcacc cccaccaaac 420
tttatttttc tatgtgtttt ttgcaacata tgagtgtttt gaaaataaag tacccatgtc 480
ttta 484

<210> 435
<211> 424
<212> DNA
<213> Homo sapiens

<400> 435
gcgccgctca gagcaggtca ctttctgcct tccacgtcct ccttcaagga agccccatgt 60
gggtagcttt caatatcgca ggttcttact cctctgcctc tataagctca aacccaccaa 120
cgatcgggca agtaaacccc ctccctcgcc gacttcggaa ctggcgagag ttcagcgcag 180
atgggcctgt ggggaggggg caagatagat gagggggagc ggcatggtgc ggggtgaccc 240
cttggagaga ggaaaaaggc cacaagaggg gctgccaccg ccactaacgg agatggccct 300
ggtagagacc tttgggggtc tggaacctct ggactcccca tgctctaact cccacactct 360
gctatcagaa acttaaactt gaggattttc tctgtttttc actcgcaata aattcagagc 420
aaac 424

<210> 436
<211> 667

<212> DNA
<213> Homo sapiens

<220>
<221> misc_feature
<222> (1)...(667)
<223> n = A,T,C or G

<400> 436
   accttgggaa nactctcaca atataaaggg tcgtagactt tactccaaat tccaaaaagg 60
   tcctggccat gtaatcctga aagttttccc aaggtagcta taaaatcctt ataagggtgc 120
   agcctcttct ggaattcctc tgatttcaaa gtctcactct caagttcttg aaaacgaggg 180
   cagttcctga aaggcaggta tagcaactga tcttcagaaa gaggaactgt gtgcaccggg 240
   atgggctgcc agagtaggat aggattccag atgctgacac cttctggggg aaacagggct 300
   gccaggtttg tcatagcact catcaaagtc cggtcaacgt ctgtgcttcg aatataaacc 360
   tgttcatgtt tataggactc attcaagaat tttctatatc tctttcttat atactctcca 420
   agttcataat gctgctccat gcccagctgg gtgagttggc caaatccttg tggccatgag 480
   gattccttta tggggtcagt gggaaaggtg tcaatgggac ttcggtctcc atgccgaaac 540
   accaaagtca caaacttcaa ctccttggct agtacacttc ggtctagcca gaaaaaaagc 600
   agaaacaaga agccaaggct aaggcttgct gccctgccag gaggaggggt gcagctctca 660
   tgttgag 667

<210> 437
<211> 693
<212> DNA
<213> Homo sapiens

<400> 437
ctacgtctca accctcattt ttaggtaagg aatcttaagt ccaaagatat taagtgactc 60
acacagccag gtaaggaaag ctggattggc acactaggac tctaccatac cgggttttgt 120
taaagctcag gttaggaggc tgataagctt ggaaggaact tcagacagct ttttcagatc 180
ataaaagata attcttagcc catgttcttc tccagagcag acctgaaatg acagcacagc 240
aggtactcct ctattttcac ccctcttgct tctactctct ggcagtcaga cctgtgggag 300
gccatgggag aaagcagctc tctggatgtt tgtacagatc atggactatt ctctgtggac 360
catttctcca ggttacccta ggtgtcacta ttggggggac agccagcatc tttagctttc 420
atttgagttt ctgtctgtct tcagtagagg aaacttttgc tcttcacact tcacatctga 480
acacctaact gctgttgctc ctgaggtggt gaaagacaga tatagagctt acagtattta 540
tcctatttct aggcactgag ggctgtgggg taccttgtgg tgccaaaaca gatcctgttt 600
taaggacatg ttgcttcaga gatgtctgta actatctggg ggctctgttg gctctttacc 660
ctgcatcatg tgctctcttg gctgaaaatg acc 693

<210> 438
<211> 360
<212> DNA
<213> Homo sapiens

<400> 438
ctgcttatca caatgaatgt tctcctgggc agcgttgtga tctttgccac cttcgtgact 60
ttatgcaatg catcatgcta tttcatacct aatgagggag ttccaggaga ttcaaccagg 120
atgtttctac acctgtgggt tatgacaaag acaactgcca aagaatcttc aagaaggagg 180
actgcaagta tatctggtgg agaagaagga cccaaaaaag acctgttctg tcagtgaatg 240
gataatctaa tgtgcttcta gtaggcacag ggctcccagg ccaggcctca ttctcctctg 300
gcctctaata gtcaataatt gtgtagccat gcctatcagt aaaaagattt ttgagcaaac 360

<210> 439
<211> 431
<212> DNA
<213> Homo sapiens

<220>
<221> misc_feature
<222> (1)...(431)

<223> n = A,T,C or G

<400> 439
gttcctnnta actcctgcca gaaacagctc tcctcaacat gagagctgca cccctcctcc 60
tggccagggc agcaagcctt agccttggct tcttgtttct gctttttttc tggctagacc 120
gaagtgtact agccaaggag ttgaagtttg tgactttggt gtttcggcat ggagaccgaa 180
gtcccattga cacctttccc actgacccca taaaggaatc ctcatggcca caaggatttg 240
gccaactcac ccagctgggc atggagcagc attatgaact tggagagtat ataagaaaga 300
gatatagaaa attcttgaat gagtcctata aacatgaaca ggtttatatt cgaagcacag 360
acgttgaccg gactttgatg agtgctatga caaacctggc agcccgtcga cgcggccgcg 420
aatttagtag t 431

<210> 440
<211> 523
<212> DNA
<213> Homo sapiens

<400> 440
agagataaag cttaggtcaa agttcataga gttcccatga actatatgac tggccacaca 60
ggatcttttg tatttaagga ttctgagatt ttgcttgagc aggattagat aaggctgttc 120
tttaaatgtc tgaaatggaa cagatttcaa aaaaaaaccc cacaatctag ggtgggaaca 180
aggaaggaaa gatgtgaata ggctgatggg caaaaaacca atttacccat cagttccagc 240
cttctctcaa ggagaggcaa agaaaggaga tacagtggag acatctggaa agttttctcc 300
actggaaaac tgctactatc tgtttttata tttctgttaa aatatatgag gctacagaac 360
taaaaattaa aacctctttg tgtcccttgg tcctggaaca tttatgttcc ttttaaagaa 420
acaaaaatca aactttacag aaagatttga tgtatgtaat acatatagca gctcttgaag 480
tatatatatc atagcaaata agtcatctga tgagaacaag cta 523

<210> 441
<211> 430
<212> DNA
<213> Homo sapiens

<400> 441
gttcctccta actcctgcca gaaacagctc tcctcaacat gagagctgca cccctcctcc 60
tggccagggc agcaagcctt agccttggct tcttgtttct gctttttttc tggctagacc 120
gaagtgtact agccaaggag ttgaagtttg tgactttggt gtttcggcat ggagaccgaa 180
gtcccattga cacctttccc actgacccca taaaggaatc ctcatggcca caaggatttg 240
gccaactcac ccagctgggc atggagcagc attatgaact tggagagtat ataagaaaga 300
gatatagaaa attcttgaat gagtcctata aacatgaaca ggtttatatt cgaagcacag 360
acgttgaccg gactttgatg agtgctatga caaacctggc agcccgtcga cgcggccgcg 420
aatttagtag 430

<210> 442
<211> 362
<212> DNA
<213> Homo sapiens

<400> 442
ctaaggaatt agtagtgttc ccatcacttg tttggagtgt gctattctaa aagattttga 60
tttcctggaa tgacaattat attttaactt tggtggggga aagagttata ggaccacagt 120
cttcacttct gatacttgta aattaatctt ttattgcact tgttttgacc attaagctat 180
atgtttagaa atggtcattt tacggaaaaa ttagaaaaat tctgataata gtgcagaata 240
aatgaattaa tgttttactt aatttatatt gaactgtcaa tgacaaataa aaattctttt 300
tgattatttt ttgttttcat ttaccagaat aaaaactaag aattaaaagt ttgattacag 360
tc 362

<210> 443
<211> 624
<212> DNA
<213> Homo sapiens

<220>
<221> misc_feature
<222> (1)...(624)
<223> n = A,T,C or G

<400> 443
tttttttttt gcaacacaat atacatcaca gtgaaatgtg taatccttgc aaattgcaag 60
ttgaaagaat taaattcaga ggaggggaga gaaagagtac tcagtaggga ctgagcacta 120
aatgcttatt ttaaaagaaa tgtaaagagc agaaagcaat tcaggctacc ctgccttttg 180
tgctggctag tactccggtc ggtgtcagca gcacgtggca ttgaacattg caatgtggag 240
cccaaaccac agaaaatggg gtgaaattgg ccaactttct attaacttgg cttcctgttt 300
tataaaatat tgtgaataat atcacctact tcaaagggca gttatgaggc ttaaatgaac 360
taacgcctac aaaacactta aacatagata acataggtgc aagtactatg tatctggtac 420
atggtaaaca tccttattat taaagtcaac gctaaaatga atgtgtgtgc atatgctaat 480
agtacagaga gagggcactt aaaccaacta agggcctgga gggaaggttt cctggaaaga 540
ngatgcttgt gctgggtcca aatcttggtc tactatgacc ttggccaaat tatttaaact 600
ttgtccctat ctgctaaaca gatc 624

<210> 444
<211> 425
<212> DNA
<213> Homo sapiens

<220>
<221> misc_feature
<222> (1)...(425)
<223> n = A,T,C or G

<400> 444
gcacatcatt nntcttgcat tctttgagaa taagaagatc agtaaatagt tcagaagtgg 60
gaagctttgt ccaggcctgt gtgtgaaccc aatgttttgc ttagaaatag aacaagtaag 120
ttcattgcta tagcataaca caaaatttgc ataagtggtg gtcagcaaat ccttgaatgc 180
tgcttaatgt gagaggttgg taaaatcctt tgtgcaacac tctaactccc tgaatgtttt 240
gctgtgctgg gacctgtgca tgccagacaa ggccaagctg gctgaaagag caaccagcca 300
cctctgcaat ctgccacctc ctgctggcag gatttgtttt tgcatcctgt gaagagccaa 360
ggaggcacca gggcataagt gagtagactt atggtcgacg cggccgcgaa tttagtagta 420
gtaga 425

<210> 445
<211> 414
<212> DNA
<213> Homo sapiens

<220>
<221> misc_feature
<222> (1)...(414)
<223> n = A,T,C or G

<400> 445
catgtttatg nttttggatt actttgggca cctagtgttt ctaaatcgtc tatcattctt 60
ttctgttttt caaaagcaga gatggccaga gtctcaacaa actgtatctt caagtctttg 120
tgaaattctt tgcatgtggc agattattgg atgtagtttc ctttaactag catataaatc 180
tggtgtgttt cagataaatg aacagcaaaa tgtggtggaa ttaccatttg gaacattgtg 240
aatgaaaaat tgtgtctcta gattatgtaa caaataacta tttcctaacc attgatcttt 300
ggatttttat aatcctactc acaaatgact aggcttctcc tcttgtattt tgaagcagtg 360
tgggtgctgg attgataaaa aaaaaaaaag tcgacgcggc cgcgaattta gtag 414

<210> 446
<211> 631
<212> DNA
<213> Homo sapiens

<220>
<221> misc_feature
<222> (1)...(631)
<223> n = A,T,C or G

<400> 446
acaaattaga anaaagtgcc agagaacacc acataccttg tccggaacat tacaatggct 60
tctgcatgca tgggaagtgt gagcattcta tcaatatgca ggagccatct tgcaggtgtg 120
atgctggtta tactggacaa cactgtgaaa aaaaggacta cagtgttcta tacgttgttc 180
ccggtcctgt acgatttcag tatgtcttaa tcgcagctgt gattggaaca attcagattg 240
ctgtcatctg tgtggtggtc ctctgcatca caagggccaa actttaggta atagcattgg 300
actgagattt gtaaactttc caaccttcca ggaaatgccc cagaagcaac agaattcaca 360
gacagaagca aaatacaggg cactacagtt cagacaatac aacaagagcg tccacgaggt 420
taatctaaag ggagcatgtt tcacagtggc tggactaccg agagcttgga ctacacaata 480
cagtattata gacaaaagaa taagacaaga gatctacaca tgttgccttg catttgtggt 540
aatctacacc aatgaaaaca tgtactacag ctatatttga ttatgtatgg atatatttga 600
aatagtatac attgtcttga tgttttttct g 631

<210> 447
<211> 585
<212> DNA
<213> Homo sapiens

<220>
<221> misc_feature
<222> (1)...(585)
<223> n = A,T,C or G

<400> 447
ccttgggaaa antntcacaa tataaagggt cgtagacttt actccaaatt ccaaaaaggt 60
cctggccatg taatcctgaa agttttccca aggtagctat aaaatcctta taagggtgca 120
gcctcttctg gaattcctct gatttcaaag tctcactctc aagttcttga aaacgagggc 180
agttcctgaa aggcaggtat agcaactgat cttcagaaag aggaactgtg tgcaccggga 240
tgggctgcca gagtaggata ggattccaga tgctgacacc ttctggggga aacagggctg 300
ccaggtttgt catagcactc atcaaagtcc ggtcaacgtc tgtgcttcga atataaacct 360
gttcatgttt ataggactca ttcaagaatt ttctatatct ctttcttata tactctccaa 420
gttcataatg ctgctccatg cccagctggg tgagttggcc aaatccttgt ggccatgagg 480
attcctttat ggggtcagtg ggaaaggtgt caatgggact tcggtctcca tgccgaaaca 540
ccaaagtcac aaacttcaac tccttggcta gtacacttcg gtcta 585

<210> 448
<211> 93
<212> DNA
<213> Homo sapiens

<220>
<221> misc_feature
<222> (1)...(93)
<223> n = A,T,C or G

<400> 448
tgctcgtggg tcattctgan nnccgaactg accntgccag ccctgccgan gggccnccat 60
ggctccctag tgccctggag agganggggc tag 93

<210> 449
<211> 706
<212> DNA
<213> Homo sapiens

<220>
<221> misc_feature
<222> (1)...(706)

<223> n = A,T,C or G

<400> 449
ccaagttcat gctntgtgct ggacgctgga cagggggcaa aagcnnttgc tcgtgggtca 60
ttctgancac cgaactgacc atgccagccc tgccgatggt cctccatggc tccctagtgc 120
cctggagagg aggtgtctag tcagagagta gtcctggaag gtggcctctg ngaggagcca 180
cggggacagc atcctgcaga tggtcggcgc cgtcccattc gccattcagg ctgcgcaact 240
gttgggaagg gcgatcggtg cgggcctctt cgctattacg ccagctggcg aaagggggat 300
gtgctgcaag gcgattaagt tgggtaacgc cagggttttc ccagtcncga cgttgtaaaa 360
cgacggccag tgaattgaat ttaggtgacn ctatagaaga gctatgacgt cgcatgcacg 420
cgtacgtaag cttggatcct ctagagcggc cgcctactac tactaaattc gcggccgcgt 480
cgacgtggga tccncactga gagagtggag agtgacatgt gctggacnct gtccatgaag 540
cactgagcag aagctggagg cacaacgcnc cagacactca cagctactca ggaggctgag 600
aacaggttga acctgggagg tggaggttgc aatgagctga gatcaggccn ctgcncccca 660
gcatggatga cagagtgaaa ctccatctta aaaaaaaaaa aaaaaa 706

<210> 450
<211> 493
<212> DNA
<213> Homo sapiens

<400> 450
gagacggagt gtcactctgt tgcccaggct ggagtgcagc aagacactgt ctaagaaaaa 60
acagttttaa aaggtaaaac aacataaaaa gaaatatcct atagtggaaa taagagagtc 120
aaatgaggct gagaacttta caaagggatc ttacagacat gtcgccaata tcactgcatg 180
agcctaagta taagaacaac ctttggggag aaaccatcat ttgacagtga ggtacaattc 240
caagtcaggt agtgaaatgg gtggaattaa actcaaatta atcctgccag ctgaaacgca 300
agagacactg tcagagagtt aaaaagtgag ttctatccat gaggtgattc cacagtcttc 360
tcaagtcaac acatctgtga actcacagac caagttctta aaccactgtt caaactctgc 420
tacacatcag aatcacctgg agagctttac aaactcccat tgccgagggt cgacgcggcc 480
gcgaatttag tag 493

<210> 451
<211> 501
<212> DNA
<213> Homo sapiens

<220>
<221> misc_feature
<222> (1)...(501)
<223> n = A,T,C or G

<400> 451
gggcgcgtcc cattcgccat tcaggctgcg caactgttgg gaagggcgat cggtgcgggc 60
ctcttcgcta ttacgccagc tggcgaaagg gggatgtgct gcaaggcgat taagttgggt 120
aacgccaggg ttttcccagt cncgacgttg taaaacgacg gccagtgaat tgaatttagg 180
tgacnctata gaagagctat gacgtcgcat gcacgcgtac gtaagcttgg atcctctaga 240
gcggccgcct actactacta aattcgcggc cgcgtcgacg tgggatccnc actgagagag 300
tggagagtga catgtgctgg acnctgtcca tgaagcactg agcagaagct ggaggcacaa 360
cgcnccagac actcacagct actcaggagg ctgagaacag gttgaacctg ggaggtggag 420
gttgcaatga gctgagatca ggccnctgcn ccccagcatg gatgacagag tgaaactcca 480
tcttaaaaaa aaaaaaaaaa a 501

<210> 452
<211> 51
<212> DNA
<213> Homo sapiens

<220>
<221> misc_feature
<222> (1)...(51)
<223> n = A,T,C or G

<400> 452
agacggtttc accnttacaa cnccttttag gatgggnntt ggggagcaag c          51

<210> 453
<211> 317
<212> DNA
<213> Homo sapiens

<220>
<221> misc_feature
<222> (1)...(317)
<223> n = A,T,C or G

<400> 453
tacatcttgc tttttcccca ttggaactag tcattaaccc atctctgaac tggtagaaaa 60
acatctgaag agctagtcta tcagcatctg gcaagtgaat tggatggttc tcagaaccat 120
ttcacccana cagcctgttt ctatcctgtt taataaatta gtttgggttc tctacatgca 180
taacaaaccc tgctccaatc tgtcacataa aagtctgtga cttgaagttt antcagcacc 240
cccaccaaac tttatttttc tatgtgtttt ttgcaacata tgagtgtttt gaaaataagg 300
tacccatgtc tttatta                                                 317

<210> 454
<211> 231
<212> DNA
<213> Homo sapiens

<400> 454
ttcgaggtac aatcaactct cagagtgtag tttccttcta tagatgagtc agcattaata 60
taagccacgc cacgctcttg aaggagtctt gaattctcct ctgctcactc agtagaacca 120
agaagaccaa attcttctgc atcccagctt gcaaacaaaa ttgttcttct aggtctccac 180
ccttcctttt tcagtgttcc aaagctcctc acaatttcat gaacaacagc t          231

<210> 455
<211> 231
<212> DNA
<213> Homo sapiens

<400> 455
taccaaagag ggcataataa tcagtctcac agtagggttc accatcctcc aagtgaaaaa 60
cattgttccg aatgggcttt ccacaggcta cacacacaaa acaggaaaca tgccaagttt 120
gtttcaacgc attgatgact tctccaagga tcttcctttg gcatcgacca cattcagggg 180
caaagaattt ctcatagcac agctcacaat acagggctcc tttctcctct a          231

<210> 456
<211> 231
<212> DNA
<213> Homo sapiens

<400> 456
ttggcaggta cccttacaaa gaagacacca taccttatgc gttattaggt ggaataatca 60
ttccattcag tattatcgtt attattcttg gagaaaccct gtctgtttac tgtaaccttt 120
tgcactcaaa ttcctttatc aggaataact acatagccac tatttacaaa gccattggaa 180
cctttttatt tggtgcagct gctagtcagt ccctgactga cattgccaag t          231

<210> 457
<211> 231
<212> DNA
<213> Homo sapiens

<220>
<221> misc_feature

<222> (1)...(231)
<223> n = A,T,C or G

<400> 457
cgaggtaccc aggggtctga aaatctctnn tttantagtc gatagcaaaa ttgttcatca 60
gcattcctta atatgatctt gctataatta gatttttctc cattagagtt catacagttt 120
tatttgattt tattagcaat ctctttcaga agacccttga gatcattaag ctttgtatcc 180
agttgtctaa atcgatgcct catttcctct gaggtgtcgc tggcttttgt g 231

<210> 458
<211> 231
<212> DNA
<213> Homo sapiens

<400> 458
aggtctggtt ccccccactt ccactcccct ctactctctc taggactggg ctgggccaag 60
agaagagggg tggttaggga agccgttgag acctgaagcc ccaccctcta ccttccttca 120
acaccctaac cttgggtaac agcatttgga attatcattt gggatgagta gaatttccaa 180
ggtcctgggt taggcatttt gggggggcag accccaggag aagaagattc t 231

<210> 459
<211> 231
<212> DNA
<213> Homo sapiens

<400> 459
ggtaccgagg ctcgctgaca cagagaaacc ccaacgcgag gaaaggaatg gccagccaca 60
ccttcgcgaa acctgtggtg gcccaccagt cctaacggga caggacagag agacagagca 120
gccctgcact gttttccctc caccacagcc atcctgtccc tcattggctc tgtgctttcc 180
actatacaca gtcaccgtcc caatgagaaa caagaaggag caccctccac a 231

<210> 460
<211> 231
<212> DNA
<213> Homo sapiens

<400> 460
gcaggtataa catgctgcaa caacagatgt gactaggaac ggccggtgac atggggaggg 60
cctatcaccc tattcttggg ggctgcttct tcacagtgat catgaagcct agcagcaaat 120
cccacctccc cacacgcaca cggccagcct ggagcccaca gaagggtcct cctgcagcca 180
gtggagcttg gtccagcctc cagtccaccc ctaccaggct taaggataga a 231

<210> 461
<211> 231
<212> DNA
<213> Homo sapiens

<400> 461
cgaggtttga gaagctctaa tgtgcagggg agccgagaag caggcggcct agggagggtc 60
gcgtgtgctc cagaagagtg tgtgcatgcc agaggggaaa caggcgcctg tgtgtcctgg 120
gtggggttca gtgaggagtg ggaaattggt tcagcagaac caagccgttg ggtgaataag 180
agggggattc catggcactg atagagccct atagtttcag agctgggaat t 231

<210> 462
<211> 231
<212> DNA
<213> Homo sapiens

<400> 462
aggtaccctc attgtagcca tgggaaaatt gatgttcagt ggggatcagt gaattaaatg 60
gggtcatgca agtataaaaa ttaaaaaaaa aagacttcat gcccaatctc atatgatgtg 120
gaagaactgt tagagagacc aacagggtag tgggttagag atttccagag tcttacattt 180

tctagaggag gtatttaatt tcttctcact catccagtgt tgtatttagg a 231

<210> 463
<211> 231
<212> DNA
<213> Homo sapiens

<400> 463
tactccagcc tggtgacaga gcgagaccct atcaccgccc cccaccccac caaaaaaaaa 60
actgagtaga caggtgtcct cttggcatgg taagtcttaa gtcccctccc agatctgtga 120
catttgacag gtgtcttttc ctctggacct cggtgtcccc atctgagtga gaaaaggcag 180
tggggaggtg gatcttccag tcgaagcggt atagaagccc gtgtgaaaag c 231

<210> 464
<211> 231
<212> DNA
<213> Homo sapiens

<400> 464
gtactctaag attttatcta agttgccttt tctgggtggg aaagtttaac cttagtgact 60
aaggacatca catatgaaga atgtttaagt tggaggtggc aacgtgaatt gcaaacaggg 120
cctgcttcag tgactgtgtg cctgtagtcc cagctactcg ggagtctgtg tgaggccagg 180
ggtgccagcg caccagctag atgctctgta acttctaggc cccattttcc c 231

<210> 465
<211> 231
<212> DNA
<213> Homo sapiens

<400> 465
catgttgttg tagctgtggt aatgctggct gcatctcaga cagggttaac ttcagctcct 60
gtggcaaatt agcaacaaat tctgacatca tatttatggt ttctgtatct ttgttgatga 120
aggatggcac aatttttgct tgtgttcata atatactcag attagttcag ctccatcaga 180
taaactggag acatgcagga cattagggta gtgttgtagc tctggtaatg a 231

<210> 466
<211> 231
<212> DNA
<213> Homo sapiens

<400> 466
caggtacctc tttccattgg atactgtgct agcaagcatg ctctccgggg tttttttaat 60
ggccttcgaa cagaacttgc cacataccca ggtataatag tttctaacat ttgcccagga 120
cctgtgcaat caaatattgt ggagaattcc ctagctggag aagtcacaaa gactataggc 180
aataatggag accagtccca caagatgaca accagtcgtt gtgtgcggct g 231

<210> 467
<211> 311
<212> DNA
<213> Homo sapiens

<400> 467
gtacaccctg gcacagtcca atctgaactg gttcggcact catctttcat gagatggatg 60
tggtggcttt tctccttttt catcaagact cctcagcagg gagcccagac cagcctgcac 120
tgtgccttaa cagaaggtct tgagattcta agtgggaatc atttcagtga ctgtcatgtg 180
gcatgggtct ctgcccaagc tcgtaatgag actatagcaa ggcggctgtg ggacgtcagt 240
tgtgacctgc tgggcctccc aatagactaa caggcagtgc cagttggacc caagagaaga 300
ctgcagcaga c 311

<210> 468
<211> 3112
<212> DNA

<213> Homo sapiens

<400> 468
cattgtgttg ggagaaaaac agaggggaga tttgtgtggc tgcagccgag ggagaccagg 60
aagatctgca tggtgggaag gacctgatga tacagagttt gataggagac aattaaaggc 120
tggaaggcac tggatgcctg atgatgaagt ggactttcaa actggggcac tactgaaacg 180
atgggatggc cagagacaca ggagatgagt tggagcaagc tcaataacaa agtggttcaa 240
cgaggacttg gaattgcatg gagctggagc tgaagtttag cccaattgtt tactagttga 300
gtgaatgtgg atgattggat gatcatttct catctctgag cctcaggttc cccatccata 360
aaatgggata cacagtatga tctataaagt gggatatagt atgatctact tcactgggtt 420
atttgaagga tgaattgaga taatttattt caggtgccta gaacaatgcc cagattagta 480
catttggtgg aactgagaaa tggcataaca ccaaatttaa tatatgtcag atgttactat 540
gattatcatt caatctcata gttttgtcat ggcccaattt atcctcactt gtgcctcaac 600
aaattgaact gttaacaaag gaatctctgg tcctgggtaa tggctgagca ccactgagca 660
tttccattcc agttggcttc ttgggtttgc tagctgcatc actagtcatc ttaaataaat 720
gaagttttaa catttctcca gtgatttttt tatctcacct ttgaagatac tatgttatgt 780
gattaaataa agaacttgag aagaacaggt ttcattaaac ataaaatcaa tgtagacgca 840
aattttctgg atgggcaata cttatgttca caggaaatgc tttaaaatat gcagaagata 900
attaaatggc aatggacaaa gtgaaaaact tagacttttt tttttttttt ggaagtatct 960
ggatgttcct tagtcactta aaggagaact gaaaaatagc agtgagttcc acataatcca 1020
acctgtgaga ttaaggctct ttgtggggaa ggacaaagat ctgtaaattt acagtttcct 1080
tccaaagcca acgtcgaatt ttgaaacata tcaaagctct tcttcaagac aaataatcta 1140
tagtacatct ttcttatggg atgcacttat gaaaaatggt ggctgtcaac atctagtcac 1200
tttagctctc aaaatggttc attttaagag aaagttttag aatctcatat ttattcctgt 1260
ggaaggacag cattgtggct tggactttat aaggtcttta ttcaactaaa taggtgagaa 1320
ataagaaagg ctgctgactt taccatctga ggccacacat ctgctgaaat ggagataatt 1380
aacatcacta gaaacagcaa gatgacaata taatgtctaa gtagtgacat gtttttgcac 1440
atttccagcc cctttaaata tccacacaca caggaagcac aaaaggaagc acagagatcc 1500
ctgggagaaa tgcccggccg ccatcttggg tcatcgatga gcctcgccct gtgcctggtc 1560
ccgcttgtga gggaaggaca ttagaaaatg aattgatgtg ttccttaaag gatgggcagg 1620
aaaacagatc ctgttgtgga tatttatttg aacgggatta cagatttgaa atgaagtcac 1680
aaagtgagca ttaccaatga gaggaaaaca gacgagaaaa tcttgatggc ttcacaagac 1740
atgcaacaaa caaaatggaa tactgtgatg acatgaggca gccaagctgg ggaggagata 1800
accacggggc agagggtcag gattctggcc ctgctgccta aactgtgcgt tcataaccaa 1860
atcatttcat atttctaacc ctcaaaacaa agctgttgta atatctgatc tctacggttc 1920
cttctgggcc caacattctc catatatcca gccacactca tttttaatat ttagttccca 1980
gatctgtact gtgacctttc tacactgtag aataacatta ctcattttgt tcaaagaccc 2040
ttcgtgttgc tgcctaatat gtagctgact gtttttccta aggagtgttc tggcccaggg 2100
gatctgtgaa caggctggga agcatctcaa gatctttcca gggttatact tactagcaca 2160
cagcatgatc attacggagt gaattatcta atcaacatca tcctcagtgt ctttgcccat 2220
actgaaattc atttcccact tttgtgccca ttctcaagac ctcaaaatgt cattccatta 2280
atatcacagg attaactttt ttttttaacc tggaagaatt caatgttaca tgcagctatg 2340
ggaatttaat tacatatttt gttttccagt gcaaagatga ctaagtcctt tatccctccc 2400
ctttgtttga ttttttttcc agtataaagt taaaatgctt agccttgtac tgaggctgta 2460
tacagccaca gcctctcccc atccctccag ccttatctgt catcaccatc aacccctccc 2520
atgcacctaa acaaaatcta acttgtaatt ccttgaacat gtcaggcata cattattcct 2580
tctgcctgag aagctcttcc ttgtctctta aatctagaat gatgtaaagt tttgaataag 2640
ttgactatct tacttcatgc aaagaaggga cacatatgag attcatcatc acatgagaca 2700
gcaaatacta aaagtgtaat ttgattataa gagtttagat aaatatatga aatgcaagag 2760
ccacagaggg aatgtttatg gggcacgttt gtaagcctgg gatgtgaagc aaaggcaggg 2820
aacctcatag tatcttatat aatatacttc atttctctat ctctatcaca atatccaaca 2880
agcttttcac agaattcatg cagtgcaaat ccccaaaggt aacctttatc catttcatgg 2940
tgagtgcgct ttagaatttt ggcaaatcat actggtcact tatctcaact ttgagatgtg 3000
tttgtccttg tagttaattg aaagaaatag ggcactcttg tgagccactt tagggttcac 3060
tcctggcaat aaagaattta caaagagcaa aaaaaaaaaa aaaaaaaaaa aa 3112

<210> 469
<211> 2229
<212> DNA
<213> Homo sapiens

<400> 469

agctctttgt aaattcttta ttgccaggag tgaaccctaa agtggctcac aagagtgccc 60
tatttctttc aattaactac aaggacaaac acatctcaaa gttgagataa gtgaccagta 120
tgatttgcca aaattctaaa gcgcactcac catgaaatgg ataaaggtta cctttgggga 180
tttgcactgc atgaattctg tgaaaagctt gttggatatt gtgatagaga tagagaaatg 240
aagtatatta tataagatac tatgaggttc cctgcctttg cttcacatcc caggcttaca 300
aacgtgcccc ataaacattc cctctgtggc tcttgcattt catatattta tctaaactct 360
tataatcaaa tacactttta gtatttgctg tctcatgtga tgatgaatct catatgtgtc 420
ccttctttgc atgaagtaag atagtcaact tattcaaaac tttacatcat tctagattta 480
agagacaagg aagagcttct caggcagaag gaataatgta tgcctgacat gttcaaggaa 540
ttacaagtta gattttgttt aggtgcatgg gaggggttga tggtgatgac agataaggct 600
ggagggatgg ggagaggctg tggctgtata cagcctcagt acaaggctaa gcattttaac 660
tttatactgg aaaaaaaatc aaacaaaggg gagggataaa ggacttagtc atctttgcac 720
tggaaaacaa aatatgtaat taaattccca tagctgcatg taacattgaa ttcttccagg 780
ttaaaaaaaa agttaatcct gtgatattaa tggaatgaca ttttgaggtc ttgagaatgg 840
gcacaaaagt gggaaatgaa tttcagtatg ggcaaagaca ctgaggatga tgttgattag 900
ataattcact ccgtaatgat catgctgtgt gctagtaagt ataaccctgg aaagatcttg 960
agatgcttcc cagcctgttc acagatcccc tgggccagaa cactccttag gaaaaacagt 1020
cagctacata ttaggcagca acacgaaggg tctttgaaca aaatgagtaa tgttattcta 1080
cagtgtagaa aggtcacagt acagatctgg gaactaaata ttaaaaatga gtgtggctgg 1140
atatatggag aatgttgggc ccagaaggaa ccgtagagat cagatattac aacagctttg 1200
ttttgagggt tagaaatatg aaatgatttg gttatgaacg cacagtttag gcagcagggc 1260
cagaatcctg accctctgcc ccgtggttat ctcctcccca gcttggctgc ctcatgtcat 1320
cacagtattc cattttgttt gttgcatgtc ttgtgaagcc atcaagattt tctcgtctgt 1380
tttcctctca ttggtaatgc tcactttgtg acttcatttc aaatctgtaa tcccgttcaa 1440
ataaatatcc acaacaggat ctgttttcct gcccatcctt taaggaacac atcaattcat 1500
tttctaatgt ccttccctca caagcgggac caggcacagg gcgaggctca tcgatgaccc 1560
aagatggcgg ccgggcattt ctcccaggga tctctgtgct tccttttgtg cttcctgtgt 1620
gtgtggatat ttaaaggggc tggaaatgtg caaaaacatg tcactactta gacattatat 1680
tgtcatcttg ctgtttctag tgatgttaat tatctccatt tcagcagatg tgtggcctca 1740
gatggtaaag tcagcagcct ttcttatttc tcacctggaa atacatacga ccatttgagg 1800
agacaaatgg caaggtgtca gcataccctg aacttgagtt gagagctaca cacaatatta 1860
ttggtttccg agcatcacaa acaccctctc tgtttcttca ctgggcacag aattttaata 1920
cttatttcag tgggctgttg gcaggaacaa atgaagcaat ctacataaag tcactagtgc 1980
agtgcctgac acacaccatt ctcttgaggt cccctctaga gatcccacag gtcatatgac 2040
ttcttgggga gcagtggctc acacctgtaa tcccagcact ttgggaggct gaggcaggtg 2100
ggtcacctga ggtcaggagt tcaagaccag cctggccaat atggtgaaac cccatctcta 2160
ctaaaaatac aaaaattagc tgggcgtgct ggtgcatgcc tgtaatccca gccccaacac 2220
aatggaatt 2229

<210> 470
<211> 2426
<212> DNA
<213> Homo sapiens

<400> 470
gtaaattctt tattgccagg agtgaaccct aaagtggctc acaagagtgc cctatttctt 60
tcaattaact acaaggacaa acacatctca aagttgagat aagtgaccag tatgatttgc 120
caaaattcta aagcgcactc accatgaaat ggataaaggt tacctttggg gatttgcact 180
gcatgaattc tgtgaaaagc ttgttggata ttgtgataga gatagagaaa tgaagtatat 240
tatataagat actatgaggt tccctgcctt tgcttcacat cccaggctta caaacgtgcc 300
ccataaacat tccctctgtg gctcttgcat ttcatatatt tatctaaact cttataatca 360
aattacactt ttagtatttg ctgtctcatg tgatgatgaa tctcatatgt gtcccttctt 420
tgcatgaagt aagatagtca acttattcaa aactttacat cattctagat ttaagagaca 480
aggaagagct tctcaggcag aaggaataat gtatgcctga catgttcaag gaattacaag 540
ttagattttg tttaggtgca tgggaggggt tgatggtgat gacagataag gctggaggga 600
tggggagagg ctgtggctgt atacagcctc agtacaaggc taagcatttt aactttatac 660
tggaaaaaaa atcaaacaaa ggggagggat aaaggactta gtcatctttg cactggaaaa 720
caaaatatgt aattaaattc ccatagctgc atgtaacatt gaattcttcc aggttaaaaa 780
aaaaagttaa tcctgtgata ttaatggaat gacattttga ggtcttgaga atgggcacaa 840
aagtgggaaa tgaatttcag tatgggcaaa gacactgagg atgatgttga ttagataatt 900
cactccgtaa tgatcatgct gtgtgctagt aagtataacc ctggaaagat cttgagatgc 960
ttcccagcct gttcacagat cccctgggcc agaacactcc ttaggaaaaa cagtcagcta 1020

catattaggc agcaacacga agggtctttg aacaaaatga gtaatgttat tctacagtgt 1080
agaaaggtca cagtacagat ctgggaacta aatattaaaa atgagtgtgg ctggatatat 1140
ggagaatgtt gggcccagaa ggaaccgtag agatcagata ttacaacagc tttgttttga 1200
gggttagaaa tatgaaatga tttggttatg aacgcacagt ttaggcagca gggccagaat 1260
cctgaccctc tgccccgtgg ttatctcctc cccagcttgg ctgcctcatg tcatcacagt 1320
attccatttt gtttgttgca tgtcttgtga agccatcaag attttctcgt ctgttttcct 1380
ctcattggta atgctcactt tgtgacttca tttcaaatct gtaatcccgt tcaaataaat 1440
atccacaaca ggatctgttt tcctgcccat cctttaagga acacatcaat tcattttcta 1500
atgtccttcc ctcacaagcg ggaccaggca cagggcgagg ctcatcgatg acccaagatg 1560
gcggccgggc atttctccca gggatctctg tgcttccttt tgtgcttcct gtgtgtgtgg 1620
atatttaaag gggctggaaa tgtgcaaaaa catgtcacta cttagacatt atattgtcat 1680
cttgctgttt ctagtgatgt taattatctc catttcagca gatgtgtggc ctcagatggt 1740
aaagtcagca gcctttctta tttctcacct ggaaatacat acgaccattt gaggagacaa 1800
atggcaaggt gtcagcatac cctgaacttg agttgagagc tacacacaat attattggtt 1860
tccgagcatc acaaacaccc tctctgtttc ttcactgggc acagaatttt aatacttatt 1920
tcagtgggct gttggcagga acaaatgaag caatctacat aaagtcacta gtgcagtgcc 1980
tgacacacac cattctcttg aggtcccctc tagagatccc acaggtcata tgacttcttg 2040
gggagcagtg gctcacacct gtaatcccag cactttggga ggctgaggca ggtgggtcac 2100
ctgaggtcag gagttcaaga ccagcctggc caatatggtg aaaccccatc tctactaaaa 2160
atacaaaaat tagctgggcg tgctggtgca tgcctgtaat cccagctact tgggaggctg 2220
aggcaggaga attgctggaa catgggaggc ggaggttgca gtgagctgta attgtgccat 2280
tgcactcgaa cctgggcgac agagtggaac tctgtttcca aaaaacaaac aaacaaaaaa 2340
ggcatagtca gatacaacgt gggtgggatg tgtaaataga agcaggatat aaagggcatg 2400
gggtgacggt tttgcccaac acaatg 2426

<210> 471
<211> 812
<212> DNA
<213> Homo sapiens

<400> 471
gaacaaaatg agtaatgtta ttctacagtg tagaaaggtc acagtacaga tctgggaact 60
aaatattaaa aatgagtgtg gctggatata tggagaatgt tgggcccaga aggaaccgta 120
gagatcagat attacaacag ctttgttttg agggttagaa atatgaaatg atttggttat 180
gaacgcacag tttaggcagc agggccagaa tcctgaccct ctgccccgtg gttatctcct 240
ccccagcttg gctgcctcat gtcatcacag tattccattt tgtttgttgc atgtcttgtg 300
aagccatcaa gattttctcg tctgttttcc tctcattggt aatgctcact ttgtgacttc 360
atttcaaatc tgtaatcccg ttcaaataaa tatccacaac aggatctgtt ttcctgccca 420
tcctttaagg aacacatcaa ttcattttct aatgtccttc cctcacaagc gggaccaggc 480
acagggcgag gctcatcgat gacccaagat ggcggccggg catttctccc agggatctct 540
gtgcttcctt ttgtgcttcc tgtgtgtgtg gatatttaaa ggggctggaa atgtgcaaaa 600
acatgtcact acttagacat tatattgtca tcttgctgtt tctagtgatg ttaattatct 660
ccatttcagc agatgtgtgg cctcagatgg taaagtcagc agcctttctt atttctcacc 720
tctgtatcat caggtccttc ccaccatgca gatcttcctg gtctccctcg gctgcagcca 780
cacaaatctc ccctctgttt ttctgatgcc ag 812

<210> 472
<211> 515
<212> DNA
<213> Homo sapiens

<220>
<221> misc_feature
<222> (1)...(515)
<223> n = A,T,C or G

<400> 472
acggagactt attttctgat attgtctgca tatgtatgtt tttaagagtc tggaaatagt 60
cttatgactt tcctatcatg cttattaata aataatacag cccagagaag atgaaaatgg 120
gttccagaat tattggtcct tgcagcccgg tgaatctcag caagaggaac caccaactga 180
caatcaggat attgaacctg gacaagagag agaaggaaca cctccgatcg aagaacgtaa 240
agtagaaggt gattgccagg aaatggatct ggaaaagact cggagtgagc gtggagatgg 300

ctctgatgta aaagagaaga ctccacctaa tcctaagcat gctaagacta aagaagcagg 360
agatgggcag ccataagtta aaaagaagac aagctgaagc tacacacatg gctgatgtca 420
cattgaaaat gtgactgaaa atttgaaaat tctctcaata aagtttgagt tttctctgaa 480
gaaaaaaaaa naaaaaaaaa aaanaaaaan aaaaa 515

## Description

### TECHNICAL FIELD

The present invention relates generally to therapy and diagnosis of cancer, such as prostate cancer. The invention is more specifically related to polypeptides comprising at least a portion of a prostate tumor protein, and to polynucleotides encoding such polypeptides. Such polypeptides and polynucleotides may be used in vaccines and pharmaceutical compositions for prevention and treatment of prostate cancer, and for the diagnosis and monitoring of such cancers.

### BACKGROUND OF THE INVENTION

Prostate cancer is the most common form of cancer among males, with an estimated incidence of 30% in men over the age of 50. Overwhelming clinical evidence shows that human prostate cancer has the propensity to metastasize to bone, and the disease appears to progress inevitably from androgen dependent to androgen refractory status, leading to increased patient mortality. This prevalent disease is currently the second leading cause of cancer death among men in the U.S.

In spite of considerable research into therapies for the disease, prostate cancer remains difficult to treat. Commonly, treatment is based on surgery and/or radiation therapy, but these methods are ineffective in a significant percentage of cases. Two previously identified prostate specific proteins - prostate specific antigen (PSA) and prostatic acid phosphatase (PAP) - have limited therapeutic and diagnostic potential. For example, PSA levels do not always correlate well with the presence of prostate cancer, being positive in a percentage of non-prostate cancer cases, including benign prostatic hyperplasia (BPH). Furthermore, PSA measurements correlate with prostate volume, and do not indicate the level of metastasis.

In spite of considerable research into therapies for these and other cancers, prostate cancer remains difficult to diagnose and treat effectively. Accordingly, there is a need in the art for improved methods for detecting and treating such cancers. The present invention fulfills these needs and further provides other related advantages.

### SUMMARY OF THE INVENTION

Briefly stated, the present invention provides compositions and methods for the diagnosis and therapy of cancer, such as prostate cancer. In one aspect, the present invention provides polypeptides comprising at least a portion of a prostate tumor protein, or a variant thereof. Certain portions and other variants are immunogenic, such that the ability of the variant to react with antigen-specific antisera is not substantially diminished. Within certain embodiments, the polypeptide comprises at least an immunogenic portion of a prostate tumor protein, or a variant thereof, wherein the tumor protein comprises an amino acid sequence that is encoded by a polynucleotide sequence selected from the group consisting of: (a) sequences recited in any one of SEQ ID NOs:1-111, 115-171, 173-175, 177, 179-305, 307-315, 326, 328, 330, 332-335, 340-375, 381, 382 or 384-472; (b) sequences that hybridize to any of the foregoing sequences under moderately stringent conditions; and (c) complements of any of the sequence of (a) or (b). In certain specific embodiments, such a polypeptide comprises at least a portion, or variant thereof, of a tumor protein that includes an amino acid sequence selected from the group consisting of sequences recited in any one of SEQ ID NO: 112-114, 172, 176, 178, 327, 329, 331, 336, 339, 376-380 and 383.

The present invention further provides polynucleotides that encode a polypeptide as described above, or a portion thereof (such as a portion encoding at least 15 amino acid residues of a prostate tumor protein), expression vectors comprising such polynucleotides and host cells transformed or transfected with such expression vectors.

Within other aspects, the present invention provides pharmaceutical compositions comprising a polypeptide or polynucleotide as described above and a physiologically acceptable carrier.

Within a related aspect of the present invention, vaccines are provided. Such vaccines comprise a polypeptide or polynucleotide as described above and a non-specific immune response enhancer.

The present invention further provides pharmaceutical compositions that comprise: (a) an antibody or antigen-binding fragment thereof that specifically binds to a prostate tumor protein; and (b) a physiologically acceptable carrier.

Within further aspects, the present invention provides pharmaceutical compositions comprising: (a) an antigen presenting cell that expresses a polypeptide as described above and (b) a pharmaceutically acceptable carrier or excipient. Antigen presenting cells include dendritic cells, macrophages, monocytes, fibroblasts and B cells.

Within related aspects, vaccines are provided that comprise: (a) an antigen presenting cell that expresses a polypeptide as described above and (b) a non-specific immune response enhancer.

The present invention further provides, in other aspects, fusion proteins that comprise at least one polypeptide as described above, as well as polynucleotides encoding such fusion proteins.

Within related aspects, pharmaceutical compositions comprising a fusion protein, or a polynucleotide encoding a fusion protein, in combination with a physiologically acceptable carrier are provided.

Vaccines are further provided, within other aspects, that comprise a fusion protein, or a polynucleotide encoding a fusion protein, in combination with a non-specific immune response enhancer.

Within further aspects, the present invention provides methods for inhibiting the development of a cancer in a patient, comprising administering to a patient a pharmaceutical composition or vaccine as recited above.

The present invention further provides, within other aspects, methods for removing tumor cells from a biological sample, comprising contacting a biological sample with T cells that specifically react with a prostate tumor protein, wherein the step of contacting is performed under conditions and for a time sufficient to permit the removal of cells expressing the protein from the sample.

Within related aspects, methods are provided for inhibiting the development of a cancer in a patient, comprising administering to a patient a biological sample treated as described above.

Methods are further provided, within other aspects, for stimulating and/or expanding T cells specific for a prostate tumor protein, comprising contacting T cells with one or more of: (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Isolated T cell populations comprising T cells prepared as described above are also provided.

Within further aspects, the present invention provides methods for inhibiting the development of a cancer in a patient, comprising administering to a patient an effective amount of a T cell population as described above.

The present invention further provides methods for inhibiting the development of a cancer in a patient, comprising the steps of: (a) incubating CD4⁺ and/or CD8⁺ T cells isolated from a patient with one or more of: (i) a polypeptide comprising at least an immunogenic portion of a prostate tumor protein; (ii) a polynucleotide encoding such a polypeptide; and (iii) an antigen-presenting cell that expressed such a polypeptide; and (b) administering to the patient an effective amount of the proliferated T cells, and thereby inhibiting the development of a cancer in the patient. Proliferated cells may, but need not, be cloned prior to administration to the patient.

Within further aspects, the present invention provides methods for determining the presence or absence of a cancer in a patient, comprising: (a) contacting a biological sample obtained from a patient with a binding agent that binds to a polypeptide as recited above; (b) detecting in the sample an amount of polypeptide that binds to the binding agent; and (c) comparing the amount of polypeptide with a predetermined cut-off value, and therefrom determining the presence or absence of a cancer in the patient. Within preferred embodiments, the binding agent is an antibody, more preferably a monoclonal antibody. The cancer may be prostate cancer.

The present invention also provides, within other aspects, methods for monitoring the progression of a cancer in a patient. Such methods comprise the steps of: (a) contacting a biological sample obtained from a patient at a first point in time with a binding agent that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b) and therefrom monitoring the progression of the cancer in the patient.

The present invention further provides, within other aspects, methods for determining the presence or absence of a cancer in a patient, comprising the steps of: (a) contacting a biological sample obtained from a patient with an oligonucleotide that hybridizes to a polynucleotide that encodes a prostate tumor protein; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are provided for monitoring the progression of a cancer in a patient, comprising the steps of: (a) contacting a biological sample obtained from a patient with an oligonucleotide that hybridizes to a polynucleotide that encodes a prostate tumor protein; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Within further aspects, the present invention provides antibodies, such as monoclonal antibodies, that bind to a polypeptide as described above, as well as diagnostic kits comprising such antibodies. Diagnostic kits comprising one or more oligonucleotide probes or primers as described above are also provided.

These and other aspects of the present invention will become apparent upon reference to the following detailed description and attached drawings. All references disclosed herein are hereby incorporated by reference in their entirety as if each was incorporated individually.

### BRIEF DESCRIPTION OF THE DRAWINGS AND SEQUENCE IDENTIFIERS

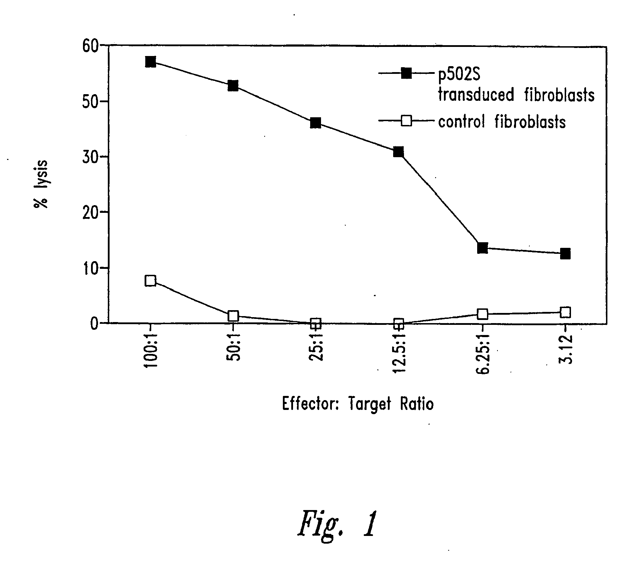

Figure 1 illustrates the ability of T cells to kill fibroblasts expressing the representative prostate tumor polypeptide P502S, as compared to control fibroblasts. The percentage lysis is shown as a series of effector:target ratios, as indicated.

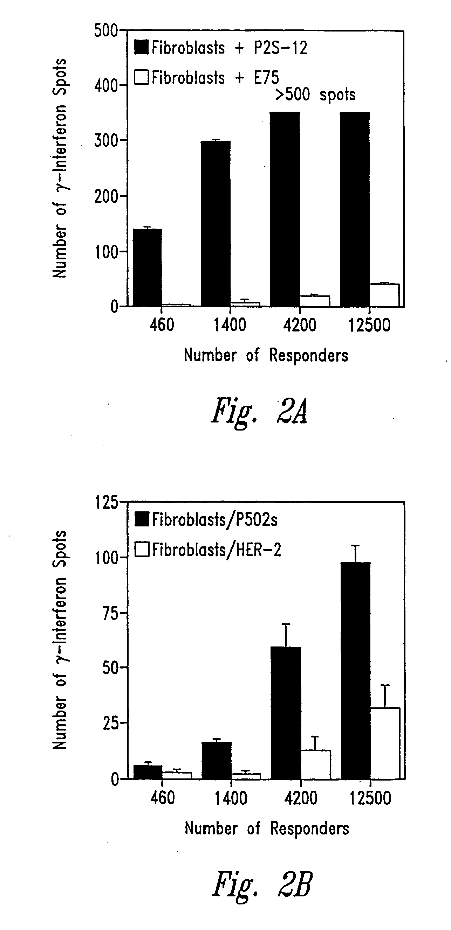

Figures 2A and 2B illustrate the ability of T cells to recognize cells expressing the representative prostate tumor polypeptide P502S. In each case, the number of γ-interferon spots is shown for different numbers of responders. In Figure 2A, data is presented for fibroblasts pulsed with the P2S-12 peptide, as compared to fibroblasts pulsed with a control E75 peptide. In Figure 2B, data is presented for fibroblasts expressing P502S, as compared to fibroblasts expressing HER-2/*neu.*

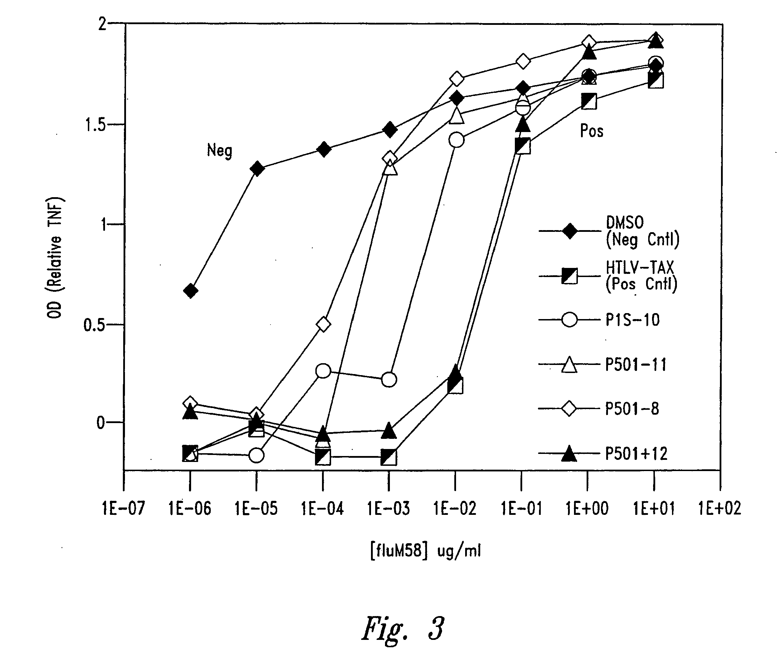

Figure 3 represents a peptide competition binding assay showing that the P1S#10 peptide, derived from P501S, binds HLA-A2. Peptide P1S#10 inhibits HLA-A2 restricted presentation of fluM58 peptide to CTL clone D150M58 in TNF release bioassay. D150M58 CTL is specific for the HLA-A2 binding influenza matrix peptide fluM58.

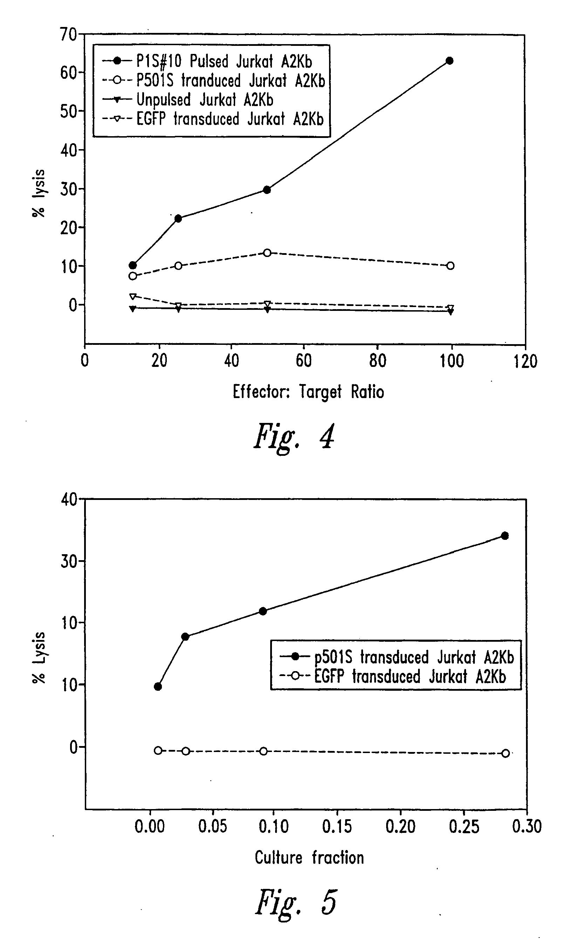

Figure 4 illustrates the ability of T cell lines generated from P1S#10 immunized mice to specifically lyse P1S#10-pulsed Jurkat A2Kb targets and P501S-transduced Jurkat A2Kb targets, as compared to EGFP-transduced Jurkat A2Kb. The percent lysis is shown as a series of effector to target ratios, as indicated.
Figure 5 illustrates the ability of a T cell clone to recognize and specifically lyse Jurkat A2Kb cells expressing the representative prostate tumor polypeptide P501S, thereby demonstrating that the P1S#10 peptide may be a naturally processed epitope of the P501S polypeptide.

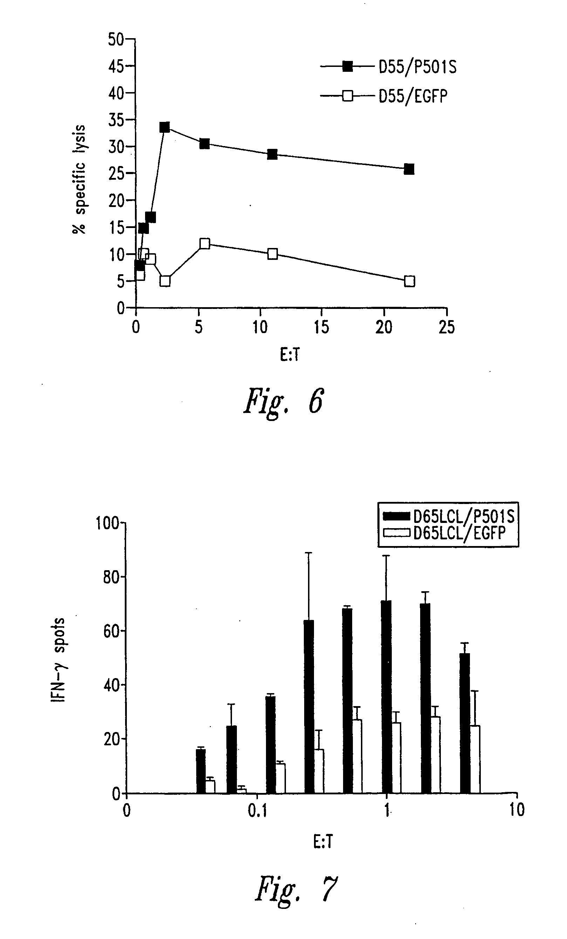

Figures 6A and 6B are graphs illustrating the specificity of a CD8⁺ cell line (3A-1) for a representative prostate tumor antigen (P501 S). Figure 6A shows the results of a ⁵¹Cr release assay. The percent specific lysis is shown as a series of effector:target ratios, as indicated. Figure 6B shows the production of interferon-gamma by 3A-1 cells stimulated with autologous B-LCL transduced with P501 S, at varying effector:target rations as indicated.

SEQ ID NO: 1 is the determined cDNA sequence for F1-13
SEQ ID NO: 2 is the determined 3' cDNA sequence for F1-12
SEQ ID NO: 3 is the determined 5' cDNA sequence for F1-12
SEQ ID NO: 4 is the determined 3' cDNA sequence for F1-16
SEQ ID NO: 5 is the determined 3' cDNA sequence for H1-1
SEQ ID NO: 6 is the determined 3' cDNA sequence for H1-9
SEQ ID NO: 7 is the determined 3' cDNA sequence for H1-4
SEQ ID NO: 8 is the determined 3' cDNA sequence for J1-17
SEQ ID NO: 9 is the determined 5' cDNA sequence for J1-17
SEQ ID NO: 10 is the determined 3' cDNA sequence for L1-12
SEQ ID NO: 11 is the determined 5' cDNA sequence for L1-12
SEQ ID NO: 12 is the determined 3' cDNA sequence for N1-1862
SEQ ID NO: 13 is the determined 5' cDNA sequence for N1-1862
SEQ ID NO: 14 is the determined 3' cDNA sequence for J1-13
SEQ ID NO: 15 is the determined 5' cDNA sequence for J1-13
SEQ ID NO: 16 is the determined 3' cDNA sequence for J1-19
SEQ ID NO: 17 is the determined 5' cDNA sequence for J1-19
SEQ ID NO: 18 is the determined 3' cDNA sequence for J1-25
SEQ ID NO: 19 is the determined 5' cDNA sequence for J1-25
SEQ ID NO: 20 is the determined 5' cDNA sequence for J1-24
SEQ ID NO: 21 is the determined 3' cDNA sequence for J1-24
SEQ ID NO: 22 is the determined 5' cDNA sequence for K1-58
SEQ ID NO: 23 is the determined 3' cDNA sequence for K1-58
SEQ ID NO: 24 is the determined 5' cDNA sequence for K1-63
SEQ ID NO: 25 is the determined 3' cDNA sequence for K1-63
SEQ ID NO: 26 is the determined 5' cDNA sequence for L1-4
SEQ ID NO: 27 is the determined 3' cDNA sequence for L1-4
SEQ ID NO: 28 is the determined 5' cDNA sequence for L1-14
SEQ ID NO: 29 is the determined 3' cDNA sequence for L1-14
SEQ ID NO: 30 is the determined 3' cDNA sequence for J1-12
SEQ ID NO: 31 is the determined 3' cDNA sequence for J1-16
SEQ ID NO: 32 is the determined 3' cDNA sequence for J1-21
SEQ ID NO: 33 is the determined 3' cDNA sequence for K1-48
SEQ ID NO: 34 is the determined 3' cDNA sequence for K1-55
SEQ ID NO: 35 is the determined 3' cDNA sequence for L1-2
SEQ ID NO: 36 is the determined 3' cDNA sequence for L1-6
SEQ ID NO: 37 is the determined 3' cDNA sequence for N1-1858
SEQ ID NO: 38 is the determined 3' cDNA sequence for N1-1860
SEQ ID NO: 39 is the determined 3' cDNA sequence for N1-1861
SEQ ID NO: 40 is the determined 3' cDNA sequence for N1-1864
SEQ ID NO: 41 is the determined cDNA sequence for P5
SEQ ID NO: 42 is the determined cDNA sequence for P8
SEQ ID NO: 43 is the determined cDNA sequence for P9
SEQ ID NO: 44 is the determined cDNA sequence for P 18
SEQ ID NO: 45 is the determined cDNA sequence for P20
SEQ ID NO: 46 is the determined cDNA sequence for P29
SEQ ID NO: 47 is the determined cDNA sequence for P30
SEQ ID NO: 48 is the determined cDNA sequence for P34
SEQ ID NO: 49 is the determined cDNA sequence for P36
SEQ ID NO: 50 is the determined cDNA sequence for P38
SEQ ID NO: 51 is the determined cDNA sequence for P39
SEQ ID NO: 52 is the determined cDNA sequence for P42
SEQ ID NO: 53 is the determined cDNA sequence for P47
SEQ ID NO: 54 is the determined cDNA sequence for P49
SEQ ID NO: 55 is the determined cDNA sequence for P50
SEQ ID NO: 56 is the determined cDNA sequence for P53
SEQ ID NO: 57 is the determined cDNA sequence for P55
SEQ ID NO: 58 is the determined cDNA sequence for P60
SEQ ID NO: 59 is the determined cDNA sequence for P64
SEQ ID NO: 60 is the determined cDNA sequence for P65
SEQ ID NO: 61 is the determined cDNA sequence for P73
SEQ ID NO: 62 is the determined cDNA sequence for P75
SEQ ID NO: 63 is the determined cDNA sequence for P76
SEQ ID NO: 64 is the determined cDNA sequence for P79
SEQ ID NO: 65 is the determined cDNA sequence for P84
SEQ ID NO: 66 is the determined cDNA sequence for P68
SEQ ID NO: 67 is the determined cDNA sequence for P80
SEQ ID NO: 68 is the determined cDNA sequence for P82
SEQ ID NO: 69 is the determined cDNA sequence for U1-3064
SEQ ID NO: 70 is the determined cDNA sequence for U1-3065
SEQ ID NO: 71 is the determined cDNA sequence for V1-3692
SEQ ID NO: 72 is the determined cDNA sequence for 1A-3905
SEQ ID NO: 73 is the determined cDNA sequence for V1-3686
SEQ ID NO: 74 is the determined cDNA sequence for R1-2330
SEQ ID NO: 75 is the determined cDNA sequence for 1B-3976
SEQ ID NO: 76 is the determined cDNA sequence for V1-3679
SEQ ID NO: 77 is the determined cDNA sequence for 1G-4736
SEQ ID NO: 78 is the determined cDNA sequence for 1G-4738
SEQ ID NO: 79 is the determined cDNA sequence for 1G-4741
SEQ ID NO: 80 is the determined cDNA sequence for 1G-4744
SEQ ID NO: 81 is the determined cDNA sequence for 1G-4734
SEQ ID NO: 82 is the determined cDNA sequence for 1H-4774
SEQ ID NO: 83 is the determined cDNA sequence for 1H-4781
SEQ ID NO: 84 is the determined cDNA sequence for 1H-4785
SEQ ID NO: 85 is the determined cDNA sequence for 1H-4787
SEQ ID NO: 86 is the determined cDNA sequence for 1H-4796
SEQ ID NO: 87 is the determined cDNA sequence for 1I-4807
SEQ ID NO: 88 is the determined cDNA sequence for 1I-4810
SEQ ID NO: 89 is the determined cDNA sequence for 1I-4811
SEQ ID NO: 90 is the determined cDNA sequence for 1J-4876
SEQ ID NO: 91 is the determined cDNA sequence for 1K-4884
SEQ ID NO: 92 is the determined cDNA sequence for 1K-4896
SEQ ID NO: 93 is the determined cDNA sequence for 1G-4761
SEQ ID NO: 94 is the determined cDNA sequence for 1G-4762
SEQ ID NO: 95 is the determined cDNA sequence for 1H-4766
SEQ ID NO: 96 is the determined cDNA sequence for 1H-4770
SEQ ID NO: 97 is the determined cDNA sequence for 1H-4771
SEQ ID NO: 98 is the determined cDNA sequence for 1H-4772
SEQ ID NO: 99 is the determined cDNA sequence for 1D-4297
SEQ ID NO: 100 is the determined cDNA sequence for 1D-4309
SEQ ID NO: 101 is the determined cDNA sequence for 1D.1-4278
SEQ ID NO: 102 is the determined cDNA sequence for 1D-4288
SEQ ID NO: 103 is the determined cDNA sequence for 1D-4283
SEQ ID NO: 104 is the determined cDNA sequence for 1D-4304
SEQ ID NO: 105 is the determined cDNA sequence for 1D-4296
SEQ ID NO: 106 is the determined cDNA sequence for 1D-4280
SEQ ID NO: 107 is the determined full length cDNA sequence for F1-12 (also referred to as P504S)
SEQ ID NO: 108 is the predicted amino acid sequence for F1-12
SEQ ID NO: 109 is the determined full length cDNA sequence for J1-17
SEQ ID NO: 110 is the determined full length cDNA sequence for L1-12
SEQ ID NO: 111 is the determined full length cDNA sequence for N1-1862
SEQ ID NO: 112 is the predicted amino acid sequence for J1-17
SEQ ID NO: 113 is the predicted amino acid sequence for L1-12
SEQ ID NO: 114 is the predicted amino acid sequence for N1-1862
SEQ ID NO: 115 is the determined cDNA sequence for P89
SEQ ID NO: 116 is the determined cDNA sequence for P90
SEQ ID NO: 117 is the determined cDNA sequence for P92
SEQ ID NO: 118 is the determined cDNA sequence for P95
SEQ ID NO: 119 is the determined cDNA sequence for P98
SEQ ID NO: 120 is the determined cDNA sequence for P102
SEQ ID NO: 121 is the determined cDNA sequence for P110
SEQ ID NO: 122 is the determined cDNA sequence for P111
SEQ ID NO: 123 is the determined cDNA sequence for P114
SEQ ID NO: 124 is the determined cDNA sequence for P115
SEQ ID NO: 125 is the determined cDNA sequence for P116
SEQ ID NO: 126 is the determined cDNA sequence for P124
SEQ ID NO: 127 is the determined cDNA sequence for P126
SEQ ID NO: 128 is the determined cDNA sequence for P130
SEQ ID NO: 129 is the determined cDNA sequence for P133
SEQ ID NO: 130 is the determined cDNA sequence for P138
SEQ ID NO: 131 is the determined cDNA sequence for P143
SEQ ID NO: 132 is the determined cDNA sequence for P151
SEQ ID NO: 133 is the determined cDNA sequence for P156
SEQ ID NO: 134 is the determined cDNA sequence for P157
SEQ ID NO: 135 is the determined cDNA sequence for P166
SEQ ID NO: 136 is the determined cDNA sequence for P176
SEQ ID NO: 137 is the determined cDNA sequence for P178
SEQ ID NO: 138 is the determined cDNA sequence for P179
SEQ ID NO: 139 is the determined cDNA sequence for P185
SEQ ID NO: 140 is the determined cDNA sequence for P192
SEQ ID NO: 141 is the determined cDNA sequence for P201
SEQ ID NO: 142 is the determined cDNA sequence for P204
SEQ ID NO: 143 is the determined cDNA sequence for P208
SEQ ID NO: 144 is the determined cDNA sequence for P211
SEQ ID NO: 145 is the determined cDNA sequence for P213
SEQ ID NO: 146 is the determined cDNA sequence for P219
SEQ ID NO: 147 is the determined cDNA sequence for P237
SEQ ID NO: 148 is the determined cDNA sequence for P239
SEQ ID NO: 149 is the determined cDNA sequence for P248
SEQ ID NO: 150 is the determined cDNA sequence for P251
SEQ ID NO: 151 is the determined cDNA sequence for P255
SEQ ID NO: 152 is the determined cDNA sequence for P256
SEQ ID NO: 153 is the determined cDNA sequence for P259
SEQ ID NO: 154 is the determined cDNA sequence for P260
SEQ ID NO: 155 is the determined cDNA sequence for P263
SEQ ID NO: 156 is the determined cDNA sequence for P264
SEQ ID NO: 157 is the determined cDNA sequence for P266
SEQ ID NO: 158 is the determined cDNA sequence for P270
SEQ ID NO: 159 is the determined cDNA sequence for P272
SEQ ID NO: 160 is the determined cDNA sequence for P278
SEQ ID NO: 161 is the determined cDNA sequence for P105
SEQ ID NO: 162 is the determined cDNA sequence for P107
SEQ ID NO: 163 is the determined cDNA sequence for P137
SEQ ID NO: 164 is the determined cDNA sequence for P194
SEQ ID NO: 165 is the determined cDNA sequence for P195
SEQ ID NO: 166 is the determined cDNA sequence for P 196
SEQ ID NO: 167 is the determined cDNA sequence for P220
SEQ ID NO: 168 is the determined cDNA sequence for P234
SEQ ID NO: 169 is the determined cDNA sequence for P235
SEQ ID NO: 170 is the determined cDNA sequence for P243
SEQ ID NO: 171 is the determined cDNA sequence for P703P-DE1
SEQ ID NO: 172 is the predicted amino acid sequence for P703P-DE1
SEQ ID NO: 173 is the determined cDNA sequence for P703P-DE2
SEQ ID NO: 174 is the determined cDNA sequence for P703P-DE6
SEQ ID NO: 175 is the determined cDNA sequence for P703P-DE13
SEQ ID NO: 176 is the predicted amino acid sequence for P703P-DE13
SEQ ID NO: 177 is the determined cDNA sequence for P703P-DE14
SEQ ID NO: 178 is the predicted amino acid sequence for P703P-DE14
SEQ ID NO: 179 is the determined extended cDNA sequence for 1G-4736
SEQ ID NO: 180 is the determined extended cDNA sequence for 1G-4738
SEQ ID NO: 181 is the determined extended cDNA sequence for 1G-4741
SEQ ID NO: 182 is the determined extended cDNA sequence for 1G-4744
SEQ ID NO: 183 is the determined extended cDNA sequence for 1H-4774
SEQ ID NO: 184 is the determined extended cDNA sequence for 1H-4781
SEQ ID NO: 185 is the determined extended cDNA sequence for 1H-4785
SEQ ID NO: 186 is the determined extended cDNA sequence for 1H-4787
SEQ ID NO: 187 is the determined extended cDNA sequence for 1H-4796
SEQ ID NO: 188 is the determined extended cDNA sequence for 1I-4807
SEQ ID NO: 189 is the determined 3' cDNA sequence for 1I-4810
SEQ ID NO: 190 is the determined 3' cDNA sequence for 1I-4811
SEQ ID NO: 191 is the determined extended cDNA sequence for 1J-4876
SEQ ID NO: 192 is the determined extended cDNA sequence for 1K-4884
SEQ ID NO: 193 is the determined extended cDNA sequence for 1K-4896
SEQ ID NO: 194 is the determined extended cDNA sequence for 1G-4761
SEQ ID NO: 195 is the determined extended cDNA sequence for 1G-4762
SEQ ID NO: 196 is the determined extended cDNA sequence for 1H-4766
SEQ ID NO: 197 is the determined 3' cDNA sequence for 1H-4770
SEQ ID NO: 198 is the determined 3' cDNA sequence for 1H-4771
SEQ ID NO: 199 is the determined extended cDNA sequence for 1H-4772
SEQ ID NO: 200 is the determined extended cDNA sequence for 1D-4309
SEQ ID NO: 201 is the determined extended cDNA sequence for 1D.1-4278
SEQ ID NO: 202 is the determined extended cDNA sequence for 1D-4288
SEQ ID NO: 203 is the determined extended cDNA sequence for 1D-4283
SEQ ID NO: 204 is the determined extended cDNA sequence for 1D-4304
SEQ ID NO: 205 is the determined extended cDNA sequence for 1D-4296
SEQ ID NO: 206 is the determined extended cDNA sequence for 1D-4280
SEQ ID NO: 207 is the determined cDNA sequence for 10-d8fwd
SEQ ID NO: 208 is the determined cDNA sequence for 10-H10con
SEQ ID NO: 209 is the determined cDNA sequence for 11-C8rev
SEQ ID NO: 210 is the determined cDNA sequence for 7.g6fwd
SEQ ID NO: 211 is the determined cDNA sequence for 7.g6rev
SEQ ID NO: 212 is the determined cDNA sequence for 8-b5fwd
SEQ ID NO: 213 is the determined cDNA sequence for 8-b5rev
SEQ ID NO: 214 is the determined cDNA sequence for 8-b6fwd
SEQ ID NO: 215 is the determined cDNA sequence for 8-b6 rev
SEQ ID NO: 216 is the determined cDNA sequence for 8-d4fwd
SEQ ID NO: 217 is the determined cDNA sequence for 8-d9rev
SEQ ID NO: 218 is the determined cDNA sequence for 8-g3fwd
SEQ ID NO: 219 is the determined cDNA sequence for 8-g3rev
SEQ ID NO: 220 is the determined cDNA sequence for 8-h11rev
SEQ ID NO: 221 is the determined cDNA sequence for g-f12fwd
SEQ ID NO: 222 is the determined cDNA sequence for g-f3rev
SEQ ID NO: 223 is the determined cDNA sequence for P509S
SEQ ID NO: 224 is the determined cDNA sequence for P510S
SEQ ID NO: 225 is the determined cDNA sequence for P703DE5
SEQ ID NO: 226 is the determined cDNA sequence for 9-A11
SEQ ID NO: 227 is the determined cDNA sequence for 8-C6
SEQ ID NO: 228 is the determined cDNA sequence for 8-H7
SEQ ID NO: 229 is the determined cDNA sequence for JPTPN13
SEQ ID NO: 230 is the determined cDNA sequence for JPTPN14
SEQ ID NO: 231 is the determined cDNA sequence for JPTPN23
SEQ ID NO: 232 is the determined cDNA sequence for JPTPN24
SEQ ID NO: 233 is the determined cDNA sequence for JPTPN25
SEQ ID NO: 234 is the determined cDNA sequence for JPTPN30
SEQ ID NO: 235 is the determined cDNA sequence for JPTPN34
SEQ ID NO: 236 is the determined cDNA sequence for PTPN35
SEQ ID NO: 237 is the determined cDNA sequence for JPTPN36
SEQ ID NO: 238 is the determined cDNA sequence for JPTPN38
SEQ ID NO: 239 is the determined cDNA sequence for JPTPN39
SEQ ID NO: 240 is the determined cDNA sequence for JPTPN40
SEQ ID NO: 241 is the determined cDNA sequence for JPTPN41
SEQ ID NO: 242 is the determined cDNA sequence for JPTPN42
SEQ ID NO: 243 is the determined cDNA sequence for JPTPN45
SEQ ID NO: 244 is the determined cDNA sequence for JPTPN46
SEQ ID NO: 245 is the determined cDNA sequence for JPTPN51
SEQ ID NO: 246 is the determined cDNA sequence for JPTPN56
SEQ ID NO: 247 is the determined cDNA sequence for PTPN64
SEQ ID NO: 248 is the determined cDNA sequence for JPTPN65
SEQ ID NO: 249 is the determined cDNA sequence for JPTPN67
SEQ ID NO: 250 is the determined cDNA sequence for JPTPN76
SEQ ID NO: 251 is the determined cDNA sequence for JPTPN84
SEQ ID NO: 252 is the determined cDNA sequence for JPTPN85
SEQ ID NO: 253 is the determined cDNA sequence for JPTPN86
SEQ ID NO: 254 is the determined cDNA sequence for JPTPN87
SEQ ID NO: 255 is the determined cDNA sequence for JPTPN88
SEQ ID NO: 256 is the determined cDNA sequence for JP1F1
SEQ ID NO: 257 is the determined cDNA sequence for JP1F2
SEQ ID NO: 258 is the determined cDNA sequence for JP1C2
SEQ ID NO: 259 is the determined cDNA sequence for JP1B1
SEQ ID NO: 260 is the determined cDNA sequence for JP1B2
SEQ ID NO: 261 is the determined cDNA sequence for JP1D3
SEQ ID NO: 262 is the determined cDNA sequence for JP1A4
SEQ ID NO: 263 is the determined cDNA sequence for JP1F5
SEQ ID NO: 264 is the determined cDNA sequence for JP1E6
SEQ ID NO: 265 is the determined cDNA sequence for JP1D6
SEQ ID NO: 266 is the determined cDNA sequence for JP1B5
SEQ ID NO: 267 is the determined cDNA sequence for JP1A6
SEQ ID NO: 268 is the determined cDNA sequence for JP1E8
SEQ ID NO: 269 is the determined cDNA sequence for JP1D7
SEQ ID NO: 270 is the determined cDNA sequence for JP1D9
SEQ ID NO: 271 is the determined cDNA sequence for JP1C10
SEQ ID NO: 272 is the determined cDNA sequence for JP1A9
SEQ ID NO: 273 is the determined cDNA sequence for JP1F12
SEQ ID NO: 274 is the determined cDNA sequence for JP1E12
SEQ ID NO: 275 is the determined cDNA sequence for JP1D11
SEQ ID NO: 276 is the determined cDNA sequence for JP1C11
SEQ ID NO: 277 is the determined cDNA sequence for JP1C12
SEQ ID NO: 278 is the determined cDNA sequence for JP1B12
SEQ ID NO: 279 is the determined cDNA sequence for JP1A12
SEQ ID NO: 280 is the determined cDNA sequence for JP8G2
SEQ ID NO: 281 is the determined cDNA sequence for JP8H1
SEQ ID NO: 282 is the determined cDNA sequence for JP8H2
SEQ ID NO: 283 is the determined cDNA sequence for JP8A3
SEQ ID NO: 284 is the determined cDNA sequence for JP8A4
SEQ ID NO: 285 is the determined cDNA sequence for JP8C3
SEQ ID NO: 286 is the determined cDNA sequence for JP8G4
SEQ ID NO: 287 is the determined cDNA sequence for JP8B6
SEQ ID NO: 288 is the determined cDNA sequence for JP8D6
SEQ ID NO: 289 is the determined cDNA sequence for JP8F5
SEQ ID NO: 290 is the determined cDNA sequence for JP8A8
SEQ ID NO: 291 is the determined cDNA sequence for JP8C7
SEQ ID NO: 292 is the determined cDNA sequence for JP8D7
SEQ ID NO: 293 is the determined cDNA sequence for P8D8
SEQ ID NO: 294 is the determined cDNA sequence for JP8E7
SEQ ID NO: 295 is the determined cDNA sequence for JP8F8
SEQ ID NO: 296 is the determined cDNA sequence for JP8G8
SEQ ID NO: 297 is the determined cDNA sequence for JP8B10
SEQ ID NO: 298 is the determined cDNA sequence for JP8C10
SEQ ID NO: 299 is the determined cDNA sequence for JP8E9
SEQ ID NO: 300 is the determined cDNA sequence for JP8E10
SEQ ID NO: 301 is the determined cDNA sequence for JP8F9
SEQ ID NO: 302 is the determined cDNA sequence for JP8H9
SEQ ID NO: 303 is the determined cDNA sequence for JP8C12
SEQ ID NO: 304 is the determined cDNA sequence for JP8E11
SEQ ID NO: 305 is the determined cDNA sequence for JP8E12
SEQ ID NO: 306 is the amino acid sequence for the peptide PS2#12
SEQ ID NO: 307 is the determined cDNA sequence for P711P
SEQ ID NO: 308 is the determined cDNA sequence for P712P
SEQ ID NO: 309 is the determined cDNA sequence for CLONE23
SEQ ID NO: 310 is the determined cDNA sequence for P774P
SEQ ID NO: 311 is the determined cDNA sequence for P775P
SEQ ID NO: 312 is the determined cDNA sequence for P715P
SEQ ID NO: 313 is the determined cDNA sequence for P710P
SEQ ID NO: 314 is the determined cDNA sequence for P767P
SEQ ID NO: 315 is the determined cDNA sequence for P768P
SEQ ID NO: 316-325 are the determined cDNA sequences of previously isolated genes
SEQ ID NO: 326 is the determined cDNA sequence for P703PDE5
SEQ ID NO: 327 is the predicted amino acid sequence for P703PDE5
SEQ ID NO: 328 is the determined cDNA sequence for P703P6.26
SEQ ID NO: 329 is the predicted amino acid sequence for P703P6.26
SEQ ID NO: 330 is the determined cDNA sequence for P703PX-23
SEQ ID NO: 331 is the predicted amino acid sequence for P703PX-23
SEQ ID NO: 332 is the determined full length cDNA sequence for P509S
SEQ ID NO: 333 is the determined extended cDNA sequence for P707P (also referred to as 11-C9)
SEQ ID NO: 334 is the determined cDNA sequence for P714P
SEQ ID NO: 335 is the determined cDNA sequence for P705P (also referred to as 9-F3)
SEQ ID NO: 336 is the predicted amino acid sequence for P705P
SEQ ID NO: 337 is the amino acid sequence of the peptide P1S#10
SEQ ID NO: 338 is the amino acid sequence of the peptide p5
SEQ ID NO: 339 is the predicted amino acid sequence of P509S
SEQ ID NO: 340 is the determined cDNA sequence for P778P
SEQ ID NO: 341 is the determined cDNA sequence for P786P
SEQ ID NO: 342 is the determined cDNA sequence for P789P
SEQ ID NO: 343 is the determined cDNA sequence for a clone showing homology to Homo sapiens MM46 mRNA
SEQ ID NO: 344 is the determined cDNA sequence for a clone showing homology to Homo sapiens TNF-alpha stimulated ABC protein (ABC50) mRNA
SEQ ID NO: 345 is the determined cDNA sequence for a clone showing homology to Homo sapiens mRNA for E-cadherin
SEQ ID NO: 346 is the determined cDNA sequence for a clone showing homology to Human nuclear-encoded mitochondrial serine hydroxymethyltransferase (SHMT)
SEQ ID NO: 347 is the determined cDNA sequence for a clone showing homology to Homo sapiens natural resistance-associated macrophage protein2 (NRAMP2)
SEQ ID NO: 348 is the determined cDNA sequence for a clone showing homology to Homo sapiens phosphoglucomutase-related protein (PGMRP)
SEQ ID NO: 349 is the determined cDNA sequence for a clone showing homology to Human mRNA for proteosome subunit p40
SEQ ID NO: 350 is the determined cDNA sequence for P777P
SEQ ID NO: 351 is the determined cDNA sequence for P779P
SEQ ID NO: 352 is the determined cDNA sequence for P790P
SEQ ID NO: 353 is the determined cDNA sequence for P784P
SEQ ID NO: 354 is the determined cDNA sequence for P776P
SEQ ID NO: 355 is the determined cDNA sequence for P780P
SEQ ID NO: 356 is the determined cDNA sequence for P544S
SEQ ID NO: 357 is the determined cDNA sequence for P745S
SEQ ID NO: 358 is the determined cDNA sequence for P782P
SEQ ID NO: 359 is the determined cDNA sequence for P783P
SEQ ID NO: 360 is the determined cDNA sequence for unknown 17984
SEQ ID NO: 361 is the determined cDNA sequence for P787P
SEQ ID NO: 362 is the determined cDNA sequence for P788P
SEQ ID NO: 363 is the determined cDNA sequence for unknown 17994
SEQ ID NO: 364 is the determined cDNA sequence for P781P
SEQ ID NO: 365 is the determined cDNA sequence for P785P
SEQ ID NO: 366-375 are the determined cDNA sequences for splice variants of B305D.
SEQ ID NO: 376 is the predicted amino acid sequence encoded by the sequence of SEQ ID NO: 366.
SEQ ID NO: 377 is the predicted amino acid sequence encoded by the sequence of SEQ ID NO: 372.
SEQ ID NO: 378 is the predicted amino acid sequence encoded by the sequence of SEQ ID NO:373.
SEQ ID NO: 379 is the predicted amino acid sequence encoded by the sequence of SEQ ID NO: 374.
SEQ ID NO: 380 is the predicted amino acid sequence encoded by the sequence of SEQ ID NO: 375.
SEQ ID NO: 381 is the determined cDNA sequence for B716P.
SEQ ID NO: 382 is the determined full-length cDNA sequence for P711P.
SEQ ID NO: 383 is the predicted amino acid sequence for P711P.
SEQ ID NO: 384 is the cDNA sequence for P1000C.
SEQ ID NO: 385 is the cDNA sequence for CGI-82.
SEQ ID NO:386 is the cDNA sequence for 23320.
SEQ ID NO:387 is the cDNA sequence for CGI-69.
SEQ ID NO:388 is the cDNA sequence for L-iditol-2-dehydrogenase.
SEQ ID NO:389 is the cDNA sequence for 23379.
SEQ ID NO:390 is the cDNA sequence for 23381.
SEQ ID NO:391 is the cDNA sequence for KIAA0122.
SEQ ID NO:392 is the cDNA sequence for 23399.
SEQ ID NO:393 is the cDNA sequence for a previously identified gene.
SEQ ID NO:394 is the cDNA sequence for HCLBP.
SEQ ID NO:395 is the cDNA sequence for transglutaminase.
SEQ ID NO:396 is the cDNA sequence for a previously identified gene.
SEQ ID NO:397 is the cDNA sequence for PAP.
SEQ ID NO:398 is the cDNA sequence for Ets transcription factor PDEF.
SEQ ID NO:399 is the cDNA sequence for hTGR.
SEQ ID NO:400 is the cDNA sequence for KIAA0295.
SEQ ID NO:401 is the cDNA sequence for 22545.
SEQ ID NO:402 is the cDNA sequence for 22547.
SEQ ID NO:403 is the cDNA sequence for 22548.
SEQ ID NO:404 is the cDNA sequence for 22550.
SEQ ID NO:405 is the cDNA sequence for 22551.
SEQ ID NO:406 is the cDNA sequence for 22552.
SEQ ID NO:407 is the cDNA sequence for 22553.
SEQ ID NO:408 is the cDNA sequence for 22558.
SEQ ID NO:409 is the cDNA sequence for 22562.
SEQ ID NO:410 is the cDNA sequence for 22565.
SEQ ID NO:411 is the cDNA sequence for 22567.
SEQ ID NO:412 is the cDNA sequence for 22568.
SEQ ID NO:413 is the cDNA sequence for 22570.
SEQ ID NO:414 is the cDNA sequence for 22571.
SEQ ID NO:415 is the cDNA sequence for 22572.
SEQ ID NO:416 is the cDNA sequence for 22573.
SEQ ID NO:417 is the cDNA sequence for 22573.
SEQ ID NO:418 is the cDNA sequence for 22575.
SEQ ID NO:419 is the cDNA sequence for 22580.
SEQ ID NO:420 is the cDNA sequence for 22581.
SEQ ID NO:421 is the cDNA sequence for 22582.
SEQ ID NO:422 is the cDNA sequence for 22583.
SEQ ID NO:423 is the cDNA sequence for 22584.
SEQ ID NO:424 is the cDNA sequence for 22585.
SEQ ID NO:425 is the cDNA sequence for 22586.
SEQ ID NO:426 is the cDNA sequence for 22587.
SEQ ID NO:427 is the cDNA sequence for 22588.
SEQ ID NO:428 is the cDNA sequence for 22589.
SEQ ID NO:429 is the cDNA sequence for 22590.
SEQ ID NO:430 is the cDNA sequence for 22591.
SEQ ID NO:431 is the cDNA sequence for 22592.
SEQ ID NO:432 is the cDNA sequence for 22593.
SEQ ID NO:433 is the cDNA sequence for 22594.
SEQ ID NO:434 is the cDNA sequence for 22595.
SEQ ID NO:435 is the cDNA sequence for 22596.
SEQ ID NO:436 is the cDNA sequence for 22847.
SEQ ID NO:437 is the cDNA sequence for 22848.
SEQ ID NO:438 is the cDNA sequence for 22849.
SEQ ID NO:439 is the cDNA sequence for 22851.
SEQ ID NO:440 is the cDNA sequence for 22852.
SEQ ID NO:441 is the cDNA sequence for 22853.
SEQ ID NO:442 is the cDNA sequence for 22854.
SEQ ID NO:443 is the cDNA sequence for 22855.
SEQ ID NO:444 is the cDNA sequence for 22856.
SEQ ID NO:445 is the cDNA sequence for 22857.
SEQ ID NO:446 is the cDNA sequence for 23601.
SEQ ID NO:447 is the cDNA sequence for 23602.
SEQ ID NO:448 is the cDNA sequence for 23605.
SEQ ID NO:449 is the cDNA sequence for 23606.
SEQ ID NO:450 is the cDNA sequence for 23612.
SEQ ID NO:451 is the cDNA sequence for 23614.
SEQ ID NO:452 is the cDNA sequence for 23618.
SEQ ID NO:453 is the cDNA sequence for 23622.
SEQ ID NO:454 is the cDNA sequence for folate hydrolase.
SEQ ID NO:455 is the cDNA sequence for LIM protein.
SEQ ID NO:456 is the cDNA sequence for a known gene.
SEQ ID NO:457 is the cDNA sequence for a known gene.
SEQ ID NO:458 is the cDNA sequence for a previously identified gene.
SEQ ID NO:459 is the cDNA sequence for 23045.
SEQ ID NO:460 is the cDNA sequence for 23032.
SEQ ID NO:461 is the cDNA sequence for 23054.
SEQ ID NOs:462-467 are cDNA sequences for known genes.
SEQ ID NOs:468-471 are cDNA sequences for P710P.
SEQ ID NO:472 is a cDNA sequence for P1001C.

### DETAILED DESCRIPTION OF THE INVENTION

As noted above, the present invention is generally directed to compositions and methods for the therapy and diagnosis of cancer, such as prostate cancer. The compositions described herein may include prostate tumor polypeptides, polynucleotides encoding such polypeptides, binding agents such as antibodies, antigen presenting cells (APCs) and/or immune system cells (*e.g.*, T cells). Polypeptides of the present invention generally comprise at least a portion (such as an immunogenic portion) of a prostate tumor protein or a variant thereof. A "prostate tumor protein" is a protein that is expressed in prostate tumor cells at a level that is at least two fold, and preferably at least five fold, greater than the level of expression in a normal tissue, as determined using a representative assay provided herein. Certain prostate tumor proteins are tumor proteins that react detectably (within an immunoassay, such as an ELISA or Western blot) with antisera of a patient afflicted with prostate cancer. Polynucleotides of the subject invention generally comprise a DNA or RNA. sequence that encodes all or a portion of such a polypeptide, or that is complementary to such a sequence. Antibodies are generally immune system proteins, or antigen-binding fragments thereof, that are capable of binding to a polypeptide as described above. Antigen presenting cells include dendritic cells, macrophages, monocytes, fibroblasts and B-cells that express a polypeptide as described above. T cells that may be employed within such compositions are generally T cells that are specific for a polypeptide as described above.

The present invention is based on the discovery of human prostate tumor proteins. Sequences of polynucleotides encoding certain tumor proteins, or portions thereof, are provided in SEQ ID NOs:1-111, 115-171, 173-175, 177, 179-305, 307-315, 326, 328, 330, 332-335, 340-375, 381, 382 or 384-472. Sequences of polypeptides comprising at least a portion of a tumor protein are provided in SEQ ID NOs:112-114, 172, 176, 178, 327, 329, 331, 336, 339, 376-380 and 383.

### PROSTATE TUMOR PROTEIN POLYNUCLEOTIDES

Any polynucleotide that encodes a prostate tumor protein or a portion or other variant thereof as described herein is encompassed by the present invention. Preferred polynucleotides comprise at least 15 consecutive nucleotides, preferably at least 30 consecutive nucleotides and more preferably at least 45 consecutive nucleotides, that encode a portion of a prostate tumor protein. More preferably, a polynucleotide encodes an immunogenic portion of a prostate tumor protein. Polynucleotides complementary to any such sequences are also encompassed by the present invention. Polynucleotides may be single-stranded (coding or antisense) or double-stranded, and may be DNA (genomic, cDNA or synthetic) or RNA molecules. RNA molecules include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i.e.*, an endogenous sequence that encodes a prostate tumor protein or a portion thereof) or may comprise a variant of such a sequence. Polynucleotide variants may contain one or more substitutions, additions, deletions and/or insertions such that the immunogenicity of the encoded polypeptide is not diminished, relative to a native tumor protein. The effect on the immunogenicity of the encoded polypeptide may generally be assessed as described herein. Variants preferably exhibit at least about 70% identity, more preferably at least about 80% identity and most preferably at least about 90% identity to a polynucleotide sequence that encodes a native prostate tumor protein or a portion thereof.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (*i*.*e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i. e., the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native prostate tumor protein (or a complementary sequence). Suitable moderately stringent conditions include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1% SDS.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Polynucleotides may be prepared using any of a variety of techniques. For example, a polynucleotide may be identified, as described in more detail below, by screening a microarray of cDNAs for tumor-associated expression (*i.e*., expression that is at least five fold greater in a prostate tumor than in normal tissue, as determined using a representative assay provided herein). Such screens may be performed using a Synteni microarray (Palo Alto, CA) according to the manufacturer's instructions (and essentially as described by Schena et al., Proc. Natl. Acad. Sci. USA 93:10614-10619, 1996 and Heller et al., Proc. Natl. Acad. Sci. USA 94:2150-2155, 1997). Alternatively, polypeptides may be amplified from cDNA prepared from cells expressing the proteins described herein, such as prostate tumor cells. Such polynucleotides may be amplified via polymerase chain reaction (PCR). For this approach, sequence-specific primers may be designed based on the sequences provided herein, and may be purchased or synthesized.

An amplified portion may be used to isolate a full length gene from a suitable library (*e.g.,* a prostate tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e.g.*, by nick-translation or end-labeling with ³²P) using well known techniques. A bacterial or bacteriophage library is then screened by hybridizing filters containing denatured bacterial colonies (or lawns containing phage plaques) with the labeled probe (*see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (*see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., PCR Methods Applic. 1:111-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g.*, NCBI BLAST searches), and such ESTs may be used to generate a contiguous full length sequence.

Certain nucleic acid sequences of cDNA molecules encoding at least a portion of a prostate tumor protein are provided in SEQ ID NOs:1-111, 115-171, 173-175, 177, 179-305, 307-315, 326, 328, 330, 332-335, 340-375, 381, 382 or 384-472. Isolation of these polynucleotides is described below. Each of these prostate tumor proteins was overexpressed in prostate tumor tissue.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis *(see* Adelman et al., DNA 2:183, 1983). Alternatively, RNA molecules may be generated by in *vitro* or *in vivo* transcription of DNA sequences encoding a prostate tumor protein, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo* (*e.g.,* by transfecting antigen-presenting cells, such as dendritic cells, with a cDNA construct encoding a prostate tumor polypeptide, and administering the transfected cells to the patient).

A portion of a sequence complementary to a coding sequence (*i.e.*, an antisense polynucleotide) may also be used as a probe or to modulate gene expression. cDNA constructs that can be transcribed into antisense RNA may also be introduced into cells of tissues to facilitate the production of antisense RNA. An antisense polynucleotide may be used, as described herein, to inhibit expression of a tumor protein. Antisense technology can be used to control gene expression through triple-helix formation, which compromises the ability of the double helix to open sufficiently for the binding of polymerases, transcription factors or regulatory molecules (*see* Gee et al., In Huber and Carr, Molecular and Immunologic Approaches, Futura Publishing Co. (Mt. Kisco, NY; 1994)). Alternatively, an antisense molecule may be designed to hybridize with a control region of a gene (*e.g.,* promoter, enhancer or transcription initiation site), and block transcription of the gene; or to block translation by inhibiting binding of a transcript to ribosomes.

A portion of a coding sequence, or of a complementary sequence, may also be designed as a probe or primer to detect gene expression. Probes may be labeled with a variety of reporter groups, such as radionuclides and enzymes, and are preferably at least 10 nucleotides in length, more preferably at least 20 nucleotides in length and still more preferably at least 30 nucleotides in length. Primers, as noted above, are preferably 22-30 nucleotides in length.

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (*e.g.*, avian pox virus). Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (*i.e.*, an artificial membrane vesicle). The preparation and use of such systems is well known in the art.

### PROSTATE TUMOR POLYPEPTIDES

Within the context of the present invention, polypeptides may comprise at least an immunogenic portion of a prostate tumor protein or a variant thereof, as described herein. As noted above, a "prostate tumor protein" is a protein that is expressed by prostate tumor cells. Proteins that are prostate tumor proteins also react detectably within an immunoassay (such as an ELISA) with antisera from a patient with prostate cancer. Polypeptides as described herein may be of any length. Additional sequences derived from the native protein and/or heterologous sequences may be present, and such sequences may (but need not) possess further immunogenic or antigenic properties.

An "immunogenic portion," as used herein is a portion of a protein that is recognized (*i.e*., specifically bound) by a B-cell and/or T-cell surface antigen receptor. Such immunogenic portions generally comprise at least 5 amino acid residues, more preferably at least 10, and still more preferably at least 20 amino acid residues of a prostate tumor protein or a variant thereof. Certain preferred immunogenic portions include peptides in which an N-terminal leader sequence and/or transmembrane domain have been deleted. Other preferred immunogenic portions may contain a small N- and/or C-terminal deletion (*e.g.*, 1-30 amino acids, preferably 5-15 amino acids), relative to the mature protein.

Immunogenic portions may generally be identified using well known techniques, such as those summarized in Paul, Fundamental Immunology, 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e.*, they react with the protein in an ELISA or other immunoassay, and do not react detectably with unrelated proteins). Such antisera and antibodies may be prepared as described herein, and using well known techniques. An immunogenic portion of a native prostate tumor protein is a portion that reacts with such antisera and/or T-cells at a level that is not substantially less than the reactivity of the full length polypeptide (*e.g.*, in an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, Antibodies: A. Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As noted above, a composition may comprise a variant of a native prostate tumor protein. A polypeptide "variant," as used herein, is a polypeptide that differs from a native prostate tumor protein in one or more substitutions, deletions, additions and/or insertions, such that the immunogenicity of the polypeptide is not substantially diminished. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence or transmembrane domain, have been removed. Other preferred variants include variants in which a small portion (*e.g.*, 1-30 amino acids, preferably 5-15 amino acids) has been removed from the N- and/or C-terminal of the mature protein. Polypeptide variants preferably exhibit at least about 70%, more preferably at least about 90% and most preferably at least about 95% identity (determined as described above) to the identified polypeptides.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g.*, poly-His), or to enhance binding of the polypeptide to a solid support. For example, a polypeptide may be conjugated to an immunoglobulin Fc region.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division (Foster City, CA), and may be operated according to the manufacturer's instructions.

Within certain specific embodiments, a polypeptide may be a fusion protein that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and the second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins (*see,* for example, Stoute et al. New Engl. J Med., 336:86-91, 1997).

Within preferred embodiments, an immunological fusion partner is derived from protein D, a surface protein of the gram-negative bacterium Haemophilus influenza B (WO 91/18926). Preferably, a protein D derivative comprises approximately the first third of the protein (*e.g.*, the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described *(see* Biotechnology 10:795-798, 1992). Within a preferred embodiment, a repeat portion of LYTA may be incorporated into a fusion protein. A repeat portion is found in the C-terminal region starting at residue 178. A particularly preferred repeat portion incorporates residues 188-305.

In general, polypeptides (including fusion proteins) and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### BINDING AGENTS

The present invention further provides agents, such as antibodies and antigen-binding fragments thereof, that specifically bind to a prostate tumor protein. As used herein, an antibody, or antigen-binding fragment thereof, is said to "specifically bind" to a prostate tumor protein if it reacts at a detectable level (within, for example, an ELISA) with a prostate tumor protein, and does not react detectably with unrelated proteins under similar conditions. As used herein, "binding" refers to a noncovalent association between two separate molecules such that a complex is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ L/mol. The binding constant may be determined using methods well known in the art.

Binding agents may be further capable of differentiating between patients with and without a cancer, such as prostate cancer, using the representative assays provided herein. In other words, antibodies or other binding agents that bind to a prostate tumor protein will generate a signal indicating the presence of a cancer in at least about 20% of patients with the disease, and will generate a negative signal indicating the absence of the disease in at least about 90% of individuals without the cancer. To determine whether a binding agent satisfies this requirement, biological samples (*e.g.*, blood, sera, urine and/or tumor biopsies) from patients with and without a cancer (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e.g.,* mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e.,* reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e.g.*, covalently bonded) to a suitable monoclonal antibody either directly or indirectly (*e.g.*, via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g.*, a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g.*, U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g.*, U.S. Patent No. 4,489,710, to Spitler), by irradiation of a photolabile bond (*e.g.*, U.S. Patent No. 4,625,014, to Senter et al.), by hydrolysis of derivatized amino acid side chains (*e.g.*, U.S. Patent No. 4,638,045, to Kohn et al.), by serum complement-mediated hydrolysis (*e.g.*, U.S. Patent No. 4,671,958, to Rodwell et al.), and acid-catalyzed hydrolysis (*e.g.*, U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (*e.g.,* U.S. Patent No. 4,507,234, to Kato et al.), peptides and polysaccharides such as aminodextran (*e.g.*, U.S. Patent No. 4,699,784, to Shih et al.). A carrier may also bear an agent by noncovalent bonding or by encapsulation, such as within a liposome vesicle (*e.g.*, U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

### T CELLS

Immunotherapeutic compositions may also, or alternatively, comprise T cells specific for a prostate tumor protein. Such cells may generally be prepared *in vitro* or *ex vivo,* using standard procedures. For example, T cells may be isolated from bone marrow, peripheral blood, or a fraction of bone marrow or peripheral blood of a patient, using a commercially available cell separation system, such as the CEPRATE^{™} system, available from CellPro Inc., Bothell WA (*see also* U.S. Patent No. 5,240,856; U.S. Patent No. 5,215,926; WO 89/06280; WO 91/16116 and WO 92/07243). Alternatively, T cells may be derived from related or unrelated humans, non-human mammals, cell lines or cultures.

T cells may be stimulated with a prostate tumor polypeptide, polynucleotide encoding a prostate tumor polypeptide and/or an antigen presenting cell (APC) that expresses such a polypeptide. Such stimulation is performed under conditions and for a time sufficient to permit the generation of T cells that are specific for the polypeptide. Preferably, a prostate tumor polypeptide or polynucleotide is present within a delivery vehicle, such as a microsphere, to facilitate the generation of specific T cells.

T cells are considered to be specific for a prostate tumor polypeptide if the T cells kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (*e.g.*, by pulse-labeling cultures of T cells with tritiated thymidine and measuring the amount of tritiated thymidine incorporated into DNA). Contact with a prostate tumor polypeptide (100 ng/ml - 100 µg/ml, preferably 200 ng/ml - 25 µg/ml) for 3 - 7 days should result in at least a two fold increase in proliferation of the T cells. Contact as described above for 2-3 hours should result in activation of the T cells, as measured using standard cytokine assays in which a two fold increase in the level of cytokine release (*e.g.*, TNF or IFN-γ) is indicative of T cell activation (*see* Coligan et al., Current Protocols in Immunology, vol. 1, Wiley Interscience (Greene 1998)). T cells that have been activated in response to a prostate tumor polypeptide, polynucleotide or polypeptide-expressing APC may be CD4⁺ and/or CD8⁺. Prostate tumor protein-specific T cells may be expanded using standard techniques. Within preferred embodiments, the T cells are derived from either a patient or a related, or unrelated, donor and are administered to the patient following stimulation and expansion.

For therapeutic purposes, CD4⁺ or CD8⁺ T cells that proliferate in response to a prostate tumor polypeptide, polynucleotide or APC can be expanded in number either *in vitro* or *in vivo.* Proliferation of such T cells *in vitro* may be accomplished in a variety of ways. For example, the T cells can be re-exposed to a prostate tumor polypeptide, or a short peptide corresponding to an immunogenic portion of such a polypeptide, with or without the addition of T cell growth factors, such as interleukin-2, and/or stimulator cells that synthesize a prostate tumor polypeptide. Alternatively, one or more T cells that proliferate in the presence of a prostate tumor protein can be expanded in number by cloning. Methods for cloning cells are well known in the art, and include limiting dilution.

### PHARMACEUTICAL COMPOSITIONS AND VACCINES

Within certain aspects, polypeptides, polynucleotides, T cells and/or binding agents disclosed herein may be incorporated into pharmaceutical compositions or immunogenic compositions (*i.e.*, vaccines). Pharmaceutical compositions comprise one or more such compounds and a physiologically acceptable carrier. Vaccines may comprise one or more such compounds and a non-specific immune response enhancer. A non-specific immune response enhancer may be any substance that enhances an immune response to an exogenous antigen. Examples of non-specific immune response enhancers include adjuvants, biodegradable microspheres (*e.g.*, polylactic galactide) and liposomes (into which the compound is incorporated; *see e.g.,* Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other tumor antigens may be present, either incorporated into a fusion polypeptide or as a separate compound, within the composition or vaccine.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface or secretes such an epitope. In a preferred embodiment, the DNA may be introduced using a viral expression system (*e.g.*, vaccinia or other pox virus, retrovirus, or adenovirus), which may involve the use of a non-pathogenic (defective), replication competent virus. Suitable systems are disclosed, for example, in Fisher-Hoch et al., Proc. Natl. Acad. Sci. USA 86:317-321, 1989; Flexner et al., Ann. N. Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.*, polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (*e.g.,* neutral buffered saline or phosphate buffered saline), carbohydrates (*e.g.,* glucose, mannose, sucrose or dextrans), mannitol, proteins, polypeptides or amino acids such as glycine, antioxidants, chelating agents such as EDTA or glutathione, adjuvants (*e.g.*, aluminum hydroxide) and/or preservatives. Alternatively, compositions of the present invention may be formulated as a lyophilizate. Compounds may also be encapsulated within liposomes using well known technology.

Any of a variety of non-specific immune response enhancers may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Within the vaccines provided herein, the adjuvant composition is preferably designed to induce an immune response predominantly of the Th1 type. High levels of Th1-type cytokines (*e.g*., IFN-γ, IL-2 and IL-12) tend to favor the induction of cell mediated immune responses to an administered antigen. In contrast, high levels of Th2-type cytokines (*e.g.*, IL-4, IL-5, IL-6, IL-10 and TNF-β) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Ribi ImmunoChem Research Inc. (Hamilton, MT; *see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555. Another preferred adjuvant is a saponin, preferably QS21, which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210. Any vaccine provided herein may be prepared using well known methods that result in a combination of antigen, immune response enhancer and a suitable carrier or excipient.

The compositions described herein may be administered as part of a sustained release formulation (*i.e*., a formulation such as a capsule or sponge that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets tumor cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i.e.*, matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity (*see* Timmerman and Levy, Ann. Rev. Med. 50:507-529, 1999). In general, dendritic cells may be identified based on their typical shape (stellate *in situ,* with marked cytoplasmic processes (dendrites) visible *in vitro*) and based on the lack of differentiation markers of B cells (CD 19 and CD20), T cells (CD3), monocytes (CD14) and natural killer cells (CD56), as determined using standard assays. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (*see* Zitvogel et al., Nature Med. 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor, mannose receptor and DEC-205 marker. The mature phenotype is typically characterized by a lower expression of these markers, but a high expression of cell surface molecules responsible for T cell activation such as class I and class II MHC, adhesion molecules (*e.g.,* CD54 and CD11) and costimulatory molecules (*e.g.,* CD40, CD80 and CD86).

APCs may generally be transfected with a polynucleotide encoding a prostate tumor protein (or portion or other variant thereof) such that the prostate tumor polypeptide, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the prostate tumor polypeptide, DNA (naked or within a plasmid vector) or RNA; or with antigen-expressing recombinant bacterium or viruses (*e.g*., vaccinia, fowlpox, adenovirus or lentivirus vectors). Prior to loading, the polypeptide may be covalently conjugated to an immunological partner that provides T cell help (*e.g*., a carrier molecule). Alternatively, a dendritic cell may be pulsed with a non-conjugated immunological partner, separately or in the presence of the polypeptide.

### CANCER THERAPY

In further aspects of the present invention, the compositions described herein may be used for immunotherapy of cancer, such as prostate cancer. Within such methods, pharmaceutical compositions and vaccines are typically administered to a patient. As used herein, a "patient" refers to any warm-blooded animal, preferably a human. A patient may or may not be afflicted with cancer. Accordingly, the above pharmaceutical compositions and vaccines may be used to prevent the development of a cancer or to treat a patient afflicted with a cancer. A cancer may be diagnosed using criteria generally accepted in the art, including the presence of a malignant tumor. Pharmaceutical compositions and vaccines may be administered either prior to or following surgical removal of primary tumors and/or treatment such as administration of radiotherapy or conventional chemotherapeutic drugs.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides disclosed herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 (*see,* for example, Cheever et al., Immunological Reviews 157:177, 1997).

Alternatively, a vector expressing a polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions disclosed herein, as well as dosage, will vary from individual to individual, and may be readily established using standard techniques. In general, the pharmaceutical compositions and vaccines may be administered by injection (*e.g.*, intracutaneous, intramuscular, intravenous or subcutaneous), intranasally (*e.g.*, by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i.e.,* untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g.*, more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 100 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (*e.g.*, more frequent remissions, complete or partial, or longer disease-free survival) in treated patients as compared to non-treated patients. Increases in preexisting immune responses to a prostate tumor protein generally correlate with an improved clinical outcome. Such immune responses may generally be evaluated using standard proliferation, cytotoxicity or cytokine assays, which may be performed using samples obtained from a patient before and after treatment.

### METHODS FOR DETECTING CANCER

In general, a cancer may be detected in a patient based on the presence of one or more prostate tumor proteins and/or polynucleotides encoding such proteins in a biological sample (for example, blood, sera, urine and/or tumor biopsies) obtained from the patient. In other words, such proteins may be used as markers to indicate the presence or absence of a cancer such as prostate cancer. In addition, such proteins may be useful for the detection of other cancers. The binding agents provided herein generally permit detection of the level of antigen that binds to the agent in the biological sample. Polynucleotide primers and probes may be used to detect the level of mRNA encoding a tumor protein, which is also indicative of the presence or absence of a cancer. In general, a prostate tumor sequence should be present at a level that is at least three fold higher in tumor tissue than in normal tissue

There are a variety of assay formats known to those of ordinary skill in the art for using a binding agent to detect polypeptide markers in a sample. *See, e.g.,* Harlow and Lane, Antibodies: A Laboratory Manual, Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length prostate tumor proteins and portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e.*, incubation time) is a period of time that is sufficient to detect the presence of polypeptide within a sample obtained from an individual with prostate cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as prostate cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates (*i.e.,* sensitivity) and false positive rates (100%-specificity) that correspond to each possible cut-off value for the diagnostic test result. The cut-off value on the plot that is the closest to the upper left-hand corner (*i.e.*, the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use prostate tumor polypeptides to detect antibodies that bind to such polypeptides in a biological sample. The detection of such prostate tumor protein specific antibodies may correlate with the presence of a cancer.

A cancer may also, or alternatively, be detected based on the presence of T cells that specifically react with a prostate tumor protein in a biological sample. Within certain methods, a biological sample comprising CD4⁺ and/or CD8⁺ T cells isolated from a patient is incubated with a prostate tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with prostate tumor polypeptide (*e.g.*, 5 - 25 µg/ml). It may be desirable to incubate another aliquot of a T cell sample in the absence of prostate tumor polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a prostate tumor protein in a biological sample. For example, at least two oligonucleotide primers may be employed in a polymerase chain reaction (PCR) based assay to amplify a portion of a prostate tumor cDNA derived from a biological sample, wherein at least one of the oligonucleotide primers is specific for (*i.e.*, hybridizes to) a polynucleotide encoding the prostate tumor protein. The amplified cDNA is then separated and detected using techniques well known in the art, such as gel electrophoresis. Similarly, oligonucleotide probes that specifically hybridize to a polynucleotide encoding a prostate tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a prostate tumor protein that is at least 10 nucleotides, and preferably at least 20 nucleotides, in length. Preferably, oligonucleotide primers and/or probes will hybridize to a polynucleotide encoding a polypeptide disclosed herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence recited in SEQ ID NO: 1-111, 115-171, 173-175, 177, 179-305, 307-315, 326, 328, 330, 332-335, 340-375 and 381. Techniques for both PCR based assays and hybridization assays are well known in the art (*see,* for example, Mullis et al., Cold Spring Harbor Symp. Quant. Biol., 51:263, 1987; Erlich ed., PCR Technology, Stockton Press, NY, 1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, the disclosed compositions may be used as markers for the progression of cancer. In this embodiment, assays as described above for the diagnosis of a cancer may be performed over time, and the change in the level of reactive polypeptide(s) or polynucleotide evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple prostate tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

### DIAGNOSTIC KITS

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a prostate tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a prostate tumor protein in a biological sample. Such kits generally comprise at least one oligonucleotide probe or primer, as described above, that hybridizes to a polynucleotide encoding a prostate tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a prostate tumor protein.

The following Examples are offered by way of illustration and not by way of limitation.

### EXAMPLES

### EXAMPLE 1

### ISOLATION AND CHARACTERIZATION OF PROSTATE TUMOR POLYPEPTIDES

This Example describes the isolation of certain prostate tumor polypeptides from a prostate tumor cDNA library.

A human prostate tumor cDNA expression library was constructed from prostate tumor poly A⁺ RNA using a Superscript Plasmid System for cDNA Synthesis and Plasmid Cloning kit (BRL Life Technologies, Gaithersburg, MD 20897) following the manufacturer's protocol. Specifically, prostate tumor tissues were homogenized with polytron (Kinematica, Switzerland) and total RNA was extracted using Trizol reagent (BRL Life Technologies) as directed by the manufacturer. The poly A⁺ RNA was then purified using a Qiagen oligotex spin column mRNA purification kit (Qiagen, Santa Clarita, CA 91355) according to the manufacturer's protocol. First-strand cDNA was synthesized using the NotI/Oligo-dT18 primer. Double-stranded cDNA was synthesized, ligated with EcoRI/BAXI adaptors (Invitrogen, San Diego, CA) and digested with NotI. Following size fractionation with Chroma Spin-1000 columns (Clontech, Palo Alto, CA), the cDNA was ligated into the EcoRI/NotI site of pCDNA3.1 (Invitrogen) and transformed into ElectroMax *E. coli* DH10B cells (BRL Life Technologies) by electroporation.

Using the same procedure, a normal human pancreas cDNA expression library was prepared from a pool of six tissue specimens (Clontech). The cDNA libraries were characterized by determining the number of independent colonies, the percentage of clones that carried insert, the average insert size and by sequence analysis. The prostate tumor library contained 1.64 x 10⁷ independent colonies, with 70% of clones having an insert and the average insert size being 1745 base pairs. The normal pancreas cDNA library contained 3.3 x 10⁶ independent colonies, with 69% of clones having inserts and the average insert size being 1120 base pairs. For both libraries, sequence analysis showed that the majority of clones had a full length cDNA sequence and were synthesized from mRNA, with minimal rRNA and mitochondrial DNA contamination.

cDNA library subtraction was performed using the above prostate tumor and normal pancreas cDNA libraries, as described by Hara et al. (Blood, 84:189-199, 1994) with some modifications. Specifically, a prostate tumor-specific subtracted cDNA library was generated as follows. Normal pancreas cDNA library (70 µg) was digested with EcoRI, NotI, and SfuI, followed by a filling-in reaction with DNA polymerase Klenow fragment. After phenol-chloroform extraction and ethanol precipitation, the DNA was dissolved in 100 µl of H₂O, heat-denatured and mixed with 100 µl (1.00 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (50 µl) was added and the biotinylation reaction was repeated. After extraction with butanol five times, the DNA was ethanol-precipitated and dissolved in 23 µl H₂O to form the driver DNA.

To form the tracer DNA, 10 µg prostate tumor cDNA library was digested with BamHI and XhoI, phenol chloroform extracted and passed through Chroma spin-400 columns (Clontech). Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O mixed with 8 µl driver DNA and 20 µl of 2 x hybridization buffer, and subjected to a hybridization at 68 °C for 2 hours (short hybridization [SH]). After removal of biotinylated double-stranded DNA, subtracted cDNA was ligated into BamHI/XhoI site of chloramphenicol resistant pBCSK⁺ (Stratagene, La Jolla, CA 92037) and transformed into ElectroMax *E. coli* DH10B cells by electroporation to generate a prostate tumor specific subtracted cDNA library (referred to as "prostate subtraction 1").

To analyze the subtracted cDNA library, plasmid DNA was prepared from 100 independent clones, randomly picked from the subtracted prostate tumor specific library and grouped based on insert size. Representative cDNA clones were further characterized by DNA sequencing with a Perkin Elmer/Applied Biosystems Division Automated Sequencer Model 373A (Foster City, CA). Six cDNA clones, hereinafter referred to as F1-13, F1-12, F1-16, H1-1, H1-9 and H1-4, were shown to be abundant in the subtracted prostate-specific cDNA library. The determined 3' and 5' cDNA sequences for F1-12 are provided in SEQ ID NO: 2 and 3, respectively, with determined 3' cDNA sequences for F1-13, F1-16, H1-1, H1-9 and H1-4 being provided in SEQ ID NO: 1 and 4-7, respectively.

The cDNA sequences for the isolated clones were compared to known sequences in the gene bank using the EMBL and GenBank databases (release 96). Four of the prostate tumor cDNA clones, F1-13, F1-16, H1-1, and H1-4, were determined to encode the following previously identified proteins: prostate specific antigen (PSA), human glandular kallikrein, human tumor expression enhanced gene, and mitochondria cytochrome C oxidase subunit II. H1-9 was found to be identical to a previously identified human autonomously replicating sequence. No significant homologies to the cDNA sequence for F1-12 were found.

Subsequent studies led to the isolation of a full-length cDNA sequence for F1-12. This sequence is provided in SEQ ID NO: 107, with the corresponding predicted amino acid sequence being provided in SEQ ID NO: 108.

To clone less abundant prostate tumor specific genes, cDNA library subtraction was performed by subtracting the prostate tumor cDNA library described above with the normal pancreas cDNA library and with the three most abundant genes in the previously subtracted prostate tumor specific cDNA library: human glandular kallikrein, prostate specific antigen (PSA), and mitochondria cytochrome C oxidase subunit II. Specifically, 1 µg each of human glandular kallikrein, PSA and mitochondria cytochrome C oxidase subunit II cDNAs in pCDNA3.1 were added to the driver DNA and subtraction was performed as described above to provide a second subtracted cDNA library hereinafter referred to as the "subtracted prostate tumor specific cDNA library with spike".

Twenty-two cDNA clones were isolated from the subtracted prostate tumor specific cDNA library with spike. The determined 3' and 5' cDNA sequences for the clones referred to as J1-17 L1-12, N1-1862, J1-13, J1-19, J1-25, J1-24, K1-58, K1-63, L1-4 and L1-14 are provided in SEQ ID NOS: 8-9, 10-11, 12-13, 14-15, 16-17, 18-19, 20-21, 22-23, 24-25, 26-27 and 28-29, respectively. The determined 3' cDNA sequences for the clones referred to as J1-12, J1-16, J1-21, K1-48, K1-55, L1-2, L1-6, N1-1858, N1-1860, N1-1861, N1-1864 are provided in SEQ ID NOS: 30-40, respectively. Comparison of these sequences with those in the gene bank as described above, revealed no significant homologies to three of the five most abundant DNA species, (J1-17, L1-12 and N1-1862; SEQ ID NOS: 8-9, 10-11 and 12-13, respectively). Of the remaining two most abundant species, one (J1-12; SEQ ID NO:30) was found to be identical to the previously identified human pulmonary surfactant-associated protein, and the other (K1-48; SEQ ID NO:33) was determined to have some homology to R. *norvegicus* mRNA for 2-arylpropionyl-CoA epimerase. Of the 17 less abundant cDNA clones isolated from the subtracted prostate tumor specific cDNA library with spike, four (J1-16, K1-55, L1-6 and N1-1864; SEQ ID NOS:31, 34, 36 and 40, respectively) were found to be identical to previously identified sequences, two (J1-21 and N1-1860; SEQ ID NOS: 32 and 38, respectively) were found to show some homology to non-human sequences, and two (L1-2 and N1-1861; SEQ ID NOS: 35 and 39, respectively) were found to show some homology to known human sequences. No significant homologies were found to the polypeptides J1-13, J1-19, J1-24, J1-25, K1-58, K1-63, L1-4, L1-14 (SEQ ID NOS: 14-15, 16-17, 20-21, 18-19, 22-23, 24-25, 26-27, 28-29, respectively).

Subsequent studies led to the isolation of full length cDNA sequences for J1-17, L1-12 and N1-1862 (SEQ ID NOS: 109-111, respectively). The corresponding predicted amino acid sequences are provided in SEQ ID NOS: 112-114. L1-12 is also referred to as P501S.

In a further experiment, four additional clones were identified by subtracting a prostate tumor cDNA library with normal prostate cDNA prepared from a pool of three normal prostate poly A+ RNA (referred to as "prostate subtraction 2"). The determined cDNA sequences for these clones, hereinafter referred to as U1-3064, U1-3065, V1-3692 and 1A-3905, are provided in SEQ ID NO: 69-72, respectively. Comparison of the determined sequences with those in the gene bank revealed no significant homologies to U1-3065.

A second subtraction with spike (referred to as "prostate subtraction spike 2") was performed by subtracting a prostate tumor specific cDNA library with spike with normal pancreas cDNA library and further spiked with PSA, J1-17, pulmonary surfactant-associated protein, mitochondrial DNA, cytochrome c oxidase subunit II, N1-1862, autonomously replicating sequence, L1-12 and tumor expression enhanced gene. Four additional clones, hereinafter referred to as V1-3686, R1-2330, 1B-3976 and V1-3679, were isolated. The determined cDNA sequences for these clones are provided in SEQ ID NO:73-76, respectively. Comparison of these sequences with those in the gene bank revealed no significant homologies to V1-3686 and R1-2330.

Further analysis of the three prostate subtractions described above (prostate subtraction 2, subtracted prostate tumor specific cDNA library with spike, and prostate subtraction spike 2) resulted in the identification of sixteen additional clones, referred to as 1G-4736, 1G-4738, 1G-4741, 1G-4744, 1G-4734, 1H-4774, 1H-4781, 1H-4785, 1H-4787, 1H-4796, 1I-4810, 1I-4811, 1J-4876, 1K-4884 and 1K-4896. The determined cDNA sequences for these clones are provided in SEQ ID NOS: 77-92, respectively. Comparison of these sequences with those in the gene bank as described above, revealed no significant homologies to 1G-4741, 1G-4734, 11-4807, 1J-4876 and 1K-4896 (SEQ ID NOS: 79, 81, 87, 90 and 92, respectively). Further analysis of the isolated clones led to the determination of extended cDNA sequences for 1G-4736, 1G-4738, 1G-4741, 1G-4744, 1H-4774, 1H-4781, 1H-4785, 1H-4787, 1H-4796, 1I-4807, 1J-4876, 1K-4884 and 1K-4896, provided in SEQ ID NOS: 179-188 and 191-193, respectively, and to the determination of additional partial cDNA sequences for 1I-4810 and 1I-4811, provided in SEQ ID NOS: 189 and 190, respectively.

Additional studies with prostate subtraction spike 2 resulted in the isolation of three more clones. Their sequences were determined as described above and compared to the most recent GenBank. All three clones were found to have homology to known genes, which are Cysteine-rich protein, KIAA0242, and KIAA0280 (SEQ ID NO: 317, 319, and 320, respectively). Further analysis of these clones by Synteni microarray (Synteni, Palo Alto, CA) demonstrated that all three clones were over-expressed in most prostate tumors and prostate BPH, as well as in the majority of normal prostate tissues tested, but low expression in all other normal tissues.

An additional subtraction was performed by subtracting a normal prostate cDNA library with normal pancreas cDNA (referred to as "prostate subtraction 3"). This led to the identification of six additional clones referred to as 1G-4761, 1G-4762, 1H-4766, 1H-4770, 1H-4771 and 1H-4772 (SEQ ID NOS: 93-98). Comparison of these sequences with those in the gene bank revealed no significant homologies to 1G-4761 and 1H-4771 (SEQ ID NOS: 93 and 97, respectively). Further analysis of the isolated clones led to the determination of extended cDNA sequences for 1G-4761, 1 G-4762, 1H-4766 and 1H-4772 provided in SEQ ID NOS: 194-196 and 199, respectively, and to the determination of additional partial cDNA sequences for 1H-4770 and 1H-4771, provided in SEQ ID NOS: 197 and 198, respectively.

Subtraction of a prostate tumor cDNA library, prepared from a pool of polyA+ RNA from three prostate cancer patients, with a normal pancreas cDNA library (prostate subtraction 4) led to the identification of eight clones, referred to as 1D-4297, 1D-4309, 1D.1-4278, 1D-4288, 1D-4283, ID-4304, ID-4296 and 1D-4280 (SEQ ID NOS: 99-107). These sequences were compared to those in the gene bank as described above. No significant homologies were found to 1D-4283 and 1D-4304 (SEQ ID NOS: 103 and 104, respectively). Further analysis of the isolated clones led to the determination of extended cDNA sequences for 1D-4309, 1D.1-4278, 1D-4288, 1D-4283, 1D-4304, 1D-4296 and 1D-4280, provided in SEQ ID NOS: 200-206, respectively.

cDNA clones isolated in prostate subtraction 1 and prostate subtraction 2, described above, were colony PCR amplified and their mRNA expression levels in prostate tumor, normal prostate and in various other normal tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Two clones (referred to as P509S and P510S) were found to be over-expressed in prostate tumor and normal prostate and expressed at low levels in all other normal tissues tested (liver, pancreas, skin, bone marrow, brain, breast, adrenal gland, bladder, testes, salivary gland, large intestine, kidney, ovary, lung, spinal cord, skeletal muscle and colon). The determined cDNA sequences for P509S and P510S are provided in SEQ ID NO: 223 and 224, respectively. Comparison of these sequences with those in the gene bank as described above, revealed some homology to previously identified ESTs.

Additional, studies led to the isolation of the full-length cDNA sequence for P509S. This sequence is provided in SEQ ID NO: 332, with the corresponding predicted amino acid sequence being provided in SEQ ID NO: 339.

### EXAMPLE 2

### DETERMINATION OF TISSUE SPECIFICITY OF PROSTATE TUMOR POLYPEPTIDES

Using gene specific primers, mRNA expression levels for the representative prostate tumor polypeptides F1-16, H1-1, J1-17 (also referred to as P502S), L1-12 (also referred to as P501S), F1-12 (also referred to as P504S) and N1-1862 (also referred to as P503S) were examined in a variety of normal and tumor tissues using RT-PCR.

Briefly, total RNA was extracted from a variety of normal and tumor tissues using Trizol reagent as described above. First strand synthesis was carried out using 1-2 µg of total RNA with Superscript II reverse transcriptase (BRL Life Technologies) at 42 °C for one hour. The cDNA was then amplified by PCR with gene-specific primers. To ensure the semi-quantitative nature of the RT-PCR, β-actin was used as an internal control for each of the tissues examined. First, serial dilutions of the first strand cDNAs were prepared and RT-PCR assays were performed using β-actin specific primers. A dilution was then chosen that enabled the linear range amplification of the β-actin template and which was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in four different types of tumor tissue (prostate tumor from 2 patients, breast tumor from 3 patients, colon tumor, lung tumor), and sixteen different normal tissues, including prostate, colon, kidney, liver, lung, ovary, pancreas, skeletal muscle, skin, stomach, testes, bone marrow and brain. F1-16 was found to be expressed at high levels in prostate tumor tissue, colon tumor and normal prostate, and at lower levels in normal liver, skin and testes, with expression being undetectable in the other tissues examined. H1-1 was found to be expressed at high levels in prostate tumor, lung tumor, breast tumor, normal prostate, normal colon and normal brain, at much lower levels in normal lung, pancreas, skeletal muscle, skin, small intestine, bone marrow, and was not detected in the other tissues tested. J1-17 (P502S) and L1-12 (P501S) appear to be specifically over-expressed in prostate, with both genes being expressed at high levels in prostate tumor and normal prostate but at low to undetectable levels in all the other tissues examined. N1-1862 (P503S) was found to be over-expressed in 60% of prostate tumors and detectable in normal colon and kidney. The RT-PCR results thus indicate that F1-16, H1-1, J1-17 (P502S), N1-1862 (P503S) and L1-12 (P501S) are either prostate specific or are expressed at significantly elevated levels in prostate.

Further RT-PCR studies showed that F1-12 (P504S) is over-expressed in 60% of prostate tumors, detectable in normal kidney but not detectable in all other tissues tested. Similarly, R1-2330 was shown to be over-expressed in 40% of prostate tumors, detectable in normal kidney and liver, but not detectable in all other tissues tested. U1-3064 was found to be over-expressed in 60% of prostate tumors, and also expressed in breast and colon tumors, but was not detectable in normal tissues.

RT-PCR characterization of R1-2330, U1-3064 and 1D-4279 showed that these three antigens are over-expressed in prostate and/or prostate tumors.

Northern analysis with four prostate tumors, two normal prostate samples, two BPH prostates, and normal colon, kidney, liver, lung, pancrease, skeletal muscle, brain, stomach, testes, small intestine and bone marrow, showed that L1-12 (P501S) is over-expressed in prostate tumors and normal prostate, while being undetectable in other normal tissues tested. J1-17 (P502S) was detected in two prostate tumors and not in the other tissues tested. N1-1862 (P503S) was found to be over-expressed in three prostate tumors and to be expressed in normal prostate, colon and kidney, but not in other tissues tested. F1-12(P504S) was found to be highly expressed in two prostate tumors and to be undetectable in all other tissues tested.

The microarray technology described above was used to determine the expression levels of representative antigens described herein in prostate tumor, breast tumor and the following normal tissues: prostate, liver, pancreas, skin, bone marrow, brain, breast, adrenal gland, bladder, testes, salivary gland, large intestine, kidney, ovary, lung, spinal cord, skeletal muscle and colon. L1-12 (P501S) was found to be over-expressed in normal prostate and prostate tumor, with some expression being detected in normal skeletal muscle. Both J1-12 and F1-12 (P504S) were found to be over-expressed in prostate tumor, with expression being lower or undetectable in all other tissues tested. N1-1862 (P503S) was found to be expressed at high levels in prostate tumor and normal prostate, and at low levels in normal large intestine and normal colon, with expression being undetectable in all other tissues tested. R1-2330 was found to be over-expressed in prostate tumor and normal prostate, and to be expressed at lower levels in all other tissues tested. 1D-4279 was found to be over-expressed in prostate tumor and normal prostate, expressed at lower levels in normal spinal cord, and to be undetectable in all other tissues tested.

Further microarray analysis to specifically address the extent to which P501S (SEQ ID NO: 110) was expressed in breast tumor revealed moderate over-expression not only in breast tumor; but also in metastatic breast tumor (2/31), with negligible to low expression in normal tissues. This data suggests that P501S may be over-expressed in various breast tumors as well as in prostate tumors.

The expression levels of 32 ESTs (expressed sequence tags) described by Vasmatzis et al. (Proc. Natl. Acad. Sci. USA 95:300-304, 1998) in a variety of tumor and normal tissues were examined by microarray technology as described above. Two of these clones (referred to as P1000C and P1001C) were found to be over-expressed in prostate tumor and normal prostate, and expressed at low to undetectable levels in all other tissues tested (normal aorta, thymus, resting and activated PBMC, epithelial cells, spinal cord, adrenal gland, fetal tissues, skin, salivary gland, large intestine, bone marrow, liver, lung, dendritic cells, stomach, lymph nodes, brain, heart, small intestine, skeletal muscle, colon and kidney. The determined cDNA sequences for P1000C and P1001C are provided in SEQ ID NO: 384 and 472, respectively. The sequence of P1001C was found to show some homology to the previously isolated Human mRNA for JM27 protein. No significant homologies were found to the sequence of P1000C.

The expression of the polypeptide encoded by the full length cDNA sequence for F1-12 (also referred to as P504S; SEQ ID NO: 108) was investigated by immunohistochemical analysis. Rabbit-anti-P504S polyclonal antibodies were generated against the full length P504S protein by standard techniques. Subsequent isolation and characterization of the polyclonal antibodies were also performed by techniques well known in the art. Immunohistochemical analysis showed that the P504S polypeptide was expressed in 100% of prostate carcinoma samples tested (n=5).

The rabbit-anti-P504S polyclonal antibody did not appear to label benign prostate cells with the same cytoplasmic granular staining, but rather with light nuclear staining. Analysis of normal tissues revealed that the encoded polypeptide was found to be expressed in some, but not all normal human tissues. Positive cytoplasmic staining with rabbit-anti-P504S polyclonal antibody was found in normal human kidney, liver, brain, colon and lung-associated macrophages, whereas heart and bone marrow were negative.

This data indicates that the P504S polypeptide is present in prostate cancer tissues, and that there are qualitative and quantitative differences in the staining between benign prostatic hyperplasia tissues and prostate cancer tissues, suggesting that this polypeptide may be detected selectively in prostate tumors and therefore be useful in the diagnosis of prostate cancer.

### EXAMPLE 3

### ISOLATION AND CHARACTERIZATION OF PROSTATE TUMOR POLYPEPTIDES BY PCR-BASED SUBTRACTION

A cDNA subtraction library, containing cDNA from normal prostate subtracted with ten other normal tissue cDNAs (brain, heart, kidney, liver, lung, ovary, placenta, skeletal muscle, spleen and thymus) and then submitted to a first round of PCR amplification, was purchased from Clontech. This library was subjected to a second round of PCR amplification, following the manufacturer's protocol. The resulting cDNA fragments were subcloned into the vector pT7 Blue T-vector (Novagen, Madison, WI) and transformed into XL-1 Blue MRF' *E. coli* (Stratagene). DNA was isolated from independent clones and sequenced using a Perkin Elmer/Applied Biosystems Division Automated Sequencer Model 373A.

Fifty-nine positive clones were sequenced. Comparison of the DNA sequences of these clones with those in the gene bank, as described above, revealed no significant homologies to 25 of these clones, hereinafter referred to as P5, P8, P9, P18, P20, P30, P34, P36, P38, P39, P42, P49, P50, P53, P55, P60, P64, P65, P73, P75, P76, P79 and P84. The determined cDNA sequences for these clones are provided in SEQ ID NO: 41-45, 47-52 and 54-65, respectively. P29, P47, P68, P80 and P82 (SEQ ID NO: 46, 53 and 66-68, respectively) were found to show some degree of homology to previously identified DNA sequences. To the best of the inventors' knowledge, none of these sequences have been previously shown to be present in prostate.

Further studies using the PCR-based methodology described above resulted in the isolation of more than 180 additional clones, of which 23 clones were found to show no significant homologies to known sequences. The determined cDNA sequences for these clones are provided in SEQ ID NO: 115-123, 127, 131, 137, 145, 147-151, 153, 156-158 and 160. Twenty-three clones (SEQ ID NO: 124-126, 128-130, 132-136, 138-144, 146, 152, 154, 155 and 159) were found to show some homology to previously identified ESTs. An additional ten clones (SEQ ID NO: 161-170) were found to have some degree of homology to known genes. Larger cDNA clones containing the P20 sequence represent splice variants of a gene referred to as P703P. The determined DNA sequence for the variants referred to as DE1, DE13 and DE14 are provided in SEQ ID NOS: 171, 175 and 177, respectively, with the corresponding predicted amino acid sequences being provided in SEQ ID NO: 172, 176 and 178, respectively. The determined cDNA sequence for an extended spliced form of P703 is provided in SEQ ID NO: 225. The DNA sequences for the splice variants referred to as DE2 and DE6 are provided in SEQ ID NOS: 173 and 174, respectively.

mRNA Expression levels for representative clones in tumor tissues (prostate (n=5), breast (n=2), colon and lung) normal tissues (prostate (n=5), colon, kidney, liver, lung (n=2), ovary (n=2), skeletal muscle, skin, stomach, small intestine and brain), and activated and non-activated PBMC was determined by RT-PCR as described above. Expression was examined in one sample of each tissue type unless otherwise indicated.

P9 was found to be highly expressed in normal prostate and prostate tumor compared to all normal tissues tested except for normal colon which showed comparable expression. P20, a portion of the P703P gene, was found to be highly expressed in normal prostate and prostate tumor, compared to all twelve normal tissues tested. A modest increase in expression of P20 in breast tumor (n=2), colon tumor and lung tumor was seen compared to all normal tissues except lung (1 of 2). Increased expression of P 18 was found in normal prostate, prostate tumor and breast tumor compared to other normal tissues except lung and stomach. A modest increase in expression of P5 was observed in normal prostate compared to most other normal tissues. However, some elevated expression was seen in normal lung and PBMC. Elevated expression of P5 was also observed in prostate tumors (2 of 5), breast tumor and one lung tumor sample. For P30, similar expression levels were seen in normal prostate and prostate tumor, compared to six of twelve other normal tissues tested. Increased expression was seen in breast tumors, one lung tumor sample and one colon tumor sample, and also in normal PBMC. P29 was found to be over-expressed in prostate tumor (5 of 5) and normal prostate (5 of 5) compared to the majority of normal tissues. However, substantial expression of P29 was observed in normal colon and normal lung (2 of 2). P80 was found to be over-expressed in prostate tumor (5 of 5) and normal prostate (5 of 5) compared to all other normal tissues tested, with increased expression also being seen in colon tumor.

Further studies resulted in the isolation of twelve additional clones, hereinafter referred to as 10-d8, 10-h10, 11-c8, 7-g6, 8-b5, 8-b6, 8-d4, 8-d9, 8-g3, 8-h11, 9-f12 and 9-f3. The determined DNA sequences for 10-d8, 10-h10, 11-c8, 8-d4, 8-d9, 8-h11, 9-f12 and 9-f3 are provided in SEQ ID NO: 207, 208, 209, 216, 217, 220, 221 and 222, respectively. The determined forward and reverse DNA sequences for 7-g6, 8-b5, 8-b6 and 8-g3 are provided in SEQ ID NO: 210 and 211; 212 and 213; 214 and 215; and 218 and 219, respectively. Comparison of these sequences with those in the gene bank revealed no significant homologies to the sequence of 9-f3. The clones 10-d8, 11-c8 and 8-h1 1 were found to show some homology to previously isolated ESTs, while 10-h10, 8-b5, 8-b6, 8-d4, 8-d9, 8-g3 and 9-f12 were found to show some homology to previously identified genes. Further characterization of 7-G6 and 8-G3 showed identity to the known genes PAP and PSA, respectively.

mRNA expression levels for these clones were determined using the micro-array technology described above. The clones 7-G6, 8-G3, 8-B5, 8-B6, 8-D4, 8-D9, 9-F3, 9-F12, 9-H3, 10-A2, 10-A4, 11-C9 and 11-F2 were found to be over-expressed in prostate tumor and normal prostate, with expression in other tissues tested being low or undetectable. Increased expression of 8-F11 was seen in prostate tumor and normal prostate, bladder, skeletal muscle and colon. Increased expression of 10-H10 was seen in prostate tumor and normal prostate, bladder, lung, colon, brain and large intestine. Increased expression of 9-B1 was seen in prostate tumor, breast tumor, and normal prostate, salivary gland, large intestine and skin, with increased expression of 11-C8 being seen in prostate tumor, and normal prostate and large intestine.

An additional cDNA fragment derived from the PCR-based normal prostate subtraction, described above, was found to be prostate specific by both micro-array technology and RT-PCR. The determined cDNA sequence of this clone (referred to as 9-A11) is provided in SEQ ID NO: 226. Comparison of this sequence with those in the public databases revealed 99% identity to the known gene HOXB13.

Further studies led to the isolation of the clones 8-C6 and 8-H7. The determined cDNA sequences for these clones are provided in SEQ ID NO: 227 and 228, respectively. These sequences were found to show some homology to previously isolated ESTs.

PCR and hybridization-based methodologies were employed to obtain longer cDNA sequences for clone P20 (also referred to as P703P), yielding three additional cDNA fragments that progressively extend the 5' end of the gene. These fragments, referred to as P703PDE5, P703P6.26, and P703PX-23 (SEQ ID NO: 326, 328 and 330, with the predicted corresponding amino acid sequences being provided in SEQ ID NO: 327, 329 and 331, respectively) contain additional 5' sequence. P703PDE5 was recovered by screening of a cDNA library (#141-26) with a portion of P703P as a probe. P703P6.26 was recovered from a mixture of three prostate tumor cDNAs and P703PX_23 was recovered from cDNA library (#438-48). Together, the additional sequences include all of the putative mature serine protease along with part of the putative signal sequence. Further studies using a PCR-based subtraction library of a prostate tumor pool subtracted against a pool of normal tissues (referred to as JP: PCR subtraction) resulted in the isolation of thirteen additional clones, seven of which did not share any significant homology to known GenBank sequences. The determined cDNA sequences for these seven clones (P711P, P712P, novel 23, P774P, P775P, P710P and P768P) are provided in SEQ ID NO: 307-311, 313 and 315, respectively. The remaining six clones (SEQ ID NO: 316 and 321-325) were shown to share some homology to known genes. By microarray analysis, all thirteen clones showed three or more fold over-expression in prostate tissues, including prostate tumors, BPH and normal prostate as compared to normal non-prostate tissues. Clones P711P, P712P, novel 23 and P768P showed over-expression in most prostate tumors and BPH tissues tested (n=29), and in the majority of normal prostate tissues (n=4), but background to low expression levels in all normal tissues. Clones P774P, P775P and P710P showed comparatively lower expression and expression in fewer prostate tumors and BPH samples, with negative to low expression in normal prostate.

The full-length cDNA for P711P was obtained by employing the partial sequence of SEQ ID NO: 307 to screen a prostate cDNA library. Specifically, a directionally cloned prostate cDNA library was prepared using standard techniques. One million colonies of this library were plated onto LB/Amp plates. Nylon membrane filters were used to lift these colonies, and the cDNAs which were picked up by these filters were denatured and cross-linked to the filters by UV light. The P711P cDNA fragment of SEQ ID NO: 307 was radio-labeled and used to hybridize with these filters. Positive clones were selected, and cDNAs were prepared and sequenced using an automatic Perkin Elmer/Applied Biosystems sequencer. The determined full-length sequence of P711P is provided in SEQ ID NO: 382, with the corresponding predicted amino acid sequence being provided in SEQ ID NO: 383.

Using PCR and hybridization-based methodologies, additional cDNA sequence information was derived for two clones described above, 11-C9 and 9-F3, herein after referred to as P707P and P714P, respectively (SEQ ID NO: 333 and 334). After comparison with the most recent GenBank, P707P was found to be a splice variant of the known gene HoxB 13. In contrast, no significant homologies to P714P were found.

Clones 8-B3, P89, P98, P130 and P201 (as disclosed in U.S. Patent Application No. 09/020,956, filed February 9, 1998) were found to be contained within one contiguous sequence, referred to as P705P (SEQ ID NO: 335, with the predicted amino acid sequence provided in SEQ ID NO: 336), which was determined to be a splice variant of the known gene NKX 3.1.

### EXAMPLE 4

### SYNTHESIS OF POLYPEPTIDES

Polypeptides may be synthesized on a Perkin Elmer/Applied Biosystems 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1 % TFA) may be used to elute the peptides. Following lyophilization of the pure fractions, the peptides may be characterized using electrospray or other types of mass spectrometry and by amino acid analysis.

### EXAMPLE 5

### FURTHER ISOLATION AND CHARACTERIZATION OF PROSTATE TUMOR POLYPEPTIDES BY PCR-BASED SUBTRACTION

A cDNA library generated from prostate primary tumor mRNA as described above was subtracted with cDNA from normal prostate. The subtraction was performed using a PCR-based protocol (Clontech), which was modified to generate larger fragments. Within this protocol, tester and driver double stranded cDNA were separately digested with five restriction enzymes that recognize six-nucleotide restriction sites (MluI, MscI, PvuII, SalI and StuI). This digestion resulted in an average cDNA size of 600 bp, rather than the average size of 300 bp that results from digestion with RsaI according to the Clontech protocol. This modification did not affect the subtraction efficiency. Two tester populations were then created with different adapters, and the driver library remained without adapters.

The tester and driver libraries were then hybridized using excess driver cDNA. In the first hybridization step, driver was separately hybridized with each of the two tester cDNA populations. This resulted in populations of (a) unhybridized tester cDNAs, (b) tester cDNAs hybridized to other tester cDNAs, (c) tester cDNAs hybridized to driver cDNAs and (d) unhybridized driver cDNAs. The two separate hybridization reactions were then combined, and rehybridized in the presence of additional denatured driver cDNA. Following this second hybridization, in addition to populations (a) through (d), a fifth population (e) was generated in which tester cDNA with one adapter hybridized to tester cDNA with the second adapter. Accordingly, the second hybridization step resulted in enrichment of differentially expressed sequences which could be used as templates for PCR amplification with adaptor-specific primers.

The ends were then filled in, and PCR amplification was performed using adaptor-specific primers. Only population (e), which contained tester cDNA that did not hybridize to driver cDNA, was amplified exponentially. A second PCR amplification step was then performed, to reduce background and further enrich differentially expressed sequences.

This PCR-based subtraction technique normalizes differentially expressed cDNAs so that rare transcripts that are overexpressed in prostate tumor tissue may be recoverable. Such transcripts would be difficult to recover by traditional subtraction methods.

In addition to genes known to be overexpressed in prostate tumor, seventy-seven further clones were identified. Sequences of these partial cDNAs are provided in SEQ ID NO: 29 to 305. Most of these clones had no significant homology to database sequences. Exceptions were JPTPN23 (SEQ ID NO: 231; similarity to pig valosin-containing protein), JPTPN30 (SEQ ID NO: 234; similarity to rat mRNA for proteasome subunit), JPTPN45 (SEQ ID NO: 243; similarity to rat *norvegicus* cytosolic NADP-dependent isocitrate dehydrogenase), JPTPN46 (SEQ ID NO: 244; similarity to human subclone H8 4 d4 DNA sequence), JP1D6 (SEQ ID NO: 265; similarity to *G. gallus* dynein light chain-A), JP8D6 (SEQ ID NO: 288; similarity to human BAC clone RG016J04), JP8F5 (SEQ ID NO: 289; similarity to human subclone H8 3 b5 DNA sequence), and JP8E9 (SEQ ID NO: 299; similarity to human Alu sequence).

Additional studies using the PCR-based subtraction library consisting of a prostate tumor pool subtracted against a normal prostate pool (referred to as PT-PN PCR subtraction) yielded three additional clones. Comparison of the cDNA sequences of these clones with the most recent release of GenBank revealed no significant homologies to the two clones referred to as P715P and P767P (SEQ ID NO: 312 and 314). The remaining clone was found to show some homology to the known gene KIAA0056 (SEQ ID NO: 318). Using microarray analysis to measure mRNA expression levels in various tissues, all three clones were found to be over-expressed in prostate tumors and BPH tissues. Specifically, clone P715P was over-expressed in most prostate tumors and BPH tissues by a factor of three or greater, with elevated expression seen in the majority of normal prostate samples and in fetal tissue, but negative to low expression in all other normal tissues. Clone P767P was over-expressed in several prostate tumors and BPH tissues, with moderate expression levels in half of the normal prostate samples, and background to low expression in all other normal tissues tested.

Further analysis, by microarray as described above, of the PT-PN PCR subtraction library and of a DNA subtraction library containing cDNA from prostate tumor subtracted with a pool of normal tissue cDNAs, led to the isolation of 27 additional clones (SEQ ID NO: 340-365 and 381) which were determined to be over-expressed in prostate tumor. The clones of SEQ ID NO: 341, 342, 345, 347, 348, 349, 351, 355-359, 361, 362 and 364 were also found to be expressed in normal prostate. Expression of all 26 clones in a variety of normal tissues was found to be low or undetectable, with the exception of P544S (SEQ ID NO: 356) which was found to be expressed in small intestine. Of the 26 clones, 10 (SEQ ID NO: 340-349) were found to show some homology to previously identified sequences. No significant homologies were found to the clones of SEQ ID NO: 350-365.

### EXAMPLE 6

### PEPTIDE PRIMING OF MICE AND PROPAGATION OF CTL LINES

6.1. This Example illustrates the preparation of a CTL cell line specific for cells expressing the P502S gene.

Mice expressing the transgene for human HLA A2.1 (provided by Dr L. Sherman, The Scripps Research Institute, La Jolla, CA) were immunized with P2S#12 peptide (VLGWVAEL; SEQ ID NO: 306), which is derived from the P502S gene (also referred to herein as J1-17, SEQ ID NO: 8), as described by Theobald et al., Proc. Natl. Acad. Sci. USA 92:11993-11997, 1995 with the following modifications. Mice were immunized with 100µg of P2S#12 and 120µg of an I-A^{b} binding peptide derived from hepatitis B Virus protein emulsified in incomplete Freund's adjuvant. Three weeks later these mice were sacrificed and using a nylon mesh single cell suspensions prepared. Cells were then resuspended at 6 x 10⁶ cells/ml in complete media (RPMI-1640; Gibco BRL, Gaithersburg, MD) containing 10% FCS, 2mM Glutamine (Gibco BRL), sodium pyruvate (Gibco BRL), non-essential amino acids (Gibco BRL), 2 x 10⁻⁵ M 2-mercaptoethanol, 50U/ml penicillin and streptomycin, and cultured in the presence of irradiated (3000 rads) P2S#12-pulsed (5mg/ml P2S#12 and 10mg/ml β2-microglobulin) LPS blasts (A2 transgenic spleens cells cultured in the presence of 7µg/ml dextran sulfate and 25µg/ml LPS for 3 days). Six days later, cells (5 x 10⁵/ml) were restimulated with 2.5 x 10⁶/ml peptide pulsed irradiated (20,000 rads) EL4A2Kb cells (Sherman et al, Science 258:815-818, 1992) and 3 x 10⁶/ml A2 transgenic spleen feeder cells. Cells were cultured in the presence of 20U/ml IL-2. Cells continued to be restimulated on a weekly basis as described, in preparation for cloning the line.

P2S#12 line was cloned by limiting dilution analysis with peptide pulsed EL4 A2Kb tumor cells (1 x 10⁴ cells/ well) as stimulators and A2 transgenic spleen cells as feeders ( 5 x 10⁵ cells/ well) grown in the presence of 30U/ml IL-2. On day 14, cells were restimulated as before. On day 21, clones that were growing were isolated and maintained in culture. Several of these clones demonstrated significantly higher reactivity (lysis) against human fibroblasts (HLA A2.1 expressing) transduced with P502S than against control fibroblasts. An example is presented in Figure 1.

This data indicates that P2S #12 represents a naturally processed epitope of the P502S protein that is expressed in the context of the human HLA A2.1 molecule.

6.2. This Example illustrates the preparation of murine CTL lines and CTL clones specific for cells expressing the P501 S gene.

This series of experiments were performed similarly to that described above. Mice were immunized with the P1S#10 peptide (SEQ ID NO: 337), which is derived from the P501S gene (also referred to herein as L1-12, SEQ ID NO: 110). The P1S#10 peptide was derived by analysis of the predicted polypeptide sequence for P501S for potential HLA-A2 binding sequences as defined by published HLA-A2 binding motifs (Parker, KC, et al, J. Immunol., 152:163, 1994). P1S#10 peptide was synthesized as described in Example 4, and empirically tested for HLA-A2 binding using a T cell based competition assay. Predicted A2 binding peptides were tested for their ability to compete HLA-A2 specific peptide presentation to an HLA-A2 restricted CTL clone (D150M58), which is specific for the HLA-A2 binding influenza matrix peptide fluM58. D150M58 CTL secretes TNF in response to self-presentation of peptide fluM58. In the competition assay, test peptides at 100-200 µg/ml were added to cultures of D150M58 CTL in order to bind HLA-A2 on the CTL. After thirty minutes, CTL cultured with test peptides, or control peptides, were tested for their antigen dose response to the fluM58 peptide in a standard TNF bioassay. As shown in Figure 3, peptide P1S#10 competes HLA-A2 restricted presentation of fluM58, demonstrating that peptide P1S#10 binds HLA-A2.

Mice expressing the transgene for human HLA A2.1 were immunized as described by Theobald et al. (Proc. Natl. Acad. Sci. USA 92:11993-11997, 1995) with the following modifications. Mice were immunized with 62.5µg of P1S #10 and 120µg of an I-A^{b} binding peptide derived from Hepatitis B Virus protein emulsified in incomplete Freund's adjuvant. Three weeks later these mice were sacrificed and single cell suspensions prepared using a nylon mesh. Cells were then resuspended at 6 x 10⁶ cells/ml in complete media (as described above) and cultured in the presence of irradiated (3000 rads) PIS#10-pulsed (2µ g/ml P1S#10 and 10mg/ml β2-microglobulin) LPS blasts (A2 transgenic spleens cells cultured in the presence of 7µg/ml dextran sulfate and 25µg/ml LPS for 3 days). Six days later cells (5 x 10⁵/ml) were restimulated with 2.5 x 10⁶/ml peptide-pulsed irradiated (20,000 rads) EL4A2Kb cells, as described above, and 3 x 10⁶/ml A2 transgenic spleen feeder cells. Cells were cultured in the presence of 20 U/ml IL-2. Cells were restimulated on a weekly basis in preparation for cloning. After three rounds of *in vitro* stimulations, one line was generated that recognized P1S#10-pulsed Jurkat A2Kb targets and P501S-transduced Jurkat targets as shown in Figure 4.

A P1S#10-specific CTL line was cloned by limiting dilution analysis with peptide pulsed EL4 A2Kb tumor cells (1 x 10⁴ cells/ well) as stimulators and A2 transgenic spleen cells as feeders ( 5 x 10⁵ cells/ well) grown in the presence of 30U/ml IL-2. On day 14, cells were restimulated as before. On day 21, viable clones were isolated and maintained in culture. As shown in Figure 5, five of these clones demonstrated specific cytolytic reactivity against P501S-transduced Jurkat A2Kb targets. This data indicates that P1S#10 represents a naturally processed epitope of the P501S protein that is expressed in the context of the human HLA-A2.1 molecule.

### EXAMPLE 7

### ABILITY OF HUMAN T CELLS TO RECOGNIZE PROSTATE TUMOR POLYPEPTIDES

This Example illustrates the ability of T cells specific for a prostate tumor polypeptide to recognize human tumor.

Human CD8⁺ T cells were primed *in vitro* to the P2S-12 peptide (SEQ ID NO: 306) derived from P502S (also referred to as J1-17) using dendritic cells according to the protocol of Van Tsai et al. (Critical Reviews in Immunology 18:65-75, 1998). The resulting CD8⁺ T cell microcultures were tested for their ability to recognize the P2S-12 peptide presented by autologous fibroblasts or fibroblasts which were transduced to express the P502S gene in a γ-interferon ELISPOT assay (*see* Lalvani et al., J. Exp. Med. 186:859-865, 1997). Briefly, titrating numbers of T cells were assayed in duplicate on 10⁴ fibroblasts in the presence of 3 µg/ml human β₂-microglobulin and 1 µg/ml P2S-12 peptide or control E75 peptide. In addition, T cells were simultaneously assayed on autologous fibroblasts transduced with the P502S gene or as a control, fibroblasts transduced with HER-2/*neu*. Prior to the assay, the fibroblasts were treated with 10 ng/ml γ-interferon for 48 hours to upregulate class I MHC expression. One of the microcultures (#5) demonstrated strong recognition of both peptide pulsed fibroblasts as well as transduced fibroblasts in a γ-interferon ELISPOT assay. Figure 2A demonstrates that there was a strong increase in the number of γ-interferon spots with increasing numbers of T cells on fibroblasts pulsed with the P2S-12 peptide (solid bars) but not with the control E75 peptide (open bars). This shows the ability of these T cells to specifically recognize the P2S-12 peptide. As shown in Figure 2B, this microculture also demonstrated an increase in the number of γ-interferon spots with increasing numbers of T cells on fibroblasts transduced to express the P502S gene but not the HER-2/*neu* gene. These results provide additional confirmatory evidence that the P2S-12 peptide is a naturally processed epitope of the P502S protein. Furthermore, this also demonstrates that there exists in the human T cell repertoire, high affinity T cells which are capable of recognizing this epitope. These T cells should also be capable of recognizing human tumors which express the P502S gene.

### EXAMPLE 8

### PRIMING OF CTL IN VIVO USING NAKED DNA IMMUNIZATION WITH A PROSTATE ANTIGEN

The prostate tumor antigen L1-12, as described above, is also referred to as P501S. HLA A2Kb Tg mice (provided by Dr L. Sherman, The Scripps Research Institute, La Jolla, CA) were immunized with 100 µg VR10132-P501S either intramuscularly or intradermally. The mice were immunized three times, with a two week interval between immunizations. Two weeks after the last immunization, immune spleen cells were cultured with Jurkat A2Kb-P501S transduced stimulator cells. CTL lines were stimulated weekly. After two weeks of *in vitro* stimulation, CTL activity was assessed against P501 S transduced targets. Two out of 8 mice developed strong anti-P501S CTL responses. These results demonstrate that P501S contains at least one naturally processed A2-restricted CTL epitope.

### EXAMPLE 9

### GENERATION OF HUMAN CTL IN VITRO USING WHOLE GENE PRIMING AND STIMULATION TECHNIQUES WITH PROSTATE TUMOR ANTIGEN

Using *in vitro* whole-gene priming with P501S-retrovirally transduced autologous fibroblasts (see, for example, Yee et al, The Journal of Immunology, 157(9):4079-86, 1996), human CTL lines were derived that specifically recognize autologous fibroblasts transduced with P501S (also known as L1-12), as determined by interferon-γ ELISPOT analysis as described above. Using a panel of HLA-mismatched fibroblast lines transduced with P501S, these CTL lines were shown to be restricted HLA-A2 class I allele. Specifically, dendritic cells (DC) were differentiated from monocyte cultures derived from PBMC of normal human donors by growing for five days in RPMI medium containing 10% human serum, 50 ng/ml human GM-CSF and 30 ng/ml human IL-4. Following culture, DC were infected overnight with recombinant P501S vaccinia virus at a multiplicity of infection (M.O.I) of five, and matured overnight by the addition of 3 µg/ml CD40 ligand. Virus was inactivated by UV irradiation. CD8+ T cells were isolated using a magnetic bead system, and priming cultures were initiated using standard culture techniques. Cultures were restimulated every 7-10 days using autologous primary fibroblasts retrovirally transduced with P501S. Following four stimulation cycles, CD8+ T cell lines were identified that specifically produced interferon-γ when stimulated with P501 S-transduced autologous fibroblasts. The P501S-specific activity could be sustained by the continued stimulation of the cultures with P501S-transduced fibroblasts in the presence of IL-15. A panel of HLA-mismatched fibroblast lines transduced with P501 S were generated to define the restriction allele of the response. By measuring interferon-γ in an ELISPOT assay, the P501S specific response was shown to be restricted by HLA-A2. These results demonstrate that a CD8+ CTL response to P501 S can be elicited.

### EXAMPLE 10

### IDENTIFICATION OF A NATURALLY PROCESSED CTL EPITOPE CONTAINED WITHIN A PROSTATE TUMOR ANTIGEN

The 9-mer peptide p5 (SEQ ID NO: 338) was derived from the P703P antigen (also referred to as P20). The p5 peptide is immunogenic in human HLA-A2 donors and is a naturally processed epitope. Antigen specific CD8+ T cells can be primed following repeated *in vitro* stimulations with monocytes pulsed with p5 peptide. These CTL specifically recognize p5-pulsed target cells in both ELISPOT (as described above) and chromium release assays. Additionally, immunization of HLA-A2 transgenic mice with p5 leads to the generation of CTL lines which recognize a variety of P703P transduced target cells expressing either HLA-A2Kb or HLA-A2. Specifically, HLA-A2 transgenic mice were immunized subcutaneously in the footpad with 100 µg of p5 peptide together with 140 µg of hepatitis B virus core peptide (a Th peptide) in Freund's incomplete adjuvant. Three weeks post immunization, spleen cells from immunized mice were stimulated *in vitro* with peptide-pulsed LPS blasts. CTL activity was assessed by chromium release assay five days after primary *in vitro* stimulation. Retrovirally transduced cells expressing the control antigen P703P and HLA-A2Kb were used as targets. CTL lines that specifically recognized both p5-pulsed targets as well as P703P-expressing targets were identified.

Human *in vitro* priming experiments demonstrated that the p5 peptide is immunogenic in humans. Dendritic cells (DC) were differentiated from monocyte cultures derived from PBMC of normal human donors by culturing for five days in RPMI medium containing 10% human serum, 50 ng/ml human GM-CSF and 30 ng/ml human IL-4. Following culture, the DC were pulsed with p5 peptide and cultured with GM-CSF and IL-4 together with CD8+ T cell enriched PBMC. CTL lines were restimulated on a weekly basis with p5-pulsed monocytes. Five to six weeks after initiation of the CTL cultures, CTL recognition of p5-pulsed target cells was demonstrated.

### EXAMPLE 11

### EXPRESSION OF A BREAST TUMOR-DERIVED ANTIGEN IN PROSTATE

Isolation of the antigen B305D from breast tumor by differential display is described in US Patent Application No. 08/700,014, filed August 20, 1996. Several different splice forms of this antigen were isolated. The determined cDNA sequences for these splice forms are provided in SEQ ID NO: 366-375, with the predicted amino acid sequences corresponding to the sequences of SEQ ID NO: 292, 298 and 301-303 being provided in SEQ ID NO: 299-306, respectively.

The expression levels of B305D in a variety of tumor and normal tissues were examined by real time PCR and by Northern analysis. The results indicated that B305D is highly expressed in breast tumor, prostate tumor, normal prostate tumor and normal testes, with expression being low or undetectable in all other tissues examined (colon tumor, lung tumor, ovary tumor, and normal bone marrow, colon, kidney, liver, lung, ovary, skin, small intestine, stomach).

### EXAMPLE 12

### ELICITATION OF PROSTATE TUMOR ANTIGEN-SPECIFIC CTL RESPONSES IN HUMAN BLOOD

This Example illustrates the ability of a prostate tumor antigen to elicit a CTL response in blood of normal humans.

Autologous dendritic cells (DC) were differentiated from monocyte cultures derived from PBMC of normal donors by growth for five days in RPMI medium containing 10% human serum, 50 ng/ml GMCSF and 30 ng/ml IL-4. Following culture, DC were infected overnight with recombinant P501S-expressing vaccinia virus at an M.O.I. of 5 and matured for 8 hours by the addition of 2 micrograms/ml CD40 ligand. Virus was inactivated by UV irradiation, CD8⁺ cells were isolated by positive selection using magnetic beads, and priming cultures were initiated in 24-well plates. Following five stimulation cycles, CD8+ lines were identified that specifically produced interferon-gamma when stimulated with autologous P501 S-transduced fibroblasts. The P501S-specific activity of cell line 3A-1 could be maintained following additional stimulation cycles on autologous B-LCL transduced with P501S. Line 3A-1 was shown to specifically recognize autologous B-LCL transduced to express P501S, but not EGFP-transduced autologous B-LCL, as measured by cytotoxity assays (⁵¹Cr release) and interferon-gamma production (Interferon-gamma Elispot; *see* above and Lalvani et al., J. Exp. Med. 186:859-865, 1997). The results of these assays are presented in Figures 6A and 6B.

### EXAMPLE 13

### IDENTIFICATION OF PROSTATE TUMOR ANTIGENS BY MICROARRAY ANALYSIS

This Example describes the isolation of certain prostate tumor polypeptides from a prostate tumor cDNA library.

A human prostate tumor cDNA expression library as described above was screened using microarray analysis to identify clones that display at least a three fold over-expression in prostate tumor and/or normal prostate tissue, as compared to non-prostate normal tissues (not including testis). 372 clones were identified, and 319 were successfully sequenced. Table I presents a summary of these clones, which are shown in SEQ ID NOs:385-400. Of these sequences SEQ ID NOs:386, 389, 390 and 392 correspond to novel genes, and SEQ ID NOs: 393 and 396 correspond to previously identified sequences. The others (SEQ ID NOs:385, 387, 388, 391, 394, 395 and 397-400) correspond to known sequences, as shown in Table I.

**Table I**

| Summary of Prostate Tumor Antigens | | |
|---|---|---|
| Known Genes | Previously identified Genes | Novel Genes |
| T-cell gamma chain | P504S | 23379 (SEQ ID NO:389) |
| | | |
| Kallikrein | P1000C | 23399 (SEQ ID NO:392) |
| | | |
| Vector | P501S | 23320 (SEQ ID NO:386) |
| | | |
| CGI-82 protein mRNA (23319; SEQ ID NO:385) | P503S | 23381 (SEQ ID NO:390) |
| | | |
| PSA | P510S | |
| | | |
| Ald. 6 Dehyd. | P784P | |
| | | |
| L-iditol-2 dehydrogenase (23376; SEQ ID NO:388) | P502S | |
| | | |
| Ets transcription factor PDEF (22672; SEQ ID NO:398) | P706P | |
| | | |
| hTGR (22678; SEQ ID NO:399) | 19142.2, bangur.seq (22621; SEQ ID NO:396) | |
| | | |
| KIAA0295(22685; SEQ ID NO:400) | 5566.1 Wang(23404; SEQ ID NO:393) | |
| | | |
| Prostatic Acid Phosphatase(22655; SEQ ID NO:397) | P712P | |
| | | |
| transglutaminase (22611; SEQ ID NO:395) | P778P | |
| | | |
| HDLBP (23508; SEQ ID NO:394) | | |
| | | |
| CGI-69 Protein(23367; SEQ ID NO:387) | | |
| | | |
| KIAA0122(23383; SEQ ID NO:391) | | |
| | | |
| TEEG | | |
| | | |

CGI-82 showed 4.06 fold over-expression in prostate tissues as compared to other normal tissues tested. It was over-expressed in 43% of prostate tumors, 25% normal prostate, not detected in other normal tissues tested. L-iditol-2 dehydrogenase showed 4.94 fold over-expression in prostate tissues as compared to other normal tissues tested. It was over-expressed in 90% of prostate tumors, 100% of normal prostate, and not detected in other normal tissues tested. Ets transcription factor PDEF showed 5.55 fold over-expression in prostate tissues as compared to other normal tissues tested. It was over-expressed in 47% prostate tumors, 25% normal prostate and not detected in other normal tissues tested. hTGR1 showed 9.11 fold over-expression in prostate tissues as compared to other normal tissues tested. It was over-expressed in 63% of prostate tumors and is not detected in normal tissues tested including normal prostate. KIAA0295 showed 5.59 fold over-expression in prostate tissues as compared to other normal tissues tested. It was over-expressed in 47% of prostate tumors, low to undetectable in normal tissues tested including normal prostate tissues. Prostatic acid phosphatase showed 9.14 fold over-expression in prostate tissues as compared to other normal tissues tested. It was over-expressed in 67% of prostate tumors, 50% of normal prostate, and not detected in other normal tissues tested. Transglutaminase showed 14.84 fold over-expression in prostate tissues as compared to other normal tissues tested. It was over-expressed in 30% of prostate tumors, 50% of normal prostate, and is not detected in other normal tissues tested. High density lipoprotein binding protein (HDLBP) showed 28.06 fold over-expression in prostate tissues as compared to other normal tissues tested. It was over-expressed in 97% of prostate tumors, 75% of normal prostate, and is undetectable in all other normal tissues tested. CGI-69 showed 3.56 fold over-expression in prostate tissues as compared to other normal tissues tested. It is a low abundant gene, detected in more than 90% of prostate tumors, and in 75% normal prostate tissues. The expression of this gene in normal tissues was very low. KIAA0122 showed 4.24 fold over-expression in prostate tissues as compared to other normal tissues tested. It was over-expressed in 57% of prostate tumors, it was undetectable in all normal tissues tested including normal prostate tissues. 19142.2 bangur showed 23.25 fold over-expression in prostate tissues as compared to other normal tissues tested. It was over-expressed in 97% of prostate tumors and 100% of normal prostate. It was undetectable in other normal tissues tested. 5566.1 Wang showed 3.31 fold over-expression in prostate tissues as compared to other normal tissues tested. It was over-expressed in 97% of prostate tumors, 75% normal prostate and was also over-expressed in normal bone marrow, pancreas, and activated PBMC. Novel clone 23379 showed 4.86 fold over-expression in prostate tissues as compared to other normal tissues tested. It was detectable in 97% of prostate tumors and 75% normal prostate and is undetectable in all other normal tissues tested. Novel clone 23399 showed 4.09 fold over-expression in prostate tissues as compared to other normal tissues tested. It was over-expressed in 27% of prostate tumors and was undetectable in all normal tissues tested including normal prostate tissues. Novel clone 23320 showed 3.15 fold over-expression in prostate tissues as compared to other normal tissues tested. It was detectable in all prostate tumors and 50% of normal prostate tissues. It was also expressed in normal colon and trachea. Other normal tissues do not express this gene at high level.

### EXAMPLE 14

### IDENTIFICATION OF PROSTATE TUMOR ANTIGENS BY ELECTRONIC SUBTRACTION

This Example describes the use of an electronic subtraction technique to identify prostate tumor antigens.

Potential prostate-specific genes present in the GenBank human EST database were identified by electronic subtraction (similar to that described by Vasmatizis et al., Proc. Natl. Acad. Sci. USA95:300-304, 1998). The sequences of EST clones (43,482) derived from various prostate libraries were obtained from the GenBank public human EST database. Each prostate EST sequence was used as a query sequence in a BLASTN (National Center for Biotechnology Information) search against the human EST database. All matches considered identical (length of matching sequence > 100 base pairs, density of identical matches over this region > 70%) were grouped (aligned) together in a cluster. Clusters containing more than 200 ESTs were discarded since they probably represented repetitive elements or highly expressed genes such as those for ribosomal proteins. If two or more clusters shared common ESTs, those clusters were grouped together into a "supercluster," resulting in 4,345 prostate superclusters.

Records for the 479 human cDNA libraries represented in the GenBank release were downloaded to create a database of these cDNA library records. These 479 cDNA libraries were grouped into three groups, Plus (normal prostate and prostate tumor libraries, and breast cell lines, in which expression was desired), Minus (libraries from other normal adult tissues, in which expression was not desirable), and Other (fetal tissue, infant tissue, tissues found only in women, non-prostate tumors and cell lines other than prostate cell lines, in which expression was considered to be irrelevant). A summary of these library groups is presented in Table II.

**Table II**

| Prostate cDNA Libraries and ESTs | | |
|---|---|---|
| Library | # of Libraries | # of ESTs |
| Plus | 25 | 43,482 |
| Normal | 11 | 18,875 |
| Tumor | 11 | 21,769 |
| Cell lines | 3 | 2,838 |
| Minus | 166 | |
| Other | 287 | |

Each supercluster was analyzed in terms of the ESTs within the supercluster. The tissue source of each EST clone was noted and used to classify the superclusters into four groups: Type 1- EST clones found in the Plus group libraries only; no expression detected in Minus or Other group libraries; Type 2- EST clones found in the Plus and Other group libraries only; no expression detected in the Minus group; Type 3- EST clones found in the Plus, Minus and Other group libraries, but the expression in the Plus group is higher than in either the Minus or Other groups; and Type 4- EST clones found in Plus, Minus and Other group libraries, but the expression in the Plus group is higher than the expression in the Minus group. This analysis identified 4,345 breast clusters (*see* Table III). From these clusters, 3,172 EST clones were ordered from Research Genetics, Inc., and were received as frozen glycerol stocks in 96-well plates.

**Table III**

| Prostate Cluster Summary | | |
|---|---|---|
| Type | # of Superclusters | # of ESTs Ordered |
| 1 | 688 | 677 |
| 2 | 2899 | 2484 |
| 3 | 85 | 11 |
| 4 | 673 | 0 |
| Total | 4345 | 3172 |

The inserts were PCR-amplified using amino-linked PCR primers for Synteni microarray analysis. When more than one PCR product was obtained for a particular clone, that PCR product was not used for expression analysis. In total, 2,528 clones from the electronic subtraction method were analyzed by microarray analysis to identify electronic subtraction breast clones that had high tumor vs. normal tissue mRNA. Such screens were performed using a Synteni (Palo Alto, CA) microarray, according to the manufacturer's instructions (and essentially as described by Schena et al., Proc. Natl. Acad. Sci. USA 93:10614-10619, 1996 and Heller et al., Proc. Natl. Acad. Sci. USA94:2150-2155, 1997). Within these analyses, the clones were arrayed on the chip, which was then probed with fluorescent probes generated from normal and tumor prostate cDNA, as well as various other normal tissues. The slides were scanned and the fluorescence intensity was measured.

Clones with an expression ratio greater than 3 (*i.e.,* the level in prostate tumor cDNA was at least three times the level in normal prostate cDNA) were identified as prostate tumor-specific sequences (Table IV). The sequences of these clones are provided in SEQ ID NOs:401-453, with certain novel sequences shown in SEQ ID NOs:407, 413, 416-419, 422, 426, 427 and 450.

**Table IV**

| Prostate-tumor Specific Clones | | |
|---|---|---|
| SEQ ID NO. | Sequence Designation | Comments |
| 401 | 22545 | previously identified P1000C |
| 402 | 22547 | previously identified P704P |
| 403 | 22548 | known |
| 404 | 22550 | known |
| 405 | 22551 | PSA |
| 406 | 22552 | prostate secretory protein 94 |
| 407 | 22553 | novel |
| 408 | 22558 | previously identified P509S |
| 409 | 22562 | glandular kallikrein |
| 410 | 22565 | previously identified P1000C |
| 411 | 22567 | PAP |
| 412 | 22568 | B1006C (breast tumor antigen) |
| 413 | 22570 | novel |
| 414 | 22571 | PSA |
| 415 | 22572 | previously identified P706P |
| 416 | 22573 | novel |
| 417 | 22574 | novel |
| 418 | 22575 | novel |
| 419 | 22580 | novel |
| 420 | 22581 | PAP |
| 421 | 22582 | prostatic secretory protein 94 |
| 422 | 22583 | novel |
| 423 | 22584 | prostatic secretory protein 94 |
| 424 | 22585 | prostatic secretory protein 94 |
| 425 | 22586 | known |
| 426 | 22587 | novel |
| 427 | 22588 | novel |
| 428 | 22589 | PAP |
| 429 | 22590 | known |
| 430 | 22591 | PSA |
| 431 | 22592 | known |
| 432 | 22593 | Previously identified P777P |
| 433 | 22594 | T cell receptor gamma chain |
| 434 | 22595 | Previously identified P705P |
| 435 | 22596 | Previously identified P707P |
| 436 | 22847 | PAP |
| 437 | 22848 | known |
| 438 | 22849 | prostatic secretory protein 57 |
| 439 | 22851 | PAP |
| 440 | 22852 | PAP |
| 441 | 22853 | PAP |
| 442 | 22854 | previously identified P509S |
| 443 | 22855 | previously identified P705P |
| 444 | 22856 | previously identified P774P |
| 445 | 22857 | PSA |
| 446 | 23601 | previously identified P777P |
| 447 | 23602 | PSA |
| 448 | 23605 | PSA |
| 449 | 23606 | PSA |
| 450 | 23612 | novel |
| 451 | 23614 | PSA |
| 452 | 23618 | previously identified P1000C |
| 453 | 23622 | previously identified P705P |

### EXAMPLE 15

### FURTHER IDENTIFICATION OF PROSTATE TUMOR ANTIGENS BY MICROARRAY ANALYSIS

This Example describes the isolation of additional prostate tumor polypeptides from a prostate tumor cDNA library.

A human prostate tumor cDNA expression library as described above was screened using microarray analysis to identify clones that display at least a three fold over-expression in prostate tumor and/or normal prostate tissue, as compared to non-prostate normal tissues (not including testis). 142 clones were identified and sequenced. Certain of these clones are shown in SEQ ID NOs:454-467. Of these sequences SEQ ID NOs:459-461 correspond to novel genes. The others (SEQ ID NOs:454-458 and 461-467) correspond to known sequences.

### EXAMPLE 16

### FURTHER CHARACTERIZATION OF PROSTATE TUMOR ANTIGEN P710P

This Example describes the full length cloning ofP710P.

The prostate cDNA library described above was screened with the P710P fragment described above. One million colonies were plated on LB/Ampicillin plates. Nylon membrane filters were used to lift these colonies, and the cDNAs picked up by these filters were then denatured and cross-linked to the filters by UV light. The P710P fragment was radiolabeled and used to hybridize with the filters. Positive cDNA clones were selected and their cDNAs recovered and sequenced by an automatic ABI Sequencer. Four sequences were obtained, and are presented in SEQ ID NOs:468-471.

From the foregoing, it will be appreciated that, although specific embodiments of the invention have been described herein for the purposes of illustration, various modifications may be made without deviating from the spirit and scope of the invention. Accordingly, the present invention is not limited except as by the appended claims.

## Claims

1. An isolated polypeptide comprising a sequence recited in any one of SEQ ID NO: 337, 108, 112, 113, 114, 172, 176, 178, 327, 329, 331, 339 and 383.

2. An isolated polypeptide comprising at least an immunogenic portion of a prostate tumor protein, or a variant thereof, wherein the tumor protein comprises an amino acid sequence that is encoded by a polynucleotide sequence selected from the group consisting of:
(a) sequences recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472;
(b) sequences that hybridize to any of the foregoing sequences under moderately stringent conditions; and
(c) complements of any of the sequence of (a) or (b).

3. An isolated polypeptide according to claim 2, wherein the polypeptide comprises an amino acid sequence that is encoded by a polynucleotide sequence recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472, or a complement of any of the foregoing polynucleotide sequences.

4. An isolated polynucleotide encoding a polypeptide according to claim 1.

5. An isolated polynucleotide encoding at least 15 amino acid residues of a prostate tumor protein, or a variant thereof that differs in one or more substitutions, deletions, additions and/or insertions such that the ability of the variant to react with antigen-specific antisera is not substantially diminished, wherein the tumor protein comprises an amino acid sequence that is encoded by a polynucleotide comprising a sequence recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472, or a complement of any of the foregoing sequences.

6. An isolated polynucleotide encoding a prostate tumor protein, or a variant thereof, wherein the tumor protein comprises an amino acid sequence that is encoded by a polynucleotide comprising a sequence recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472, or a complement of any of the foregoing sequences.

7. An isolated polynucleotide comprising a sequence recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472.

8. An isolated polynucleotide comprising a sequence that hybridizes, under moderately stringent conditions, to a sequence recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472.

9. An isolated polynucleotide complementary to a polynucleotide according to any one of claims 4-8.

10. An expression vector comprising a polynucleotide according to any one of claims 4-8.

11. A host cell transformed or transfected with an expression vector according to claim 10.

12. An expression vector comprising a polynucleotide according claim 9.

13. A host cell transformed or transfected with an expression vector according to claim 12.

14. A pharmaceutical composition comprising a polypeptide according to claim 1 or 2, in combination with a physiologically acceptable carrier.

15. A vaccine comprising a polypeptide according to claim 1 or 2, in combination with a non-specific immune response enhancer.

16. A vaccine according to claim 15, wherein the non-specific immune response enhancer is an adjuvant.

17. A vaccine according to claim 15, wherein the non-specific immune response enhancer induces a predominantly Type I response.

18. A pharmaceutical composition comprising a polynucleotide according to claim 4 or 5, in combination with a physiologically acceptable carrier.

19. A vaccine comprising a polynucleotide according to claim 4 or 5, in combination with a non-specific immune response enhancer.

20. A vaccine according to claim 19, wherein the non-specific immune response enhancer is an adjuvant.

21. A vaccine according to claim 19, wherein the non-specific immune response enhancer induces a predominantly Type I response.

22. An isolated antibody, or antigen-binding fragment thereof, that specifically binds to a prostate tumor protein that comprises an amino acid sequence of a polypeptide according to claim 1, or an amino acid sequence that is encoded by a polynucleotide sequence recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472 or a complement of any of the foregoing polynucleotide sequences.

23. A pharmaceutical composition comprising an antibody or fragment thereof according to claim 22, in combination with a physiologically acceptable carrier.

24. A pharmaceutical composition comprising an antigen-presenting cell that expresses a polypeptide according to claim 1 or 2, in combination with a pharmaceutically acceptable carrier or excipient.

25. A pharmaceutical composition according to claim 24, wherein the antigen presenting cell is a dendritic cell or a macrophage.

26. A vaccine comprising an antigen-presenting cell that expresses a polypeptide according to claim 1 or 2, in combination with a non-specific immune response enhancer.

27. A vaccine according to claim 26, wherein the non-specific immune response enhancer is an adjuvant.

28. A vaccine according to claim 26, wherein the non-specific immune response enhancer induces a predominantly Type I response.

29. A vaccine according to claim 26, wherein the antigen-presenting cell is a dendritic cell.

30. A method for inhibiting the development of a cancer in a patient, comprising administering to a patient an effective amount of a polypeptide according to claim 1 or 2, and thereby inhibiting the development of a cancer in the patient.

31. A method for inhibiting the development of a cancer in a patient, comprising administering to a patient an effective amount of a polynucleotide according to claim 4 or 5, and thereby inhibiting the development of a cancer in the patient.

32. A method for inhibiting the development of a cancer in a patient, comprising administering to a patient an effective amount of an antibody or antigen-binding fragment thereof according to claim 22, and thereby inhibiting the development of a cancer in the patient.

33. A method for inhibiting the development of a cancer in a patient, comprising administering to a patient an effective amount of an antigen-presenting cell that expresses a polypeptide according to claim 1 or 2, and thereby inhibiting the development of a cancer in the patient.

34. A method according to claim 33, wherein the antigen-presenting cell is a dendritic cell.

35. A method according to any one of claims 30-33, wherein the cancer is prostate cancer.

36. A fusion protein comprising at least one polypeptide according to claim 1 or 2.

37. A fusion protein according to claim 36, wherein the fusion protein comprises an expression enhancer that increases expression of the fusion protein in a host cell transfected with a polynucleotide encoding the fusion protein.

38. A fusion protein according to claim 36, wherein the fusion protein comprises a T helper epitope that is not present within the polypeptide of claim 1 or 2.

39. A fusion protein according to claim 36, wherein the fusion protein comprises an affinity tag.

40. An isolated polynucleotide encoding a fusion protein according to claim 36.

41. A pharmaceutical composition comprising a fusion protein according to claim 36, in combination with a physiologically acceptable carrier.

42. A vaccine comprising a fusion protein according to claim 36, in combination with a non-specific immune response enhancer.

43. A vaccine according to claim 42, wherein the non-specific immune response enhancer is an adjuvant.

44. A vaccine according to claim 42, wherein the non-specific immune response enhancer induces a predominantly Type I response.

45. A pharmaceutical composition comprising a polynucleotide according to claim 40, in combination with a physiologically acceptable carrier.

46. A vaccine comprising a polynucleotide according to claim 40, in combination with a non-specific immune response enhancer.

47. A vaccine according to claim 46, wherein the non-specific immune response enhancer is an adjuvant.

48. A vaccine according to claim 46, wherein the non-specific immune response enhancer induces a predominantly Type I response.

49. A method for inhibiting the development of a cancer in a patient, comprising administering to a patient an effective amount of a pharmaceutical composition according to claim 41 or claim 45.

50. A method for inhibiting the development of a cancer in a patient, comprising administering to a patient an effective amount of a vaccine according to claim 42 or claim 46.

51. A method for removing tumor cells from a biological sample, comprising contacting a biological sample with T cells that specifically react with a prostate tumor protein, wherein the tumor protein comprises an amino acid sequence of a polypeptide according to claim 1, or an amino acid sequence that is encoded by a polynucleotide sequence selected from the group consisting of:
(i) polynucleotides recited in any one of SEQ ID NOs:1-111, 115-171, 173-175, 177, 179-305, 307-315, 326, 328, 330, 332-335, 340-375, 381, 382 or 384-472; and
(ii) complements of the foregoing polynucleotides;
wherein the step of contacting is performed under conditions and for a time sufficient to permit the removal of cells expressing the prostate tumor protein from the sample.

52. A method according to claim 51, wherein the biological sample is blood or a fraction thereof.

53. A method for inhibiting the development of a cancer in a patient, comprising administering to a patient a biological sample treated according to the method of claim 51.

54. A method for stimulating and/or expanding T cells specific for a prostate tumor protein, comprising contacting T cells with one or more of:
(i) a polypeptide according to claim 1 or 2;
(ii) a polypeptide encoded by a polynucleotide comprising a sequence provided in any one of SEQ ID NOs:1-111, 115-171, 173-175, 177, 179-305, 307-315, 326, 328, 330, 332-335, 340-375, 381, 382 or 384-472;
(iii) a polynucleotide encoding a polypeptide of (i) or (ii); and/or
(iv) an antigen presenting cell that expresses a polypeptide of (i) or (ii);
under conditions and for a time sufficient to permit the stimulation and/or expansion of T cells.

55. An isolated T cell population, comprising T cells prepared according to the method of claim 54.

56. A method for inhibiting the development of a cancer in a patient, comprising administering to a patient an effective amount of a T cell population according to claim 55.

57. A method for inhibiting the development of a cancer in a patient, comprising the steps of:
(a) incubating CD4⁺ and/or CD8+ T cells isolated from a patient with at least one component selected from the group consisting of:
(i) a polypeptide according to claim 1 or 2;
(ii) a polypeptide encoded by a polynucleotide comprising a sequence of any one of SEQ ID NOs:1-111, 115-171, 173-175, 177, 179-305, 307-315, 326, 328, 330, 332-335, 340-375, 381, 382 or 384-472;
(iii) a polynucleotide encoding a polypeptide of (i) or (ii); or
(iv) an antigen-presenting cell that expresses a polypeptide of (i) or (ii);
such that T cells proliferate; and
(b) administering to the patient an effective amount of the proliferated T cells, and thereby inhibiting the development of a cancer in the patient.

58. A method for inhibiting the development of a cancer in a patient, comprising the steps of:
(a) incubating CD4⁺ and/or CD8+ T cells isolated from a patient with at least one component selected from the group consisting of:
(i) a polypeptide according to claim 1 or 2;
(ii) a polypeptide encoded by a polynucleotide comprising a sequence of any one of SEQ ID NOs: 1-111, 115-171, 173-175, 177, 179-305, 307-315, 326, 328, 330, 332-335, 340-375, 381, 382 or 384-472;
(iii) a polynucleotide encoding a polypeptide of (i) or (ii); or
(iv) an antigen-presenting cell that expresses a polypeptide of (i) or (ii);
such that T cells proliferate;
(b) cloning at least one proliferated cell; and
(c) administering to the patient an effective amount of the cloned T cells, and thereby inhibiting the development of a cancer in the patient.

59. A method for determining the presence or absence of a cancer in a patient, comprising the steps of:
(a) contacting a biological sample obtained from a patient with a binding agent that binds to a prostate tumor protein, wherein the tumor protein comprises an amino acid sequence of a polypeptide according to claim 1, or an amino acid sequence that is encoded by a polynucleotide sequence selected from the group consisting of:
(i) polynucleotides recited in any one of SEQ ID NOs:1-111, 115-171, 173-175, 177, 179-305, 307-315, 326, 328, 330, 332-335, 340-375, 381, 382 or 384-472; and
(ii) complements of the foregoing polynucleotides;
(b) detecting in the sample an amount of polypeptide that binds to the binding agent; and
(c) comparing the amount of polypeptide to a predetermined cut-off value, and therefrom determining the presence or absence of a cancer in the patient.

60. A method according to claim 59, wherein the binding agent is an antibody.

61. A method according to claim 60, wherein the antibody is a monoclonal antibody.

62. A method according to claim 59, wherein the cancer is prostate cancer.

63. A method for monitoring the progression of a cancer in a patient, comprising the steps of:
(a) contacting a biological sample obtained from a patient at a first point in time with a binding agent that binds to a prostate tumor protein, wherein the tumor protein comprises an amino acid sequence of a polypeptide according to claim 1, or an amino acid sequence that is encoded by a polynucleotide sequence recited in any one of SEQ ID NOs:1-111, 115-171, 173-175, 177, 179-305, 307-315, 326, 328, 330, 332-335, 340-375, 381, 382 or 384-472, or a complement of any of the foregoing polynucleotides;
(b) detecting in the sample an amount of polypeptide that binds to the binding agent;
(c) repeating steps (a) and (b) using a biological sample obtained from the patient at a subsequent point in time; and
(d) comparing the amount of polypeptide detected in step (c) to the amount detected in step (b) and therefrom monitoring the progression of the cancer in the patient.

64. A method according to claim 63, wherein the binding agent is an antibody.

65. A method according to claim 64, wherein the antibody is a monoclonal antibody.

66. A method according to claim 63, wherein the cancer is a prostate cancer.

67. A method for determining the presence or absence of a cancer in a patient, comprising the steps of:
(a) contacting a biological sample obtained from a patient with an oligonucleotide that hybridizes to a polynucleotide that encodes a prostate tumor protein, wherein the tumor protein comprises an amino acid sequence of a polypeptide according to claim 1, or an amino acid sequence that is encoded by a polynucleotide sequence recited in any one of SEQ ID NOs:1-111, 115-171, 173-175, 177, 179-305, 307-315, 326, 328, 330, 332-335, 340-375, 381, 382 or 384-472, or a complement of any of the foregoing polynucleotides;
(b) detecting in the sample an amount of a polynucleotide that hybridizes to the oligonucleotide; and
(c) comparing the amount of polynucleotide that hybridizes to the oligonucleotide to a predetermined cut-off value, and therefrom determining the presence or absence of a cancer in the patient.

68. A method according to claim 67, wherein the amount of polynucleotide that hybridizes to the oligonucleotide is determined using a polymerase chain reaction.

69. A method according to claim 67, wherein the amount of polynucleotide that hybridizes to the oligonucleotide is determined using a hybridization assay.

70. A method for monitoring the progression of a cancer in a patient, comprising the steps of:
(a) contacting a biological sample obtained from a patient with an oligonucleotide that hybridizes to a polynucleotide that encodes a prostate tumor protein, wherein the tumor protein comprises an amino acid sequence of a polypeptide according to claim 1, or an amino acid sequence that is encoded by a polynucleotide sequence recited in any one of SEQ ID NOs: 1-111, 115-171, 173-175, 177, 179-305, 307-315, 326, 328, 330, 332-335, 340-375, 381, 382 or 384-472, or a complement of any of the foregoing polynucleotides;
(b) detecting in the sample an amount of a polynucleotide that hybridizes to the oligonucleotide;
(c) repeating steps (a) and (b) using a biological sample obtained from the patient at a subsequent point in time; and
(d) comparing the amount of polynucleotide detected in step (c) to the amount detected in step (b) and therefrom monitoring the progression of the cancer in the patient.

71. A method according to claim 70, wherein the amount of polynucleotide that hybridizes to the oligonucleotide is determined using a polymerase chain reaction.

72. A method according to claim 70, wherein the amount of polynucleotide that hybridizes to the oligonucleotide is determined using a hybridization assay.

73. A diagnostic kit, comprising:
(a) one or more antibodies according to claim 22; and
(b) a detection reagent comprising a reporter group.

74. A kit according to claim 73, wherein the antibodies are immobilized on a solid support.

75. A kit according to claim 74, wherein the solid support comprises nitrocellulose, latex or a plastic material.

76. A kit according to claim 73, wherein the detection reagent comprises an anti-immunoglobulin, protein G, protein A or lectin.

77. A kit according to claim 73, wherein the reporter group is selected from the group consisting of radioisotopes, fluorescent groups, luminescent groups, enzymes, biotin and dye particles.

78. An oligonucleotide comprising 10 to 40 nucleotides that hybridize under moderately stringent conditions to a polynucleotide that encodes a prostate tumor protein, wherein the tumor protein comprises an amino acid sequence of a polypeptide according to claim 1, or an amino acid sequence that is encoded by a polynucleotide sequence recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472, or a complement of any of the foregoing polynucleotides.

79. A oligonucleotide according to claim 78, wherein the oligonucleotide comprises 10-40 nucleotides recited in any one of SEQ ID NOs:2, 3, 8-29, 41-45, 47-52, 54-65, 70, 73-74, 79, 81, 87, 90, 92, 93, 97, 103, 104, 107, 109-111, 115-160, 171, 173-175, 177, 181, 188, 191, 193, 194, 198, 203, 204, 207, 209, 220, 222-225, 227-305, 307-315, 326, 328, 330, 332, 334, 350-365, 381, 382, 384, 386, 389, 390, 392, 393, 396, 401, 402, 407, 408, 410, 413, 415-419, 422, 426, 427, 432, 434, 435, 442-444, 446, 450, 452, 453, 459-461, 468-471 or 472.

80. A diagnostic kit, comprising:
(a) an oligonucleotide according to claim 78; and
(b) a diagnostic reagent for use in a polymerase chain reaction or hybridization assay.
